# EUROPEAN PATENT APPLICATION

(11) **EP 4 624 465 A1**
(43) Date of publication of application: **01.10.2025**
(21) Application number: 23894566.1
(22) Date of filing: 20.11.2023
(51) Int. Cl.: C07D 401/14, A61K 31/437, A61K 31/4427, A61K 31/501, A61K 31/506, A61K 31/5377, A61P 1/16, A61P 11/00, A61P 13/12, A61P 19/02, A61P 35/00, A61P 35/02, C07D 403/14, C07D 413/14, C07D 417/14, C07D 471/04

(54) **COMPOUND SERVING AS DDR1 KINASE INHIBITOR, AND MEDICINE**

(30) Priority: 21.11.2022 JP 2022185827
(71) Applicant: Nippon Shinyaku Co., Ltd., Kyoto-shi Kyoto 601-8550 (JP)
(72) Inventor: HASHIMOTO, Kosuke, Kyoto-shi, Kyoto 601-8550 (JP); ASADA, Junshi, Kyoto-shi, Kyoto 601-8550 (JP); SUGANOMATA, Mei, Kyoto-shi, Kyoto 601-8550 (JP); HONDA, Yohei, Kyoto-shi, Kyoto 601-8550 (JP); TSUZUKI, Yota, Kyoto-shi, Kyoto 601-8550 (JP); KOSUGI, Keiji, Kyoto-shi, Kyoto 601-8550 (JP)
(74) Representative: Vossius & Partner Patentanwälte Rechtsanwälte mbB
(86) International application number: PCT/JP2023/041679
(87) International publication number: WO 2024/111560

(57) **Abstract**

Provided is a compound having DDR1 inhibitory activity and represented by general formula (I), or a pharmaceutically acceptable salt thereof, or a solvate thereof, and pharmaceutical use of the same.

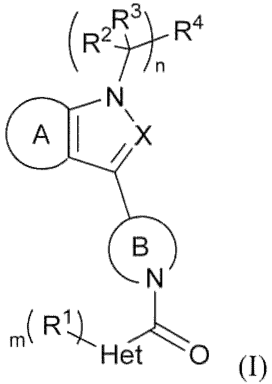

## Description

### TECHNICAL FIELD

The present invention relates to substituted bicyclic heterocyclic compounds useful as DDR1 kinase inhibitors and pharmaceuticals containing such compounds.

### BACKGROUND ART

Discoidin Domain Receptor 1 (also called DDR1) is a tyrosine kinase receptor belonging to the discoidin domain receptor family and is known to be involved in cell proliferation and differentiation as well as migration, invasion and adhesion. It is mainly expressed on epithelial cells in the lung, kidney, mammary gland, gastrointestinal tract, and the like.

DDR1 transduces collagen signals into the cell by binding to the ligand collagen through its discoidin domain. DDR1 is unique in that it recognizes, as a ligand, not only fibrillar collagens such as type I collagens but also basement membrane collagens such as type IV collagens. In DDR1 bound to collagen, specific tyrosine residues in the kinase domain on the cytoplasmic side of the receptor are phosphorylated, and the binding of molecules with Src homology 2 or phosphotyrosine binding domains activates various downstream signaling pathways.

Activation of DDR1 is involved in various renal diseases. For example, it is involved in the pathological conditions of renal diseases, including Alport syndrome, which is caused by mutations in the type IV collagen gene (see Non-Patent Documents 1 and 2), IgA nephropathy (see Non-Patent Document 3), Goodpasture syndrome (see Non-Patent Document 4), lupus nephritis (see Non-Patent Document 4), ANCA (anti-neutrophil cytoplasmic antibody) related nephritis (see Non-Patent Document 5), diabetic nephropathy (see Non-Patent Document 6), and the like.

Activation of DDR1 has also been reported to be involved in various fibrotic diseases, including pulmonary fibrosis (see Non-Patent Document 7) and liver cirrhosis (see Non-Patent Document 8).

Furthermore, DDR1 has been reported to be involved in the function of Th17 cells involved in autoimmune diseases or allergies (see Non-Patent Document 9). Th17 cells are involved in the pathogenesis of diseases such as rheumatoid arthritis, multiple sclerosis, ulcerative colitis and Crohn's disease.

DDR1 has also been reported to be involved in cancer-related pathogenesis, for example, that of acute myeloid leukemia (see Non-Patent Document 10), acute lymphoid leukemia (see Non-Patent Document 11), chronic lymphocytic leukemia (see Non-Patent Document 12), Hodgkin lymphoma (see Non-Patent Document 13), glioma (see Non-Patent Document 14), non-small-cell lung cancer (see Non-Patent Document 15), breast cancer (see Non-Patent Document 16), ovarian cancer (see Non-Patent Document 17), prostate cancer (see Non-Patent Document 18), colorectal cancer (see Non-Patent Document 19), and the like.

In addition, DDR1 has been reported to be involved in other pathological conditions such as systemic lupus erythematosus (see Non-Patent Document 5), osteoarthritis (see Non-Patent Document 20), heparin-induced thrombocytopenia (see Non-Patent Document 21), atherosclerosis (see Non-Patent Document 22) and vitiligo vulgaris (see Non-Patent Document 23).

Many of the treatments for these diseases are limited to symptomatic treatment, control of disease progression, and improvement of quality of life, and efficient treatments for the diseases are extremely difficult. Therefore, DDR1 inhibitors may provide a new treatment for these DDR1-related diseases.

### PRIOR ART DOCUMENTS

### NON-PATENT DOCUMENTS

[Non-Patent Document 1] Gross et al., Matrix Biol. 2010 Jun; 29(5): 346-56.
[Non-Patent Document 2] Richter et al., ACS Chem Biol. 2019 Jan 18; 14(1): 37-49.
[Non-Patent Document 3] Hahn et al., Int J Mol Med. 2010 May; 25(5):785-91.
[Non-Patent Document 4] Kerroch et al., FASEB J. 2012 Oct; 26(10): 4079-91.
[Non-Patent Document 5] Moll et al., J Transl Med. 2018 Jun 1; 16(1): 148.
[Non-Patent Document 6] Salem et al., J Am Soc Nephrol. 2019 Oct;30(10):2000-2016.
[Non-Patent Document 7] Matsuyama et al., J Immunol. 2005 May 15; 174(10): 6490-8.
[Non-Patent Document 8] Shackel et al., Am J Pathol. 2002 Feb; 160(2): 641-54.
[Non-Patent Document 9] Azreq et al., Oncotarget. 2016 Jul 19; 7(29): 44975-44990.
[Non-Patent Document 10] Favreau et al., Cancer Med. 2014 Apr; 3(2): 265-72.
[Non-Patent Document 11] Guo et al., Exp Ther Med. 2019 Mar; 17(3): 1593-1600.
[Non-Patent Document 12] Barisione et al., Blood Cancer J. 2017 Jan 6; 6(1): e513.
[Non-Patent Document 13] Cader et al., Blood. 2013 Dec 19; 122(26): 4237-45.
[Non-Patent Document 14] Ram et al., J Neurooncol. 2006 Feb; 76(3): 239-48.
[Non-Patent Document 15] Miao et al., Med Oncol. 2013; 30(3): 626.
[Non-Patent Document 16] Zhong et al., Oncol Rep. 2019 Dec; 42(6): 2844-2854.
[Non-Patent Document 17] Heinzelmann-Schwarz et al., Clin Cancer Res. 2004 Jul 1; 10(13): 4427-36.
[Non-Patent Document 18] Azizi et al., J Cell Physiol. 2019 Nov; 234(11): 19539-19552.
[Non-Patent Document 19] Le et al., Front Cell Dev Biol. 2020 Jun 3; 8: 412.
[Non-Patent Document 20] Han et al., PLoS One. 2020 Apr 24; 15(4): e0231501.
[Non-Patent Document 21] Witten et al., J Mol Med. 2018 Aug; 96(8): 765-775.
[Non-Patent Document 22] Franco et al., Circ Res. 2008 May 23; 102(10): 1202-11.
[Non-Patent Document 23] Almasi-Nasrabadi et al., Gene. 2019 Jun 5; 700: 17-22.

### SUMMARY OF INVENTION

### PROBLEMS TO BE SOLVED BY THE INVENTION

The object of the present invention is to provide a compound having DDR1 kinase inhibitory activity, or a pharmaceutically acceptable salt thereof, or a solvate thereof.

### MEANS OF SOLVING THE PROBLEMS

The present inventors have found that the compounds described below or pharmaceutically acceptable salts or solvates thereof have excellent DDR1 kinase inhibitory activity, and thus have completed the present invention.

That is, the present invention includes the following embodiments (Item 1) to (Item 22).

### (Item 1)

A compound of formula (I): 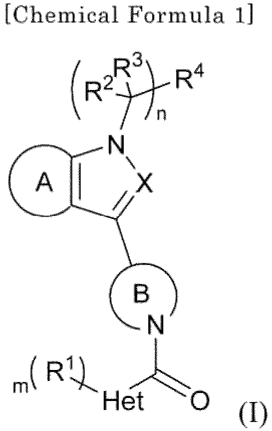 wherein:
A is 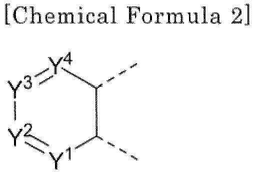
wherein
Y¹, Y², Y³ and Y⁴ are each independently N or C-R⁵, wherein at most two of the Y¹, Y², Y³ and Y⁴ are N, and the others are C-R⁵,
R⁵ is hydrogen, halogen, cyano, alkoxy, or optionally-substituted C₁-C₆ alkyl, and
dashed lines represent bonding points,
   or 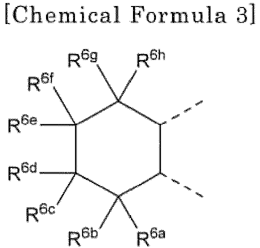
wherein
R^{6a}, R^{6b}, R^{6c}, R^{6d}, R^{6e}, R^{6f}, R^{6g} and R^{6h} are each independently hydrogen, halogen, cyano or optionally-substituted C₁-C₆ alkyl, and
dashed lines represent bonding points;
B is optionally-substituted, 3- to 8-membered saturated or unsaturated monocyclic nitrogen-containing heterocyclic ring;
X is N or C-R⁷, wherein R⁷ is hydrogen, halogen, cyano or optionally-substituted C₁ -C₆ alkyl;
R¹ s are each independently selected from the group consisting of hydrogen, halogen, cyano, oxo, C₁-C₆ alkyl, C₂-C₆ alkenyl, C₂-C₆ alkynyl, C₁-C₆ alkoxy, hydroxy, carboxy, alkylcarbonyloxy, amino, monoalkylamino, dialkylamino, aminoalkyl, alkylcarbonylamino, C₃ -C₁₀ cycloalkyl, C₃-C₆ cycloalkenyl, heterocycloalkyl, aryl, and heteroaryl, each of which may be optionally substituted;
R² and R³ are each independently hydrogen, halogen or optionally-substituted C₁ -C₆ alkyl;
R⁴ is selected from the group consisting of hydrogen, halogen, cyano, C₁-C₆ alkyl, C₂ -C₆ alkenyl, C₂-C₆ alkynyl, C₁-C₆ alkoxy, hydroxy, carboxy, alkylcarbonyloxy, amino, monoalkylamino, dialkylamino, aminoalkyl, alkylcarbonylamino, monocyclic or bicyclic saturated carbocyclic ring, monocyclic or bicyclic unsaturated carbocyclic ring, monocyclic or bicyclic saturated heterocyclic ring, and monocyclic or bicyclic unsaturated heterocyclic ring, each of which may be optionally substituted; or
R⁴ is -CONR⁸R⁹, wherein:
   R⁸ and R⁹ are each independently selected from the group consisting of hydrogen, C₁-C₆ alkyl, C₁-C₆ alkoxy, C₂-C₆ alkenyl, C₂-C₆ alkynyl, C₃-C₁₀ cycloalkyl, C₃-C₆ cycloalkenyl, heterocycloalkyl, aryl, and heteroaryl, each of which may be optionally substituted; or
   R⁸ and R⁹, together with the atom to which they are attached, form optionally-substituted, 5- to 10-membered monocyclic or bicyclic saturated heterocyclic ring or 5- to 10-membered monocyclic or bicyclic unsaturated heterocyclic ring,
m is an integer of 0 to 3;
n is an integer of 0 to 3; and
Het is 5- to 10-membered monocyclic or bicyclic saturated heterocyclic ring or 5- to 10-membered monocyclic or bicyclic unsaturated heterocyclic ring,
or a pharmaceutically acceptable salt thereof, or a solvate thereof.

### (Item 2)

The compound according to Item 1, wherein the formula (I) is formula (II): 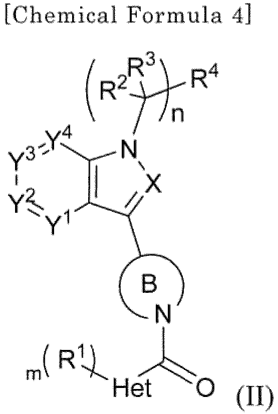
wherein B, R¹, R², R³, R⁴, Het, X, Y¹, Y², Y³, Y⁴, m and n are as defined in the formula (I),
or a pharmaceutically acceptable salt thereof, or a solvate thereof.

### (Item 3)

The compound according to Item 1, wherein the formula (I) is formula (III): 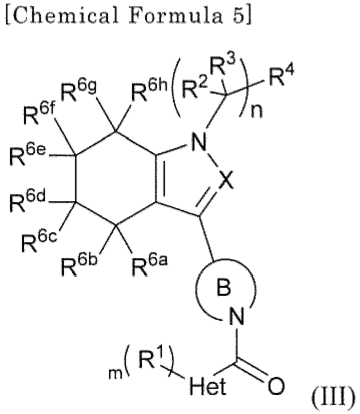
wherein B, R¹, R², R³, R⁴, R^{6a}, R^{6b}, R^{6c}, R^{6d}, R^{6e}, R^{6f}, R^{6g}, R^{6h}, Het, X, m and n are as defined in the formula (I),
or a pharmaceutically acceptable salt thereof, or a solvate thereof.

### (Item 4)

The compound according to any one of Items 1 to 3, wherein R¹ s are each independently selected from the group consisting of hydrogen, halogen, oxo, C₁-C₆ alkyl, C₁-C₆ alkoxy, amino, monoalkylamino, dialkylamino, aminoalkyl, alkylcarbonylamino, C₃-C₁₀ cycloalkyl, heterocycloalkyl, aryl, and heteroaryl, each of which may be optionally substituted,
or a pharmaceutically acceptable salt thereof, or a solvate thereof.

### (Item 5)

The compound according to any one of Items 1 to 4, wherein R⁴ is selected from the group consisting of hydrogen, halogen, cyano, C₁-C₆ alkyl, C₂-C₆ alkenyl, C₂-C₆ alkynyl, C₁-C₆ alkoxy, hydroxy, carboxy, alkylcarbonyloxy, amino, monoalkylamino, dialkylamino, aminoalkyl, alkylcarbonylamino, C₃-C₁₀ cycloalkyl, C₃-C₆ cycloalkenyl, heterocycloalkyl, aryl, and heteroaryl, each of which may be optionally substituted,
or a pharmaceutically acceptable salt thereof, or a solvate thereof.

### (Item 6)

The compound according to any one of Items 1 to 4, wherein R⁴ is -CONR⁸R⁹, wherein:
R⁸ and R⁹ are each independently selected from the group consisting of hydrogen, C₁-C₆ alkyl, C₁-C₆ alkoxy, C₂-C₆ alkenyl, C₂-C₆ alkynyl, C₃-C₁₀ cycloalkyl, C₃-C₆ cycloalkenyl, heterocycloalkyl, aryl, and heteroaryl, each of which may be optionally substituted; or
R⁸ and R⁹, together with the atom to which they are attached, form optionally-substituted, 5- to 10-membered monocyclic or bicyclic saturated heterocyclic ring or 5- to 10-membered monocyclic or bicyclic unsaturated heterocyclic ring,
or a pharmaceutically acceptable salt thereof, or a solvate thereof.

### (Item 7)

The compound according to any one of Items 1 to 6, wherein X is N,
or a pharmaceutically acceptable salt thereof, or a solvate thereof.

### (Item 8)

The compound according to any one of Items 1 to 6, wherein X is C-R⁷, wherein R⁷ is hydrogen, halogen, cyano or optionally-substituted C₁-C₆ alkyl,
or a pharmaceutically acceptable salt thereof, or a solvate thereof.

### (Item 9)

A compound selected from the group consisting of the following compounds (1) to (295), or a pharmaceutically acceptable salt thereof, or a solvate thereof:
(1) 2-(4-chloro-3-{1-[2-(cyclopropylamino)pyridine-4-carbonyl]azetidin-3-yl}-5-fluoro-1H-indazol-1-yl)-N-(2,2,2-trifluoroethyl)acetamide hydrochloride
(2) 2-{3-[1-(2-aminopyridine-4-carbonyl)azetidin-3-yl]-4,6-difluoro-1H-indazol-1-yl}-1-[(2S)-2-(trifluoromethyl)pyrrolidin-1-yl]ethan-1-one hydrochloride
(3) 2-{3-[1-(2-amino-5-fluoropyridine-4-carbonyl)azetidin-3-yl]-1H-indazol-1-yl}-1-[(2S)-2-(trifluoromethyl)pyrrolidin-1-yl]ethan-1-one
(4) 2-{3-[1-(2-aminopyrimidine-4-carbonyl)azetidin-3-yl]-5-chloro-1H-indazol-1-yl}-N-methyl-N-(2,2,2-trifluoroethyl)acetamide
(5) 2-{3-[1-(2-amino-5-fluoropyridine-4-carbonyl)azetidin-3-yl]-6-fluoro-1H-indazol-1-yl}-N-methyl-N-(2,2,2-trifluoroethyl)acetamide
(6) 2-{3-[1-(2-aminopyridine-4-carbonyl)-3-methylazetidin-3-yl]-1H-indazol-1-yl}-1-[(2S)-2-(trifluoromethyl)pyrrolidin-1-yl]ethan-1-one
(7) 2-{3-[1-(2-amino-4-methyl-1,3-thiazole-5-carbonyl)azetidin-3-yl]-1H-indazol-1-yl}-1-(3,3-dimethylmorpholin-4-yl)ethan-1-one
(8) 2-{3-[1-(2-amino-3-methylpyridine-4-carbonyl)azetidin-3-yl]-1H-indazol-1-yl}-1-[(2R)-2-(trifluoromethyl)morpholin-4-yl]ethan-1-one
(9) 4-[4-(4-fluoro-1-{[5-(trifluoromethyl)pyridin-2-yl]methyl}-1H-indazol-3-yl)piperidine-1-carbonyl]pyridin-2-amine
(10) 2-{3-[1-(2-amino-5-fluoropyridine-4-carbonyl)piperidin-4-yl]-4-fluoro-1H-indol-1-yl}-1-[(2S)-2-(trifluoromethyl)pyrrolidin-1-yl]ethan-1-one
(11) 2-{3-[1-(2-aminopyridine-4-carbonyl)azetidin-3-yl]-1H-indol-1-yl}-1-[(2S)-2-(trifluoromethyl)pyrrolidin-1-yl]ethan-1-one
(12) 2-{3-[1-(2-aminopyridine-4-carbonyl)azetidin-3-yl]-5-methyl-1H-pyrazolo[4,3-b]pyridin-1-yl}-1-[(2S)-2-(trifluoromethyl)pyrrolidin-1-yl]ethan-1-one
(13) 2-{3-[1-(2-aminopyridine-4-carbonyl)azetidin-3-yl]-1H-pyrazolo[4,3-c]pyridin-1-yl}-1-[(2S)-2-(trifluoromethyl)pyrrolidin-1-yl]ethan-1-one hydrochloride
(14) 4-{3-[4-fluoro-1-({5-[1-(trifluoromethyl)cyclopropyl]-1,2,4-oxadiazol-3-yl}methyl)-1H-pyrazolo[3,4-c]pyridin-3-yl]azetidine-1-carbonyl}pyridin-2-amine
(15) 2-{3-[1-(2-aminopyridine-4-carbonyl)azetidin-3-yl]-4-methyl-1H-pyrazolo[3,4-b]pyridin-1-yl}-1-[(2S)-2-(trifluoromethyl)pyrrolidin-1-yl]ethan-1-one
(16) 2-{3-[1-(6-aminopyridazine-4-carbonylazetidin-3-yl]-4,6-difluoro-1H-indazol-1-yl}-1-[(2S)-2-(trifluoromethyl)pyrrolidin-1-yl]ethan-1-one
(17) 2-[4,6-difluoro-3-(1-{1H-pyrazolo[3,4-b]pyridine-4-carbonyl}azetidin-3-yl)-1H-indazol-1-yl]-1-[(2S)-2-(trifluoromethyl)pyrrolidin-1-yl]ethan-1-one
(18) 5-fluoro-4-[3-(1-{[3-(trifluoromethyl)-1,2,4-oxadiazol-5-yl]methyl}-1H-indazol-3-yl)azetidine-1-carbonyl]pyridin-2-amine
(19) 4-{3-[1-({1-[(3,3-difluorocyclobutyl)methyl]-1H-1,2,3-triazol-4-yl}methyl)-4-fluoro-1H-indazol-3-yl]azetidine-1-carbonyl}pyridin-2-amine hydrochloride
(20) 4-[3-(4-fluoro-1-{[5-(4-fluorophenyl)-1,3,4-oxadiazol-2-yl]methyl}-1H-indazol-3-yl)azetidine-1-carbonyl]pyridin-2-amine
(21) 2-{3-[1-(2-amino-5-fluoropyrimidine-4-carbonyl)azetidin-3-yl]-5-chloro-1H-indazol-1-yl)-N-methyl-N-(2,2,2-trifluoroethyl)acetamide
(22) 2-{3-[1-(2-amino-5-fluoropyrimidine-4-carbonyl)azetidin-3-yl]-1H-indazol-1-yl}-1-[(2S)-2-(trifluoromethyl)pyrrolidin-1-yl]ethan-1-one
(23) 2-{3-[1-(2-amino-5-fluoropyrimidine-4-carbonyl)azetidin-3-yl]-4-fluoro-1H-indazol-1-yl}-N-methyl-N-(2,2,2-trifluoroethyl)acetamide
(24) 2-{3-[1-(2-amino-5-fluoropyridine-4-carbonyl)azetidin-3-yl]-4,5,6,7-tetrahydro-1H-indazol-1-yl}-1-[(2S)-2-(trifluoromethyl)pyrrolidin-1-yl]ethan-1-one
(25) 2-{3-[1-(2-amino-5-fluoropyridine-4-carbonyl)azetidin-3-yl]-5,5-difluoro-4,5,6,7-tetrahydro-1H-indazol-1-yl}-1-[(2S)-2-(trifluoromethyl)pyrrolidin-1-yl]ethan-1-one
(26) 2-(4-chloro-3-{1-[2-(cyclopropylamino)pyridine-4-carbonyl]azetidin-3-yl}-5-fluoro-1H-indazol-1-yl)-N-(2,2,2-trifluoroethyl)acetamide p-toluenesulfonate
(27) 2-{3-[1-(2-aminopyridine-4-carbonyl)azetidin-3-yl]-4,6-difluoro-1H-indazol-1-yl}-1-[(2S)-2-(trifluoromethyl)pyrrolidin-1-yl]ethan-1-one methanesulfonate
(28) 2-{3-[1-(2-amino-5-fluoropyridine-4-carbonyl)azetidin-3-yl)-1H-indazol-1-yl}-1-[(2S)-2-(trifluoromethyl)pyrrolidin-1-yl]ethan-1-one hydrochloride
(29) 2-{3-[1-(2-amino-5-fluoropyridine-4-carbonyl)azetidin-3-yl]-1H-indazol-1-yl}-1-[(2S)-2-(trifluoromethyl)pyrrolidin-1-yl]ethan-1-one methanesulfonate
(30) 2-(3-{1-[2-(cyclopropylamino)pyridine-4-carbonyl]azetidin-3-yl}-4,6-difluoro-1H-indazol-1-yl)-N-(2,2,2-trifluoroethyl)acetamide hydrochloride
(31) 2-(3-{1-[6-(cyclopropylamino)pyrimidine-4-carbonyl]azetidin-3-yl}-4,6-difluoro-1H-indazol-1-yl)-N-(2,2,2-trifluoroethyl)acetamide
(32) 2-[4,6-difluoro-3-(1-{2-[(oxetan-3-yl)amino]pyridine-4-carbonyl}azetidin-3-yl)-1H-indazol-1-yl]-N-(2,2,2-trifluoroethyl)acetamide
(33) 2-[4,6-difluoro-3-(1-{1H-pyrrolo[2,3-b]pyridine-4-carbonyl}azetidin-3-yl)-1H-indazol-1-yl]-1-[(2S)-2-(trifluoromethyl)pyrrolidin-1-yl]ethan-1-one hydrochloride
(34) 4-(3-{4-fluoro-1-[(1-phenyl-1H-1,2,3-triazol-4-yl)methyl]-1H-indazol- 3-yl}azetidine-1-carbonyl)pyridin-2-amine
(35) 4-(3-{1-[(1-benzyl-1H-1,2,3-triazol-4-yl)methyl]-4-fluoro-1H-indazol-3-yl}azetidine-1-carbonyl)pyridin-2-amine hydrochloride
(36) 2-{3-[1-(2-aminopyridine-4-carbonyl)azetidin-3-yl]-4,6-difluoro-1H-indazol-1-yl}-1-[(2R)-2-(hydroxymethyl)pyrrolidin-1-yl]ethan-1-one
(37) 4-[3-(1-{[1-(cyclopropylmethyl)-1H-1,2,3-triazol-4-yl]methyl}-4-fluoro-1H-indazol-3-yl)azetidine-1-carbonyl]pyridin-2-amine hydrochloride
(38) 4-(3-(4-fluoro-1-({1-((oxetan-3-yl)methyl]-1H-1,2,3-triazol-4-yl}methyl)-1H-indazol-3-yl]azetidine-1-carbonyl}pyridin-2-amine
(39) 4-{4-[1-({1-[(3,3-difluorocyclobutyl)methyl]-1H-1,2,3-triazol-4-yl}methyl)-4-fluoro-1H-indazol-3-yl]piperidine-1-carbonyl}pyridin-2-amine
(40) 2-{3-[1-(2-aminopyridine-4-carbonyl)azetidin-3-yl]-1H-indazol-1-yl}-1-[(2S)-2-(trifluoromethyl)pyrrolidin-1-yl]ethan-1-one
(41) 2-{3-[1-(2-aminopyridine-4-carbonyl)azetidin-3-yl]-1H-indazol-1-yl}-1-[(2S)-2-(trifluoromethyl)pyrrolidin-1-yl]ethan-1-one hydrochloride
(42) 2-{3-[1-(2-amino-5-fluoropyridine-4-carbonyl)azetidin-3-yl]-4,6-difluoro-1H-indazol-1-yl}-1-[(2R)-2-(trifluoromethyl)morpholin-4-yl]ethan-1-one
(43) 2-{3-[1-(2-amino-5-fluoropyridine-4-carbonyl)azetidin-3-yl]-4,6-difluoro-1H-indazol-1-yl}-1-[(2R)-2-(trifluoromethyl)morpholin-4-yl]ethan-1-one p-toluenesulfonate
(44) 2-(3-[1-(2-amino-3-methylpyridine-4-carbonyl)azetidin-3-yl]-4-fluoro-1H-pyrazolo[3,4-c]pyridin-1-yl}-1-[(2S)-2-[(trifluoromethoxy)methyl]pyrrolidin-1-yl]ethan-1-one di-p-toluenesulfonate
(45) 2-{3-[1-(2-aminopyridine-4-carbonyl)azetidin-3-yl]-1H-indazol-1-yl}-N-methyl-N-(2,2,2-trifluoroethyl)acetamide
(46) 2-{3-[1-(2-aminopyridine-4-carbonyl)azetidin-3-yl]-1H-indazol-1-yl}-N-methyl-N-(2,2,2-trifluoroethyl)acetamide p-toluenesulfonate
(47) 2-{3-[1-(2-amino-5-fluoropyridine-4-carbonyl)-1,2,3,6-tetrahydropyridin-4-yl]-1H-indazol-1-yl}-1-[(2S)-2-(trifluoromethyl)pyrrolidin-1-yl]ethan-1-one
(48) 2-{3-[1-(2-amino-5-fluoropyridine-4-carbonyl)piperidin-4-yl]-1H-indazol-1-yl}-N-methyl-N-(2,2,2-trifluoroethyl)acetamide
(49) 2-{3-[1-(2-amino-5-fluoropyridine-4-carbonyl)piperidin-4-yl]-1H-indazol-1-yl}-1-[(2S)-2-(trifluoromethyl)pyrrolidin-1-yl]ethan-1-one
(50) 2-{3-[1-(2-aminopyridine-4-carbonyl)azetidin-3-yl]-4,6-difluoro-1H-indazol-1-yl}-1-[(2R)-2-(trifluoromethyl)pyrrolidin-1-yl]ethan-1-one
(51) 2-{3-[1-(2-amino-3-methylpyridine-4-carbonyl)azetidin-3-yl]-4,6-difluoro-1H-indazol-1-yl}-1-[(2R)-2-(trifluoromethyl)morpholin-4-yl]ethan-1-one hydrochloride
(52) 4-[3-(1-{[1-(2,2,2-trifluoroethyl)-1H-1,2,3-triazol-4-yl]methyl}-4-fluoro-1H-indazol-3-yl)azetidine-1-carbonyl]pyridin-2-amine hydrochloride
(53) 2-{3-[1-(2-aminopyridine-4-carbonyl)azetidin-3-yl]-4-methyl-1H-pyrazolo[4,3-c]pyridin-1-yl}-1-[(2S)-2-(trifluoromethyl)pyrrolidin-1-yl]ethan-1-one
(54) 2-{3-[1-(2-aminopyridine-4-carbonyl)azetidin-3-yl]-4-fluoro-1H-pyrazolo[3,4-c]pyridin-1-yl}-1-[(2S)-2-(trifluoromethyl)pyrrolidin-1-yl]ethan-1-one hydrochloride
(55) 4-{3-[4-fluoro-1-({3-[3-(trifluoromethyl)phenyl]-1,2,4-oxadiazol-5-yl}methyl)-1H-indazol-3-yl]azetidine-1-carbonyl}pyridin-2-amine
(56) 2-{3-[1-(2-aminopyridine-4-carbonyl)azetidin-3-yl]-4,6-difluoro-1H-indazol-1-yl}-1-[(2S)-2-[(trifluoromethoxy)methyl]pyrrolidin-1-yl]ethan-1-one
(57) 4-(3-{1-[(4,4-difluorocyclohexyl)methyl]-4,6-difluoro-1H-indazol-3-yl}azetidine-1-carbonyl)pyridin-2-amine
(58) 2-{3-[1-(2-amino-5-fluoropyridine-4-carbonyl)azetidin-3-yl]-4-fluoro-1H-pyrazolo[3,4-c]pyridin-1-yl}-1-[(2S)-2-[(trifluoromethoxy)methyl]pyrrolidin-1-yllethan-1-one di-p-toluenesulfonate
(59) 2-{3-[1-(2-amino-5-fluoropyridine-4-carbonyl)azetidin-3-yl]-4-fluoro-1H-indazol-1-yl}-N-methyl-N-(2,2,2-trifluoroethyl)acetamide
(60) 2-{3-[1-(2-amino-5-fluoropyridine-4-carbonyl)azetidin-3-yl]-1H-indazol-1-yl}-1-[(2R)-2-(trifluoromethyl)pyrrolidin-1-yl]ethan-1-one
(61) 2-[4-chloro-3-(1-{1H-pyrrolo[2,3-b]pyridine-4-carbonyl}azetidin-3-yl)-1H-indazol-1-yl]-N-(2,2,2-trifluoroethyl)acetamide
(62) 2-{3-[1-(2-aminopyridine-4-carbonyl)azetidin-3-yl]-4-chloro-1H-indazol-1-yl}-N-(2,2,2-trifluoroethyl)acetamide
(63) 2-(4-chloro-3-{1-[2-(methylamino)pyridine-4-carbonyl]azetidin-3-yl}-1H-indazol-1-yl)-N-(2,2,2-trifluoroethyl)acetamide
(64) 2-(4-chloro-3-{1-[2-(ethylamino)pyridine-4-carbonyl]azetidin-3-yl}-1H-indazol-1-yl)-N-(2,2,2-trifluoroethyl)acetamide hydrochloride
(65) 2-(3-{1-[2-(cyclopropylamino)pyridine-4-carbonyl]azetidin-3-yl}-4-fluoro-1H-indazol-1-yl),-N-(2,2,2-trifluoroethyl)acetamide hydrochloride
(66) 2-(3-{1-[2-(cyclopropylamino)pyridine-4-carbonyl]azetidin-3-yl}-4,7-difluoro-1H-indazol-1-yl)-N-(2,2,2-trifluoroethyl)acetamide hydrochloride
(67) 2-{3-[1-(2-aminopyridine-4-carbonyl)azetidin-3-yl]-4-chloro-5-fluoro-1H-indazol-1-yl}-N-(2,2,2-trifluoroethyl)acetamide hydrochloride
(68) 2-(3-{1-[2-(cyclobutylamino)pyridine-4-carbonyl]azetidin-3-yl}-4-fluoro-1H-indazol-1-yl)-N-(2,2,2-trifluoroethyl)acetamide hydrochloride
(69) 2-(4-bromo-3-{1-[2-(cyclopropylamino)pyridine-4-carbonyl]azetidin-3-yl}-5-fluoro-1H-indazol-1-yl)-N-(2,2,2-trifluoroethyl)acetamide hydrochloride
(70) 2-{3-[1-(2-aminopyridine-4-carbonyl)azetidin-3-yl]-4-bromo-5-fluoro-1H-indazol-1-yl}-N-(2,2,2-trifluoroethyl)acetamide hydrochloride
(71) 2-[4-fluoro-3-(1-{2-[(oxan-4-yl)amino]pyridine-4-carbonyl}azetidin-3-yl)-1H-indazol-1-yl]-N-(2,2,2-trifluoroethyl)acetamide hydrochloride
(72) 2-(3-{1-[2-(cyclopropylamino)pyridine-4-carbonyl]azetidin-3-yl}-4,6-difluoro-1H-indazol-1-yl)-1-[(2S)-2-(trifluoromethyl)pyrrolidin-1-yl]ethan-1-one hydrochloride
(73) 2-{3-[1-(2-aminopyridine-4-carbonyl)azetidin-3-yl]-4,6-difluoro-1H-indazol-1-yl}-1-[(2R)-2-(trifluoromethyl)morpholin-4-yl]ethan-1-one hydrochloride
(74) 2-[4,6-difluoro-3-(1-{2-[(1-methylcyclopropyl)amino)pyridine-4-carbonyl}azetidin-3-yl)-1H-indazol-1-yl]-1-[(2S)-2-(trifluoromethyl)pyrrolidin-1-yllethan-1-one hydrochloride
(75) 2-[3-(1-{2-[(4,4-difluorocyclohexyl)amino)pyridine-4-carbonyl}azetidin-3-yl)-4,6-difluoro-1H-indazol-1-yl]-1-[(2S)-2-(trifluoromethyl)pyrrolidin-1-yl]ethan-1-one hydrochloride
(76) 2-{3-[1-(2-aminopyridine-4-carbonyl)azetidin-3-yl]-4,6-difluoro-1H-indazol-1-yl}-1-(pyrrolidin-1-yl)ethan-1-one
(77) 2-{3-[1-(2-amino-3-methylpyridine-4-carbonyl)azetidin-3-yl]-4,6-difluoro-1H-indazol-1-yl}-1-[(2S)-2-(trifluoromethyl)pyrrolidin-1-yl]ethan-1-one hydrochloride
(78) 2-{3-[1-(2-amino-5-methylpyridine-4-carbonyl)azetidin-3-yl]-4,6-difluoro-1H-indazol-1-yl}-1-[(2S)-2-(trifluoromethyl)pyrrolidin-1-yl]ethan-1-one hydrochloride
(79) 2-{3-[1-(2-aminopyridine-4-carbonyl)azetidin-3-yl]-4,6-difluoro-1H-indazol-1-yl}-1-[(2S)-2-methylmorpholin-4-yl]ethan-1-one hydrochloride
(80) 2-{3-[1-(2-aminopyridine-4-carbonyl)azetidin-3-yl]-4,6-difluoro-1H-indazol-1-yl}-1-[(3R)-3-methylmorpholin-4-yl]ethan-1-one hydrochloride
(81) 2-{3-[1-(2-aminopyridine-4-carbonyl)piperidin-4-yl]-4-fluoro-1H-indazol-1-yl}-1-[(2S)-2-(trifluoromethyl)pyrrolidin-1-yl]ethan-1-one
(82) 2-{3-[1-(2-aminopyridine-4-carbonyl)azetidin-3-yl])-4,6-difluoro-1H-indazol-1-yl}-1-[(2S)-2-(trifluoromethyl)morpholin-4-yl]ethan-1-one
(83) 2-{3-[1-(2-aminopyridine-4-carbonyl)azetidin-3-yl]-4,6-difluoro-1H-indazol-1-yl}-1-[(3R)-3-(propan-2-yl)morpholin-4-yl]ethan-1-one
(84) 2-{3-[1-(2-aminopyridine-4-carbonyl)azetidin-3-yl]-4,6-difluoro-1H-indazol-1-yl}-1-[(2R,6S)-2,6-dimethylmorpholin-4-yl]ethan-1-one
(85) 2-{3-[1-(6-aminopyridine-3-carbonyl)piperidin-4-yl]-4-fluoro-1H-indazol-1-yl}-1-[(2S)-2-(trifluoromethyl)pyrrolidin-1-yl]ethan-1-one
(86) 2-(3-{1-[2-(cyclopropylamino)pyridine-4-carbonyl]piperidin-4-yl}-4-fluoro-1H-indazol-1-yl)-1-[(2S)-2-(trifluoromethyl)pyrrolidin-1-yl]ethan-1-one
(87) 2-{3-[1-(2-aminopyridine-4-carbonyl)azetidin-3-yl]-4-fluoro-1H-indazol-1-yl}-N-methyl-N-(2,2,2-trifluoroethyl)acetamide
(88) 2-[4,6-difluoro-3-(1-{2-[(oxan-4-yl)amino]pyridine-4-carbonyl}azetidin-3-yl)-1H-indazol-1-yl]-1-[(28)-2-(trifluoromethyl)pyrrolidin-1-yl]ethan-1-one
(89) 2-{3-[1-(2-amino-3-methylpyridine-4-carbonyl)azetidin-3-yl]-4,6-difluoro-1H-indazol-1-yl}-1-[(2S)-2-(trifluoromethyl)morpholin-4-yl]ethan-1-one hydrochloride
(90) 2-{3-[1-(2-aminopyridine-4-carbonyl)piperidin-4-yl]-4-fluoro-1H-indazol-1-yl}-1-[(2S)-2-(trifluoromethyl)morpholin-4-yl]ethan-1-one
(91) 2-{3-[1-(2-aminopyridine-4-carbonyl)piperidin-4-yl]-4-fluoro-1H-indazol-1-yl}-1-[(2R)-2-(trifluoromethyl)morpholin-4-yl]ethan-1-one
(92) 4-[3-(1-{[1-(cyclobutylmethyl)-1H-1,2,3-triazol-4-yl]methyl)-4-fluoro-1H-indazol-3-yl)azetidine-1-carbonyl]pyridin-2-amine hydrochloride
(93) 2-(3-{1-[2-(cyclopropylamino)pyridine-4-carbonyl]azetidin-3-yl}-4-methyl-1H-pyrazolo[4,3-c]pyridin-1-yl)-1-[(2S)-2-(trifluoromethyl)pyrrolidin-1-yl]ethan-1-one
(94) 2-{3-[1-(2-amino-3-methylpyridine-4-carbonyl)azetidin-3-yl]-4-methyl-1H-pyrazolo[4,3-c]pyridin-1-yl}-1-[(2S)-2-(trifluoromethyl)pyrrolidin-1-yl]ethan-1-one
(95) 2-{3-[1-(2-aminopyridine-4-carbonyl)azetidin-3-yl]-4-fluoro-1H-indazol-1-yl}·1-[(2R)-2-(trifluoromethyl)morpholin-4-yl]ethan-1-one hydrochloride
(96) 2-{3-[1-(2-aminopyridine-4-carbonyl)azetidin-3-yl]-4-fluoro-1H-indazol-1-yl}-1-[(2S)-2-(trifluoromethyl)pyrrolidin-1-yl]ethan-1-one
(97) 2-S3-[1-(2-amino-1,3-thiazole-5-carbonyl)azetidin-3-yl]-4,6-difluoro-1H-indazol-1-yl}-1-[(2S)-2-(trifluoromethyl)pyrrolidin-1-yl]ethan-1-one
(98) 2-(3-{1-[2-(cyclopropylamino)-1,3-thiazole-5-carbonyl]azetidin-3-yl}-4,6-difluoro-1H-indazol-1-yl)-1-[(2S)-2-(trifluoromethyl)pyrrolidin-1-yl]ethan-1-one
(99) 2-(3-{1-[2-(cyclopropylamino)-4-methyl-1,3-thiazole-5-carbonyl]azetidin-3-yl}-4,6-difluoro-1H-indazol-1-yl)-1-[(2S)-2-(trifluoromethyl)pyrrolidin-1-yl]ethan-1-one
(100) 2-{3-[1-(2-aminopyridine-4-carbonyl)azetidin-3-yl]-4-fluoro-1H-pyrazolo[3,4-c]pyridin-1-yl}-1-[(2R)-2-(trifluoromethyl)morpholin-4-yl]ethan-1-one
(101) 2-[4,6-difluoro-3-(1-{imidazo[1,2-a]pyridine-3-carbonyl}azetidin-3-yl)-1H-indazol-1-yl]-1-[(2S)-2-(trifluoromethyl)pyrrolidin-1-yl]ethan-1-one
(102) 2-[4,6-difluoro-3-(1-{pyrazolo[3,2-b][1,3]thiazol-7-carbonyl}azetidin-3-yl)-1H-indazol-1-yl]-1-[(2S)-2-(trifluoromethyl)pyrrolidin-1-yl]ethan-1-one
(103) 2-{3-[1-(2-aminopyridine-4-carbonyl)azetidin-3-yl]-4,6-difluoro-1H-indazol-1-yl}-1-[(2S)-2-(trifluoromethyl)pyrrolidin-1-yl}propane-1-one hydrochloride
(104) 4-[3-(4-fluoro-1-{[3-(4-fluorophenyl)-1,2,4-oxadiazol-5-yl]methyl}-1H-indazol-3-yl)azetidine-1-carbonyl]pyridin-2-amine
(105) 2-{3-[1-(2-amino-1,3-oxazole-5-carbonyl)azetidin-3-yl]-4,6-difluoro-1H-indazol-1-yl}-1-[(2S)-2-(trifluoromethyl)pyrrolidin-1-yl]ethan-1-one
(106) 2-{3-[1-(2-amino-4-methyl-1,3-thiazole-5-carbonyl)azetidin-3-yl]-4,6-difluoro-1H-indazol-1-yl}-1-[(2S)-2-(trifluoromethyl)pyrrolidin-1-yl]ethan-1-one
(107) 2-{3-[1-(2-aminopyridine-4-carbonyl)azetidin-3-yl]-4,6-difluoro-1H-indazol-1-yl}-2-fluoro-1-[(2S)-2-(trifluoromethyl)pyrrolidin-1-yl]ethan-1-one
(108) 2-{3-[1-(2-aminopyridine-4-carbonyl)piperidin-4-yl]-4-fluoro-1H-indol-1-yl}-1-[(2S)-2-(trifluoromethyl)pyrrolidin-1-yl]ethan-1-one
(109) 4-(3-{4-fluoro-1-[(3-methyl-1,2,4-oxadiazol-5-yl)methyl]-1H-indazol-3-yl}azetidine-1-carbonyl)pyridin-2-amine
(110) 2-{3-[1-(2-amino-4-methyl-1,3-oxazole-5-carbonyl)azetidin-3-yl]-4,6-difluoro-1H-indazol-1-yl}-1-[(2S)-2-(trifluoromethyl)pyrrolidin-1-yl]ethan-1-one
(111) 2-{3-[1-(2-aminopyridine-4-carbonyl)azetidin-3-yl]-4,6-difluoro-1H-indazol-1-yl}-1-[(2S)-4,4-difluoro-2-(fluoromethyl)pyrrolidin-1-yl]ethan-1-one
(112) 4-[3-(4-fluoro-1-{[4-(trifluoromethyl)phenyl]methyl}-1H-indazol-3-yl)azetidine-1-carbonyl]pyridin-2-amine hydrochloride
(113) 4-[3-(4-fluoro-1-{[3-(trifluoromethyl)phenyl]methyl}-1H-indazol-3-yl)azetidine-1-carbonyl]pyridin-2-amine hydrochloride
(114) 4-[3-(4-fluoro-1-{[2-(trifluoromethyl)phenyl]methyl}-1H-indazol-3-yl)azetidine-1-carbonyl]pyridin-2-amine hydrochloride
(115) 4-[3-(4-fluoro-1-{[3-(trifluoromethyl)-1,2,4-oxadiazol-5-yl]methyl}-1H-indazol-3-yl)azetidine-1-carbonyl]pyridin-2-amine hydrochloride
(116) 4-[3-(4-fluoro-1-{[5-(4-fluorophenyl)-1,2,4-oxadiazol-3-yl)methyl}-1H-indazol-3-yl)azetidine-1-carbonyl]pyridin-2-amine
(117) 4-{3-{4-fluoro-1-({5-[3-(trifluoromethyl)phenyl]-1,2,4-oxadiazol-3-yl}methyl)-1H-indazol-3-yl]azetidine-1-carbonyl}pyridin-2-amine
(118) 2-{3-(1-(2-amino-1,3-thiazole-5-carbonyl)piperidin-4-yl]-4-fluoro-1H-indazol-1-yl}-1-[(2S)-2-(trifluoromethyl)pyrrolidin-1-yl]ethan-1-one
(119) 2-{3-[1-(2-amino-4-methyl-1,3-thiazole-5-carbonyl)piperidin-4-yl]-4-fluoro-1H-indazol-1-yl}-1-[(2S)-2-(trifluoromethyl)pyrrolidin-1-yl]ethan-1-one
(120) 2-{3-[1-(2-aminopyridine-4-carbonyl)azetidin-3-yl]-1H-pyrazolo[4,3-b]pyridin-1-yl}-1-[(2S)-2-(trifluoromethyl)pyrrolidin-1-yl]ethan-1-one
(121) 2-(3-{1-[2-(cyclopropylamino)pyridine-4-carbonyl]azetidin-3-yl}-1H-pyrazolo(4,3-h]pyridin-1-yl)-1-[(2S)-2-(trifluoromethyl)pyrrolidin-1-yl]ethan-1-one
(122) 2-{3-[1-(2-amino-4-methyl-1,3-thiazole-5-carbonyl)azetidin-3-yl]-1H-pyrazolo[4,3-b]pyridin-1-yl}-1-[(2S)-2-(trifluoromethyl)pyrrolidin-1-yl]ethan-1-one
(123) 2-{3-[1-(2-aminopyridine-4-carbonyl)azetidin-3-yl)-4,6-difluoro-1H-indazol-1-yl}-1-[(3S)-3-(trifluoromethyl)morpholin-4-yl]ethan-1-one
(124) 2-{3-[1-(2-aminopyridine-4-carbonyl)-1,2,3,6-tetrahydropyridin-4-yl]-4-fluoro-1H-indol-1-yl}-1-[(2S)-2-(trifluoromethyl)pyrrolidin-1-yl]ethan-1-one
(125) 2-{3-[1-(2-aminopyridine-4-carbonyl)azetidin-3-yl]-4,6-difluoro-1H-indazol-1-yl}-1-[(3S)-3-(difluoromethoxy)pyrrolidin-1-yl]ethan-1-one
(126) 2-{3-[1-(2-aminopyridine-4-carbonyl)azetidin-3-yl}-4,6-difluoro-1H-indazol-1-yl}-1-[(3R)-3-(difluoromethoxy)pyrrolidin-1-yl]ethan-1-one
(127) 2-{3-[1-(2-aminopyridine-4-carbonyl)azetidin-3-yl]-4,6-difluoro-1H-indazol-1-yl}-1-(2,2-dimethylpyrrolidin-1-yl)ethan-1-one
(128) 4-[3-(4-fluoro-1-{[5-(trifluoromethyl)-1,3,4-oxadiazol-2-yl]methyl}-1H-indazol-3-yl)azetidine-1-carbonyl]pyridin-2-amine
(129) 2-{3-[1-(2-aminopyrimidine-4-carbonyl)azetidin-3-yl]-4,6-difluoro-1H-indazol-1-yl}-1-[(2S)-2-(trifluoromethyl)pyrrolidin-1-yl]ethan-1-one
(130) 2-(3-{1-[2-(cyclopropylamino)pyridine-4-carbonyl]azetidin-3-yl}-5-methyl-1H-pyrazolo[4,3-b]pyridin-1-yl)-1-[(2S)-2-(trifluoromethyl)pyrrolidin-1-yl]ethan-1-one
(131) 2-{3-[1-(6-aminopyrimidine-4-carbonyl)azetidin-3-yl]-4,6-difluoro-1H-indazol-1-yl}-1-[(2S)-2-(trifluoromethyl)pyrrolidin-1-yl]ethan-1-one
(132) 2-{4,6-difluoro-3-[1-(pyridine-4-carbonyl)azetidin-3-yl]-1H-indazol-1-yl}-1-[(2S)-2-(trifluoromethyl)pyrrolidin-1-yl]ethan-1-one
(133) 2-(4,6-difluoro-3-[1-(3-methoxypyridine-4-carbonyl)azetidin-3-yl]-1H-indazol-1-yl}-1-[(2S)-2-(trifluoromethyl)pyrrolidin-1-yl]ethan-1-one
(134) 4-[4-(4-fluoro-1-{[6-(trifluoromethyl)pyridin-3-yl]methyl}-1H-indazol-3-yl)piperidine-1-carbonyl]pyridin-2-amine
(135) 2-{3-[1-(2-aminopyridine-4-carbonyl)piperidin-4-yl]-4-fluoro-1H-indol-1-yl}-1-[(2R)-2-(trifluoromethyl)morpholin-4-yl]ethan-1-one
(136) 2-(3-[1-(2-aminopyridine-4-carbonyl)piperidin-4-yl]-4-fluoro-1H-indol-1-yl}-1-[(2S)-2-(trifluoromethyl)morpholin-4-yl]ethan-1-one
(137) 4-[3-(4-fluoro-1-{[5-(oxan-4-yl)-1,2,4-oxadiazol-3-yl]methyl}-1H-indazol-3-yl)azetidine-1-carbonyl]pyridin-2-amine
(138) 4-[3-(4-fluoro-1-([3-(pyridin-4-yl)-1,2,4-oxadiazol-5-yl]methyl}-1H-indazol-3-yl)azetidine-1-carbonyl]pyridin-2-amine
(139) 2-(3-{1-[2-amino-4-(trifluoromethyl)-1,3-thiazole-5-carbonyl]azetidin-3-yl}-4,6-difluoro-1H-indazol-1-yl)-1-[(2S)-2-(trifluoromethyl)pyrrolidin-1-yl]ethan-1-one
(140) 2-(4,6-difluoro-3-[1-(1H-indazole-5-carbonyl)azetidin-3-yl]-1H-indazol-1-yl}-1-[(2S)-2-(trifluoromethyl)pyrrolidin-1-yl]ethan-1-one
(141) 2-[4,6-difluoro-3-(1-{1H-pyrazolo[4,3-b]pyridine-5-carbonyl}azetidin-3-yl)-1H-indazol-1-yl]-1-[(2S)-2-(trifluoromethyl)pyrrolidin-1-yl]ethan-1-one
(142) 2-{4,6-difluoro-3-[1-(3-fluoropyridine-4-carbonyl)azetidin-3-yl]-1H-indazol-1-yl}-1-[(2S)-2-(trifluoromethyl)pyrrolidin-1-yl]ethan-1-one
(143) 2-(3-[1-(3-chloropyridine-4-carbonyl)azetidin-3-yl]-4,6-difluoro-1H-indazol-1-yl}-1-[(2S)-2-(trifluoromethyl)pyrrolidin-1-yl]ethan-1-one
(144) 2-{3-[1-(2-aminopyridine-4-carbonyl)azetidin-3-yl]-4-chloro-5-fluoro-1H-indazol-1-yl}-1-[(2S)-2-(trifluoromethyl)pyrrolidin-1-yl]ethan-1-one
(145) 2-{3-[1-(2-amino-3-fluoropyridine-4-carbonyl)azetidin-3-yl]-4,6-difluoro-1H-indazol-1-yl}-1-[(2S)-2-(trifluoromethyl)pyrrolidin-1-yl]ethan-1-one
(146) 4-(3-[4-fluoro-1-({5-[1-(trifluoromethyl)cyclobutyl]-1,2,4-oxadiazol-3-yl}methyl)-1H-indazol-3-yl]azetidine-1-carbonyl}pyridin-2-amine hydrochloride
(147) 4-{3-[4-fluoro-1-({5-[1-(trifluoromethyl)cyclopropyl]-1,2,4-oxadiazol-3-yl}methyl)-1H-indazol-3-yl]azetidine-1-carbonyl}pyridin-2-amine
(148) 4-[3-(4-fluoro-1-{[5-(2-fluorophenyl)-1,2,4-oxadiazol-3-yl]methyl}-1H-indazol-3-yl)azetidine-1-carbonyl]pyridin-2-amine
(149) 2-{3-(1-(2-amino-4-chloro-1,3-thiazole-5-carbonyl)azetidin-3-yl]-4,6-difluoro-1H-indazol-1-yl}-1-[(2S)-2-(trifluoromethyl)pyrrolidin-1-yl]ethan-1-one
(150) 4-[3-(4-fluoro-1-{[5-(trifluoromethyl)pyridin-2-yl]methyl}-1H-pyrazolo[3,4-c]pyridin-3-yl)azetidine-1-carbonyl]pyridin-2-amine
(151) 4-[3-(4-fluoro-1-{[4-(trifluoromethyl)phenyl]methyl}-1H-pyrazolo[3,4-c]pyridin-3-yl)azetidine-1-carbonyl]pyridin-2-amine
(152) 4-(3-[4,6-difluoro-1-(3-phenylprop-2-yn-1-yl)-1H-indazol-3-yl]azetidine-1-carbonyl}pyridin-2-amine
(153) 4-[3-(4-fluoro-1-{[5-(5-fluoropyridin-2-yl)-1,2,4-oxadiazol-3-yl]methyl}-1H-indazol-3-yl)azetidine-1-carbonyl]pyridin-2-amine
(154) 4-[3-(4-fluoro-1-{[5-(6-methylpyridazin-3-yl)-1,2,4-oxadiazol-3-yl]methyl}-1H-indazol-3-yl)azetidine-1-carbonyl]pyridin-2-amine
(155) 2-{3-[1-(2-amino-5-fluoropyridine-4-carbonyl)azetidin-3-yl]-4,6-difluoro-1H-indazol-1-yl}-1-[(2S)-2-(trifluoromethyl)pyrrolidin-1-yl]ethan-1-one
(156) 4-{3-[1-(3-cyclopropylprop-2-yn-1-yl)-4,6-difluoro-1H-indazol-3-yl]azetidine-1-carbonyl)pyridin-2-amine
(157) 4-(3-{4,6-difluoro-1-[(1-phenylazetidin-3-yl)methyl]-1H-indazol-3-yl}azetidine-1-carbonyl)pyridin-2-amine
(158) 2-{3-[1-(2-aminopyridine-4-carbonyl)azetidin-3-yl]-4,6-difluoro-1H-indazol-1-yl}-1-(1,2,3,4-tetrahydroquinolin-1-yl)ethan-1-one
(159) 2-{3-[1-(2-aminopyridine-4-carbonyl)azetidin-3-yl]-4,6-difluoro-1H-indazol-1-yl}-1-(3,4-dihydro-2H-1,4-benzoxazin-4-yl)ethan-1-one
(160) 4-[3-(4-fluoro-1-{[5-(2-fluorophenyl)-1,2,4-oxadiazol-3-yl]methyl}-1H-pyrazolo[3,4-c]pyridin-3-yl)azetidine-1-carbonyl]pyridin-2-amine
(161) 2-{3-[1-(2-aminopyridine-4-carbonyl)azetidin-3-yl]-4,6-difluoro-1H-indazol-1-yl}-1-(6-bromo-2,3-dihydro-1H-indol-1-yl)ethan-1-one
(162) 2-{4,6-difluoro-3-(1-(oxane-4-carbonyl)azetidin-3-yl]-1H-indazol-1-yl}-1-[(2S)-2-(trifluoromethyl)pyrrolidin-1-yl]ethan-1-one
(163) 2-{4,6-difluoro-3-[1-(2-hydroxypyridine-4-carbonyl)azetidin-3-yl]-1H-indazol-1-yl}-1-[(2S)-2-(trifluoromethyl)pyrrolidin-1-yl]ethan-1-one
(164) 2-{4,6-difluoro-3-[1-(1H-pyrazole-4-carbonyl)azetidin-3-yl]-1H-indazol-1-yl}-1-[(2S)-2-(trifluoromethyl)pyrrolidin-1-yl]ethan-1-one
(165) 2-{4,6-difluoro-3-[1-(5-methyl-1H-pyrazole-4-carbonyl)azetidin-3-yl]-1H-indazol-1-yl}-1-[(2S)-2-(trifluoromethyl)pyrrolidin-1-yl]ethan-1-one
(166) 2-(4,6-difluoro-3-{1-[5-(trifluoromethyl)-1H-pyrazole-4-carbonyl]azetidin-3-yl}-1H-indazol-1-yl)-1-[(2S)-2-(trifluoromethyl)pyrrolidin-1-yl]ethan-1-one
(167) 4-[3-(4,6-difluoro-1-{[1-(2,2,2-trifluoroethyl)azetidin-3-yl]methyl}-1H-indazol-3-yl)azetidine-1-carbonyl]pyridin-2-amine
(168) 4-[3-(5-methyl-1-{[4-(trifluoromethyl)phenyl]methyl}-1H-pyrazolo[3,4-c]pyridin-3-yl)azetidine-1-carbonyl]pyridin-2-amine
(169) 2-{3-[1-(1H-1,2,3-benztriazole-5-carbonyl)azetidin-3-yl]-4,6-difluoro-1H-indazol-1-yl}-1-[(2S)-2-(trifluoromethyl)pyrrolidin-1-yl]ethan-1-one
(170) 4-[3-(4-fluoro-1-{[3-(3-fluoropyridin-4-yl)-1,2,4-oxadiazol-5-yl}methyl}-1H-pyrazolo[3,4-c]pyridin-3-yl)azetidine-1-carbonyl]pyridin-2-amine
(171) 4-(S-{1-[(2,3-dihydro-1H-inden-2-yl)methyl]-4,6-difluoro-1H-indazol-3-yl}azetidine-1-carbonyl)pyridin-2-amine
(172) 2-{3-[1-(2-aminopyridine-4-carbonyl)azetidin-3-yl]-4,6-difluoro-1H-indazol-1-yl}-1-(6-cyclopropyl-2,3-dihydro-1H-indol-1-yl)ethan-1-one
(173) 2-{3-[1-(2-amino-4-methyl-1,3-thiazole-5-carbonyl)azetidin-3-yl]-4,6-difluoro-1H-indazol-1-yl}-1-[(2R)-2-(trifluoromethyl)morpholin-4-yl]ethan-1-one
(174) 2-{3-[1-(2-amino-5-fluoropyridine-4-carbonyl)piperidin-4-yl]-4-fluoro-1H-indazol-1-yl}-1-[(2S)-2-(trifluoromethyl)pyrrolidin-1-yl)ethan-1-one
(175) 2-{3-[1-(2-amino-5-fluoropyridine-4-carbonyl)azetidin-3-yl]-4,6-difluoro-1H-indazol-1-yl}-1-[(2S)-2-[(trifluoromethoxy)methyl]pyrrolidin-1-yl]ethan-1-one
(176) 2-{3-[1-(2-amino-5-fluoropyridine-4-carbonyl)azetidin-3-yl]-4,6-difluoro-1H-indazol-1-yl}-1-(2,2-dimethylmorpholin-4-yl)ethan-1-one
(177) 2-{3-[1-(2-amino-5-fluoropyridine-4-carbonyl)azetidin-3-yl]-4,6-difluoro-1H-indazol-1-yl}-1-(3,3-dimethylmorpholin-4-yl)ethan-1-one
(178) 2-{3-[1-(2-amino-3-methylpyridine-4-carbonyl)azetidin-3-yl]-5-methyl-1H-pyrazolo[3,4-c]pyridin-1-yl}-1-[(2R)-2-(trifluoromethyl)morpholin-4-yl]ethan-1-one dihydrochloride
(179) 2-{3-[1-(2-amino-5-fluoropyridine-4-carbonyl)azetidin-3-yl]-4,6-difluoro-1H-indazol-1-yl}-1-[(2S)-2-(methoxymethyl)pyrrolidin-1-yl]ethan-1-one
(180) 2-{3-[1-(2-amino-3-methylpyridine-4-carbonyl)azetidin-3-yl]-4-fluoro-1H-pyrazolo[3,4-c]pyridin-1-yl}-1-[(2R)-2-(trifluoromethyl)morpholin-4-yl]ethan-1-one
(181) 2-{3-[1-(2-amino-5-fluoropyridine-4-carbonyl)azetidin-3-yl]-4-fluoro-1H-pyrazolo[3,4-c]pyridin-1-yl}-1-[(2R)-2-(trifluoromethyl)morpholin-4-yl]ethan-1-one
(182) 2-{3-[1-(2-amino-5-fluoropyridine-4-carbonyl)azetidin-3-yl]-4,6-difluoro-1H-indazol-1-yl}-1-[(3S,5S)-3,5-dimethylmorpholin-4-yl]ethan-1-one
(183) 2-13-[1-(2-amino-4-methyl-1,3-thiazole-5-carbonyl)azetidin-3-yl]-4-fluoro-1H-pyrazolo[3,4-c]pyridin-1-yl}-1-[(2S)-2-[(trifluoromethoxy)methyl]pyrrolidin-1-yl]ethan-1-one p-toluenesulfonate
(184) 2-{3-[1-(2-amino-3-fluoropyridine-4-carbonyl)azetidin-3-yl]-4,6-difluoro-1H-indazol-1-yl}-1-[(2R)-2-(trifluoromethyl)morpholin-4-yl]ethan-1-one p-toluenesulfonate
(185) 2-{3-[1-(2-amino-5-fluoropyridine-4-carbonyl)azetidin-3-yl)-5-methyl-1H-pyrazolo[4,3-b]pyridin-1-yl}-1-[(2R)-2-(trifluoromethyl)morpholin-4-yl]ethan-1-one p-toluenesulfonate
(186) 2-{3-[1-(2-amino-5-fluoropyridine-4-carbonyl)azetidin-3-yl]-5-methyl-1H-pyrazolo[4,3-b]pyridin-1-yl}-1-(3,3-dimethylmorpholin-4-yl)ethan-1-one p-toluenesulfonate
(187) 2-{3-[1-(2-amino-5-fluoropyridine-4-carbonyl)azetidin-3-yl]-1H-indazol-1-yl}-1-(3,3-dimethylmorpholin-4-yl)ethan-1-one
(188) 2-{3-[1-(2-aminopyridine-4-carbonyl)azetidin-3-yl]-1H-indazol-1-yl}-1-(3,3-dimethylmorpholin-4-yl)ethan-1-one
(189) 2-{3-[1-(2-aminopyridine-4-carbonyl)azetidin-3-yl]-4,6-difluoro-1H-indazol-1-yl)-N-tert-butylacetamide
(190) 2-{3-[1-(2-amino-3-fluoropyridine-4-carbonyl)azetidin-3-yl]-1H-indazol-1-yl}-1-(3,3-dimethylmorpholin-4-yl)ethan-1-one
(191) 2-{3-[1-(2-amino-3-methylpyridine-4-carbonyl)azetidin-3-yl]-1H-indazol-1-yl}-1-(3,3-dimethylmorpholin-4-yl)ethan-1-one
(192) 2-{3-[1-(2-amino-5-fluoropyridine-4-carbonyl)azetidin-3-yl]-1H-indazol-1-yl}-1-[(2R)-2-(trifluoromethyl)morpholin-4-yl]ethan-1-one
(193) 2-{3-(1-(2-aminopyridine-4-carbonyl)azetidin-3-yl]-1H-indazol-1-yl}-1-[(2R)-2-(trifluoromethyl)morpholin-4-yl]ethan-1-one
(194) 2-{3-[1-(2-amino-3-fluoropyridine-4-carbonyl)azetidin-3-yl]-1H-indazol-1-yl}-1-[(2R)-2-(trifluoromethyl)morpholin-4-yl]ethan-1-one
(195) 2-{3-[1-(2-amino-3-methylpyridine-4-carbonyl)azetidin-3-yl]-1H-indazol-1-yl}-1-[(2S)-2-(trifluoromethyl)pyrrolidin-1-yl]ethan-1-one
(196) 2-{3-[1-(2-amino-3-fluoropyridine-4-carbonyl)azetidin-3-yl]-1H-indazol-1-yl}-1-[(2S)-2-(trifluoromethyl)pyrrolidin-1-yl]ethan-1-one
(197) 2-{3-[1-(2-amino-4-methyl-1,3-thiazole-5-carbonyl)azetidin-3-yl]-1H-indazol-1-yl}-1-[(2S)-2-(trifluoromethyl)pyrrolidin-1-yl]ethan-1-one
(198) 2-{3-[1-(2-aminopyridine-4-carbonyl)azetidin-3-yl]-1H-indazol-1-yl}-N-tert-butylacetamide
(199) 2-{3-[1-(2-amino-5-fluoropyridine-4-carbonyl)azetidin-3-yl]-1H-indazol-1-yl}-N-tert-butylacetamide
(200) 2-{3-[1-(2-aminopyridine-4-carbonyl)azetidin-3-yl]-5-methyl-1H-indazol-1-yl}-1-[(2S)-2-(trifluoromethyl)pyrrolidin-1-yl]ethan-1-one
(201) 2-{3-[1-(2-amino-5-fluoropyridine-4-carbonyl)azetidin-3-yl]-5-methyl-1H-indazol-1-yl}-1-[(2S)-2-(trifluoromethyl)pyrrolidin-1-yl]ethan-1-one
(202) 2-{3-[1-(2-aminopyridine-4-carbonyl)azetidin-3-yl]-5-fluoro-1H-indazol-1-yl}-1-[(2S)-2-(trifluoromethyl)pyrrolidin-1-yl]ethan-1-one
(203) 2-{3-[1-(2-amino-5-fluoropyridine-4-carbonyl)azetidin-3-yl]-5-fluoro-1H-indazol-1-yl}-1-[(2S)-2-(trifluoromethyl)pyrrolidin-1-yl]ethan-1-one
(204) 2-{3-[1-(2-amino-5-fluoropyridine-4-carbonyl)azetidin-3-yl]-5-chloro-1H-indazol-1-yl}-1-[(2R)-2-(trifluoromethyl)morpholin-4-yl]ethan-1-one
(205) 2-{3-[1-(2-aminopyridine-4-carbonyl)azetidin-3-yl]-5-chloro-1H-indazol-1-yl}-1-[(2R)-2-(trifluoromethyl)morpholin-4-yl]ethan-1-one
(206) 2-{3-[1-(2-amino-5-fluoropyridine-4-carbonyl)azetidin-3-yl]-5-chloro-1H-indazol-1-yl}-1-[(2S)-2-(trifluoromethyl)pyrrolidin-1-yl]ethan-1-one
(207) 2-{3-[1-(2-aminopyridine-4-carbonyl)azetidin-3-yl]-5-chloro-1H-indazol-1-yl}-1-[(2S)-2-(trifluoromethyl)pyrrolidin-1-yl]ethan-1-one
(208) 2-{3-(1-(2-aminopyridine-4-carbonyl)azetidin-3-yl]-1H-indazol-1-yl}-N-(1-methoxy-2-methylpropan-2-yl)acetamide
(209) 2-{3-[1-(2-aminopyridine-4-carbonyl)azetidin-3-yl]-1H-indazol-1-yl}-N-(4-methyloxan-4-yl)acetamide
(210) 2-{3-[1-(2-amino-5-chloropyridine-4-carbonyl)azetidin-3-yl]-1H-indazol-1-yl}-1-[(2S)-2-(trifluoromethyl)pyrrolidin-1-yl]ethan-1-one
(211) 2-{3-[1-(2-aminopyridine-4-carbonyl)azetidin-3-yl]-1H-indazol-1-yl}-N-(2,2,2-trifluoroethyl)acetamide
(212) 2-{3-[1-(2-aminopyridine-4-carbonyl)azetidin-3-yl)-1H-indazol-1-yl}-N-[(2R)-1,1,1-trifluoropropan-2-yl]acetamide
(213) 2-{3-[1-(2-aminopyridine-4-carbonyl)azetidin-3-yl]-1H-indazol-1-yl)-N-[(2S)-1,1,1-trifluoropropan-2-yl]acetamide
(214) 2-{3-[1-(2-aminopyridine-4-carbonyl)azetidin-3-yl]-1H-indazol-1-yl}-N-(3,3-difluorocyclobutyl)acetamide
(215) 2-{3-[1-(2-aminopyrimidine-4-carbonyl)azetidin-3-yl]-1H-indazol-1-yl}-1-[(2R)-2-(trifluoromethyl)morpholin-4-yl]ethan-1-one
(216) 4-[3-(4-fluoro-1-{[5-(trifluoromethyl)-1,2,4-oxadiazol-3-yl]methyl}-1H-indazol-3-yl)azetidine-1-carbonyl]pyridin-2-amine
(217) 4-[3-(1-{[5-(trifluoromethyl)-1,2,4-oxadiazol-3-yl]methyl}-1H-indazol-3-yl)azetidine-1-carbonyl]pyridin-2-amine
(218) 4-[3-(1-{[3-(trifluoromethyl)-1,2,4-oxadiazol-5-yl]methyl}-1H-indazol-3-yl)azetidine-1-carbonyl]pyridin-2-amine
(219) 2-{3-[1-(2-aminopyridine-4-carbonyl)azetidin-3-yl]-1H-indazol-1-yl}-1-[(2S)-2-(hydroxymethyl)pyrrolidin-1-yl]ethan-1-one
(220) tert-butyl 2-{3-[1-(2-amino-5-fluoropyridine-4-carbonyl)azetidin-3-yl]-1H-indazol-1-yl}acetate
(221) 2-{3-[1-(2-aminopyridine-4-carbonyl)azetidin-3-yl]-1H-indazol-1-yl}-1-(2,2-dimethylpyrrolidin-1-yl)ethan-1-one
(222) 2-{3-[1-(2-aminopyridine-4-carbonyl)azetidin-3-yl]-1H-indazol-1-yl}-1-[(2R)-2-ethylpyrrolidin-1-yl]ethan-1-one
(223) 2-{3-[1-(2-aminopyridine-4-carbonyl)azetidin-3-yl]-1H-indazol-1-yl}-1-{2-azabicyclo[3.1.0]hexan-2-yl}ethan-1-one
(224) 2-{3-[1-(2-aminopyridine-4-carbonyl)azetidin-3-yl]-1H-indazol-1-yl}-N-(dicyclopropylmethyl)acetamide
(225) 2-{3-[1-(2-aminopyridine-4-carbonyl)azetidin-3-yl]-1H-indazol-1-yl}-N-(2,2-difluorocyclopentyl)acetamide
(226) 2-{3-[1-(2-aminopyridine-4-carbonyl)azetidin-3-yl]-1H-indazol-1-yl}-N-(4-fluorophenyl)acetamide
(227) 2-{3-[1-(2-aminopyridine-4-carbonyl)azetidin-3-yl]-1H-indazol-1-yl}-N-[2-(trifluoromethoxy)ethyl]acetamide
(228) 2-{3-[1-(2-aminopyridine-4-carbonyl)azetidin-3-yl]-1H-indazol-1-yl}-1-[(2S)-2-(methoxymethyl)pyrrolidin-1-yl]ethan-1-one
(229) 2-{3-[1-(2-aminopyridine-4-carbonyl)azetidin-3-yl]-1H-indazol-1-yl}-N-(cyclopropylmethyl)-N-methylacetamide
(230) 2-{3-[1-(2-amino-5-fluoropyridine-4-carbonyl)azetidin-3-yl]-1H-indazol-1-yl}-1-[(3S)-3-methoxypyrrolidin-1-yl]ethan-1-one
(231) 2-{3-[1-(2-amino-5-fluoropyridine-4-carbonyl)azetidin-3-yl]-1H-indazol-1-yl}-1-[(3R)-3-methoxypyrrolidin-1-yl]ethan-1-one
(232) 2-{3-[1-(2-amino-5-fluoropyridine-4-carbonyl)azetidin-3-yl)-1H-indazol-1-yl}-1-{2-oxa-6-azaspiro[3.4]octan-6-yl}ethan-1-one
(233) 2-{3-[1-(2-aminopyridine-4-carbonyl)azetidin-3-yl]-1H-indazol-1-yl}-N-(oxan-4-yl)acetamide
(234) 2-{3-[1-(2-aminopyridine-4-carbonyl)azetidin-3-yl]-1H-indazol-1-yl}-N-(2-cyclopropylpropan-2-yl)acetamide
(235) 2-{3-[1-(2-aminopyridine-4-carbonyl)azetidin-3-yl]-1H-indazol-1-yl}-N-(4,4-difluorocyclohexyl)acetamide
(236) (2S)-1-(2-{3-[1-(2-amino-5-fluoropyridine-4-carbonyl)azetidin-3-yl]-1H-indazol-1-yl}acetyl)pyrrolidine-2-carbonitrile
(237) 2-{3-[1-(2-amino-5-fluoropyridine-4-carbonyl)azetidin-3-yl]-1H-indazol-1-yl}-1-[(2S)-4,4-difluoro-2-(fluoromethyl)pyrrolidin-1-yl]ethan-1-one
(238) 2-{3-[1-(2-aminopyridine-4-carbonyl)azetidin-3-yl]-4-fluoro-1H-indazol-1-yl}-N-{bicyclo[2.2.1]heptan-2-yllacetamide
(239) 2-{3-[1-(2-aminopyridine-4-carbonyl)azetidin-3-yl]-4-fluoro-1H-indazol-1-yl}-N-{bicyclo[1.1.1]pentan-1-yl}acetamide
(240) 2-{3-[1-(2-amino-5-fluoropyridine-4-carbonyl)azetidin-3-yl}-1H-indazol-1-yl}-N-methyl-N-(2,2,2-trifluoroethyl)acetamide
(241) 2-{3-[1-(2-aminopyrimidine-4-carbonyl)azetidin-3-yl]-5-fluoro-1H-indazol-1-yl}-1-[(2S)-2-(trifluoromethyl)pyrrolidin-1-yl]ethan-1-one
(242) 2-{3-[1-(2-aminopyridine-4-carbonyl)azetidin-3-yl]-4-fluoro-1H-indazol-1-yl}-N-(2,2-difluoroethyl)acetamide
(243) 2-{3-[1-(2-amino-5-fluoropyridine-4-carbonyl)azetidin-3-yl]-1H-indazol-1-yl}-N-[trans-2-fluorocyclopropyl]acetamide
(244) 2-{3-[1-(2-amino-5-fluoropyridine-4-carbonyl)azetidin-3-yl]-1H-indazol-1-yl}-N-(1-cyanocyclopropyl)acetamide
(245) 2-{3-[1-(2-amino-5-fluoropyridine-4-carbonyl)azetidin-3-yl]-1H-indazol-1-yl}-N-[(1R,2S)-2-fluorocyclopropyl]acetamide
(246) 2-{3-[1-(2-amino-5-fluoropyridine-4-carbonyl)azetidin-3-yl]-1H-indazol-1-yl}-N-[(1S,2R)-2-fluorocyclopropyl]acetamide
(247) 2-{3-[1-(2-amino-5-fluoropyridine-4-carbonyl)azetidin-3-yl]-1H-indazol-1-yl}-N-(1-methyl-1H-pyrazol-5-yl)acetamide
(248) 2-{3-[1-(2-amino-5-fluoropyridine-4-carbonyl)azetidin-3-yl]-1H-indazol-1-yl}-N-(2,2-difluorocyclopropyl)acetamide
(249) 2-{3-[1-(2-amino-5-fluoropyridine-4-carbonyl)azetidin-3-yl]-1H-indazol-1-yl}-N-ethyl-N-(2,2,2-trifluoroethyl)acetamide
(250) 2-{3-[1-(2-amino-5-fluoropyridine-4-carbonyl)azetidin-3-yl]-1H-indazol-1-yl}-N-(3,3-difluorocyclobutyl)-N-methylacetamide
(251) 2-{3-[1-(2-amino-5-fluoropyridine-4-carbonyl)azetidin-3-yl]-1H-indazol-1-yl}-N-cyclopropyl-N-(2,2,2-trifluoroethyl)acetamide
(252) 2-{3-[1-(2-amino-5-fluoropyridine-4-carbonyl)azetidin-3-yl]-1H-indazol-1-yl}-N-cyclopropyl-N-methylacetamide
(253) 2-{3-[1-(2-amino-5-fluoropyridine-4-carbonyl)azetidin-3-yl]-1H-indazol-1-yl}-N-methyl-N-(propan-2-yl)acetamide
(254) 2-{3-[1-(2-amino-5-fluoropyridine-4-carbonyl)azetidin-3-yl]-1H-indazol-1-yl}-N-methyl-N-(oxetan-3-yl)acetamide
(255) 2-{3-[1-(2-amino-5-fluoropyridine-4-carbonyl)azetidin-3-yl]-1H-indazol-1-yl}-N-methyl-N-(oxan-4-yl)acetamide
(256) 2-{3-[1-(2-amino-5-fluoropyridine-4-carbonyl)azetidin-3-yl]-1H-indazol-1-yl}-N-[(3,3-difluorocyclobutyl)methyl]-N-methylacetamide
(257) 2-{3-[1-(2-amino-5-fluoropyridine-4-carbonyl)azetidin-3-yl]-1H-indazol-1-yl}-1-(4,4-difluoropiperidin-1-yl)ethan-1-one
(258) 2-{3-[1-(2-amino-5-fluoropyridine-4-carbonyl)azetidin-3-yl]-1H-indazol-1-yl}-1-{8-oxa-3-azabicyclo[3.2.1]octan-3-yl}ethan-1-one
(259) 2-{3-[1-(2-amino-5-fluoropyridine-4-carbonyl)azetidin-3-yl]-1H-indazol-1-yl}-1-[3-(trifluoromethyl)piperidin-1-yl]ethan-1-one
(260) 2-{3-[1-(2-amino-5-fluoropyridine-4-carbonyl)azetidin-3-yl]-1H-indazol-1-yl}-1-(4-methylpiperazin-1-yl)ethan-1-one
(261) 2-{3-[1-(2-amino-5-fluoropyridine-4-carbonyl)azetidin-3-yl]-1H-indazol-1-yl}-N-methyl-N-[(2S)-1,1,1-trifluoropropan-2-yl]acetamide
(262) 2-{3-[1-(2-amino-5-fluoropyridine-4-carbonyl)azetidin-3-yl]-1H-indazol-1-yl}-N-methyl-N-[(2R)-1,1,1-trifluoropropan-2-yl]acetamide
(263) 2-(3-[1-(2-amino-5-fluoropyridine-4-carbonyl)azetidin-3-yl]-1H-indazol-1-yl}-N,N-diethylacetamide
(264) 2-{3-[1-(2-amino-5-fluoropyridine-4-carbonyl)azetidin-3-yl)-1H-indazol-1-yl}-N-methyl-N-[2-(morpholin-4-yl)ethyl]acetamide
(265) 2-{3-[1-(2-amino-5-fluoropyridine-4-carbonyl)azetidin-3-yl]-1H-indazol-1-yl}-N-(2-methoxyethyl)-N-methylacetamide
(266) 2-{3-[1-(2-amino-5-fluoropyridine-4-carbonyl)azetidin-3-yl]-1H-indazol-1-yl}-1-[(2R,6S)-2,6-dimethylpiperidin-1-yl]ethan-1-one
(267) 2-{3-[1-(2-amino-5-fluoropyridine-4-carbonyl)azetidin-3-yl]-1H-indazol-1-yl}-N,N-bis(propan-2-yl)acetamide
(268) 2-{3-[1-(2-amino-5-fluoropyridine-4-carbonyl)azetidin-3-yl]-1H-indazol-1-yl}-N-tert-butyl-N-methylacetamide
(269) 2-{3-[1-(2-amino-5-fluoropyridine-4-carbonyl)azetidin-3-yl]-1H-indazol-1-yl}-N-(2,2-difluoroethyl)-N-methylacetamide
(270) 2-{3-[1-(2-amino-5-fluoropyridine-4-carbonyl)-3-methylazetidin-3-yl]-1H-indazol-1-yl}-N-methyl-N-(2,2,2-trifluoroethyl)acetamide
(271) 2-{3-[1-(2-amino-5-fluoropyridine-4-carbonyl)-3-methylazetidin-3-yl]-1H-indazol-1-yl}-1-[(2S)-2-(trifluoromethyl)pyrrolidin-1-yl]ethan-1-one
(272) 2-{3-[1-(2-amino-5-fluoropyridine-4-carbonyl)azetidin-3-yl]-1H-indazol-1-yl}-1-((2S)-2-(difluoromethyl)pyrrolidin-1-yl]ethan-1-one
(273) 2-{3-[1-(2-aminopyridine-4-carbonyl)-3-methylazetidin-3-yl]-1H-indazol-1-yl}-N-methyl-N-(2,2,2-trifluoroethyl)acetamide
(274) 2-{3-[1-(2-amino-5-fluoropyridine-4-carbonyl)azetidin-3-yl]-5-chloro-1H-indazol-1-yl}-N-methyl-N-(2,2,2-trifluoroethyl)acetamide
(275) 2-{3-[1-(2-amino-5-fluoropyridine-4-carbonyl)azetidin-3-yl]-6-fluoro-1H-indazol-1-yl}-1-[(2S)-2-(trifluoromethyl)pyrrolidin-1-yl]ethan-1-one
(276) 2-{3-[1-(2-amino-5-fluoropyrimidine-4-carbonyl)azetidin-3-yl]-1H-indazol-1-yl}-N-methyl-N-(2,2,2-trifluoroethyl)acetamide
(277) 2-{3-[1-(2-amino-5-fluoropyridine-4-carbonyl)azetidin-3-yl]-6-fluoro-1H-indazol-1-yl}-N-methyl-N-(2,2,2-trifluoroethyl)acetamide
(278) 2-{3-[1-(2-amino-5-fluoropyrimidine-4-carbonyl)azetidin-3-yl]-5-fluoro-1H-indazol-1-yl}-N-methyl-N-(2,2,2-trifluoroethyl)acetamide
(279) 2-{3-[1-(2-amino-5-fluoropyridine-4-carbonyl)azetidin-3-yl]-5,6-difluoro-1H-indazol-1-yl}-N-methyl-N-(2,2,2-trifluoroethyl)acetamide
(280) 2-{3-[1-(2-amino-5-fluoropyridine-4-carbonyl)azetidin-3-yl]-5,6-difluoro-1H-indazol-1-yl}-1-[(2S)-2-(trifluoromethyl)pyrrolidin-1-yl]ethan-1-one
(281) 2-{3-[1-(2-amino-5-fluoropyridine-4-carbonyl)-3-hydroxyazetidin-3-yl]-1H-indazol-1-yl}-1-[(2S)-2-(trifluoromethyl)pyrrolidin-1-yl]ethan-1-one
(282) 2-{3-[1-(2-aminopyrimidine-4-carbonyl)azetidin-3-yl]-5,6-difluoro-1H-indazol-1-yl)-N-methyl-N-(2,2,2-trifluoroethyl)acetamide
(283) 2-{3-[1-(2-aminopyrimidine-4-carbonyl)azetidin-3-yl]-5,6-difluoro-1H-indazol-1-yl}-1-[(2S)-2-(trifluoromethyl)pyrrolidin-1-yl]ethan-1-one
(284) 2-{3-[1-(2-aminopyrimidine-4-carbonyl)azetidin-3-yl]-6-fluoro-1H-indazol-1-yl}-N-methyl-N-(2,2,2-trifluoroethyl)acetamide
(285) 2-{3-[1-(2-amino-5-fluoropyridine-4-carbonyl)-3-fluoroazetidin-3-yl]-1H-indazol-1-yl}-1-[(2S)-2-(trifluoromethyl)pyrrolidin-1-yl]ethan-1-one
(286) 2-{3-[1-(2-aminopyrimidine-4-carbonyl)azetidin-3-yl]-6-fluoro-1H-indazol-1-yl}-1-[(2S)-2-(trifluoromethyl)pyrrolidin-1-yl]ethan-1-one
(287) 2-{3-[1-(2-aminopyrimidine-4-carbonyl)azetidin-3-yl]-4-chloro-5-fluoro-1H-indazol-1-yl}-N-methyl-N-(2,2,2-trifluoroethyl)acetamide
(288) 2-{3-[1-(2-aminopyrimidine-4-carbonyl)azetidin-3-yl]-5-methyl-1H-indazol-1-yl}-N-methyl-N-(2,2,2-trifluoroethyl)acetamide
(289) 2-{3-[1-(2-aminopyrimidine-4-carbonyl)azetidin-3-yl]-4,6-difluoro-1H-indazol-1-yl}-N-methyl-N-(2,2,2-trifluoroethyl)acetamide
(290) 2-{3-[1-(2-aminopyrimidine-4-carbonyl)azetidin-3-yl}-5-chloro-1H-indazol-1-yl}-1-1(2S)-2-(trifluoromethyl)pyrrolidin-1-yl]ethan-1-one
(291) 2-{3-[1-(2-amino-5-fluoropyridine-4-carbonyl)azetidin-3-yl]-5-methoxy-1H-indazol-1-yl}-1-[(2S)-2-(trifluoromethyl)pyrrolidin-1-yl]ethan-1-one
(292) 2-{3-[1-(2-aminopyrimidine-4-carbonyl)azetidin-3-yl]-5-methyl-1H-indazol-1-yl}-1-[(2S)-2-(trifluoromethyl)pyrrolidin-1-yl]ethan-1-one
(293) 2-{3-[1-(2-aminopyrimidine-4-carbonyl)azetidin-3-yl]-5-fluoro-1H-indazol-1-yl}-N-methyl-N-(2,2,2-trifluoroethyl)acetamide
(294) 2-{3-[1-(2-amino-5-fluoropyridine-4-carbonyl)azetidin-3-yl]-5,5-dimethyl-4,5,6,7-tetrahydro-1H-indazol-1-yl}-1-[(2S)-2-(trifluoromethyl)pyrrolidin-1-yl]ethan-1-one, and
(295) 2-{3-[1-(2-aminopyrimidine-4-carbonyl)azetidin-3-yl]-5,5-dimethyl-4,5,6,7-tetrahydro-1H-indazol-1-yl}-1-[(2S)-2-(trifluoromethyl)pyrrolidin-1-yl]ethan-1-one.

### (Item 10)

2-{3-[1-(2-aminopyridine-4-carbonyl)azetidin-3-yl]-4,6-difluoro-1H-indazol-1-yl}-1-[(2S)-2-(trifluoromethyl)pyrrolidin-1-yl]ethan-1-one, or a pharmaceutically acceptable salt thereof, or a solvate thereof.

### (Item 11)

2-{3-[1-(2-aminopyridine-4-carbonyl)azetidin-3-yl]-4,6-difluoro-1H-indazol-1-yl}-1-[(2S)-2-(trifluoromethyl)pyrrolidin-1-yl]ethan-1-one hydrochloride, or a solvate thereof.

### (Item 12)

2-{3-[1-(2-amino-5-fluoropyridine-4-carbonyl)azetidin-3-yl]-6-fluoro-1H-indazol-1-yl}-N-methyl-N-(2,2,2-trifluoroethyl)acetamide, or a pharmaceutically acceptable salt thereof, or a solvate thereof.

### (Item 13)

2-{3-[1-(2-aminopyridine-4-carbonyl)-3-methylazetidin-3-yl]-1H-indazol-1-yl}-1-[(2S)-2-(trifluoromethyl)pyrrolidin-1-yl]ethan-1-one, or a pharmaceutically acceptable salt thereof, or a solvate thereof.

### (Item 14)

2-{3-[1-(2-amino-5-fluoropyridine-4-carbonyl)azetidin-3-yl]-1H-indazol-1-yl}-1-[(2S)-2-(trifluoromethyl)pyrrolidin-1-yl]ethan-1-one, or a pharmaceutically acceptable salt thereof, or a solvate thereof.

### (Item 15)

2-{3-[1-(2-amino-5-fluoropyridine-4-carbonyl)azetidin-3-yl]-1H-indazol-1-yl}-1-[(2S)-2-(trifluoromethyl)pyrrolidin-1-yl]ethan-1-one methanesulfonate, or a solvate thereof.

### (Item 16)

A pharmaceutical composition comprising the compound according to any one of Items 1 to 15, or a pharmaceutically acceptable salt thereof, or a solvate thereof.

### (Item 17)

A DDR1 kinase inhibitor comprising the compound according to any one of Items 1 to 15, or a pharmaceutically acceptable salt thereof, or a solvate thereof as an active ingredient.

### (Item 18)

A method for inhibiting a DDR1 kinase, which comprises administering the compound according to any one of Items 1 to 15, or a pharmaceutically acceptable salt thereof, or a solvate thereof to a subject in need thereof.

### (Item 19)

A prophylactic or therapeutic agent for a disease in which a DDR1 kinase is involved, comprising the compound according to any one of Items 1 to 15, or a pharmaceutically acceptable salt thereof, or a solvate thereof.

### (Item 20)

A therapeutic agent for Alport syndrome, IgA nephropathy, Goodpasture syndrome, anti-glomerular basement membrane nephritis, lupus nephritis, ANCA-related nephritis, diabetic nephropathy, purpura nephritis, focal segmental glomerulosclerosis, chronic kidney failure, minimal change nephrotic syndrome, mesangial proliferative glomerulonephritis, membranoproliferative glomerulonephritis, crescentic glomerulonephritis, membranous nephropathy, pulmonary fibrosis, myelofibrosis, liver fibrosis, acute myeloid leukemia, chronic myelogenous leukemia, acute lymphoid leukemia, chronic lymphocytic leukemia, Hodgkin lymphoma, non-Hodgkin lymphoma, glioma, non-small-cell lung cancer, small-cell lung cancer, breast cancer, ovarian cancer, prostate cancer, colorectal cancer or osteoarthrosis, comprising the compound according to any one of Items 1 to 15, or a pharmaceutically acceptable salt thereof, or a solvate thereof as an active ingredient.

### (Item 21)

A method for preventing or treating a disease in which a DDR1 kinase is involved, which comprises administering the compound according to any one of Items 1 to 15, or a pharmaceutically acceptable salt thereof, or a solvate thereof to a subject in need thereof.

### (Item 22)

A method for preventing or treating Alport syndrome, IgA nephropathy, Goodpasture syndrome, anti-glomerular basement membrane nephritis, lupus nephritis, ANCA-related nephritis, diabetic nephropathy, purpura nephritis, focal segmental glomerulosclerosis, chronic kidney failure, minimal change nephrotic syndrome, mesangial proliferative glomerulonephritis, membranoproliferative glomerulonephritis, crescentic glomerulonephritis, membranous nephropathy, pulmonary fibrosis, myelofibrosis, liver fibrosis, acute myeloid leukemia, chronic myelogenous leukemia, acute lymphoid leukemia, chronic lymphocytic leukemia, Hodgkin lymphoma, non-Hodgkin lymphoma, glioma, non-small-cell lung cancer, small-cell lung cancer, breast cancer, ovarian cancer, prostate cancer, colorectal cancer or osteoarthrosis, which comprises administering the compound according to any one of Items 1 to 15, or a pharmaceutically acceptable salt thereof, or a solvate thereof to a subject in need thereof.

### (Item 23)

A compound according to any one of Items 1 to 15, or a pharmaceutically acceptable salt thereof, or a solvate thereof for use in preventing or treating a disease in which a DDR1 kinase is involved.

### (Item 24)

A compound according to any one of Items 1 to 15, or a pharmaceutically acceptable salt thereof, or a solvate thereof for use in preventing or treating Alport syndrome, IgA nephropathy, Goodpasture syndrome, anti-glomerular basement membrane nephritis, lupus nephritis, ANCA-related nephritis, diabetic nephropathy, purpura nephritis, focal segmental glomerulosclerosis, chronic kidney failure, minimal change nephrotic syndrome. mesangial proliferative glomerulonephritis, membranoproliferative glomerulonephritis, crescentic glomerulonephritis, membranous nephropathy, pulmonary fibrosis, myelofibrosis, liver fibrosis, acute myeloid leukemia, chronic myelogenous leukemia, acute lymphoid leukemia, chronic lymphocytic leukemia, Hodgkin lymphoma, non-Hodgkin lymphoma, glioma, non-small-cell lung cancer, small-cell lung cancer, breast cancer, ovarian cancer, prostate cancer, colorectal cancer or osteoarthrosis.

### (Item 25)

Use of the compound according to any one of Items 1 to 15, or a pharmaceutically acceptable salt thereof, or a solvate thereof for the manufacture of a prophylactic agent or therapeutic agent for a disease in which a DDR1 kinase is involved.

### (Item 26)

Use of the compound according to any one of Items 1 to 15, or a pharmaceutically acceptable salt thereof, or a solvate thereof for the manufacture of a prophylactic agent or therapeutic agent for Alport syndrome, IgA nephropathy, Goodpasture syndrome, anti-glomerular basement membrane nephritis, lupus nephritis, ANCA-related nephritis, diabetic nephropathy, purpura nephritis, focal segmental glomerulosclerosis, chronic kidney failure, minimal change nephrotic syndrome, mesangial proliferative glomerulonephritis, membranoproliferative glomerulonephritis, crescentic glomerulonephritis, membranous nephropathy, pulmonary fibrosis, myelofibrosis, liver fibrosis, acute myeloid leukemia, chronic myelogenous leukemia, acute lymphoid leukemia, chronic lymphocytic leukemia, Hodgkin lymphoma, non-Hodgkin lymphoma, glioma, non-small-cell lung cancer, small-cell lung cancer, breast cancer, ovarian cancer, prostate cancer, colorectal cancer or osteoarthrosis.

The definitions of the terms as used herein are as follows.

"Halogen" as used herein refers to a fluorine atom, a chlorine atom, a bromine atom, or an iodine atom. Particularly, a fluorine atom or a chlorine atom is preferable.

"Alkyl" as used herein includes, for example, a straight or branched alkyl having 1 to 8 carbon atoms (C₁-C₈), preferably 1 to 6 carbon atoms (C₁-C₆).
Specific examples thereof include methyl, ethyl, propyl, isopropyl, butyl, isobutyl, sec-butyl, tert-butyl, pentyl, isopentyl, neopentyl, tert-pentyl, isoamyl, - CH(CH₂CH₃)₂, hexyl, isohexyl, -CH₂CH₂C(CH₃)₃, -CH₂CH(CH₂CH₃)₂, heptyl, isoheptyl. octyl, isooctyl, and the like.

"Alkenyl" as used herein includes, for example, a straight or branched alkenyl having 1 or 2 double bonds and 2 to 8 carbon atoms (C₂-C₈). Specific examples thereof include ethenyl, 1-propenyl, 2-propenyl, 1-methyl-2-propenyl, 1-butenyl, 2-butenyl, 3-butenyl, 1-pentenyl, 2-pentenyl, 3-pentenyl, 4-pentenyl, 1-methyl-4-pentenyl, and the like. A preferred example is an alkenyl having 2 to 6 carbon atoms, and a more preferred example is an alkenyl having 2 to 4 carbon atoms.

"Alkynyl" as used herein includes, for example, a straight or branched alkynyl having 1 or 2 triple bonds and 2 to 8 carbon atoms (C₂-C₈). Specific examples thereof include ethynyl, 1-propynyl, 2-propynyl, 1-methyl-2-propynyl, 1-butynyl, 2-butynyl, 3-butynyl, 1-pentynyl, 2-pentynyl, 3-pentynyl, 4-pentynyl, 1-methyl-4-pentynyl, and the like. A preferred example is an alkynyl having 2 to 6 carbon atoms, and a more preferred example is an alkynyl having 2 to 4 carbon atoms.

The alkyl part in "alkylcarbonyl", "monoalkylamino", "dialkylamino", and "aminoalkyl" as used herein includes, the same examples as the above "alkyl".

"Haloalkyl" as used herein includes, for example, the above "alkyl" wherein 1 to 3 hydrogen atoms are substituted by the above "halogen". Specific examples thereof include fluoromethyl, chloromethyl, fluoroethyl, difluoromethyl, dichloromethyl, difluoroethyl (e.g., 2,2-difluoroethyl), trifluoromethyl, trichloromethyl, trifluoroethyl (e.g., 2,2,2-trifluoroethyl), and the like.

"Alkoxy" as used herein includes, for example, a straight or branched alkoxy having 1 to 8 carbon atoms (C₁-C₈), preferably 1 to 6 carbon atoms (C₁-C₆). Specific examples thereof include methoxy, ethoxy, n-propoxy, isopropoxy, n-butoxy, isobutoxy, sec-butoxy, tert-butoxy, n-pentyloxy, n-hexyloxy, n-heptyloxy, n-octyloxy, and the like.

"Haloalkoxy" as used herein includes, for example, the above "alkoxy" wherein 1 to 3 hydrogen atoms are substituted by the above "halogen". Specific examples thereof include fluoromethoxy, chloromethoxy, fluoroethoxy, difluoromethoxy, dichloromethoxy, difluoroethoxy (e.g., 2,2-difluoroethoxy), trifluoromethoxy, trichloromethoxy, trifluoroethoxy (e.g., 2,2,2-trifluoroethoxy), and the like.

"Cycloalkyl" as used herein includes, for example, monocyclic, bicyclic or tricyclic saturated hydrocarbon group having 3 to 10 carbon atoms (C₃-C₁₀). Specific examples thereof include cyclopropyl, cyclobutyl, cyclopentyl, cyclohexyl, cycloheptyl, bicyclo[2.1.0]pentyl, bicyclo[2.2.1]heptyl, bicyclo[2.2.2]octyl and bicyclo[3.2.1]octyl, adamantyl (also called tricyclo[3.3.1.1^{3.7}]decanyl), and the like.

"Halocycloalkyl" as used herein includes, for example, the above "cycloalkyl" wherein 1 to 3 hydrogen atoms are substituted by the above "halogen". Specific examples thereof include 2-fluorocyclopropyl. 3,3-difluorocyclobutyl, 2,2-difluorocyclopentyl, 4,4-difluorocyclohexyl, and the like.

"Cycloalkenyl" as used herein includes, for example, a monocyclic, bicyclic or tricyclic, unsaturated hydrocarbon group having 3 to 10 carbon atoms (C₃-C₁₀) and 1 or 2 double bonds in the molecule. Specific examples thereof include cyclopropenyl, cyclobutenyl, cyclopentenyl, cyclohexenyl, cycloheptenyl, and the like.

"Heterocycloalkyl" as used herein includes, for example, a monocyclic or bicyclic saturated heterocyclic ring having 1 to 4 heteroatoms selected from nitrogen atom, sulfur atom, and oxygen atom in the ring and being composed of 4 to 10 ring-constituting atoms. Specific examples thereof include azetidinyl, pyrrolidinyl, piperidinyl, piperazinyl, oxetanyl, tetrahydrofuranyl, tetrahydropyranyl, 1,3-dioxolanyl, 1,4-dioxolanyl, tetrahydrothiophenyl, and the like.

"Aryl" as used herein includes, for example, a monocyclic, bicyclic or tricyclic, aromatic hydrocarbon group having 6 to 14 carbon atoms. Specific examples thereof include phenyl, 1-naphthyl, 2-naphthyl. 1-anthryl, 2-anthryl, 9-anthryl, 1-phenanthryl, 2-phenanthryl, 3-phenanthryl, 4-phenanthryl, 10-phenanthryl, and the like. Particularly, phenyl is preferable.

"Heteroaryl" as used herein includes, for example, a monocyclic or bicyclic aromatic heterocyclic ring having 1 to 4 heteroatoms selected from nitrogen atom, sulfur atom, and oxygen atom in the ring and being composed of 5 to 10 ring-constituting atoms. Specific examples thereof include furyl (e.g., 2-furyl, 3-furyl), thienyl (e.g., 2-thienyl, 3-thienyl), pyrrolyl (e.g., 2-pyrrolyl, 3-pyrrolyl), imidazolyl (e.g., 2-imidazolyl, 4-imidazolyl), pyrazolyl (e.g., 3-pyrazolyl, 4-pyrazolyl), triazolyl (e.g., 1,2,4-triazol-3-yl, 1,2,4-triazol-5-yl, 1,2,3-triazol-4-yl), tetrazolyl (e.g., 5-tetrazolyl), oxazolyl (e.g., 2-oxazolyl, 4-oxazolyl, 5-oxazolyl), isoxazolyl (e.g., 3-isoxazolyl, 4-isoxazolyl, 5-isoxazolyl), oxadiazolyl (e.g., 1,3,4-oxadiazol-2-yl), thiazolyl (e.g., 2-thiazolyl, 4-thiazolyl, 5-thiazolyl), thiadiazolyl, isothiazolyl (e.g., 3-isothiazolyl, 4-isothiazolyl, 5-isothiazolyl), pyridyl (e.g., 2-pyridyl, 3-pyridyl, 4-pyridyl), pyridazinyl (e.g., 3-pyridazinyl, 4-pyridazinyl), pyrimidinyl (e.g., 2-pyrimidinyl, 4-pyrimidinyl, 5-pyrimidinyl), pyrazinyl (e.g., 2-pyrazinyl), benzimidazolyl (e.g., 2-benzimidazolyl, 4-benzimidazolyl, 5-benzimidazolyl, 6-benzimidazolyl, 7-benzimidazolyl), indazolyl (e.g., 3-indazolyl, 4-indazolyl, 5-indazolyl, 6-indazolyl, 7-indazolyl), isoquinolyl (e.g., 1-isoquinolyl, 3-isoquinolyl, 4-isoquinolyl, 5-isoquinolyl, 6-isoquinolyl, 7-isoquinolyl, 8-isoquinolyl), and the like. More preferred are furyl (e.g., 2-furyl, 3-furyl), imidazolyl (e.g., 2-imidazolyl, 4-imidazolyl), thiazolyl (e.g., 2-thiazolyl, 4-thiazolyl, 5-thiazolyl), pyridyl (e.g., 2-pyridyl, 3-pyridyl, 4-pyridyl), pyridazinyl (e.g., 3-pyridazinyl, 4-pyridazinyl), and pyrimidinyl (e.g., 2-pyrimidinyl, 4-pyrimidinyl, 5-pyrimidinyl).

"Carbon ring" refers to a cyclic structure composed of carbon atoms, and examples thereof include the above "cycloalkyl", "cycloalkenyl", and "aryl".

"Heterocyclic ring" refers to, for example, a cyclic structure composed of carbon atoms and one or more heteroatoms selected from nitrogen atom, sulfur atom, and oxygen atom, and examples thereof include the above "heterocycloalkyl" and "heteroaryl".

"3- to 8-Membered saturated or unsaturated monocyclic nitrogen-containing heterocyclic ring" as used herein refers to, for example, saturated or unsaturated monocyclic heterocyclic ring having 1 to 4 nitrogen atoms in the ring and being composed of 3 to 8 ring-constituing atoms. Specific examples thereof include aziridinyl, azetidinyl, pyrrolidinyl, piperidinyl, piperazinyl, azepanyl, azocanyl, pyrrolyl (e.g., 2-pyrrolyl, 3-pyrrolyl), imidazolyl (e.g., 2-imidazolyl, 4-imidazolyl), pyrazolyl (e.g., 3-pyrazolyl, 4-pyrazolyl), triazolyl (e.g., 1,2,4-triazol-3-yl, 1,2,4-triazol-5-yl, 1,2,3-triazol-4-yl), tetrazolyl (e.g., 5-tetrazolyl), pyridyl (e.g., 2-pyridyl, 3-pyridyl, 4-pyridyl), 1,2,3,6-tetrahydropyridyl, pyridazinyl (e.g., 3-pyridazinyl, 4-pyridazinyl), pyrimidinyl (e.g., 2-pyrimidinyl, 4-pyrimidinyl, 5-pyrimidinyl), pyrazinyl (e.g., 2-pyrazinyl), and the like.

"5- to 10-Membered, monocyclic or bicyclic saturated heterocyclic ring" as used herein refers to, for example, monocyclic or bicyclic saturated heterocyclic ring having 1 to 4 heteroatoms selected from nitrogen atom, sulfur atom, and oxygen atom in the ring and being composed of 5 to 10 ring-constituting atoms. Specific examples thereof include azetidinyl, pyrrolidinyl, piperidinyl, piperazinyl, oxetanyl, tetrahydrofuranyl, tetrahydropyranyl, 1,3-dioxolanyl, 1,4-dioxolanyl, tetrahydrothiophenyl, and the like.

"5- to 10-Membered, monocyclic or bicyclic unsaturated heterocyclic ring" as used herein refers to, for example, monocyclic or bicyclic, aromatic heterocyclic ring or non-aromatic unsaturated heterocyclic ring having 1 to 4 heteroatoms selected from nitrogen atom, sulfur atom, and oxygen atom in the ring and being composed of 5 to 10 ring-constituting atoms. Specific examples thereof include furyl (e.g., 2-furyl, 3-furyl), thienyl (e.g., 2-thienyl, 3-thienyl), pyrrolyl (e.g., 2-pyrrolyl, 3-pyrrolyl), imidazolyl (e.g., 2-imidazolyl, 4-imidazolyl), pyrazolyl (e.g., 3-pyrazolyl, 4-pyrazolyl), triazolyl (e.g., 1,2,4-triazol-3-yl, 1,2,4-triazol-5-yl, 1,2,3-triazol-4-yl), tetrazolyl (e.g., 5-tetrazolyl), oxazolyl (e.g., 2-oxazolyl, 4-oxazolyl, 5-oxazolyl), isoxazolyl (e.g., 3-isoxazolyl, 4-isoxazolyl, 5-isoxazolyl), oxadiazolyl (e.g., 1,3,4-oxadiazol-2-yl), thiazolyl (e.g., 2-thiazolyl, 4-thiazolyl, 5-thiazolyl), thiadiazolyl, isothiazolyl (e.g., 3-isothiazolyl, 4-isothiazolyl, 5-isothiazolyl), pyridyl (e.g., 2-pyridyl, 3-pyridyl, 4-pyridyl), pyridazinyl (e.g., 3-pyridazinyl, 4-pyridazinyl), pyrimidinyl (e.g., 2-pyrimidinyl, 4-pyrimidinyl, 5-pyrimidinyl), pyrazinyl (e.g., 2-pyrazinyl), benzimidazolyl (e.g., 2-benzimidazolyl, 4-benzimidazolyl, 5-benzimidazolyl, 6-benzimidazolyl, 7-benzimidazolyl), indazolyl (e.g., 3-indazolyl, 4-indazolyl, 5-indazolyl, 6-indazolyl, 7-indazolyl), pyrazolo[5,1-b]thiazolyl, azaindazolyl (e.g., 3-azaindazolyl, 4-azaindazolyl, 5-azaindazolyl, 6-azaindazolyl, 7-azaindazolyl), benztriazolyl (e.g., 5-benztriazolyl, 6-benztriazolyl), azaindolyl (e.g., 5-azaindolyl, 6-azaindolyl, 7-azaindolyl), isoquinolyl (e.g., 1-isoquinolyl, 3-isoquinolyl, 4-isoquinolyl, 5-isoquinolyl, 6-isoquinolyl, 7-isoquinolyl, 8-isoquinolyl), pyridonyl (e.g., 2-pyridon-3-yl, 2-pyridon-4-yl, 2-pyridon-5-yl, 2-pyridon-6-yl), and the like. More preferred are furyl (e.g., 2-furyl, 3-furyl), imidazolyl (e.g., 2-imidazolyl, 4-imidazolyl), thiazolyl (e.g., 2-thiazolyl, 4-thiazolyl, 5-thiazolyl), oxazolyl (e.g.. 2-oxazolyl, 4-oxazolyl, 5-oxazolyl), pyridyl (e.g., 2-pyridyl, 3-pyridyl, 4-pyridyl), pyridazinyl (e.g., 3-pyridazinyl, 4-pyridazinyl), pyrimidinyl (e.g., 2-pyrimidinyl, 4-pyrimidinyl, 5-pyrimidinyl), pyrazinyl (e.g., 2-pyrazinyl), benzimidazolyl (e.g., 2-benzimidazolyl, 4-benzimidazolyl, 5-benzimidazolyl, 6-benzimidazolyl, 7-benzimidazolyl), indazolyl (e.g., 3-indazolyl, 4-indazolyl, 5-indazolyl, 6-indazolyl, 7-indazolyl), pyrazolo[5,1-blthiazolyl, azaindazolyl (e.g., 3-azaindazolyl, 4-azaindazolyl, 5-azaindazolyl, 6-azaindazolyl, 7-azaindazolyl), benztriazolyl (e.g., 5-benztriazolyl, 6-benztriazolyl), azaindolyl (e.g., 5-azaindolyl, 6-azaindolyl, 7-azaindolyl), and pyridonyl (e.g., 2-pyridon-3-yl, 2-pyridon-4-yl, 2-pyridon-5-yl, 2-pyridon-6-yl).

"Cyano" refers to a group represented by -CN.

"Oxo" refers to a group represented by =O.

"Carbonyl" refers to a group represented by -(C=O)-.

"Hydroxy" refers to a group represented by -OH.

"Optionally-substituted" means that a specified group may or may not be substituted, i.e., it may be optionally substituted or unsubstituted. For example, "optionally-substituted alkyl" refers to both a substituted alkyl or an unsubstituted alkyl.

Examples of a substituent that substitutes a specified group include the above-defined alkyl, haloalkyl, alkoxy, cycloalkyl, halocycloalkyl, heterocycloalkyl, halogen, cyano, oxo, hydroxy, amino, aryl, heteroaryl, and the like, and combined substituents of these substituents (for example, alkyl, alkoxy, cycloalkyl, heterocycloalkyl, amino, aryl, heteroaryl, and the like, which is substituted by at least one substituent selected from the group consisting of alkyl, haloalkyl, alkoxy, haloalkoxy, cycloalkyl, heterocycloalkyl, halogen, cyano, oxo, hydroxy, amino, aryl and heteroaryl).

The number of substituents for a specified group is, for example, 1 to 3, preferably 1 or 2, more preferably 1.

In the compounds of formulae (I) to (III), preferable embodiments of each group indicated by a symbol are shown below. Examples of the compounds of formulae (I) to (III) include those having all combinations of embodiments of each of the groups shown below.

A is preferably 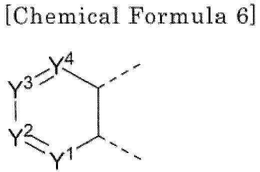 wherein
Y¹, Y², Y³ and Y⁴ are each independently N or C-R⁵, wherein at most one of the Y¹, Y², Y³ and Y⁴ is N, and the others are C-R⁵,
R⁵ is hydrogen, halogen (e.g., fluorine atom, chlorine atom), alkoxy (e.g., methoxy), or optionally-substituted C₁-C₆ alkyl (e.g., methyl), and
dashed lines represent bonding points,
   or 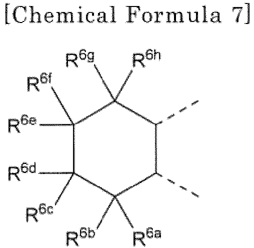
wherein
R^{6a}, R^{6b}, R^{6c}, R^{6d}, R^{6e}, R^{6f}, R^{6g} and R^{6h} are each independently hydrogen, halogen (e.g., fluorine atom), or optionally-substituted C₁-C₆ alkyl (e.g., methyl), and
dashed lines represent bonding points.

A is more preferably 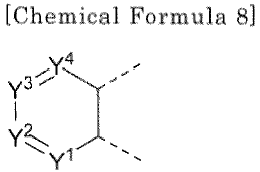 wherein
Y¹, Y², Y³ and Y⁴ are C-R⁵,
R⁵ is hydrogen, and
dashed lines represent bonding points.

B is preferably optionally-substituted, 4- to 6-membered saturated or unsaturated monocyclic nitrogen-containing heterocyclic ring, and more preferably azetidinyl, piperidinyl or 1,2,3,6-tetrahydropyridyl, particularly preferably azetidinyl, which may be optionally substituted by C₁-C₆ alkyl (e.g., methyl).

X is preferably N or C-R⁷, wherein R⁷ is preferably hydrogen, more preferably N.

R¹ s are preferably each independently selected from the group consisting of hydrogen, halogen, oxo, C₁-C₆ alkyl, C₁-C₆ alkoxy, amino, monoalkylamino, dialkylamino, aminoalkyl, alkylcarbonylamino, C₃-C₁₀ cycloalkyl, heterocycloalkyl, aryl, and heteroaryl, each of which may be optionally substituted.

R¹ s are more preferably each independently hydrogen, halogen (e.g., fluorine atom, chlorine atom), oxo, C₁-C₆ alkyl (e.g., methyl), amino, or monoalkylamino (e.g., methylamino, ethylamino), wherein the amino may be optionally substituted by cycloalkyl (e.g., cyclopropyl, cyclobutyl, 1-methylcyclopropyl), or heterocycloalkyl (e.g., oxetanyl, tetrahydropyranyl), and C₁-C₆ alkyl may be optionally substituted by 1 to 3 halogens (e.g., fluorine atom).

R¹ s are more preferably each independently hydrogen, halogen (e.g., fluorine atom) or amino.

R² and R³ are preferably each independently hydrogen or halogen (e.g., fluorine atom).

R⁴ is preferably selected from the group consisting of hydrogen, halogen, cyano, C₁-C₆ alkyl, C₂-C₆ alkenyl, C₂-C₆ alkynyl, C₁-C₆ alkoxy, hydroxy, carboxy, alkylcarbonyloxy, amino, monoalkylamino, dialkylamino, aminoalkyl, alkylcarbonylamino, C₃-C₁₀ cycloalkyl, C₃-C₆ cycloalkenyl, heterocycloalkyl, aryl, and heteroaryl, each of which may be optionally substituted.

R⁴ is more preferably selected from the group consisting of C₂-C₆ alkynyl (e.g., ethynyl), carboxy, monocyclic or bicyclic saturated carbocyclic ring (e.g., cyclohexyl), monocyclic or bicyclic unsaturated carbocyclic ring (e.g., phenyl, 2,3-dihydro-1H-indenyl), monocyclic or bicyclic saturated heterocyclic ring (e.g., azetidinyl), and monocyclic or bicyclic unsaturated heterocyclic ring (e.g., pyridyl, 1,2,4-oxadiazolyl, 1,3,4-oxadiazolyl, 1,2,3-triazolyl),
wherein:
(1) the C₂-C₆ alkynyl may be optionally substituted by C₃-C₁₀ cycloalkyl (e.g., cyclopropyl) or aryl (e.g., phenyl),
(2) the carboxy may be optionally substituted by C₁-C₆ alkyl (e.g., tert-butyl),
(3) the monocyclic or bicyclic saturated carbocyclic ring may be optionally substituted by halogen (e.g., fluorine atom),
(4) the monocyclic or bicyclic unsaturated carbocyclic ring may be optionally substituted by haloalkyl (e.g., trifluoromethyl),
(5) the monocyclic or bicyclic saturated heterocyclic ring may be optionally substituted by aryl (e.g., phenyl) or haloalkyl (e.g., 2,2,2-trifluoroethyl), and
the monocyclic or bicyclic unsaturated heterocyclic ring may be optionally substituted by substituent selected from the following groups:
(i) haloalkyl (e.g., trifluoromethyl, 2,2,2-trifluoroethyl),
(ii) C₃-C₆ cycloalkyl (e.g., cyclopropyl, cyclobutyl) substituted by haloalkyl (e.g., trifluoromethyl),
(iii) heterocycloalkyl (e.g., tetrahydropyranyl),
(iv) aryl (e.g., phenyl), which may be optionally substituted by halogen (e.g., fluorine atom) or haloalkyl (e.g., trifluoromethyl),
(v) heteroaryl (e.g., pyridyl, pyridazinyl), which may be optionally substituted by halogen (e.g., fluorine atom) or C₁-C₆ alkyl (e.g., methyl), and
(vi) C₁-C₆ alkyl (e.g., methyl), which may be optionally substituted by halocycloalkyl (e.g., 3,3-difluorocyclobutyl, 4,4-difluorocyclohexyl), aryl (e.g., phenyl), C₃-C₁₀ cycloalkyl (e.g., cyclopropyl) or heterocycloalkyl (e.g., oxetanyl).

Also, R⁴ is preferably -CONR⁸R⁹, wherein R⁸ and R⁹ are each independently selected from the group consisting of hydrogen, C₁-C₆ alkyl, C₁-C₆ alkoxy, C₂-C₆ alkenyl, C₂-C₆ alkynyl, C₃-C₁₀ cycloalkyl, C₃-C₆ cycloalkenyl, heterocycloalkyl, aryl, and heteroaryl, each of which may be optionally substituted; or R⁸ and R⁹, together with the atom to which they are attached, form optionally-substituted, 5- to 10-membered monocyclic or bicyclic saturated heterocyclic ring or 5- to 10-membered monocyclic or bicyclic unsaturated heterocyclic ring.

More preferably, R⁸ and R⁹ are each independently selected from the group consisting of hydrogen, C₁-C₆ alkyl, C₃-C₆ cycloalkyl, heterocycloalkyl, and heteroaryl, each of which may be optionally substituted;
wherein more preferably,
   (1) C₁-C₆ alkyl is, for example, methyl, ethyl, isopropyl, or tert-butyl, which may be optionally substituted by substituent selected from the following groups:
      (i) halogen (e.g., fluorine atom),
      (ii) C₁-C₆ alkoxy (e.g., methoxy),
      (iii) haloalkoxy (e.g., trifluoromethoxy),
      (iv) heterocycloalkyl (e.g., tetrahydropyranyl, morpholinyl)
      (v) halocycloalkyl (e.g., 3,3-difluorocyclobutyl), and
      (vi) aryl (e.g., phenyl), which may be optionally substituted by halogen (e.g., fluorine atom),
   (2) C₃-C₆ cycloalkyl is particularly cyclobutyl. cyclopentyl, or cyclohexyl, which may be optionally substituted by halogen (e.g., fluorine atom) or cyano,
   (3) heterocycloalkyl is, for example, oxetanyl or tetrahydropyranyl, which may be optionally substituted, and
   (4) heteroaryl is, for example, pyrazolyl, which may be optionally substituted by halogen (e.g., fluorine atom).
R⁸ and R⁹, preferably, together with the atom to which they are attached, form optionally-substituted, 5- to 10-membered monocyclic or bicyclic saturated heterocyclic ring or 5- to 10-membered monocyclic or bicyclic unsaturated heterocyclic ring,
wherein more preferably,
   (1) 5- to 10-membered monocyclic or bicyclic saturated heterocyclic ring is, for example, pyrrolidinyl, piperidinyl, piperazinyl, morpholinyl, azabicyclo[3.1.0]hexanyl, azaspiro[3.4]octanyl, bicyclo[2.2.1]heptanyl, bicyclo[1.1.1]pentanyl, or azabicyclo[3.2.1]octanyl, which may be optionally substituted by substituent selected from the following groups:
      (i) halogen (e.g., fluorine atom, bromine atom)
      (ii) C₁-C₆ alkyl (e.g., methyl, ethyl, isopropyl), which may be optionally substituted by hydroxy group, C₁-C₆ alkoxy (e.g., methoxy), haloalkoxy (e.g., trifluoromethoxy) or heterocycloalkyl (e.g., morpholinyl),
      (iii) C₁-C₆ alkoxy (e.g., methoxy),
      (iv) haloalkyl (e.g., fluoromethyl, difluoromethyl, trifluoromethyl),
      (v) C₃-C₆ cycloalkyl (e.g., cyclopropyl),
      (vi) haloalkoxy (e.g., difluoromethoxy), and
      (vii) cyano,
         and
   (2) 5- to 10-membered monocyclic or bicyclic unsaturated heterocyclic ring is, for example, 1,2,3,4-tetrahydroquinolinyl, 3,4-dihydro-2H-1,4-benzoxazinyl, or 2,3-dihydro-1H-indolyl.

Particularly preferably, R⁴ is -CONR⁸R⁹, wherein R⁸ and R⁹, together with the atom to which they are attached, form 5-membered monocyclic saturated heterocyclic ring (e.g., pyrrolidinyl) substituted by haloalkyl (e.g., trifluoromethyl).

m is preferably an integer of 0 to 2, more preferably an integer of 2.

n is preferably an integer of 0 to 1, more preferably an integer of 1.

Het is preferably 5- to 10-membered monocyclic or bicyclic unsaturated heterocyclic ring, more preferably, pyridyl, pyrimidinyl, 1,3-thiazolyl, pyridazinyl. 1H-pyrazolo[3,4-b]pyridyl, 1H-pyrrolo[2,3-b]pyridyl, 1,3-thiazolyl, imidazo[1,2-a]pyridyl, pyrazolo[3,2-b][1,3]thiazolyl, 1,3-oxazolyl, 1H-indazolyl, pyrazolyl, and 1H-1,2,3-benzotriazolyl, more preferably pyridyl.

### DESCRIPTION OF EMBODIMENTS

The compound of the present invention can be prepared from a known compound per se or an intermediate easily preparable from a known compound, according to, for example, the following methods, Examples described below or known methods. In the preparation of the compound of the present invention, if the starting material has a substituent that affects the reaction, the reaction is generally carried out after the starting material is protected by an appropriate protecting group by a known method in advance. The protecting group can be deprotected by a known method after the reaction.

Abbreviations used herein have the following meanings.

In the Examples, the following abbreviations are used.
TFA: Trifluoroacetic acid
AZADOL^{®}: 2-Azaadamantane-N-hydroxyl
Pd-C: Palladium-carbon
PdCl₂(PPh₃)₂: Bis(triphenylphosphine)palladium(II) dichloride
Pd(OAc)₂: Palladium(II) acetate
Pd(dppf)Cl₂·CH₂Cl₂: [1,1'-Bis(diphenylphosphino)ferrocene]dichloropalladium(II) dichloromethane adduct
Xantphos: 4,5-Bis(diphenylphosphino)-9,9-dimethylxanthene
BINAP: 2,2'-Bis(diphenylphosphino)-1,1'-binaphthyl
Boc: Tert-butoxycarbonyl
HATU: O-(7-azabenztriazol-1-yl)-N,N,N',N'-tetramethyluronium hexafluorophosphate
HBTU: O-(benztriazol-1-yl)-N,N,N',N'-tetramethyluronium hexafluorophosphate
EDCI: 1-Ethyl-3-(3-dimethylaminopropyl)carbodiimide
EDCI ·HCl: 1-Ethyl-3-(3-dimethylaminopropyl)carbodiimide hydrochloride
DMTMM: 4-(4,6-Dimethoxy-1,3,5-triazin-2-yl)-4-methylmorpholinium chloride
HOBt: 1-Hydroxybenztriazole
THF: Tetrahydrofuran
DMF: Dimethylformamide
DMA: Dimethylacetamide
DMSO: Dimethyl sulfoxide
NMP: N-methylpyrrolidone
DIPEA: N,N-diisopropylethylamine
DMAP: 4-Dimethylaminopyridine
NMM: 4-Methylmorpholine
DAST: (Diethylamino)sulfur-trifluoride
MS: Mass spectrometry
LCMS: High performance liquid chromatography-Mass spectrometry
ESI: Electron Spray Ionization
M: Molar concentration (mol/L)

Unless otherwise specified, the reaction in each step of the following processes is conducted according to methods as described in, for example, "Comprehensive Organic Transformations: A Guide to Functional Group Preparations", 2nd Ed. by R. C. Larock, John Wiley & Sons, Inc., 1999; The Chemical Society of Japan, "Experimental Chemistry", 4th edition, Maruzen, 1992; L. Kuerti and B. Czako, "Strategic Applications of Named Reactions in Organic Synthesis", translated by Kiyoshi Tomioka, Kagaku-Dojin Publishing Company, Inc., 2006; G.S. Zweifel and M.H. Nantz, "Modern Organic Synthesis: An Introduction", translated by Tamejiro Hiyama, Kagaku-Dojin Publishing Company, Inc., 2009, or methods in similar manners as described in the Examples, with modifications or combinations as appropriate.

### Synthesis of compound (G-1)

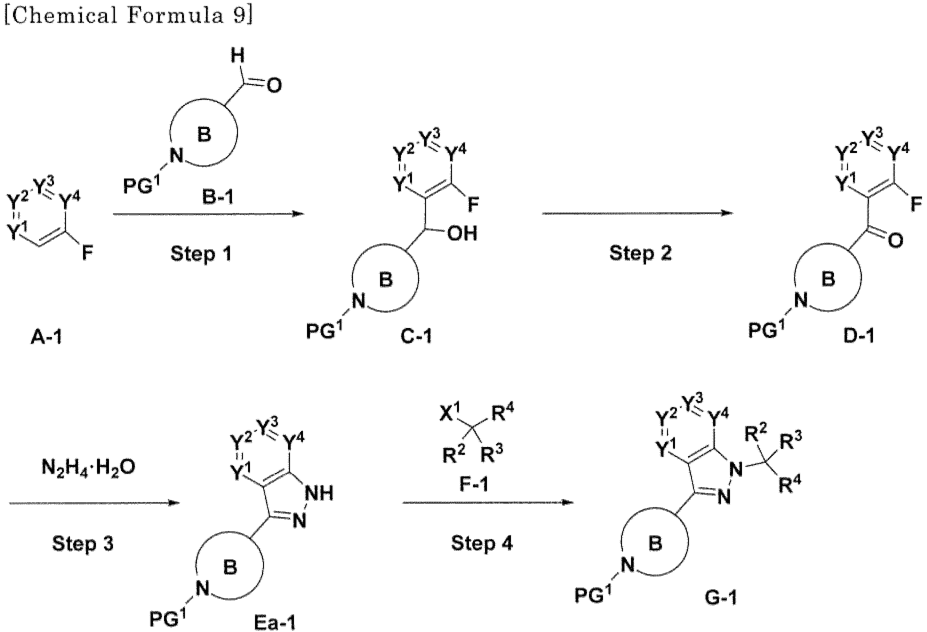

Here, Y¹, Y², Y³, Y⁴, R¹, R², R³, R⁴, and B are as defined above. PG¹ represents protecting group such as tert-butoxycarbonyl (hereinafter referred to as "Boc"). benzyloxycarbonyl (hereinafter referred to as "Cbz"), benzyl (hereinafter referred to as "Bn"), and the like. X¹ represents chloro, bromo, or triflate.

### Step1

In this process. compound A-1, which is commercially available or synthesized by a known method, is reacted with compound B-1, which is commercially available or synthesized by a known method, in the presence of lithium diisopropylamide (LDA) in a solvent such as THF or diethyl ether at -78°C to 0°C, preferably -78°C to -60°C, more preferably -78°C, for 30 minutes to 12 hours, preferably 30 minutes to 6 hours, more preferably 30 minutes to 3 hours to afford compound C-1.

LDA may be commercially available or prepared from diisopropylamine and n-butyl lithium (n-BuLi).

### Step2

This step is the oxidation of the hydroxyl group of compound C-1 to afford D-1.

This reaction is carried out using 2-azadamantane-N-oxyl (AZADOL Registered Trademark), 2,2,6,6-tetramethylpiperidine-1-oxyl radical (TEMPO), Dess-Martin reagent, etc., in a solvent such as dichloromethane or dichloroethane at 0°C to room temperature for 30 minutes to 24 hours, preferably 30 minutes to 6 hours, more preferably 30 minutes to 3 hours to afford compound D-1.

In reactions using AZADOL and TEMPO, co-oxidants such as iodobenzene diacetate, sodium hypochlorite (NaOCl), etc. are generally used.

### Step3

In this step, compound D-1 is reacted in the presence of hydrazine monohydrate in a solvent such as dioxane, THF, etc., at room temperature to reflux temperature, preferably in dioxane at 100°C for 1 hour to 48 hours, preferably 8 hours to 24 hours to afford compound Ea-1.

### Step4

This step is the alkylation of compound Ea-1 with an alkylating agent F-1 in the presence of a base to afford compound G-1, which can be performed according to a known method for alkylation reaction. The reaction of compound Ea-1 with an alkylating agent F-1 is carried out in the presence of a base such as sodium hydride, potassium hydride, potassium carbonate, sodium carbonate, cesium carbonate, etc., in a solvent such as dimethylformamide, tetrahydrofuran, etc., at 0°C to 120°C, preferably at room temperature to 120°C, for 1 to 48 hours, preferably 1 to 12 hours.

The above compounds C-1 and D-1 can be prepared by the following process.

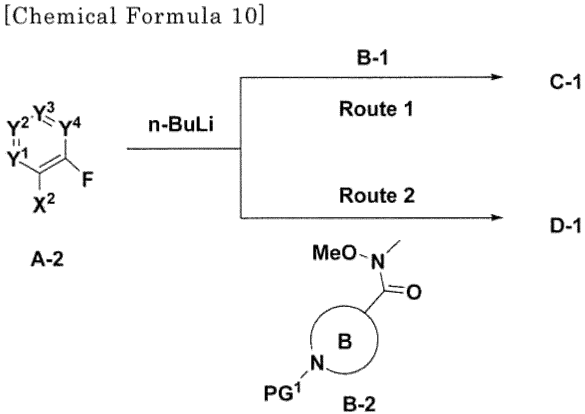

Here, Y¹, Y², Y³, Y⁴, B, and PG¹ are as defined above. X² represents Br or I.

### Route 1

In this process, compound A-2, which is commercially available or synthesized by a known method, is reacted with compound B-1 in the presence of butyllithium (n-BuLi) in a solvent such as THF, diethyl ether, etc., at -78°C to 0°C, preferably -78°C to -60°C, more preferably -78°C, for 30 minutes to 12 hours, preferably 30 minutes to 6 hours, more preferably 30 minutes to 3 hours, to afford compound C-1.

### Route 2

In this process, compound A-1, which is commercially available or synthesized by a known method, is reacted with compound B-2, which is commercially available or synthesized by a known method, in the presence of butyllithium (n-BuLi) in a solvent such as THF, diethyl ether, etc., at -78°C to 0°C, preferably -78°C to 60°C, more preferably -78°C, for 30 minutes to 12 hours, preferably 30 minutes to 6 hours, more preferably 30 minutes to 3 hours, to afford compound D-1.

### Synthesis of compound (G-2)

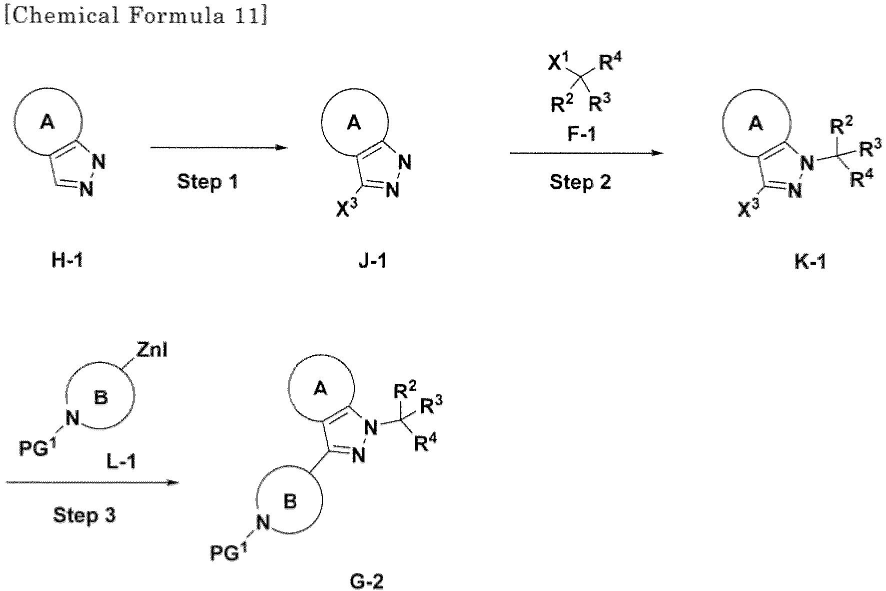

Here, A, R¹, R², R³, R⁴, B, X¹, and PG¹ are as defined above. X³ represents I, Cl, or Br.

### Step1

This process is the halogenation of compound H-1, which is commercially available or synthesized by a known method, to afford compound J-1. Halogenation can be performed by reactions normally used for halogenation of aromatic rings. Compound H-1 is reacted with chlorine, bromine, iodine, N-chlorosuccinimide (NCS), and N-bromosuccinimide (NBS) in a solvent such as dichloromethane, dichloroethane, carbon tetrachloride, DMF, etc., at 0°C to room temperature for 30 minutes to 48 hours, preferably 1 hour to 12 hours, to afford compound J-1.

### Step2

In this step, compound J-1 is alkylated with an alkylating agent F-1 in the presence of a base to afford compound K-1. Compound K-1 can be prepared by conducting the reaction under the same conditions as Step 4 in the synthesis of compound (G-1) above.

### Step3

This process is the Negishi coupling reaction of compound K-1 with compound L-1 to afford compound G-2. In this reaction, to compound K-1 are added a palladium catalyst such as tetrakistriphenylphosphine palladium (hereinafter referred to as "Pd(PPh₃)₄"), palladium(II) acetate (hereinafter referred to as "Pd(OAc)₂"), bis(triphenylphosphine)palladium(II) dichloride ("Pd(PPh₃)₂Cl₂"), [1,1'-bis(diphenylphosphino)ferrocene]dichloropalladium(II) ·dichloromethane adduct ("Pd(dppf)₂Cl₂"), etc., and compound L-1, which is commercially available or synthesized by a known method, and the mixture is reacted under a nitrogen atmosphere or argon atmosphere at 0°C to 150°C, preferably 60°C to 120°C, for 0.5 hours to 24 hours, preferably 1 hour to 12 hours, to afford compound G-2.

### Synthesis of compound (I)

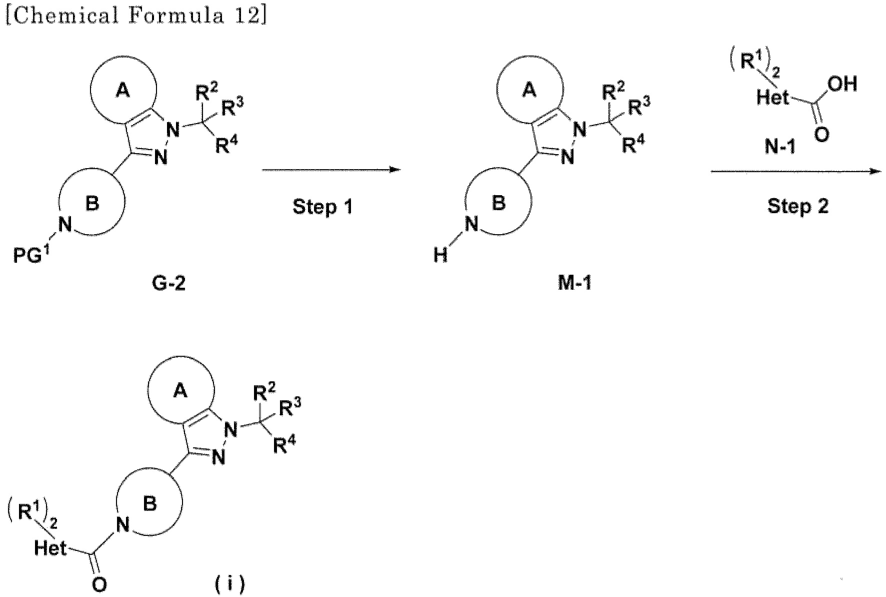

Here, A, B, R¹, R², R³, R⁴, Het, m, and PG¹ are as defined above.

### Step1

This step is the deprotection of the protecting group of compound G-2 to afford compound M-1, and can be performed with reference to Wuts and Greene, "Greene's Protective Groups in Organic Synthesis", 4th edition, John Wiley & Sons Inc., 2006, or P.J. Kocienski, "Protecting Groups", 3rd edition, Thimeme, 2005.

### Step2

This step is the condensation of compound M-1 with compound N-1 or its reactive compound N-1 in the presence of a condensing agent to afford compound (I).

In this reaction, compound M-1 is reacted with compound N-1 in the presence of an organic base such as TEA, DIPEA, N,N-dimethylaniline, or DBU and a condensing agent such as 1,1'-carbonyldiimidazole (hereinafter referred to as "CDI"), 1-ethyl-3-(3-dimethylaminopropyl)carbodiimide (hereinafter referred to as "EDCI"), diisopropylcarbodiimide ("DIC"), diethyl cyanophosphonate, O-(benzotriazol-1-yl)-N,N,N',N'-tetramethyluronium hexafluorophosphate (hereinafter referred to as "HBTU"), O-(7-azabenzotriazol-1-yl)-N,N,N',N'-tetramethyluronium hexafluorophosphate (hereinafter referred to as "HATU"), etc., in a solvent such as toluene, xylene, 1,4-dioxane, THF, DME, DMF, DMA, dichloromethane, or dichloroethane, at 0°C to 50°C, preferably at room temperature to 50°C, to afford compound (I).

In this step, the reactive compound of compound N-1 includes, for example, an acid halide (e.g., acid chloride, acid bromide), mixed acid anhydride, imidazolide, active amide, or other compounds normally used in amide condensation reactions.

In this step, other additives such as 1-hydroxybenzotriazole (hereinafter referred to as "HOBt"), N-hydroxysuccinimide, 1-hydroxy-7-azabenzotriazole (hereinafter referred to as "HOAt"), etc. can also be added, if necessary.

### Synthesis of compound (I-2)

### (When R⁴ is -CONR⁸R⁹)

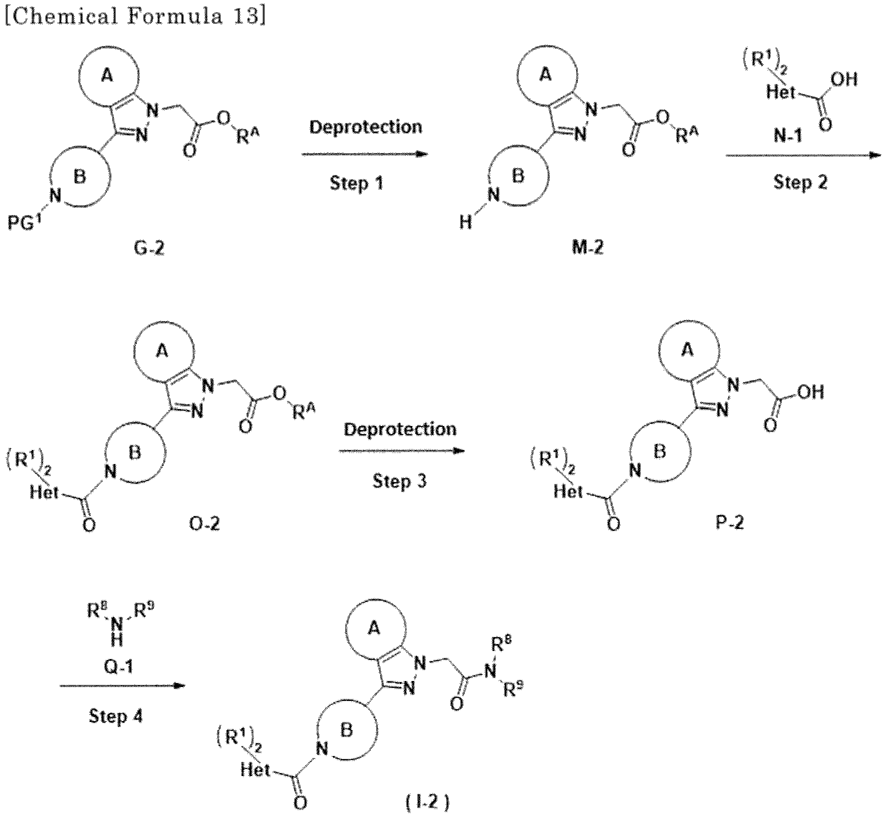

Here, A, B, PG¹, R¹, Het, R⁸, R⁹, and m are as defined above. R^{A} represents methyl, ethyl, n-propyl, or t-butyl.

### Step 1

This step is the deprotection of the protecting group of compound G-2 to afford compound M-2, and can be performed with reference to Wuts and Greene, "Greene's Protective Groups in Organic Synthesis", 4th edition, John Wiley & Sons Inc., 2006, or P.J. Kocienski, "Protecting Groups", 3rd edition. Thimeme, 2005.

### Step 2

This step is the condensation of compound M-2 with compound N-1 or its reactive compound in the presence of a condensing agent to afford compound O-2. It can be carried out by the same method as Step 2 in the synthesis of compound (I) above.

### Step 3

This step is the deprotection of the protecting group (PG²) of compound O-2 to afford compound P-2, and can be performed with reference to Wuts and Greene, "Greene's Protective Groups in Organic Synthesis", 4th edition, John Wiley & Sons Inc., 2006, or P.J. Kocienski, "Protecting Groups", 3rd edition, Thimeme, 2005.

### Step 4

This step is the condensation of compound Q-1 with compound P-2 or its reactive compound in the presence of a condensing agent to afford compound 1-2. It can be carried out by the same method as Step 2 in the synthesis of compound (I) above.

In the synthesis of compound I-2 above, the order of Step 1-Step 2 and Step 3-Step 4 can be switched to afford compound I-2. That is, the carboxylic acid obtained by deprotection (hydrolysis) of R^{A} of compound G-2 is condensed with compound Q-1. Then, the amine obtained by deprotection of PG¹ can be condensed with compound N-1 to afford compound I-2.

The compound of the present invention may be used as it is for pharmaceuticals, and can also be used in the form of a pharmaceutically acceptable salt, solvate or salt of the solvate thereof, according to a known method. Examples of pharmaceutically acceptable salts include salts with inorganic acids such as hydrochloric acid, hydrobromic acid, sulfuric acid, and phosphoric acid, and organic acids such as acetic acid, malic acid, lactic acid, citric acid, tartaric acid, maleic acid, succinic acid, fumaric acid, p-toluenesulfonic acid, benzenesulfonic acid, and methanesulfonic acid, or salts with alkali metal such as lithium, potassium and sodium, salts with alkaline earth metal such as magnesium and calcium, and salts with organic bases such as ammonium salts. These salts can be formed by methods well known in the art.

For example, a hydrochloride salt of the compound of the present invention can be prepared by dissolving the free base of the compound of the present invention in a solution of hydrogen chloride in alcohol, a solution of hydrogen chloride in ethyl acetate, a solution of hydrogen chloride in 1,4-dioxane, a solution of hydrogen chloride in cyclopentyl methyl ether, or a solution of hydrogen chloride in diethyl ether.

The compound of the present invention may incorporate solvent molecules and become a solvate by being left in the atmosphere or by recrystallization, and such a solvate is also included in the compound of the present invention. The solvates include solvates with solvent molecules such as a methanol solvate, an ethanol solvate, an isopropylalcohol solvate, a butanol solvate, a dimethyl sulfoxide solvate, an acetonitrile solvate, and the like, and a monohydrate, a dihydrate, etc.

Some of the compounds of the present invention may have an asymmetric carbon, and the respective stereo isomers and mixtures thereof are all included in the present invention. The stereo isomers can be prepared, for example, by means of optical resolution from the racemate thereof according to a known method using an optically active acid (e.g., tartaric acid, dibenzoyltartaric acid, mandelic acid, 10-camphor sulfonic acid, etc.), utilizing its basicity, or by using an optically active compound prepared in advance as a starting material. In addition, the stereo isomers may be prepared by optical resolution using a chiral column or by asymmetric synthesis.

The compound of the present invention has a DDR1 kinase inhibitory activity, as shown in Test Examples below.

Therefore, one embodiment of the present invention provides a DDRI kinase inhibitor comprising the compound of the present invention.

Also, one embodiment of the present invention provides a method for inhibiting a DDR1 kinase, which comprises administering the compound of the present invention to a subject in need thereof.

Further, one embodiment of the present invention provides the compound of the present invention for use in inhibiting a DDR1 kinase.

Furthermore, one embodiment of the present invention provides the use of the compound of the present invention for the manufacture of a DDR1 kinase inhibitor.

One embodiment of the present invention provides a prophylactic agent or therapeutic agent for a disease in which a DDR1 kinase is involved, comprising the compound of the present invention.

Also, one embodiment of the present invention provides a method for preventing or treating a disease in which a DDR1 kinase is involved, which comprises administering the compound of the present invention to a subject in need thereof.

Further, one embodiment of the present invention provides the compound of the present invention for use in preventing or treating a disease in which a DDR1 kinase is involved.

Furthermore, one embodiment of the present invention provides the use of the compound of the present invention for the manufacture of a prophylactic agent or therapeutic agent for a disease in which a DDR1 kinase is involved.

Diseases to which the compound of the present invention can be applied include, for example, Alport syndrome, IgA nephropathy, Goodpasture syndrome, anti-glomerular basement membrane nephritis, lupus nephritis, ANCA-related nephritis, diabetic nephropathy, purpura nephritis, focal segmental glomerulosclerosis, chronic kidney failure, minimal change nephrotic syndrome, mesangial proliferative glomerulonephritis, membranoproliferative glomerulonephritis, crescentic glomerulonephritis, membranous nephropathy, pulmonary fibrosis, myelofibrosis, liver fibrosis, acute myeloid leukemia, chronic myelogenous leukemia, acute lymphoid leukemia, chronic lymphocytic leukemia, Hodgkin lymphoma, non-Hodgkin lymphoma, glioma, non-small-cell lung cancer, small-cell lung cancer, breast cancer, ovarian cancer, prostate cancer, colorectal cancer or osteoarthrosis, and the like.

Therefore, one embodiment of the present invention provides a prophylactic agent or therapeutic agent for the above diseases, comprising the compound of the present invention.

Also, one embodiment of the present invention provides a method for preventing or treating the above diseases, which comprises administering the compound of the present invention to a subject in need thereof.

Further, one embodiment of the present invention provides the compound of the present invention for use in preventing or treating the above diseases.

Furthermore, one embodiment of the present invention provides the use of the compound of the present invention for the manufacture of a prophylactic agent or therapeutic agent for the above diseases.

"Subject" refers to a human or non-human animal that has or is suspected to have a disease in which DDR1 is involved. In one embodiment of the present invention, the subject is mammal. In one embodiment of the present invention, the subject is human.

The compound of the present invention can be used for a therapeutic agent for various diseases as mentioned above in a mammal such as human, mouse, rat, rabbit, dog, cat, cow, horse, pig, monkey, etc., as it is or as a pharmaceutical composition containing the compound in an amount, e.g., 0.001% to 99.5%, preferably 0.1% to 90%, in a pharmaceutically acceptable carrier, etc.

The carrier may be one or more of the customary solid, semi-solid or liquid diluents, fillers and other excipients used for formulation. The pharmaceutical composition according to the present invention is preferably administered in a unit dosage form. The pharmaceutical composition can be administered via intra-tissue, oral, intravenous, topical (transdermal, eye drops, intraperitoneal, intrathoracic, etc.) or rectal route. The pharmaceutical composition is administered in a dosage form suitable for the mode of administration.

The dose as a pharmaceutical is preferably adjusted taking into consideration the conditions such as age, weight, type and severity of disease of a patient, administration route, type of the compound of the invention, whether or not it is a salt, and the type of the salt. In general, the effective amount of the compound of the present invention or a pharmaceutically acceptable salt thereof for an adult, in the case of oral administration, is preferably within a range of 0.01 mg to 5 g/day, preferably 1 mg to 500 mg/day. In some cases, a smaller amount may be sufficient or a larger amount may be required. Usually, the dosage can be administered once a day or can be divided and administered several times a day, or in the case of intravenous administration, the dosage can be administered rapidly or sustainably within 24 hours.

The compound of the present invention can be used alone or in combination with additional therapeutic agents, such as angiotensin-converting enzyme inhibitors (ACE inhibitors) or angiotensin II AT1 receptor blockers (angiotensin receptor blocker = ARBs). Examples of ARBs include candesartan, losartan, valsartan, olmesartan, azilsartan, irbesartan, telmisartan, and the like. Examples of ACE inhibitors include ramipril, lisinopril, enalapril, imidapril, trandolapril, and the like.

One or more hydrogen, carbon and/or other atoms in the compound of the present invention may be replaced with an isotope thereof. Examples of such isotopes include ²H, ³H, ¹¹C, ¹³C, ¹⁴C, ¹⁵N, ¹⁸O, ¹⁷O, ³¹P, ³²P, ³⁵S, ¹⁸F, ¹²³I and ³³Cl, i.e., hydrogen, carbon, nitrogen, oxygen, phosphorus, sulfur, fluorine, iodine, and chlorine. The compound substituted with such an isotope may be useful as a pharmaceutical and includes all radiolabeled compounds of the compound of present invention.

The compound of the present invention can be prepared from a known compound per se or an intermediate easily preparable from a known compound, according to the following methods, Examples described below or known methods.

If the solvents, reagents and starting materials used in each step of the following processes are commercially available, such commercially available products can be used as they are. Also, the compounds obtained and the starting materials used in each step of the following processes may form a salt and can be converted by a well-known method into another type of salt or a free form. Alternatively, when the compound obtained or the starting material used in each step in the following processes is a free form. it can be converted into a desired salt by a known method. Examples of such salts include those similar to the salts as described for the compound of the present invention.

In the production of the compound of the present invention, when the starting material has a substituent capable of affecting the reaction, a protecting group may be introduced in these substituents by a known method in advance, and the target compound can be obtained by removing the protecting group after the reaction if necessary. Such protecting groups can be found, for example, in Wuts and Greene, "Greene's Protective Groups in Organic Synthesis", 4th edition, John Wiley & Sons Inc., 2006, or P.J. Kocienski, "Protecting Groups", 3rd edition, Thimeme, 2005, and may be selected as appropriate according to the reaction conditions.

The compound obtained in each step of the following processes can be isolated or purified according to a conventional method such as solvent extraction, concentration, distillation, sublimation, recrystallization, reprecipitation, chromatography, and the like. Alternatively, the compound may be used in the next step as a reaction mixture or a crude product.

The present invention is described in more detail with reference to, but is not limited to, the following Reference Examples, Examples and Test Examples.

MS was performed using LCMS. ESI was used as a method for ionization. Observed values of the mass spectrometry are expressed as m/z.

The conditions for LCMS were as follows:
Instrument: ACQUITY UPLC MS/PDA system (Waters)
Mass spectrometry: ACQUITY QDa detector or Waters 3100 MS detector
Photodiode array detector: ACQUITY PDA detector (UV-detected wave length: 210 to 400 nm)
Column: Acquity BEH C18, 1.7 µm, 2.1×50 mm
Flow rate: 0.5 mL/min
Column temperature: 40°C
Solvent;
A: 0.1% formic acid/H₂O (v/v; the same shall apply hereinafter)
B: 0.1% formic acid/acetonitrile

¹H NMR spectrum was obtained by using JNM-ECS400 Nuclear Magnetic Resonance Spectrometer (JEOL RESONANCE Ltd.). The observed peaks were shown as chemical shift values δ (ppm) (s = singlet, d = doublet, t = triplet, q = quartet, brs = broad singlet, m = multiplet, dd = double doublet, dt = double triplet).

In the experiment using microwave, Initiator 60 (Biotage) was used, which can achieve a temperature of 40 to 250°C and a pressure of up to 20 bar.

The conditions for the measurement of optical rotation are as follows. Instrument: Automatic optical rotation meter SEPA-500 (HORIBA, Ltd.)

The compounds described herein were named using naming software, ACD/NAME (Registered Trademark, Advanced Chemistry Development Inc.) according to IUPAC nomenclature rules, or ChemBioDraw (version 18.2, 19.1 or 20.1.1, Cambridge Soft), or named according to IUPAC nomenclature.

In a name of a compound, the descriptors "r" and "s" (lowercase letters) refer to the stereochemistry of a pseudoasymmetric carbon atom according IUPAC rules.

### Reference Example 1 2-[3-(azetidin-3-yl)-4-chloro-5-fluoro-1H-indazol-1-yl]-N-(2,2.2-trifluoroethyl)acetamide hydrochloride (Compound RE-1)

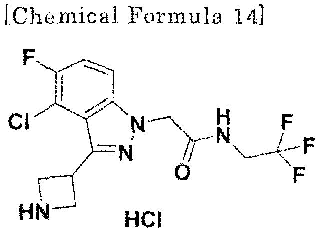

### [Step 1] Preparation of Intermediate RE-1A

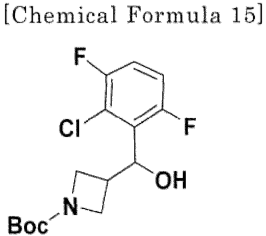

To a mixture of 2-chloro-1,4-difluorobenzene (1.2 g) and THF (11 mL) was added dropwise lithium diisopropylamide (1.08 M n-hexane/tetrahydrofuran solution, 7.50 mL) at -78°C. After stirring at -78°C for 1 hour, a solution of tert-butyl 3-formylazetidine-1-carboxylate (1.0 g) in THF (11 mL) was added dropwise and then the mixture was stirred at -78°C for 3 hours. A saturated aqueous solution of ammonium chloride was added to the reaction solution, and the mixture was extracted with ethyl acetate. The organic layer was washed with a saturated saline solution and dried over anhydrous magnesium sulfate. The solvent was removed under reduced pressure, and then the residue was purified by silica gel column chromatography to afford the title compound (1.65 g).

### [Step 2] Preparation of Intermediate RE-1B

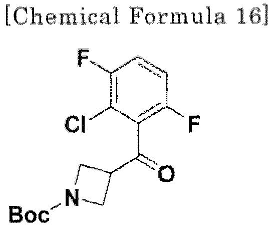

To a mixture of Intermediate RE-1A (3.23 g) and dichloromethane (48 mL) were added iodobenzene diacetate (3.74 g) and AZADOL^{®} (74 mg) sequentially, and then the mixture was stirred at room temperature for 4 hours. A saturated aqueous solution of sodium bicarbonate was added to the reaction solution, and the mixture was extracted with dichloromethane. The organic layer was washed with a saturated saline solution and dried with anhydrous magnesium sulfate. The solvent was removed under reduced pressure, and then the residue was purified by silica gel column chromatography to afford the title compound (3.09 g). MS (m/z): 332.5 [M+H]⁺

### [Step 3] Preparation of Intermediate RE-1C

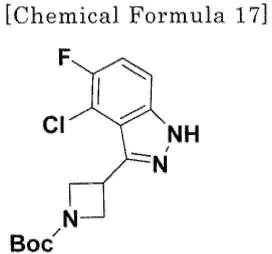

A mixture of Intermediate RE-1B (3.09 g), 1,4-dioxane (60 mL), and hydrazine monohydrate (4.54 mL) was stirred at 100°C overnight. To the reaction solution was added water, and the mixture was extracted with ethyl acetate. The organic layer was washed with a saturated saline solution and dried with anhydrous magnesium sulfate. The solvent was removed under reduced pressure, and then the residue was purified by silica gel column chromatography to afford the title compound (1.47 g). MS (m/z): 324.0 [M-H]⁻

### [Step 4] Preparation of Intermediate RE-1D

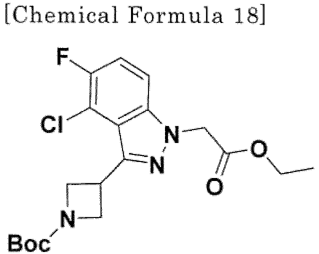

To a mixture of Intermediate RE-1C (1.47 g) and DMF (23 mL) were added cesium carbonate (1.76 g) and ethyl 2-bromoacetate (0.600 mL) sequentially under an ice bath condition, and then the mixture was stirred at room temperature for 3 hours. A saturated aqueous solution of ammonium chloride was added to the reaction solution, and the mixture was extracted with ethyl acetate. The organic layer was washed with a saturated saline solution and dried with anhydrous magnesium sulfate. The solvent was removed under reduced pressure, and then the residue was purified by silica gel column chromatography to afford the title compound (1.76 g). MS (m/z): 412.3 [M+H]⁺

### [Step 5] Preparation of Intermediate RE-1E

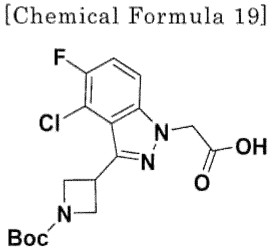

To a mixture of Intermediate RE-1D (1.75 g), methanol (8.5 mL), and THF (8.5 mL) was added 2 M aqueous sodium hydroxide solution (10.6 mL) at room temperature, and then the mixture was stirred overnight. The reaction solution was concentrated under reduced pressure, diluted with water, and acidified with 2 M hydrochloric acid under an ice bath condition. The precipitate was collected by filtration to afford the title compound (1.56 g). MS (m/z): 382.4 [M-H]⁻

### [Step 6] Preparation of Intermediate RE-1F

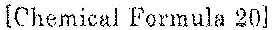

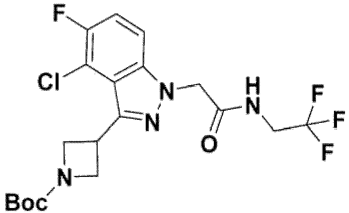

To a mixture of Intermediate RE-1E (800 mg), DMF (6.9 mL), DIPEA (1.1 mL) and 2,2,2-trifluoroethaneamine hydrochloride (367 mg) was added HATU (1.03 g), and then the mixture was stirred at room temperature for 3 hours. A saturated aqueous solution of sodium bicarbonate was added to the reaction solution, and the mixture was extracted with ethyl acetate. The organic layer was washed with a saturated saline solution and dried with anhydrous magnesium sulfate. The solvent was removed under reduced pressure, and then the residue was purified by silica gel column chromatography to afford the title compound (900 mg). MS (m/z): 463.4 [M-H]⁻

### [Step 7] Preparation of Compound RE-1

To a mixture of Intermediate RE-1F (78 mg) and methanol (1 mL) was added hydrogen chloride (2 M methanol solution, 1 mL), and then the mixture was stirred at room temperature overnight. The solvent was removed under reduced pressure to afford the title compound (72 mg). MS (m/z): 365.5 [M+H]⁺

### Reference Example 2 2-[3-(azetidin-3-yl)-4,6-difluoro-1H-indazol-1-yl]-1-[(2S)-2-(trifluoromethyl)pyrrolidin-1-yl]ethan-1-one hydrochloride (Compound RE-2)

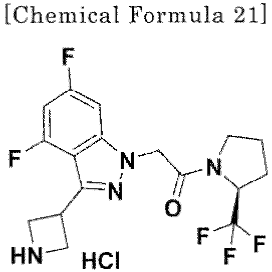

### [Step 1] Preparation of Intermediate RE-2A

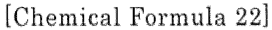

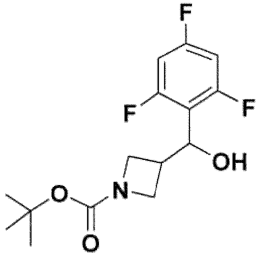

In the method according to Step 1 of Reference Example 1, 1,3,5-trifluorobenzene (7.49 g) was used instead of 2-chloro-1,4-difluorobenzene to afford the title compound (9.74 g).

### [Step 2] Preparation of Intermediate RE-2B

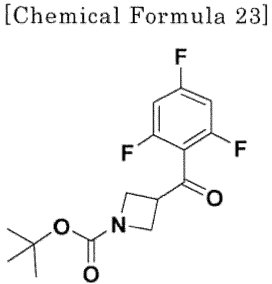

In the method according to Step 2 of Reference Example 1, Intermediate RE-2A (9.74 g) was used instead of Intermediate RE-1A to afford the title compound (9.49 g).

### [Step 3] Preparation of Intermediate RE-2C

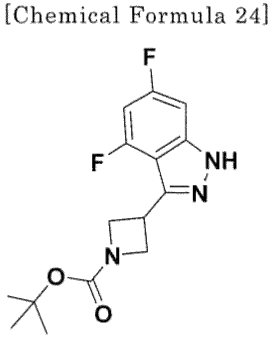

In the method according to Step 3 of Reference Example 1. Intermediate RE-2B (9.49 g) was used instead of Intermediate RE-1B to afford the title compound (8.25 g). MS (m/z): 308.3 [M-H]⁻.

### [Step 4] Preparation of Intermediate RE-2D

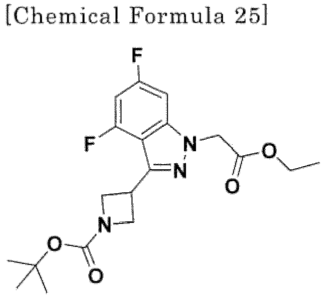

In the method according to Step 4 of Reference Example 1, Intermediate RE-2C (5.0 g) was used instead of Intermediate RE-1C to afford the title compound (6.2 g). MS (m/z): 296.0 [M-Boc+2H] ⁺

### [Step 5] Preparation of Intermediate RE-2E

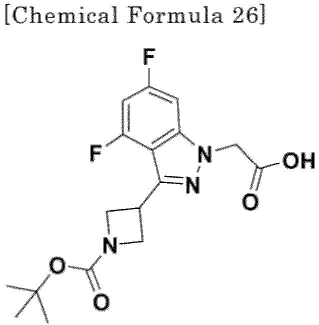

In the method according to Step 5 of Reference Example 1, Intermediate RE-2D (2.23 g) was used instead of Intermediate RE-1D to afford the title compound (1.89 g). MS (m/z): 366.4 [M-H]⁻

### [Step 6] Preparation of Intermediate RE-2F

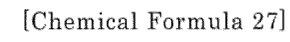

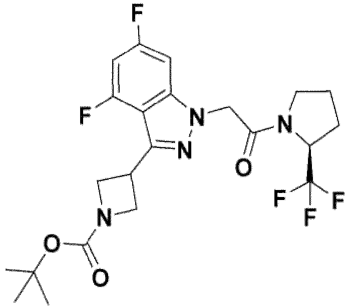

In the method according to Step 6 of Reference Example 1, Intermediate RE-2E (1.04 g) and (2S)-2-(trifluoromethyl)pyrrolidine hydrochloride (596 mg) were used instead of Intermediate RE-1E and 2,2,2-trifluoroethaneamine hydrochloride, respectively, to afford the title compound (1.32 g). MS (m/z): 511.2 [M+Na]⁺

### [Step 7] Preparation of Compound RE-2

In the method according to Step 7 of Reference Example 1, Intermediate RE-2F (125 mg) was used instead of Intermediate RE-1F to afford the title compound (117 mg). MS (m/z): 389.1 [M+H]⁺

### Reference Example 3 2-[3-(azetidin-3-yl)-4,6-difluoro-1H-indazol-1-yl]-1-[(2S)-2-(trifluoromethyl)pyrrolidin-1-yl]ethan-1-one trifluoroacetate (Compound RE-3)

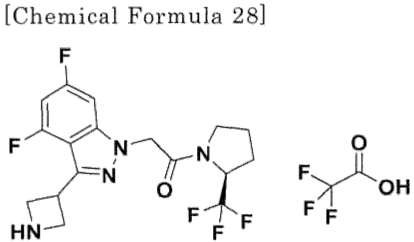

To a mixture of Intermediate RE-2F (100 mg) and dichloromethane (1 mL) was added trifluoroacetic acid (0.25 mL), and then the mixture was stirred at room temperature for 1 hour. The solvent was removed under reduced pressure to afford the title compound (103 mg). MS (m/z): 389.1 [M+H]⁺

### Reference Example 4 2-[3-(azetidin-3-yl)-1H-indazol-1-yl]-1-[(2S)-2-(trifluoromethyl)pyrrolidin-1-yl]ethan-1-one trifluoroacetate (Compound RE-4)

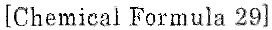

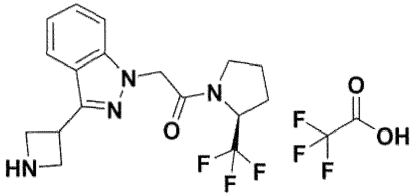

### [Step 1] Preparation of Intermediate RE-4A

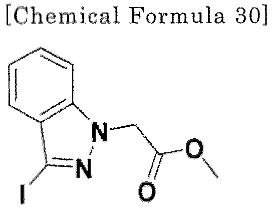

To a mixture of 3-iodo-2H-indazole (9.11 g) and DMF (75 mL) were added cesium carbonate (14.6 g) and methyl 2-bromoacetate (4.24 mL) sequentially under an ice bath condition. After stirring for 30 minutes under an ice bath condition, the mixture was stirred at room temperature for 1 hour. To the reaction solution was added a saturated aqueous solution of ammonium chloride, and the mixture was extracted with ethyl acetate. The organic layer was washed with a saturated saline solution and dried with anhydrous magnesium sulfate. The solvent was removed under reduced pressure, and then the residue was purified by silica gel column chromatography to afford the title compound (12.69 g). MS (m/z): 317.0 [M+H]⁺

### [Step 2] Preparation of Intermediate RE-4B

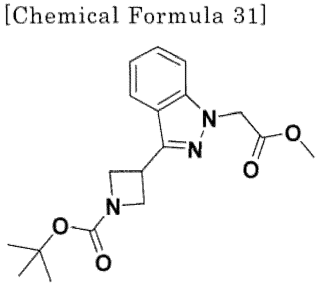

A reaction vessel containing zinc (3.62 g) was heated with a heat gun under reduced pressure for 3 minutes and replaced with argon. After bringing the reaction vessel to room temperature, DMA (40 mL) was added and degassed with argon. After that, to the mixture were added 1,2-dibromoethane (0.341 mL) and chloro(trimethyl)silane (0.602 mL) and then the mixture was stirred at room temperature for 15 minutes. A solution of tert-butyl 3-iodoazetidine-1-carboxylate (11.2 g) in DMA (40 mL) was added dropwise to the mixture through a dropping funnel. The mixture was stirred at room temperature for 1 hour to afford a solution of [1-(tert-butoxycarbonyl)azetidin-3-yl]zinc(II) iodide in DMA.

Next, a mixture of Intermediate RE-4A (5.00 g) and DMA (16 mL) was degassed with argon, and then Pd(dppf)Cl₂ ·CH₂Cl₂ (1.29 g) and copper(I) iodide (392 mg) were added thereto. To this mixture was added the previously prepared solution of [1-(tert-butoxycarbonyl)azetidin-3-yl]zinc(II) iodide in DMA at room temperature, and then the mixture was stirred at 85°C for 1 hour. A saturated aqueous solution of ammonium chloride was added to the reaction solution, and the mixture was extracted with ethyl acetate. The organic layer was washed with a saturated saline solution and dried over anhydrous magnesium sulfate. The solvent was removed under reduced pressure, and then the residue was purified by silica gel column chromatography to afford the title compound (5.05 g). MS (m/z): 368.1 [M+Na]⁺

### [Step 3] Preparation of Intermediate RE-4C

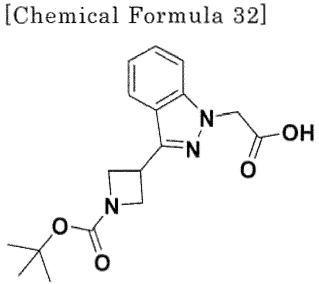

In the method according to Step 5 of Reference Example 1, Intermediate RE-4B (5.08 g) was used instead of Intermediate RE-1D to afford the title compound (4.99 g). MS (m/z): 330.4 [M-H]⁻

### [Step 4] Preparation of Intermediate RE-4D

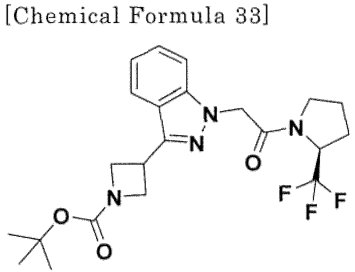

To a mixture of Intermediate RE-4C (150 mg), DMF (1.5 mL), DIPEA (0.235 mL), and (2S)-2-(trifluoromethyl)pyrrolidine hydrochloride (95 mg) was added HBTU (206 mg), and then the mixture was stirred at room temperature for 2 hours. A saturated aqueous solution of sodium bicarbonate was added to the reaction solution, and the mixture was extracted with ethyl acetate. The organic layer was washed with a saturated saline solution and dried with anhydrous magnesium sulfate. The solvent was removed under reduced pressure, and then the residue was purified by silica gel column chromatography to afford the title compound (200 mg). MS (m/z): 353.1 [M-Boc+2H]⁺

### [Step 5] Preparation of Compound RE-4

To a mixture of Intermediate RE-4D (200 mg) and dichloromethane (4 mL) was added trifluoroacetic acid (1 mL), and then the mixture was stirred at room temperature for 1 hour. The solvent was removed under reduced pressure to afford the title compound (211 mg). MS (m/z): 353.1 [M+H]⁺

### Reference Example 5 2-[3-(azetidin-3-yl)-6-chloro-1H-indazol-1-yl]-N-methyl-N-(2,2,2-trifluoroethyl)acetamide trifluoroacetate (Compound RE-5)

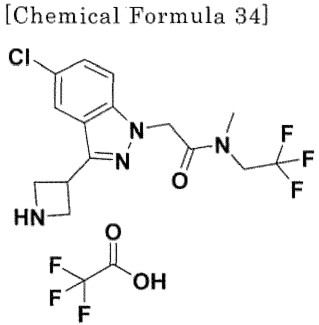

### [Step 1] Preparation of Intermediate RE-5A

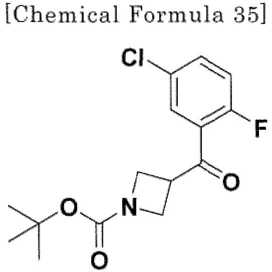

To a mixture of 2-bromo-4-chloro-1-fluorobenzene (2.57 g) and THF (20 mL) was added n-butyl lithium (1.57 M solution in n-hexane, 7.82 mL) dropwise at - 78°C. After stirring at -78°C for 1 hour. to the mixture was added dropwise a solution of tert-butyl 3·[methoxy(methyl)carbamoyl]azetidine-1-carboxylate (2.0 g) (for example, synthesized according to a method described in WO20129649) in THF (20 mL), and then the mixture was stirred at -78°C for 1 hour. A saturated aqueous solution of ammonium chloride was added to the reaction solution, and the mixture was extracted with ethyl acetate. The organic layer was washed with a saturated saline solution and dried over anhydrous magnesium sulfate. The solvent was removed under reduced pressure, and then the residue was purified by silica gel column chromatography to afford the title compound (1.21 g). MS (m/z): 214.0 [M-Boc+2H]⁺

### [Step 2] Preparation of Intermediate RE-5B

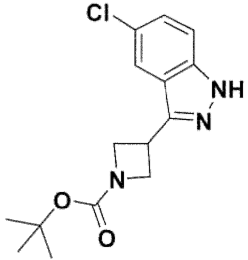

A mixture of Intermediate RE-5A (1.21 g), 1,4-dioxane (39 mL) and hydrazine monohydrate (0.940 mL) was stirred at 100°C for 2 days. To the reaction solution was added water, and the mixture was extracted with ethyl acetate. The organic layer was washed with a saturated saline solution and dried with anhydrous magnesium sulfate. The solvent was removed under reduced pressure, and then the residue was purified by silica gel column chromatography to afford the title compound (635 mg). MS (m/z): 208.0 [M-Boc+2H] ⁺

### [Step 3] Preparation of Intermediate RE-5C

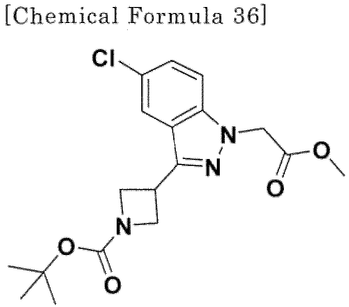

In the method according to Step 1 of Reference Example 4, Intermediate RE-5B (200 mg) was used instead of 3-iodo-2H-indazole to afford the title compound (246 mg). MS (m/z): 402.3 [M+Na] ⁺

### [Step 4] Preparation of Intermediate RE-5D

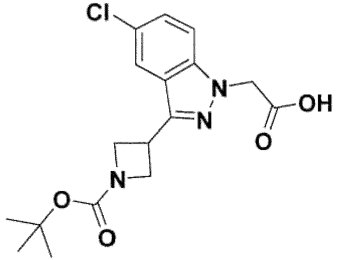

In the method according to Step 5 of Reference Example 1, Intermediate RE-5C (246 mg) was used instead of Intermediate RE-1D to afford the title compound (184 mg). MS (m/z): 364.3 [M-H]⁻

### [Step 5] Preparation of Intermediate RE-5E

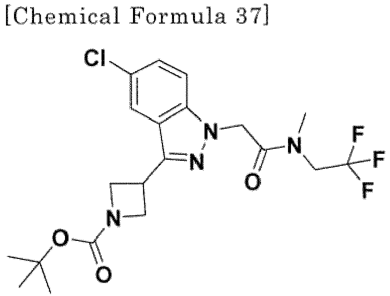

To a mixture of Intermediate RE-5D (100 mg), acetonitrile (2 mL), 1-hydroxybenzotriazole monohydrate (50 mg), NMM (0.090 mL) and 2,2,2-trifluoro-N-methylethanamine hydrochloride (49 mg) was added EDCI·HCl (63 mg), and then the mixture was stirred at room temperature for 2 hours. A saturated aqueous solution of sodium bicarbonate was added to the reaction solution, and the mixture was extracted with ethyl acetate. The organic layer was washed with a saturated saline solution and dried with anhydrous magnesium sulfate. The solvent was removed under reduced pressure to afford the title compound (123 mg). MS (m/z): 483.2 [M+Na]⁺

### [Step 6] Preparation of Compound RE-5

In the method according to Reference Example 3, Intermediate RE-5E (123 mg) was used instead of Intermediate RE-2F to afford the title compound (127 mg). MS (m/z): 361.2 [M+H]⁺

### Reference Example 6 2-[3-(azetidin-3-yl)-6-fluoro-1H-indazo]-1-yl]-N-methyl-N-(2,2,2-trifluoroethyl)acetamide trifluoroacetate (Compound RE-6)

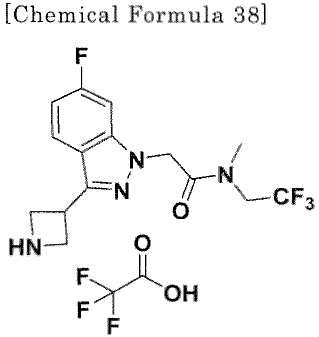

### [Step 1] Preparation of Intermediate RE-6A

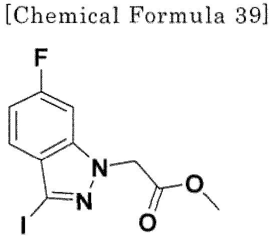

To a mixture of 6-fluoro-3-iodo-1H-indazole (for example, synthesized according to a method described in Bioorganic Med. Chem. Lett., 2010, 20, 6998-7003) (897 mg) and DMF (7 mL) were added cesium carbonate (1.34 g) and methyl 2-bromoacetate (0.39 mL) sequentially under an ice bath condition. After stirring for 10 minutes under an ice bath condition, the mixture was stirred at room temperature for 2 hours. A saturated aqueous solution of ammonium chloride was added to the reaction solution, and the mixture was extracted with a mixed solvent of ethyl acetate and hexane. The organic layer was washed with a saturated saline solution and dried with anhydrous magnesium sulfate. The solvent was distilled off at reduced pressure, and then the residue was purified by silica gel column chromatography to afford the title compound (1.17 g). MS (m/z): 335.0 [M+H]⁺

### [Step 2] Preparation of Intermediate RE-6B

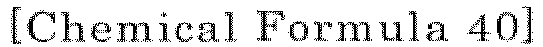

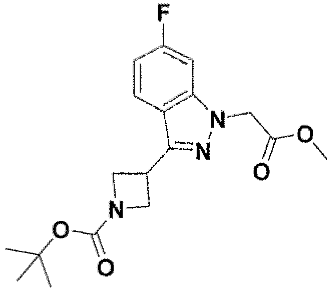

A reaction vessel containing zinc (481 mg) was heated with a heat gun under reduced pressure for 5 minutes and replaced with argon. After bringing the reaction vessel to room temperature, DMA (5.25 mL) was added and degassed with argon. Then, to the mixture were added,1,2-dibromoethane (0.045 mL) and chloro(trimethyl)silane (0.080 mL), and then the mixture was stirred for 15 minutes at room temperature. A solution of tert-butyl 3-iodoazetidine-1-carboxylate (1.49 g) in DMA (5.25 mL) was added dropwise to the mixture through a dropping funnel. Then, the mixture was stirred at room temperature for 1 hour to afford a solution of [1-(tert-butoxycarbonyl)azetidin-3-yl]zinc(II) iodide in DMA.

Next, a mixture of Intermediate RE-6A (1.17 g) and DMA (3.5 mL) was degassed with argon, and Pd(dppf)Cl₂ ·CH₂Cl₂ (286 mg) and copper(I) iodide (87 mg) were added thereto. To this mixture was added the previously prepared solution of [1-(tert-butoxycarbonyl)azetidin-3-yl]zinc(II) iodide in DMA at room temperature, and then the mixture was stirred at 85°C for 1 hour. To the reaction solution was added water, and the mixture was extracted with a mixed solvent of ethyl acetate and hexane. The organic layer was washed with a saturated saline. solution and dried with anhydrous magnesium sulfate. The solvent was removed under reduced pressure, and then the residue was purified by silica gel column chromatography to afford the title compound (1.54 g). MS (m/z): 386.3 [M+Na]⁺

### [Step 3] Preparation of Intermediate RE-6C

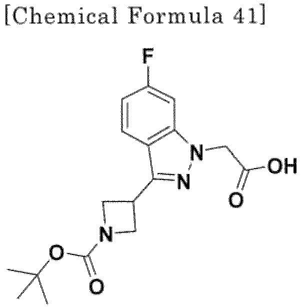

To a mixture of Intermediate RE-6B (1.54 g), methanol (6 mL) and THF (6 mL) was added 2 M aqueous sodium hydroxide solution (6.36 mL) at room temperature, and then the mixture was stirred for 2 hours. The reaction solution was concentrated under reduced pressure, diluted with water, filtered, and acidified with 2 M hydrochloric acid under an ice bath condition. The precipitate was collected by filtration to afford the title compound (1.03 g). MS (m/z): 348.2 [M-H]⁻

### [Step 4] Preparation of Intermediate RE-6D

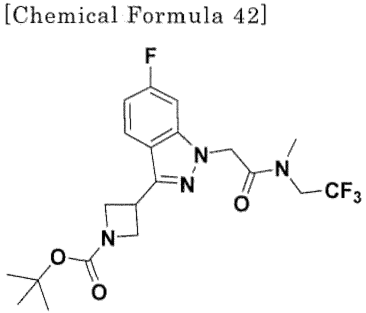

To a mixture of Intermediate RE-6C (350 mg), acetonitrile (3 mL), 1-hydroxybenzotriazole monohydrate (184 mg), NMM (0.330 mL) and 2,2,2-trifluoro-N-methylethaneamine hydrochloride (180 mg) was added EDCI·HCl (231 mg), and then the mixture was stirred at room temperature overnight. A saturated aqueous solution of sodium bicarbonate was added to the reaction solution, and the mixture was extracted with ethyl acetate. The organic layer was washed with a saturated saline solution and dried with anhydrous magnesium sulfate. The solvent was removed from the mixture under reduced pressure, and then the mixture was purified by silica gel column chromatography to afford the title compound (318 mg). MS (m/z): 467.3 [M+Na]⁺

### [Step 5] Preparation of Compound RE-6

In the method according to Reference Example 3, Intermediate RE-6D (318 mg) was used instead of Intermediate RE-2F to afford the title compound (285 mg). MS (m/z): 345.2 [M+H]⁺

### Reference Example 7 2-[3-(3-methylazetidin-3-yl)-1H-indazol-1-yl]-1-[(2S)-2-(trifluoromethyl)pyrrolidin-1-yl]ethan-1-one trifluoroacetate (Compound RE-7)

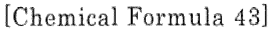

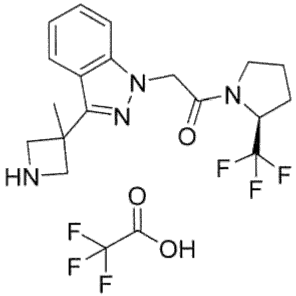

### [Step 1] Preparation of Intermediate RE-7A

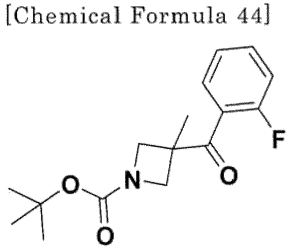

To a mixture of 3-methyl-1-[(2-methylpropan-2-yloxycarbonyl]azetidine-3-carboxylic acid (747 mg), acetonitrile (10 mL), HOBt (797 mg), NMM (2.30 mL) and N,O-dimethylhydroxylamine hydrochloride (677 mg) was added EDCI ·HCl (1.13 g), and then the mixture was stirred at room temperature overnight. To the reaction solution was added a saturated aqueous solution of sodium bicarbonate, and the mixture was extracted with ethyl acetate. The organic layer was washed with a saturated saline solution and dried with anhydrous magnesium sulfate. The solvent was removed under reduced pressure to afford tert-butyl 3-[methoxy(methyl)carbamoyl]-3-methylazetidine-1-carboxylate.

Then, in the method according to Step 1 of Reference Example 5, 1-fluoro-2-iodobenzene (1.16 g) and tert-butyl 3-[methoxy(methyl)carbamoyl]-3-methylazetidine-1-carboxylate obtained above were used instead of 2-bromo-4-chloro-1-fluorobenzene and tert-butyl 3-[methoxy(methyl)carbamoyl]azetidine-1-carboxylate, respectively, to afford the title compound (688 mg). MS (m/z): 194.2 [M-Boc+2H]⁺

### [Step 2] Preparation of Intermediate RE-7B

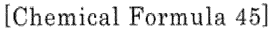

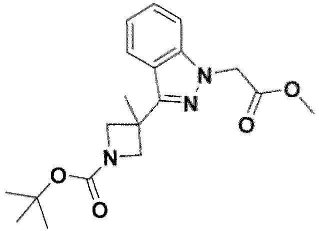

A mixture of Intermediate RE-7A (620 mg), DMA (7 mL), hydrazine monohydrate (1.03 mL) and potassium carbonate (438 mg) was reacted in a microwave reactor at 150°C for 3 hours. After cooling, a saturated aqueous solution of ammonium chloride was added to the reaction solution, and the mixture was extracted with ethyl acetate. The organic layer was washed with a saturated saline solution and dried with anhydrous magnesium sulfate. The solvent was removed under reduced pressure, and then the residue was purified by silica gel column chromatography to afford tert-butyl 3-(1H-indazol-3-yl)-3-methylazetidine-1-carboxylate.

Then, in the method according to Step 1 of Reference Example 4, tert-butyl 3-(1H-indazol-3-yl)-3-methylazetidine-1-carboxylate as described above was used instead of 3-iodo-2H-indazole to afford the title compound (629 mg). MS (m/z): 260.3 [M-Boc+2H]⁺

### [Step 3] Preparation of Intermediate RE-7C

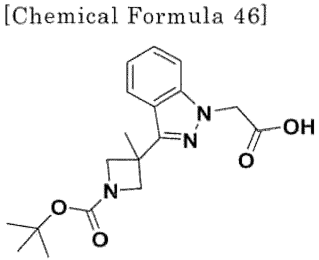

In the method according to Step 5 of Reference Example 1, Intermediate RE-7B (629 mg) was used instead of Intermediate RE-1D to afford the title compound (466 mg). MS (m/z): 344.3 [M-H]⁻

### [Step 4] Preparation of Intermediate RE-7D

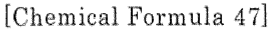

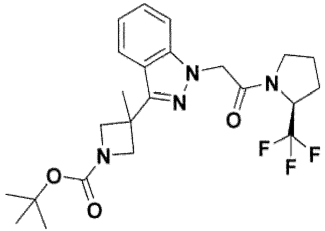

To a mixture of Intermediate RE-7C (100 mg), DMF (1.0 mL), DIPEA (0.150 mL) and (2S)-2-(trifluoromethyl)pyrrolidine hydrochloride (61 mg) was added HBTU (132 mg), and then the mixture was stirred at room temperature overnight. A saturated aqueous solution of sodium bicarbonate was added to the reaction solution, and the mixture was extracted with ethyl acetate. The reaction solution was purified by silica gel column chromatography to afford the title compound (108 mg). MS (m/z): 489.4 [M+Na]⁺

### [Step 5] Preparation of Compound RE-7

In the method according to Reference Example 3, Intermediate RE-7D (108 mg) was used instead of Intermediate RE-2F to afford the title compound (111 mg). MS (m/z): 367.3 [M+H]⁺

### Reference Example 8 2-[3-(azetidin-3-yl)-1H-indazol-1-yl]-1-(3,3-dimethylmorpholin-4-yl)ethan-1-one trifluoroacetate (Compound RE-8)

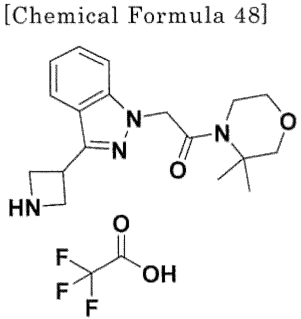

### [Step 1] Preparation of Intermediate RE-8A

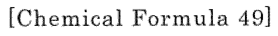

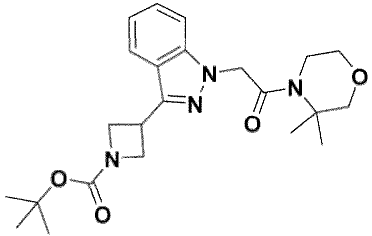

In the method according to Step 4 of Reference Example 7, Intermediate RE-4C (100 mg) and 3,3-dimethylmorpholine (45 mg) were used instead of Intermediate RE-7C and (2S)-2-(trifluoromethyl)pyrrolidine hydrochloride, respectively, to afford the title compound (100 mg). MS (m/z): 329.2 [M-Boc+2H]⁺

### [Step 2] Preparation of Compound RE-8

In the method according to Reference Example 3, Intermediate RE-8A (100 mg) was used instead of Intermediate RE-2F to afford the title compound (96 mg). MS (m/z): 329.2 [M+H]⁺

### Reference Example 9 2-[3-(azetidin-3-yl)-1H-indazol-1-yl]-1-[(2R)-2-(trifluoromethyl)morpholin-4-yl]ethan-1-one trifluoroacetate (Compound RE-9)

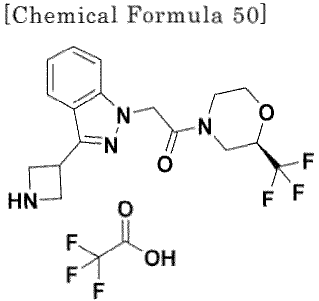

### [Step 1] Preparation of Intermediate RE-9A

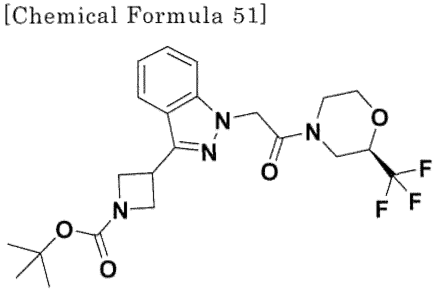

In the method according to Step 4 of Reference Example 7, Intermediate RE-4C (100 mg) and (2R)-2-(trifluoromethyl)morpholine hydrochloride (69 mg) were used instead of Intermediate RE-7C and (2S)-2-(trifluoromethyl)pyrrolidine hydrochloride, respectively, to afford the title compound (135 mg). MS (m/z): 369.1 [M-Boc+2H]⁺

### [Step 2] Preparation of Compound RE-9

In the method according to Reference Example 3, Intermediate RE-9A (135 mg) was used instead of Intermediate RE-2F to afford the title compound (145 mg). MS (m/z): 369.1 [M+H]⁺

### Reference Example 10 4-fluoro-3-(piperidin-4-yl)-1-{[5-(trifluoromethyl)pyridin-2-yl]methyl}-1H-indazole trifluoroacetate (Compound RE-10

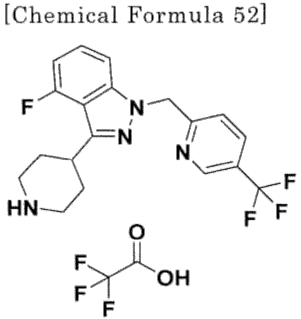

### [Step 1] Preparation of Intermediate RE-10A

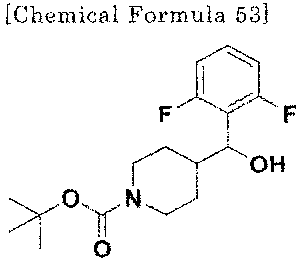

In the method according to Step 1 of Reference Example 1, 1,3-difluorobenzene (2.41 g) and tert-butyl-4-formylpiperidine-1-carboxylate (3.00 g) were used instead of 2-chloro-1,4-difluorobenzene and tert-butyl 3-formylazetidine-1-carboxylate, respectively, to afford the title compound (3.98 g).

### [Step 2] Preparation of Intermediate RE-10B

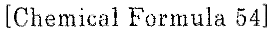

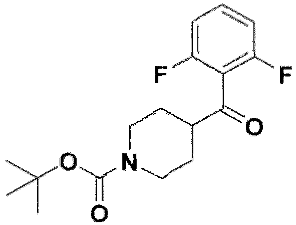

In the method according to Step 2 of Reference Example 1, Intermediate RE-10A (3.98 g) was used instead of Intermediate RE-1A to afford the title compound (3.48 g).

### [Step 3] Preparation of Intermediate RE-10C

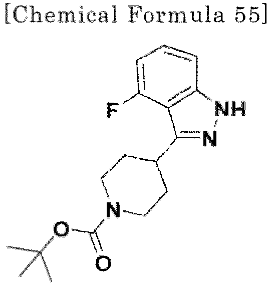

In the method according to Step 3 of Reference Example 1, Intermediate RE-10B (3.48 g) was used instead of Intermediate RE-1B to afford the title compound (3.31 g). MS (m/z): 318.3 [M-H]⁻

### [Step 4] Preparation of Intermediate RE-10D

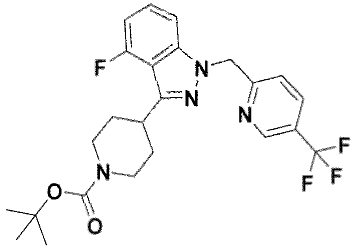

To a mixture of Intermediate RE-10C (50 mg) and acetonitrile (0.5 mL) were added cesium carbonate (77 mg) and 2-(bromomethyl)-5-(trifluoromethyl)pyridine (56 mg) sequentially, and then the mixture was stirred at room temperature for 2 hours. The reaction solution was purified by silica gel column chromatography to afford the title compound (34 mg). MS (m/z): 379.1 [M-Boc+2H]⁺

### [Step 5] Preparation of Compound RE-10

In the method according to Reference Example 3, Intermediate RE-10D (34 mg) was used instead of Intermediate RE-2F to afford the title compound (35 mg). MS (m/z): 379.2 [M+H]⁺

### Reference Example 11 2-[4-fluoro-3-(piperidin-4-yl)-1H-indol-1-yl]-1-[(2S)-2-(trifluoromethyl)pyrrolidin-1-yl]ethan-1-one hydrochloride (Compound RE-11)

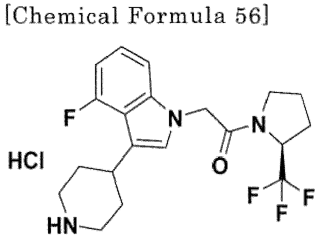

### [Step 1] Preparation of Intermediate RE-11A

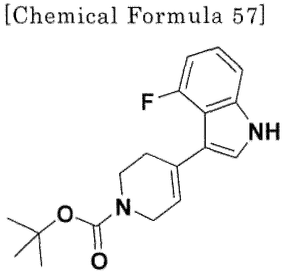

A mixture of 4-fluoro-1H-indazole (750 mg), tert-butyl 4-oxopiperidine-1-carboxylate (1.22 g), potassium hydroxide (1.25 g) and methanol (18 mL) was stirred at 80°C overnight. To the reaction solution was added water, and then the mixture was extracted with ethyl acetate and dried over anhydrous sodium sulfate. The solvent was removed from the mixture under reduced pressure, and then the mixture was purified by silica gel column chromatography to afford the title compound (660 mg). MS (m/z): 317.1 [M+H]⁺

### [Step 2] Preparation of Intermediate RE-11B

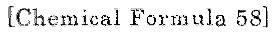

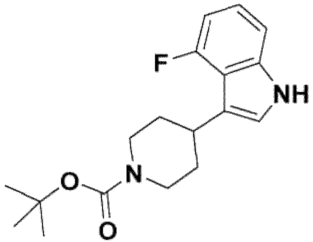

To a mixture of Intermediate RE-11A (500 mg), methanol (53 mL) and ammonium formate (1.30 g) was added 10% Pd-C (21 mg), and then the mixture was stirred at 80°C overnight. The insoluble material was filtered off by Celite^{®} filtration and then the residue was concentrated under reduced pressure to afford the title compound (400 mg). MS (m/z): 219.2 [M-Boc+2H]⁺

### [Step 3] Preparation of Intermediate RE-11C

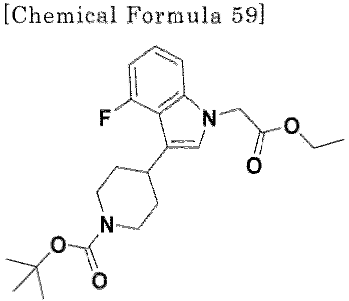

In the method according to Step 4 of Reference Example 1, Intermediate RE-11B (480 mg) was used instead of Intermediate RE-1C to afford the title compound (450 mg). MS (m/z): 305.2 [M-Boc+2H]⁺

### [Step 4] Preparation of Intermediate RE-11D

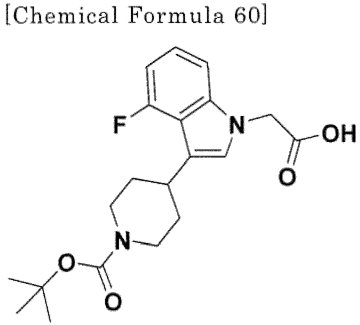

In the method according to Step 5 of Reference Example 1, Intermediate RE-11C (450 mg) was used instead of Intermediate RE-1D to afford the title compound (350 mg). MS (m/z): 375.2 [M-H]⁻

### [Step 5] Preparation of Intermediate RE-11E

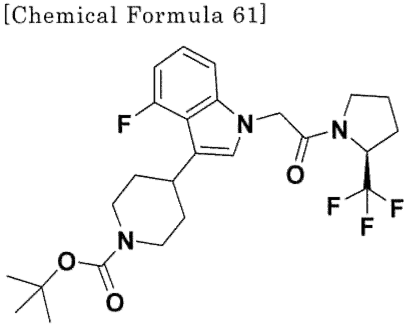

A mixture of Intermediate RE-11D (100 mg), DMF (0.5 mL), DIPEA (0.138 mL) and HATU (121 mg) was stirred at room temperature for 15 minutes. To this mixture was added (2S)-2-(trifluoromethyl)pyrrolidine hydrochloride (56 mg), and then the mixture was stirred at room temperature for 1 hour. The reaction solution was purified by silica gel column chromatography to afford the title compound (120 mg). MS (m/z): 398.2 [M-Boc+2H]⁺

### [Step 6] Preparation of Compound RE-11

In the method according to Step 7 of Reference Example 1, Intermediate RE-11E (120 mg) was used instead of Intermediate RE-1F to afford the title compound (85 mg). MS (m/z): 398.2 [M+H]⁺

### Reference Example 12 tert-butyl 3-(1-{2-oxo-2-[(2S)-2-(trifluoromethyl)pyrrolidin-1-yl]ethyl}-1H-indol-3-yl)azetidine-1-carboxylate (Compound RE-12)

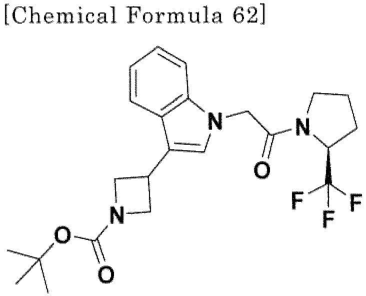

### [Step 1] Preparation of Intermediate RE-12A

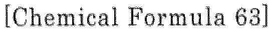

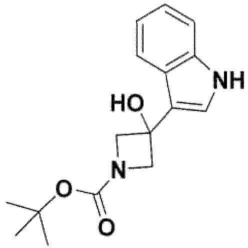

To a mixture of indole (1 g), potassium hydroxide (527 mg) and methanol (11 mL) was added tert-butyl 3-oxoazetidine-1-carboxylate (1.61 g), and then the mixture was stirred at 50°C overnight. To the reaction solution was added water, and then the mixture was extracted with ethyl acetate and dried over anhydrous sodium sulfate. The solvent was removed under reduced pressure to afford the title compound (920 mg). MS (m/z): 287.2 [M-H]⁻

### [Step 2] Preparation of Intermediate RE-12B

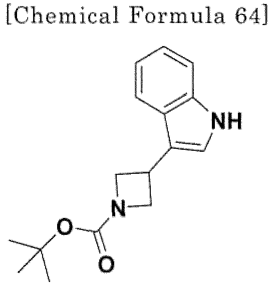

To a mixture of Intermediate RE-12A (920 mg) and dichloromethane (6.4 mL) was added triethylsilane (3.82 mL) under an ice bath condition, then trifluoroacetic acid (0.811 mL) was added slowly. After that, the mixture was stirred under an ice bath condition for 10 minutes. A saturated aqueous solution of sodium bicarbonate was added to the reaction solution, and then the mixture was extracted with dichloromethane and dried over anhydrous sodium sulfate. The solvent was removed from the mixture under reduced pressure, and then the mixture was purified by silica gel column chromatography to afford the title compound (370 mg). MS (m/z): 271.4 [M-H]⁻

### [Step 3] Preparation of Intermediate RE-12C

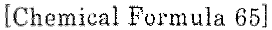

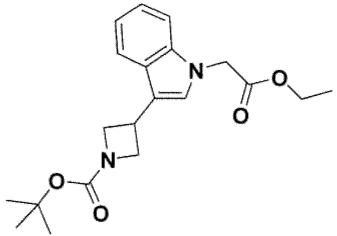

In the method according to Step 4 of Reference Example 1, Intermediate RE-12B (370 mg) was used instead of Intermediate RE-1C to afford the title compound (400 mg). MS (m/z): 259.1 [M-Boc+2H]⁺

### [Step 4] Preparation of Intermediate RE-12D

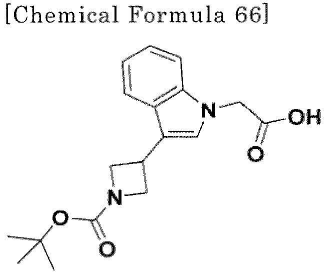

To a mixture of Intermediate RE-12C (400 mg), methanol (10 mL) and THF (10 mL) was added 2 M aqueous sodium hydroxide solution (2.79 mL) at room temperature, and then the mixture was stirred for 2 hours. The reaction solution was concentrated under reduced pressure, diluted with water, and acidified with 2 M hydrochloric acid under an ice bath condition. The solvent was removed from the mixture under reduced pressure, and then the mixture was purified by silica gel column chromatography to afford the title compound (350 mg). MS (m/z): 329.1 [M-H]⁻

### [Step 5] Preparation of Compound RE-12

In the method according to Step 5 of Reference Example 11, Intermediate RE-12D (50 mg) was used instead of Intermediate RE-11D to afford the title compound (50 mg). MS (m/z): 352.1 [M-Boc+2H]⁺

### Reference Example 13 tert-butyl 3-(5-methyl-1-{2-oxo-2-[(2S)-2-(trifluoromethyl)pyrrolidin-1-yl]ethyl}-1H-pyrazolo[4.3-b]pyridin-3-yl)azetidine-1-carboxylate (Compound RE-13)

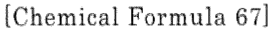

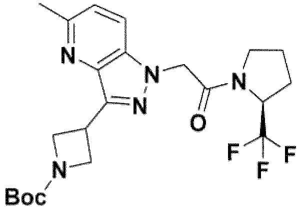

### [Step 1] Preparation of Intermediate RE-13A

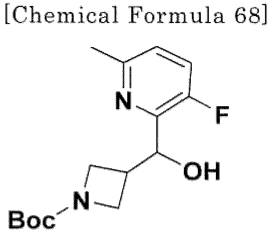

In the method according to Step 1 of Reference Example 5, 2-bromo-3-fluoro-6-methylpyridine (1.00 g) and tert-butyl 3-formylazetidine-1-carboxylate (1.17 g) were used instead of 2-bromo-4-chloro-1-fluorobenzene and tert-butyl 3-[methoxy(methyl)carbamoyllazetidine-1-carboxylate, respectively, to afford the title compound (608 mg). MS (m/z): 297.1 [M+H]⁺

### [Step 2] Preparation of Intermediate RE-13B

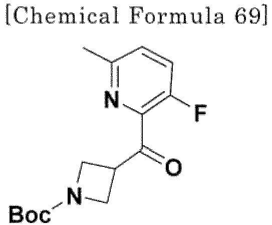

To a mixture of Intermediate RE-13A (608 mg) and dichloromethane (10 mL) were added iodobenzene diacetate (793 mg) and AZADOL^{®} (16 mg) sequentially, and then the mixture was stirred at room temperature for 2 hours. AZADOL^{®} (16 mg) was added thereto, and then the mixture was further stirred at room temperature for 2 hours. A saturated aqueous solution of sodium bicarbonate was added to the reaction solution, and the mixture was extracted with dichloromethane. The organic layer was washed with a saturated saline solution and dried over anhydrous magnesium sulfate. The solvent was removed under reduced pressure, and then the residue was purified by silica gel column chromatography to afford the title compound (542 mg). MS (m/z): 295.1 [M+H]⁺

### [Step 3] Preparation of Intermediate RE-13C

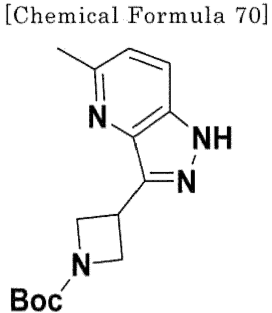

In the method according to Step 2 of Reference Example 5, Intermediate RE-13B (542 mg) was used instead of Intermediate RE-5A to afford the title compound (363 mg). MS (m/z): 289.2 [M+H]⁺

### [Step 4] Preparation of Intermediate RE-13D

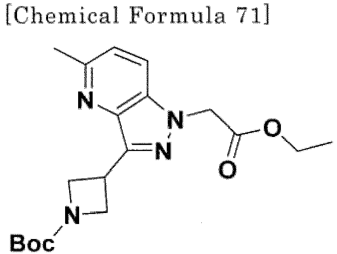

In the method according to Step 4 of Reference Example 1, Intermediate RE-13C (290 mg) was used instead of Intermediate RE-1C to afford the title compound (359 mg). MS (m/z): 375.1 [M+H]⁺

### [Step 5] Preparation of Intermediate RE-13E

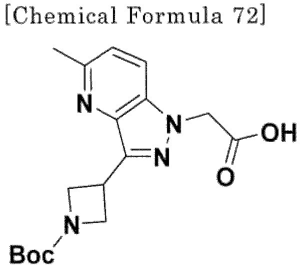

In the method according to Step 5 of Reference Example 1, Intermediate RE-13D (359 mg) was used instead of Intermediate RE-1D to afford the title compound (187 mg). MS (m/z): 347.1 [M+H]⁺

### [Step 6] Preparation of Compound RE-13

In the method according to Step 5 of Reference Example 11, Intermediate RE-13E (90 mg) was used instead of Intermediate RE-11D to afford the title compound (100 mg). MS (m/z): 468.3 [M+H]⁺

### Reference Example 14 2-[3-(azetidin-3-yl)-1H-pyrazolo[4,3-c]pyridin-1-yl]-1-[(2S)-2-(trifluoromethyl)pyrrolidin-1-yl]ethan-1-one di-trifluoroacetate (Compound RE-14)

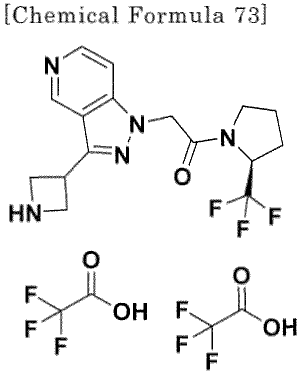

### [Step 1] Preparation of Intermediate RE-14A

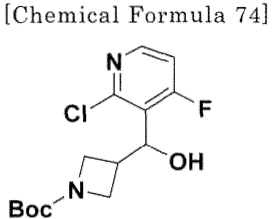

In the method according to Step 1 of Reference Example 1, 2-chloro-4 fluoropyridine (1.07 g) was used instead of 2-chloro-1,4-difluorobenzene to afford the title compound (1.62 g).

### [Step 2] Preparation of Intermediate RE-14B

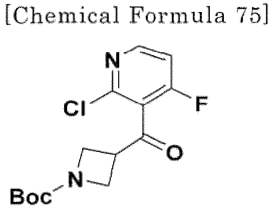

In the method according to Step 2 of Reference Example 1, Intermediate RE-14A (1.62 g) was used instead of Intermediate RE-1A to afford the title compound (1.47 g).

### [Step 3] Preparation of Intermediate RE-14C

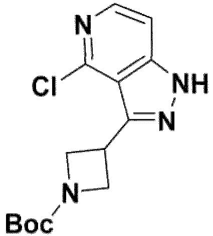

A mixture of Intermediate RE-14B (1.37 g), 1,4-dioxane (28 mL) and hydrazine monohydrate (0.65 mL) was stirred at 100°C overnight. To the reaction solution was added water, and the mixture was extracted with ethyl acetate. The organic layer was washed with a saturated saline solution and dried with anhydrous magnesium sulfate. The solvent was removed under reduced pressure, and then the residue was purified by silica gel column chromatography to afford the title compound (0.79 g). MS (m/z): 309.1 [M+H]⁺

### [Step 4] Preparation of Intermediate RE-14D

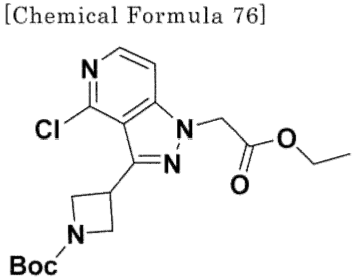

In the method according to Step 4 of Reference Example 1, Intermediate RE-14C (0.79 g) was used instead of Intermediate RE-1C to afford the title compound (1.04 g). 395.2 [M+H]⁺

### [Step 5] Preparation of Intermediate RE-14E

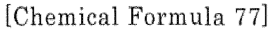

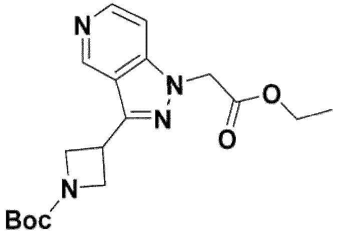

To a mixture of Intermediate RE-14D (300 mg), ethyl acetate (7.6 mL) and triethylamine (0.21 mL) was added 5% Pd-C (100 mg), and then the mixture was stirred under hydrogen atmosphere (0.1 MPa) at room temperature overnight. The insoluble material was filtered off by Celite^{®} filtration, and then the filtrate was concentrated under reduced pressure and purified by silica gel column chromatography to afford the title compound (249 mg). MS (m/z): 361.2 [M+H]⁺

### [Step 6] Preparation of Intermediate RE-14F

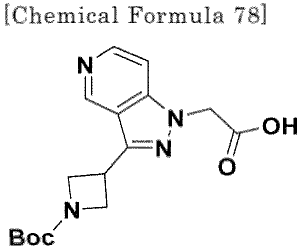

In the method according to Step 5 of Reference Example 1, Intermediate RE-14E (249 mg) was used instead of Intermediate RE-1D to afford the title compound (210 mg). MS (m/z): 333.2 [M+H]⁺

### [Step 7] Preparation of Intermediate RE-14G

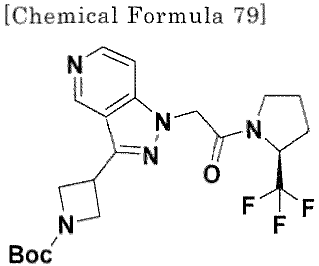

In the method according to Step 5 of Reference Example 11, Intermediate RE-14F (100 mg) was used instead of Intermediate RE-11D to afford the title compound (92 mg). MS (m/z): 454.3 [M+H]⁺

### [Step 8] Preparation of Compound RE-14

In the method according to Reference Example 3, Intermediate RE-14G (92 mg) was used instead of Intermediate RE-2F to afford a brown oil, 39 mg of which was purified by silica gel column chromatography to afford the title compound (30 mg). MS (m/z): 354.1 [M+H]⁺

### Reference Example 15 3-{[3-(azetidin-3-yl)-4-fluoro-1H-pyrazolo[3,4-c]pyridin-1-yl]methyl}-5-[1-(trifluoromethyl)cyclopropyl]-1,2,4-oxadiazole trifluoroacetate (Compound RE-15)

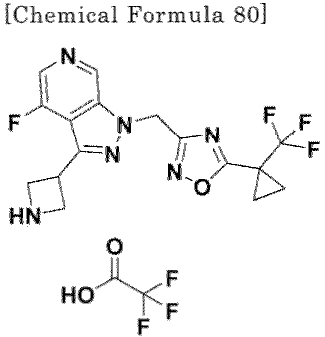

### [Step 1] Preparation of Intermediate RE-15A

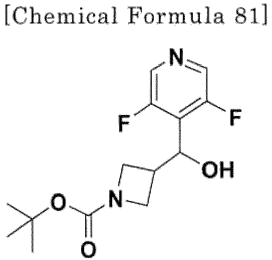

In the method according to Step 1 of Reference Example 1, 3,5-difluoropyridine (2.80 g) was used instead of 2-chloro-1,4-difluorobenzene to afford the title compound (4.92 g).

### [Step 2] Preparation of Intermediate RE-15B

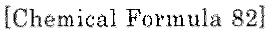

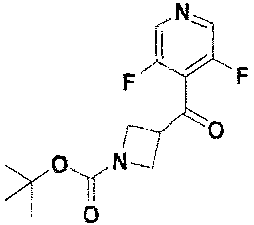

In the method according to Step 2 of Reference Example 1, Intermediate RE-15A (4.86 g) was used instead of Intermediate RE-1A to afford the title compound (4.64 g).

### [Step 3] Preparation of Intermediate RE-15C

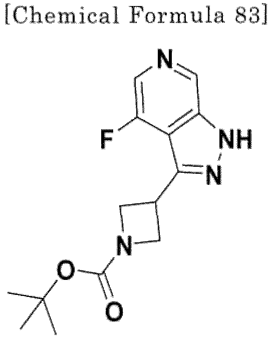

A mixture of Intermediate RE-15B (4.64 g), 1,4-dioxane (52 mL) and hydrazine monohydrate (1.16 mL) was stirred at 100°C overnight. To the reaction solution was added water, and the mixture was extracted with ethyl acetate. The organic layer was washed with a saturated saline solution and dried with anhydrous magnesium sulfate. The solvent was removed under reduced pressure, and then the residue was purified by silica gel column chromatography to afford the title compound (3.9 g). MS (m/z): 291.2 [M-H]⁻

### [Step 4] Preparation of Intermediate RE-15D

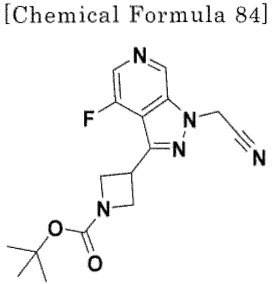

In the method according to Step 4 of Reference Example 10, Intermediate RE-15C (1.0 g) and 2-chloroacetonitrile (0.323 mL) were used instead of Intermediate RE-10C and 2-(bromomethyl)-5-(trifluoromethyl)pyridine, respectively, to afford the title compound (1.22 g).

### [Step 5] Preparation of Intermediate RE-15E

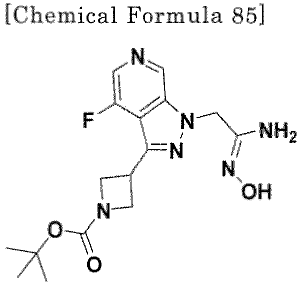

To a solution of potassium carbonate (0.95 g) in water (3.1 mL) was added a solution of hydroxylamine hydrochloride (0.48 g) and Intermediate RE-15D (1.22 g) in ethanol (11.4 mL), and then the mixture was stirred at room temperature for 3 hours. Then, the solvent was removed under reduced pressure, and ethyl acetate was added to the residue to afford a suspension, of which the insoluble material was filtered off by Celite^{®} filtration. The filtrate was concentrated under reduced pressure, and then the residue was purified by silica gel column chromatography to afford the title compound (0.72 g). MS (m/z): 365.1 [M+H]⁺

### [Step 6] Preparation of Intermediate RE-15F

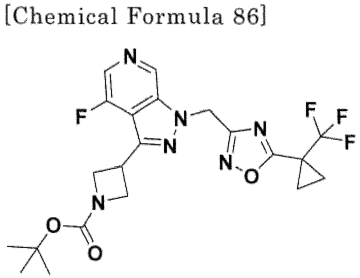

To a mixture of Intermediate RE-15E (39 mg), 1-(trifluoromethyl)cyclopropane-1-carboxylic acid (15 mg), DIPEA (0.019 mL) and DMF (0.3 mL) was added HATU (41 mg), and then the mixture was stirred at room temperature overnight. The mixture was then stirred at 120°C for 2 hours. After cooling, the reaction solution was purified by silica gel column chromatography to afford the title compound (27 mg). MS (m/z): 483.2 [M+H]⁺

### [Step 7] Preparation of Compound RE-15

In the method according to Reference Example 3, Intermediate RE-15F (27 mg) was used instead of Intermediate RE-2F to afford the title compound (28 mg). MS (m/z): 383.1 [M+H]⁺

### Reference Example 16 2-[3-(azetidin-3-yl)-4-methyl-1H-pyrazolo[3,4-b]pyridin-1-yl]-1-[(2S)-2-(trifluoromethyl)pyrrolidin-1-yl]ethan-1-one di-trifluoroacetate (Compound RE-16)

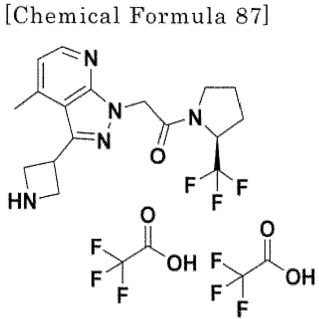

### [Step 1] Preparation of Intermediate RE-16A

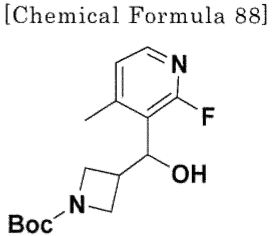

In the method according to Step 1 of Reference Example 5, 3-bromo-2-fluoro-methylpyridine (0.63 g) and tert-butyl 3-formylazetidine-1-carboxylate (0.74 g) were used instead of 2-bromo-4-chloro-1-fluorobenzene and tert-butyl 3-[methoxy(methyl)carbamoyl]azetidine-1-carboxylate to afford the title compound (0.57 g). MS (m/z): 197.1 [M-Boc+2H]⁺

### [Step 2] Preparation of Intermediate RE-16B

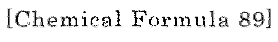

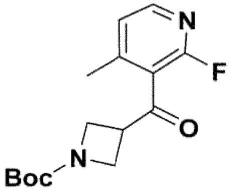

In the method according to Step 2 of Reference Example 1, Intermediate RE-16A (0.57 g) was used instead of Intermediate RE-1A to afford the title compound (241 mg). MS (m/z): 195.0 [M-Boc+2H]⁺

### [Step 3] Preparation of Intermediate RE-16C

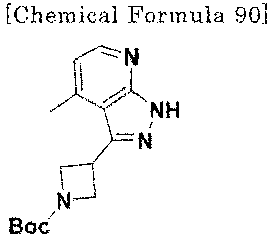

In the method according to Step 3 of Reference Example 1, Intermediate RE-16B (241 mg) was used instead of Intermediate RE-1B to afford the title compound (148 mg). MS (m/z): 289.1 [M+H]⁺

### [Step 4] Preparation of Intermediate RE-16D

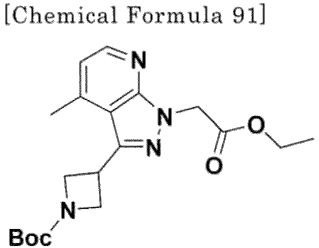

In the method according to Step 4 of Reference Example 1, Intermediate RE-16C (148 mg) was used instead of Intermediate RE-1C to afford the title compound (166 mg). MS (m/z): 375.1 [M+H]⁺

### [Step 5] Preparation of Intermediate RE-16E

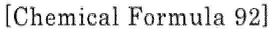

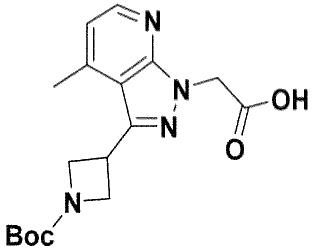

In the method according to Step 5 of Reference Example 1, Intermediate RE-16D (166 mg) was used instead of Intermediate RE-1D to afford the title compound (139 mg). MS (m/z): 347.1 [M+H]⁺

### [Step 6] Preparation of Intermediate RE-16F

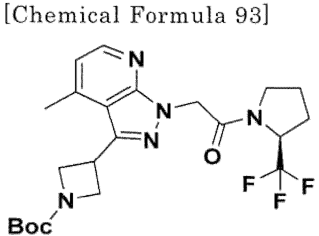

In the method according to Step 5 of Reference Example 11, Intermediate RE-16E (70 mg) was used instead of Intermediate RE-11D to afford the title compound (88 mg). MS (m/z): 412.1 [M-(tBu+2H)]⁺

### [Step 7] Preparation of Compound RE-16

In the method according to Reference Example 3, Intermediate RE-16F (88 mg) was used instead of Intermediate RE-2F to afford the title compound (130 mg). MS (m/z): 368.0 [M+H]⁺

### Reference Example 17 3-(azetidin-3-yl)-1-{[3-(trifluoromethyl)-1,2,4-oxadiazol-5-yl]methyl)-1H-indazole trifluoroacetate (Compound RE-17)

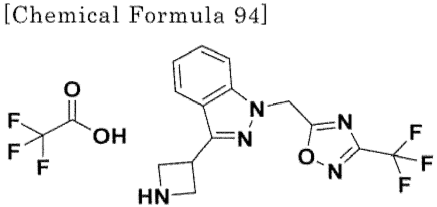

### [Step 1] Preparation of Intermediate RE-17A

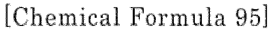

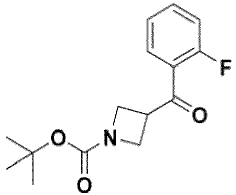

To a mixture of 1-fluoro-2-iodobenzene (8.31 g) and THF (62 mL) was added dropwise n-butyl lithium (1.57 M n-hexane solution, 23.8 mL) at -78°C. After stirring at -78°C for 1 hour, to the mixture was added dropwise a solution of tert-butyl 3-formylazetidine-1-carboxylate (4.6 g) in THF (62 mL), and then the mixture was stirred for 1 hour. A saturated aqueous solution of ammonium chloride was added to the reaction solution, and the mixture was extracted with ethyl acetate. The organic layer was washed with a saturated saline solution and dried over anhydrous magnesium sulfate. The solvent was removed under reduced pressure to afford tert-butyl 3-[(2-fluorophenyl)(hydroxy)methyl]azetidine-1-carboxylate.

To a mixture of the above tert-butyl 3-[(2-fluorophenyl)(hydroxy)methyl]azetidine-1-carboxylate and dichloromethane (124 mL) were added iodobenzene diacetate (9.6 g) and AZADOL^{®} (190 mg) sequentially, and then the mixture was stirred at room temperature for 2 hours. Iodobenzene diacetate (4.85 g) and AZADOL^{®} (190 mg) were added, and then the mixture was stirred at room temperature for 30 minutes. A saturated aqueous solution of sodium bicarbonate was added to the reaction solution, and the mixture was extracted with dichloromethane. The organic layer was washed with a saturated saline solution and dried over anhydrous magnesium sulfate. The solvent was removed under reduced pressure, and then the residue was purified by silica gel column chromatography to afford the title compound (5.29 g). MS (m/z): 280.1 [M+H]⁺

### [Step 2] Preparation of Intermediate RE-17B

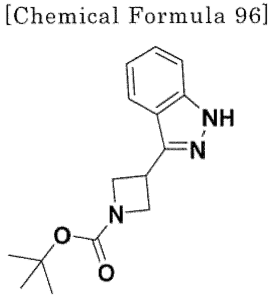

A mixture of Intermediate RE-17A (5.15 g), NMP (50 mL) and hydrazine monohydrate (8.99 mL) was stirred at 150°C for 2 days. After cooling, water was added to the reaction solution, and the mixture was extracted with ethyl acetate/n-hexane (1/1). The organic layer was washed with a saturated saline solution and dried with anhydrous magnesium sulfate. The solvent was removed under reduced pressure, and then the residue was purified by silica gel column chromatography to afford the title compound (4.39 g). MS (m/z): 274.1 [M+H]⁺

### [Step 3] Preparation of Intermediate RE-17C

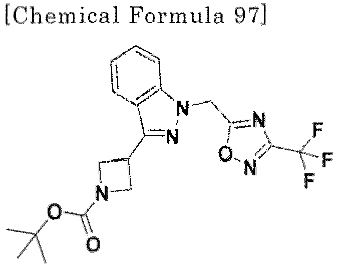

To a mixture of Intermediate RE-17B (200 mg) and acetonitrile (2.4 mL) were added cesium carbonate (358 mg) and 5-(chloromethyl)-3-(trifluoromethyl)-1,2,4-oxadiazole (205 mg) sequentially, and then the mixture was stirred at room temperature for 4 hours, then stirred at 60°C overnight. 5-(Chloromethyl)-3-(trifluoromethyl)-1,2,4-oxadiazole (137 mg) was added, and then the mixture was stirred at room temperature for 72 hours. The reaction solution was purified by silica gel column chromatography to afford the title compound (94 mg). MS (m/z): 422.2 [M-H]⁻

### [Step 4] Preparation of Compound RE-17

In the method according to Reference Example 3, Intermediate RE-17C (94 mg) was used instead of Intermediate RE-2F to afford the title compound (98 mg). MS (m/z): 324.0 [M+H]⁺

### Reference Example 18 tert-butyl 3-[1-({1-[(3,3-difluorocyclobutyl)methyl]-1H-1,2,3-triazol-4-yl}methyl)-4-fluoro-1H-indazol-3-yl]azetidine-1-carboxylate (Compound RE-18)

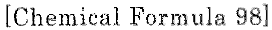

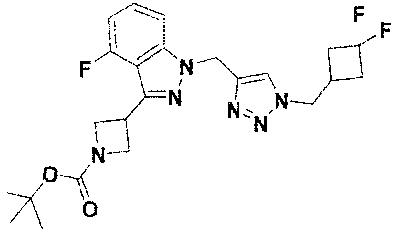

### [Step 1] Preparation of Intermediate RE-18A

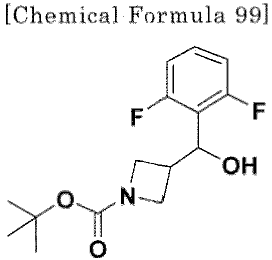

In the method according to Step 1 of Reference Example 1, 1,3-difluorobenzene (1.85 g) was used instead of 2-chloro-1,4-difluorobenzene to afford the title compound (2.72 g).

### [Step 2] Preparation of Intermediate RE-18B

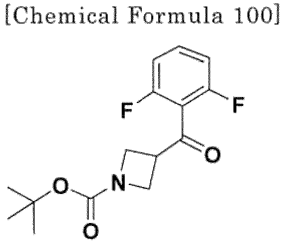

In the method according to Step 2 of Reference Example 1, Intermediate RE-18A (2.72 g) was used instead of Intermediate RE-1A to afford the title compound (2.39 g). MS (m/z): 298.0 [M+H]⁺

### [Step 3] Preparation of Intermediate RE-18C

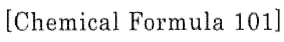

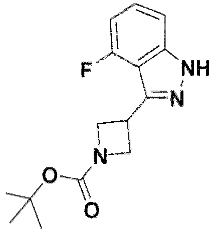

In the method according to Step 3 of Reference Example 1, Intermediate RE-18B (2.39 g) was used instead of Intermediate RE-1B to afford the title compound (2.15 g). MS (m/z): 290.3 [M-H]⁻

### [Step 4] Preparation of Intermediate RE-18D

To a mixture of Intermediate RE-18C (1.02 g) and DMF (12 mL) were added potassium carbonate (726 mg) and propargylic bromide (0.332 mL) sequentially, and the mixture was stirred at room temperature for 2 hours. A saturated aqueous solution of ammonium chloride was added to the reaction solution, and the mixture was extracted with ethyl acetate. The organic layer was washed with a saturated saline solution and dried over anhydrous magnesium sulfate. The solvent was removed under reduced pressure, and then the residue was purified by silica gel column chromatography to afford the title compound (1.01 g). MS (m/z): 230.1 [M-Boc+2H]⁺

### [Step 5] Preparation of Compound RE-18

To a mixture of (3,3-difluorocyclobutyl)methyl 4-methylbenzenesulfonate (for example, synthesized according to a method described in WO2014205234) (300 mg) and DMF (3 mL) was added sodium azide (84.7 mg), and then the mixture was stirred at 120°C overnight to afford a DMF solution of 3-(azidomethyl)-1,1-difluorocyclobutane (about 0.36 mol/L). After cooling, to a mixture of the prepared 3-(azidomethyl)-1,1-difluorocyclobutane (DMF solution, about 0.36 mol/L, 0.839 mL), Intermediate RE-18D (50 mg), DMF (2 mL) and methanol (0.2 mL) was added copper(I) iodide (2.9 mg), and then the mixture was stirred at 60°C for 3 hours. The reaction solution was diluted with ethyl acetate, washed with a saturated saline solution, and dried over anhydrous sodium sulfate. The solvent was removed under reduced pressure, and then the residue was purified by silica gel column chromatography to afford the title compound (56 mg).

### Reference Example 19 3-(azetidin-3-yl)-4-fluoro-1-{[5-(4-fluorophenyl)-1,3,4-oxadiazol-2-yl]methyl}-1H-indazole trifluoroacetate (Compound RE-19)

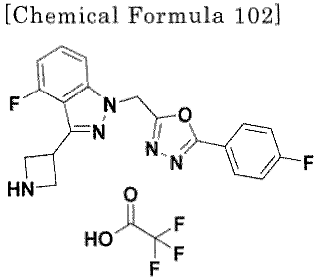

### [Step 1] Preparation of Intermediate RE-19A

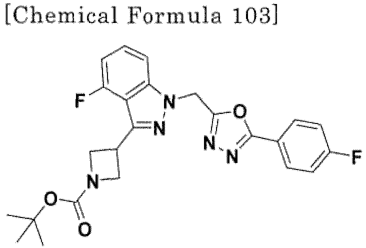

In the method according to Step 4 of Reference Example 10, Intermediate RE-18C (40 mg) and 2-(chloromethyl)-5-(4-fluorophenyl)-1,3,4-oxadiazole (35 mg) were used instead of Intermediate RE-10C and 2-(bromomethyl)-5-(trifluoromethyl)pyridine, respectively, to afford the title compound (65 mg). MS (m/z): 368.1 [M-Boc+2H]⁺

### [Step 2] Preparation of Compound RE-19

In the method according to Reference Example 3, Intermediate RE-19A (65 mg) was used instead of Intermediate RE-2F to afford the title compound (67 mg). MS (m/z): 368.1 [M+H]⁺

### Reference Example 20 2-[3-(azetidin-3-yl)-4-fluoro-1H-indazol-1-yl]-N-methyl-N-(2,2,2-trifluoroethyl)acetamide trifluoroacetate (Compound RE-20)

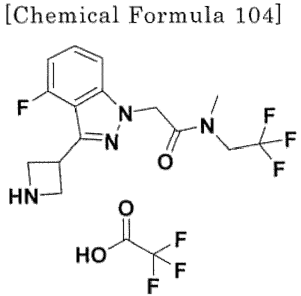

### [Step 1] Preparation of Intermediate RE-20A

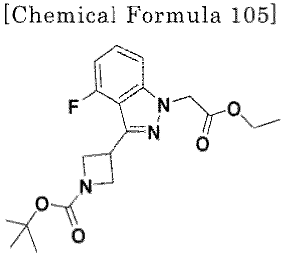

In the method according to Step 4 of Reference Example 1, Intermediate RE-18C (500 mg) was used instead of Intermediate RE-1C to afford the title compound (638 mg). MS (m/z): 278.0 [M-Boc+2H]⁺

### [Step 2] Preparation of Intermediate RE-20B

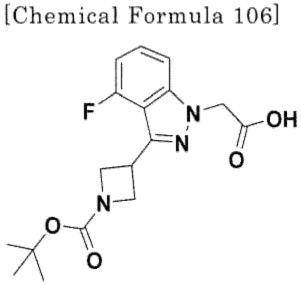

In the method according to Step 5 of Reference Example 1, Intermediate RE-20A (638 mg) was used instead of Intermediate RE-1D to afford the title compound (476 mg). MS (m/z): 348.4 [M-H]⁻

### [Step 3] Preparation of Intermediate RE-20C

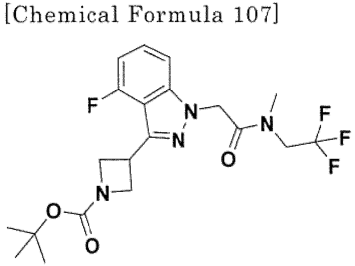

In the method according to Step 5 of Reference Example 5, Intermediate RE-20B (64 mg) was used instead of Intermediate RE-5D to afford the title compound (81 mg). MS (m/z): 468.3 [M+Na]⁺

### [Step 4] Preparation of Compound RE-20

In the method according to Reference Example 3, Intermediate RE-20C (81 mg) was used instead of Intermediate RE-2F to afford the title compound (84 mg). MS (m/z): 345.2 [M+H]⁺

### Reference Example 21 2-[3-(azetidin-3-yl)-4,5,6,7-tetrahydro-1H-indazol-1-yl]-1-[(2S)-2-(trifluoromethyl)pyrrolidin-1-yl]ethan-1-one trifluoroacetate (Compound RE-21)

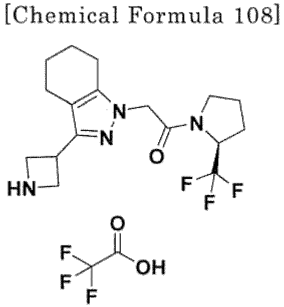

### [Step 1] Preparation of Intermediate RE-21A

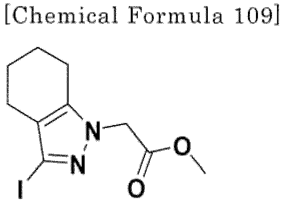

In the method according to Step 1 of Reference Example 4, 3-iodo-4,5,6,7-tetrahydro-1H-indazole (for example, synthesized according to a method described in US2014171432) (1.63 g) instead of 3-iodo-2H-indazole to afford a mixture (2.14 g) of the title compound and about 10% positional isomer. MS (m/z): 321.1 [M+H]⁺

### [Step 2] Preparation of Intermediate RE-21B

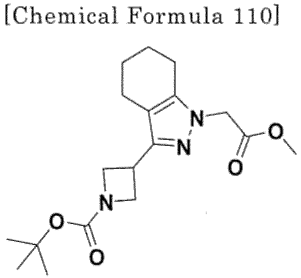

In the method according to Step 2 of Reference Example 4. Intermediate RE-21A (2.14 g) obtained in Step 1 of Reference Example 21 was used instead of Intermediate RE-4A to afford the title compound (0.39 g). MS (m/z): 372.3 [M+Na]⁺

### [Step 3] Preparation of Intermediate RE-21C

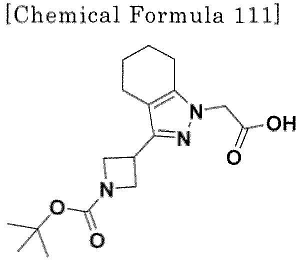

In the method according to Step 5 of Reference Example 1, Intermediate RE-21B (0.39 g) was used instead of Intermediate RE-1D to afford the title compound (34 mg). MS (m/z): 334.2 [M-H]⁻

### [Step 4] Preparation of Intermediate RE-21D

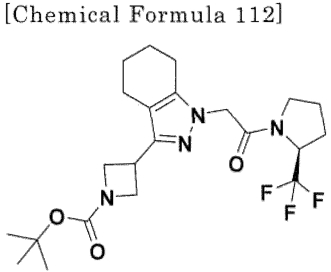

In the method according to Step 5 of Reference Example 11, Intermediate RE-21C (34 mg) was used instead of Intermediate RE-11D to afford the title compound (16 mg). MS (m/z): 457.3 [M+H]⁺

### [Step 5] Preparation of Compound RE-21

In the method according to Reference Example 3, Intermediate RE-21D (16 mg) was used instead of Intermediate RE-2F to afford the title compound (16 mg). MS (m/z): 357.2 [M+H]⁺

### Reference Example 22 2-[3-(azetidin-3-yl)-5,5-difluoro-4,5,6,7-tetrahydro-1H-indazol-1-yl]-1-[(2S)-2-(trifluoromethyl)pyrrolidin-1-yl]ethan-1-one trifluoroacetate (Compound RE-22)

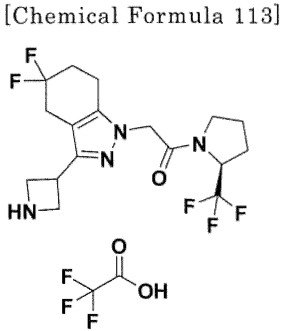

### [Step 1] Preparation of Intermediate RE-22A

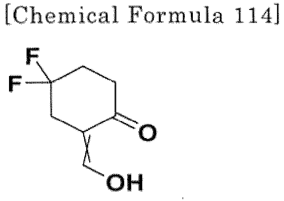

A reaction vessel containing diethyl ether (30 mL) was cooled on ice, and 60% sodium hydride (596 mg) was added thereto. Ethanol (0.087 mL) was then added thereto, and then the mixture was stirred for 20 minutes under an ice bath condition. A solution of 4,4-difluorocyclohexan-1-one (2.0 g) and ethyl formate (1.80 mL) in diethyl ether (10 mL) was added dropwise through a dropping funnel over 10 minutes. The mixture was gradually brought to room temperature and stirred overnight. Then ethanol (0.3 mL) was added to the reaction solution, and then the mixture was stirred at room temperature for 1 hour. To the reaction solution was added water, and then the mixture was washed with diethyl ether. The aqueous layer was acidified with 2 M HCl and extracted with diethyl ether. The organic layer was washed with a saturated saline solution and dried with anhydrous magnesium sulfate. The solvent was removed under reduced pressure to afford the title compound (1.35 g). MS (m/z): 163.1 [M+H]⁺

### [Step 2] Preparation of Intermediate RE-22B

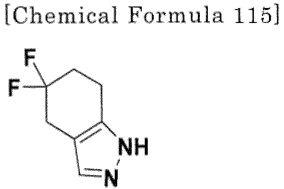

To a mixture of Intermediate RE-22A (1.35 g) and methanol (8.3 mL) was added hydrazine monohydrate (0.487 mL) dropwise, and then the mixture was stirred at room temperature overnight. The solvent was removed under reduced pressure, and then the residue was purified by silica gel column chromatography to afford the title compound (0.65 g). MS (m/z): 159.1 [M+H]⁺

### [Step 3] Preparation of Intermediate RE-22C

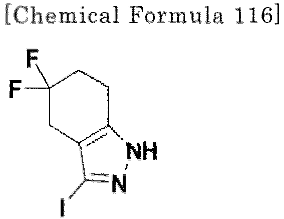

To a mixture of Intermediate RE-22B (0.65 g) and DMF (8.2 mL) were added potassium hydroxide (0.92 g) and iodine (2.1 g) sequentially under an ice bath condition, and then the mixture was stirred at room temperature overnight. A saturated aqueous solution of sodium thiosulfate was added to the reaction solution, and the mixture was extracted with ethyl acetate. The organic layer was washed with a saturated saline solution and dried over anhydrous magnesium sulfate. The solvent was removed under reduced pressure, and then the residue was purified by silica gel column chromatography to afford the title compound (468 mg). MS (m/z): 284.8 [M+H]⁺

### [Step 4] Preparation of Intermediate RE-22D

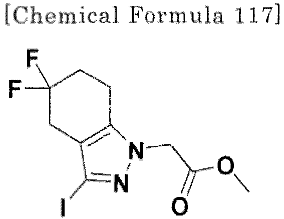

In the method according to Step 1 of Reference Example 4, Intermediate RE-22C (468 mg) was used instead of 3-iodo-2H-indazole to afford a mixture (582 mg) of the title compound and about 24% positional isomer. MS (m/z): 356.8 [M+H]⁺

### [Step 5] Preparation of Intermediate RE-22E

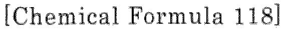

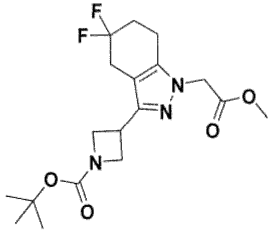

In the method according to Step 2 of Reference Example 4, Intermediate RE-22D (582 mg) was used instead of Intermediate RE-4A to afford the title compound (360 mg). MS (m/z): 408.3 [M+Na]⁺

### [Step 6] Preparation of Intermediate RE-22F

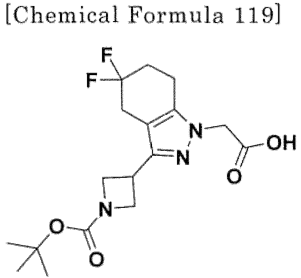

In the method according to Step 5 of Reference Example 1, Intermediate RE-22E (360 mg) was used instead of Intermediate RE-1D to afford the title compound (103 mg). MS (m/z): 370.2 [M-H]⁻

### [Step 7] Preparation of Intermediate RE-22G

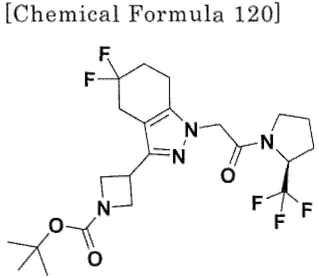

In the method according to Step 5 of Reference Example 11, Intermediate RE-22F (50 mg) was used instead of Intermediate RE-11D to afford the title compound (60 mg). MS (m/z): 515.3 [M+Na]⁺

### [Step 8] Preparation of Compound RE-22

In the method according to Reference Example 3, Intermediate RE-22G (60 mg) was used instead of Intermediate RE-2F to afford the title compound (62 mg). MS (m/z): 393.3 [M+H]⁺

### Reference Example 23 2-(cyclopropylamino)pyridine-4-carboxylic acid (Compound RE-23)

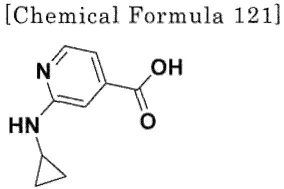

### [Step 1] Preparation of Intermediate RE-23A

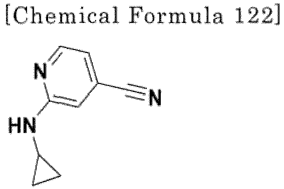

To a mixture of 2-fluoropyridine-4-carbonitrile (500 mg), NMP (4.0 mL) and DIPEA (2.1 mL) was added cyclopropaneamine (0.57 mL), and then the mixture was stirred at 80°C overnight. To the reaction solution was added water, and the mixture was extracted with ethyl acetate/n-hexane (111). The organic layer was washed with a saturated saline solution and dried over anhydrous magnesium sulfate. The solvent was removed under reduced pressure, and then the residue was purified by silica gel column chromatography to afford the title compound (550 mg).

### [Step 2] Preparation of Compound RE-23

To a mixture of Intermediate RE-23A (550 mg), ethanol (2 mL) and water (2 mL) was added potassium hydroxide (970 mg), and then the mixture was stirred at 80°C overnight. The solvent was removed under reduced pressure, and 2 M hydrochloric acid was added to the residue to pH 7 to 8. The precipitate was obtained by filtration to afford the title compound (313 mg). MS (m/z): 179.1 [M+H]⁺

### Reference Example 24 2-amino-5-fluoropyridine-4-carboxylic acid hydrochloride (Compound RE-24)

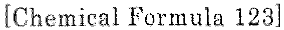

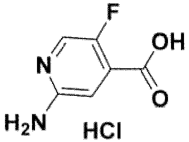

A mixture of tert-butyl 2-bromo-5-fluoropyridine-4-carboxylate (for example, synthesized according to a method described in WO202186879) (2.00 g), tert-butyl carbonate (1.02 g), Xantphos (168 mg), cesium carbonate (3.30 g) and 1,4-dioxane (24 mL) was degassed with argon. To the mixture was added Pd(OAc)₂ (33 mg), and then the mixture was stirred at 90°C overnight. The insoluble material was filtered off with Celite^{®}, and the mother liquor was washed sequentially with water and a saturated saline solution, then dried over anhydrous magnesium sulfate. The solvent was removed under reduced pressure to afford tert-butyl 2-{[(tert-butoxy)carbonyl]amino}-5-fluoropyridine-4-carboxylate.

Then, a mixture of the resulting tert-butyl 2-{[(tert-butoxy)carbonyl)amino}-5-fluoropyridine-4-carboxylate and hydrogen chloride (4 M 1,4-dioxane solution, 9 mL) was stirred at 70°C for 6 hours. After cooling, the precipitate formed was obtained by filtration to afford the title compound (1.33 g). MS (m/z): 157.0 [M+H]⁺

### Reference Example 25 2-amino-3-methylpyridine-4-carboxylic acid trifluoroacetate (Compound RE-25)

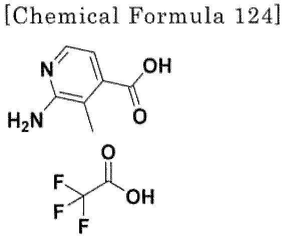

### [Step 1] Preparation of Intermediate RE-25A

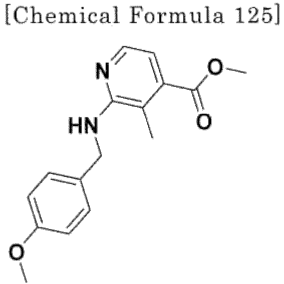

A mixture of methyl 2-chloro-3-methylpyridine-4-carboxylate (2.50g), (4-methoxyphenyl)methaneamine (2.63 mL), rac-BINAP (839 mg), cesium carbonate (13.2 g) and 1,4-dioxane (45 mL) was degassed with argon. To the mixture was added Pd(OAc)₂ (151 mg), and then the mixture was stirred at 100°C overnight. The insoluble material was filtered off with Celite^{®}, and the mother liquor was washed with a saturated saline solution and dried with anhydrous magnesium sulfate. The solvent was removed under reduced pressure, and then the residue was purified by silica gel column chromatography to afford the title compound (4.07 g). MS (m/z): 287.1 [M+H]⁺

### [Step 2] Preparation of Intermediate RE-25B

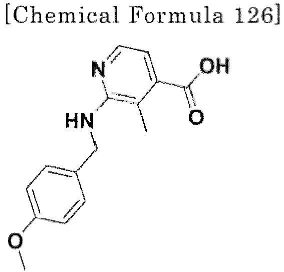

In the method according to Step 5 of Reference Example 1, Intermediate RE-25A (4.07 g) was used instead of Intermediate RE-1D to afford the title compound (4.02 g). MS (m/z): 273.0 [M+H]⁺

### [Step 3] Preparation of Compound RE-25

A mixture of Intermediate RE-25B (1.64 g) and trifluoroacetic acid (20 mL) was stirred at 70°C overnight. Then, the mixture was concentrated under reduced pressure, and the residual slurry was washed with ethyl acetate to afford the title compound (1.25 g). MS (m/z): 153.0 [M+H]⁺

### Reference Example 26 2-amino-5-fluoropyrimidine-4-carboxylic acid (Compound RE-26)

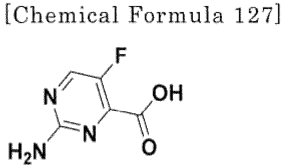

### [Step 1] Preparation of Intermediate RE-26A

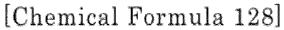

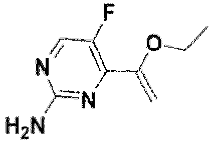

A mixture of 4-chloro-5-fluoropyrimidin-2-amine (1.36 g), tributyl (1-ethoxyvinyl)tin (3.66 g) and DMF (18 mL) was degassed with argon. After that, to the mixture was added PdCl₂(PPh₃)₂ (129 mg), and the mixture was stirred at 90°C overnight. To the reaction solution was added water, and the mixture was extracted with ethyl acetate/n-hexane (1/4). The organic layer was washed with a saturated saline solution and dried over anhydrous magnesium sulfate. The solvent was removed under reduced pressure, and then the residue was purified by silica gel column chromatography to afford the title compound (1.46 g). MS (m/z): 184.2 [M+H]⁺

### [Step 2] Preparation of Intermediate RE-26B

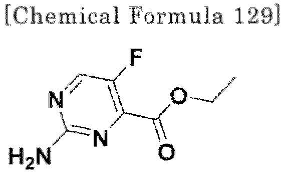

To a mixture of Intermediate RE-26A (1.46 g) and 1,4-dioxane (24 mL) were added a solution of sodium periodate (3.41 g) in water (24 mL) and potassium permanganate (252 mg) at room temperature. After that. potassium permanganate (252 mg) was added thereto three times every 2 hours, and then the mixture was stirred for another 2 hours. To the reaction solution was added a saturated aqueous solution of sodium bicarbonate, and then the mixture was diluted with ethyl acetate. The mixture was filtered through Celite^{®} to remove the insoluble material. The mother liquor was extracted with ethyl acetate. The organic layer was washed with a saturated saline solution and dried with anhydrous magnesium sulfate. The solvent was removed under reduced pressure to afford the title compound (400 mg). MS (m/z): 186.2 [M+H]⁺

### [Step 3] Preparation of Compound RE-26

In the method according to Step 5 of Reference Example 1, Intermediate RE-26B (400 mg) was used instead of Intermediate RE-1D to afford the title compound (140 mg). MS (m/z): 158.1 [M+H]⁺

### Reference Example 27 tert-butyl 6-(cyclopropylamino)pyrimidine-4-carboxylate (Compound RE-27)

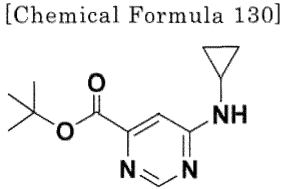

To a mixture of tert-butyl 6-chloropyrimidine-4-carboxylate (for example, synthesized according to a method described in US9598419, 2017, B1) (58 mg), DIPEA (0.01 mL) and ethanol (1 mL) was added cyclopropylamine (77 mg) and then the mixture was reacted in a microwave reactor at 140°C for 1 hour. The solvent was removed under reduced pressure, and then the residue was purified by silica gel column chromatography to afford the title compound (46 mg). ¹H-NMR (400MHz, CDCl₃) 6 8.66 (s, 1H), 7.32 (s, 1H), 5.69 (brs, 1H), 2.62 (s, 1H), 1.63 (s, 9H), 0.92-0.90 (m, 2H), 0.65-0.62 (m, 2H)

### Reference Example 28 2-(1-methylcyclopropylamino)pyridine-4-carboxylic acid (Compound RE-28)

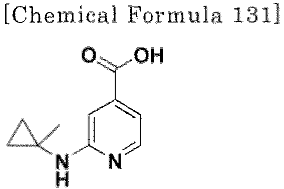

### [Step 1] Preparation of Intermediate RE-28A

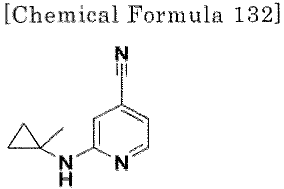

To a mixture of 2-fluoropyridine-4-carbonitrile (200 mg), DIPEA (0.85 mL) and NMP (1.6 mL) was added 1-methylcyclopropane-1-amine hydrochloride (77 mg), and then the mixture was reacted at 80°C overnight. To the reaction solution was added water, and the mixture was extracted with ethyl acetate. The organic layer was washed with a saturated saline solution and dried over anhydrous magnesium sulfate. The solvent was removed under reduced pressure, and then the residue was purified by silica gel column chromatography to afford the title compound (110 mg). ¹H-NMR (400 MHz, CDCl₃) δ 8.18 (d, 1H), 6.89 (s, 1H), 6.78 (dd, 1H), 5.53 (brs, 1H), 1.39 (s, 3H), 0.83-0.75 (m, 4H)

### [Step 2] Preparation of Compound RE-28

To a mixture of Intermediate RE-28A (110 mg), ethanol (2 mL) and water (2 mL) was added potassium hydroxide (0.2 g), and then the mixture was reacted at 80°C overnight. The solvent was removed under reduced pressure, and then the residue was adjusted to pH 7 to 8 with 2 M hydrochloric acid. The resulting precipitate was obtained by filtration, washed with water and dried to afford the title compound (63 mg). ¹H-NMR (400 MHz, DMSO-d6) δ 8.12 (d, 1H), 7.10 (s, 1H), 7.03 (s, 1H), 6.92 (dd, 1H), 1.32 (s, 3H), 0.65 (d, 4H)

### Reference Example 29 2-[(4,4-difluorocyclohexyl)amino]pyridine-4-carboxylic acid (Compound RE-29)

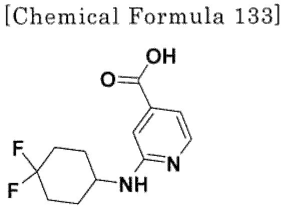

Compound RE-29 was prepared according to the same synthetic route as described for Compound RE-28, using 4,4-difluorocyclohexane-1-amine hydrochloride. ¹H-NMR (400 MHz, DMSO-d6) δ 8.09 (d, 1H), 6.99 (s, 1H), 6.86-6.83 (m, 2H), 3.95 (brs, 1H), 2.06-1.92 (m, 6H), 1.60-1.51 (m, 2H)

### Reference Example 30 2-[3-(azetidin-3-yl)-4-chloro-1H-indazol-1-yl]-N-(2.2.2-trifluoroethyl)acetamide hydrochloride (Compound RE-30)

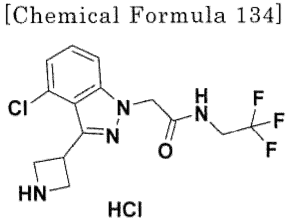

### [Step 1] Preparation of Intermediate RE-30A

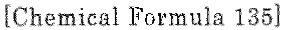

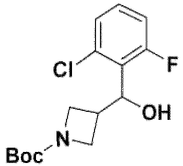

In the method according to Step 1 of Reference Example 1, 1-chloro-3-fluorobenzene was used instead of 2-chloro-1,4-difluorobenzene to afford the title compound. MS(m/z): 316.1 [M+H]⁺

### [Step 2] Preparation of Intermediate RE-30B

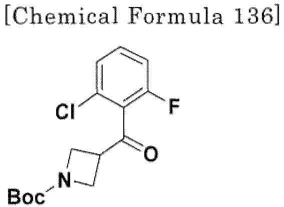

To a mixture of Intermediate RE-30A (600 mg) and dichloromethane (19 mL) were added sodium bicarbonate (479 mg) and 1,1-triacetoxy-1,1-dihydro-1,2-benzoiodoxol-3-(1H)-one (1.21 g) under ice cooling, and the mixture was stirred at room temperature overnight. A saturated aqueous solution of sodium bicarbonate was added to the reaction solution, and the mixture was extracted with ethyl acetate. The organic layer was washed with a saturated saline solution and dried with anhydrous magnesium sulfate. The solvent was removed under reduced pressure, and then the residue was purified by silica gel column chromatography to afford the title compound (557 mg). ¹H-NMR (400 MHz, CDCl₃) δ 7.39-7.33 (m, 1H), 7.24 (d, 1H), 7.07 (t, 1H), 4.23-4.20 (m, 2H), 4.09 (t, 2H), 3.97-3.88 (m, 1H), 1.45 (s, 9H)

### [Step 3] Preparation of Intermediate RE-30

Compound RE-30 was prepared starting from Intermediate RE-30B. according to procedures similar to those described in Steps 3 through 7 of Reference Example 1 for the preparation of Compound RE-1.
MS(m/z):347.0[M+H]⁺

### Reference Example 31 2-[3-(azetidin-3-yl)-4,7-difluoro-1H-indazol-1-yl]-N-(2,2,2-trifluoroethyl)acetamide hydrochloride (Compound RE-31)

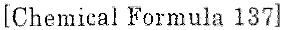

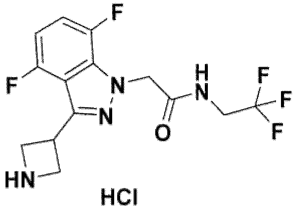

### [Step 1] Preparation of Intermediate RE-31A

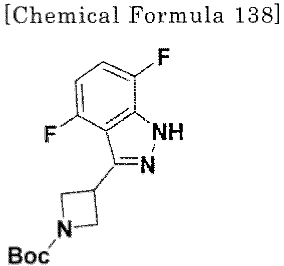

A mixture of Intermediate RE-1B (1.34 g), ethanol (10 mL) and hydrazine monohydrate (3.94 mL) was reacted in a microwave reactor at 140°C for 1 hour. To the reaction solution was added water, and the mixture was extracted with ethyl acetate. The organic layer was washed with a saturated saline solution and dried with anhydrous magnesium sulfate. The solvent was removed under reduced pressure, and then the residue was purified by silica gel column chromatography to afford the title compound (218 mg). MS (m/z): 310.5 [M+H]⁺

### [Step 2] Preparation of Intermediate RE-31B

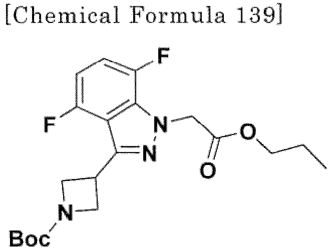

To a mixture of Intermediate RE-31A (218 mg) and DMF (3.5 mL) were added cesium carbonate (276 mg) and propyl 2-bromoacetate (153 mg) sequentially under an ice bath condition, and then the mixture was stirred at room temperature. A saturated aqueous solution of ammonium chloride was added to the reaction solution, and the mixture was extracted with ethyl acetate. The organic layer was washed with a saturated saline solution and dried over anhydrous magnesium sulfate. The solvent was removed under reduced pressure, and then the residue was purified by silica gel column chromatography to afford the title compound (238 mg). ¹H-NMR (400 MHz, CDCl₃) δ 7.00-6.90 (m, 1H), 6.70-6.60 (m, 1H), 5.22 (s, 2H), 4.40-4.27 (m, 4H), 4.25-4.17 (m, 1H), 4.16-4.11 (m, 2H), 1.70-1.59 (m, 2H), 1.46 (s, 9H), 0.89 (t, 3H)

### [Step 3] Preparation of Compound RE-31

Compound RE-31 was prepared starting from Intermediate RE-31A, according to procedures similar to those described in Steps 5 through 7 of Reference Example 1 for the preparation of Compound RE-1. MS(m/z): 349.0 [M+H]⁺

### Reference Example 32 2-[3-(azetidin-3-yl)-4-bromo-5-fluoro-1H-indazol-1-yl]-N-(2,2,2-trifluoroethyl)acetamide hydrochloride (Compound RE-32)

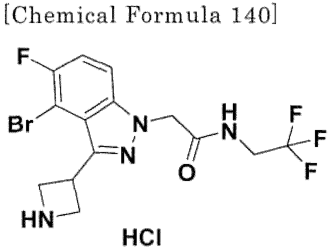

### [Step 1] Preparation of Intermediate RE-32A

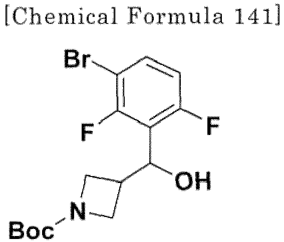

In the method according to Step 1 of Reference Example 1, 2-bromo-1,3-difluorobenzene was used instead of 2-chloro-1,4-difluorobenzene to afford the title compound. ¹H-NMR (400 MHz, CDCla) δ 7.12-7.02 (m, 2H), 5.38 (t, 1H), 4.19-4.10 (m, 1H), 4.06-3.98 (m, 1H), 3.87-3.80 (m, 1H), 3.65-3.60 (m, 1H), 3.30-3.18 (m, 1H), 2.59-2.51 (m, 1H), 1.44 (s. 9H)

### [Step 2] Preparation of Intermediate RE-32B

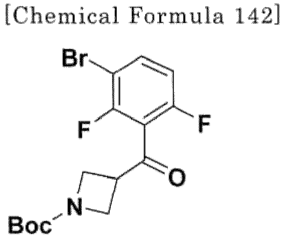

In the method according to Step 2 of Reference Example 30, Intermediate RE-32A was used instead of Intermediate RE-30A to afford the title compound. ¹H-NMR (400 MHz, CDCl₂) δ 7.22-7.16 (m, 1H), 7.14-7.09 (m, 1H), 4.37-4.30 (m, 2H), 4.10 (t, 2H), 3.97-3.88 (m, 1H), 1.45 (s, 9H)

### [Step 3] Preparation of Intermediate RE-32C

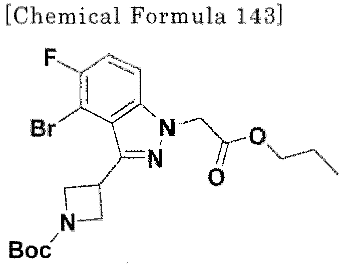

Intermediate RE-32C was prepared starting from Intermediate RE-32B, according to procedures similar to those described in Steps 1 to 2 of Reference Example 31 for the preparation of Intermediate RE-31B.
¹H-NMR (400 MHz, CDCl₃) δ 7.22-7.16 (m, 2H), 5.10 (s. 2H), 4.37-4.30 (m, 4H), 4.15-4.11 (m, 3H), 1.70-1.61 (m, 2H), 1.45 (s, 9H), 0.89 (t, 3H)

### [Step 4] Preparation of Compound RE-32

Compound RE-32 was prepared starting from Intermediate RE-32C, according to procedures similar to those described in Steps 5 through 7 of Reference Example 1 for the preparation of Compound RE-1. MS(m/z):407.1[M-H]⁻

### Reference Example 33 tert-butyl 3-(4-methyl-1-12-oxo-2-[(2S)-2-(trifluoromethyl)pyrrolidin-1-yl]ethyl}-1H-pyrazolo[4,3-c]pyridin-3-yl)azetidine-1-carboxylate (Compound RE-33)

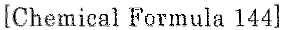

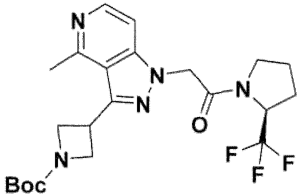

### [Step 1] Preparation of Intermediate RE-33A

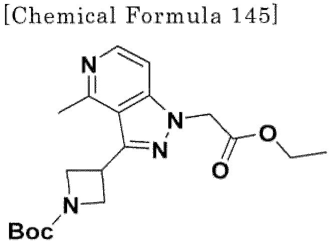

A mixture of Compound RE-14D (200 mg), trimethylboroxine (70 mg), potassium carbonate (280 mg), Pd(dppf)Cl₂·CH₂Cl₂ (41 mg) and 1,4-dioxane (2.5 mL) was degassed, and then stirred at 110°C under argon atmosphere for 5 hours. A saturated aqueous solution of ammonium chloride was added to the reaction solution, and the mixture was extracted with ethyl acetate. The organic layer was washed with a saturated saline solution and dried with anhydrous magnesium sulfate. The solvent was removed under reduced pressure, and then the residue was purified by silica gel column chromatography to afford the title compound (98 mg). MS (m/z): 375.1 [M+H]⁺

### [Step 2] Preparation of Compound RE-33

To a mixture of Intermediate RE-33A (98 mg), methanol (1 mL) and THF (1 mL) was added 2 M aqueous sodium hydroxide solution (1 mL) at room temperature, and then the mixture was stirred overnight. The reaction solution was concentrated under reduced pressure, diluted with water, and acidified with 2 M hydrochloric acid under an ice bath condition. A saturated aqueous solution of sodium bicarbonate was added to make it neutral, and the solvent was concentrated. To the residue was added DMF (2 mL), followed by DIPEA (0.2 mL), (2S)-2-(trifluoromethyl)pyrrolidine hydrochloride (60 mg), and HATU (129 mg), and then the mixture was stirred at room temperature for 2 hours. A saturated aqueous solution of sodium bicarbonate was added to the reaction solution, and the mixture was extracted with ethyl acetate. The organic layer was washed with a saturated saline solution and dried with anhydrous magnesium sulfate. The solvent was removed under reduced pressure, and then the residue was purified by silica gel column chromatography to afford the title compound (97 mg). MS (m/z): 468.2 [M+H]⁺

### Reference Example 34 tert-butyl 3-(1-{2-oxo-2-[(2S)-2-(trifluoromethyl)pyrrolidin-1-yl]ethyl}-1H-pyrazolo[4,3-b]pyridin-3-yl)azetidine-1-carboxylate (Compound RE-34)

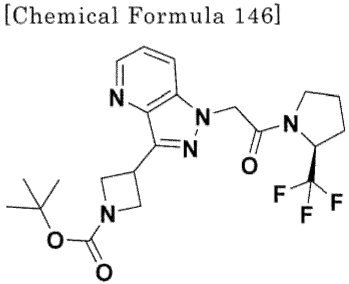

### [Step 1] Preparation of Intermediate RE-34A

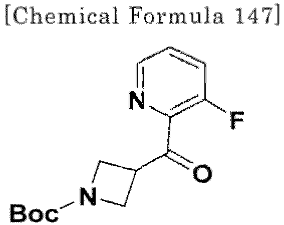

To a mixture of 3-fluoropyridine (674 mg) and THF (9 mL) was added dropwise lithium diisopropylamide (1.08 M n-hexane/tetrahydrofuran solution, 6.4 mL) at -78°C. After stirring at -78°C for 1 hour, a solution of tert-butyl 3-[methoxy(methyl)carbamoyl]azetidine-1-carboxylate (1.13 g) (for example, synthesized according to a method described in WO20129649) in THF (9 mL) was added dropwise thereto, and then the mixture was stirred at -78°C for 2 hours. After stirring at room temperature for 2 hours, a saturated aqueous solution of ammonium chloride was added to the reaction solution, and the mixture was extracted with ethyl acetate. The organic layer was washed with a saturated saline solution and dried with anhydrous magnesium sulfate. The solvent was removed under reduced pressure, and then the residue was purified by silica gel column chromatography to afford the title compound (490 mg). MS (m/z): 281.0 [M+H]⁺

### [Step 2] Preparation of Intermediate RE-34B

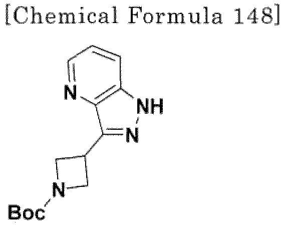

In the method according to Step 1 of Reference Example 31, Intermediate RE-34A was used instead of Intermediate RE-1B to afford the title compound. MS (m/z): 275.1 [M+H]⁺

### [Step 3] Preparation of Compound RE-34

Compound RE-34 was prepared starting from Intermediate RE-34B, according to procedures similar to those described in Steps 1 to 2 of Reference Example 33 for the preparation of Compound RE-33. MS(m/z):454.3[M+H]⁺

### Reference Example 35 tert-butyl 3-(5-methyl-1-{[4-(trifluoromethyl)phenyl]methyl}-1H-pyrazolo[3,4-c]pyridin-3-yl)azetidine-1-carboxylate (Compound RE-35)

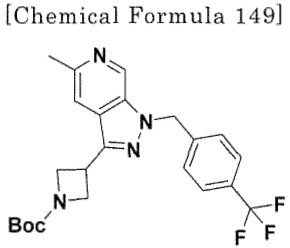

### [Step 1] Preparation of Intermediate RE-35A

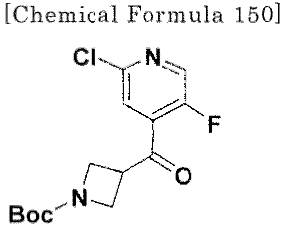

In the method according to Step 1 of Reference Example 34, 2-chloro-5-fluoropyridine was used instead of 3-fluoropyridine to afford the title compound. ¹H-NMR (400 MHz, CDCl₃) 6 8.43 (d, 1H), 7.76 (d, 1H), 4.22-4.15 (m, 4H), 4.08-3.95 (m, 1H), 1.44 (s, 9H)

### [Step 2] Preparation of Intermediate RE-35B

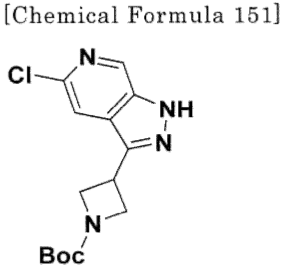

In the method according to Step 1 of Reference Example 31, Intermediate RE-35A was used instead of Intermediate RE-1B to afford the title compound. MS(m/z): 309.1 [M+H]⁺

### [Step 3] Preparation of Intermediate RE-35C

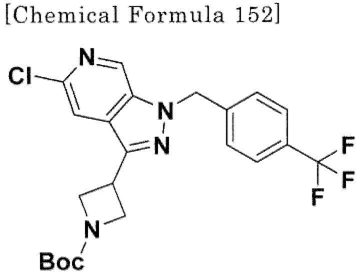

To a mixture of Intermediate RE-35B (100 mg) and DMF (2 mL) were added cesium carbonate (211 mg) and 1-(bromomethyl)-4-(trifluoromethyl)benzene (116 mg), and the mixture was stirred for 1 hour. A saturated aqueous solution of ammonium chloride was added to the reaction solution, and the mixture was extracted with ethyl acetate. The organic layer was washed with a saturated saline solution and dried over anhydrous magnesium sulfate. The solvent was removed under reduced pressure, and then the residue was purified by silica gel column chromatography to afford the title compound (134 mg). MS (m/z): 467.2 [M+H]⁺

### [Step 4] Preparation of Compound RE-35

A mixture of Intermediate RE-35C (134 mg), trimethylboroxine (72 mg), potassium carbonate (159 mg), Pd(dppf)Cl₂·CH₂Cl₂ (23 mg) and 1,4-dioxane (1.4 mL) was degassed, and then stirred at 110°C under argon atmosphere for 5 hours. After that, Pd(dppf)Cl₂·CH₂Cl₂ (23 mg) was added thereto, and then the mixture was stirred at 90°C overnight. To the reaction solution was added water, and the mixture was extracted with ethyl acetate. The organic layer was washed with a saturated saline solution and dried over anhydrous magnesium sulfate. The solvent was removed under reduced pressure, and then the residue was purified by silica gel column chromatography to afford the title compound (58 mg). MS (m/z): 447.3 [M+H]⁺

### Reference Example 36 tert-butyl 3-(5-methyl-1-{2-oxo-2-[(2R)-2-(trifluoromethyl)morpholin-4-yl]ethyl}-1H-pyrazolo[3,4-c]pyridin-3-yl)azetidine-1-carboxylate (Compound RE-36)

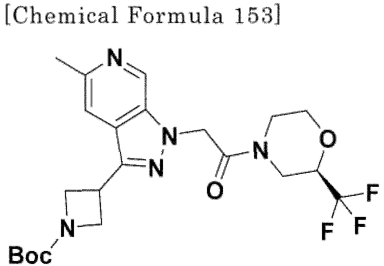

### [Step 1] Preparation of Intermediate RE-36A

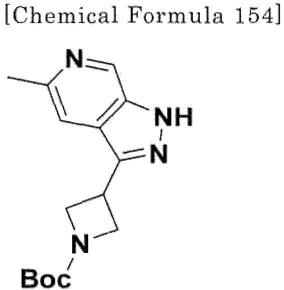

A mixture of Intermediate RE-35B (280 mg), trimethylboroxine (171 mg), potassium carbonate (376 mg), Pd(dppf)Cl₂·CH₂Cl₂ (148 mg) and 1,4-dioxane (3 mL) was reacted in a microwave reactor at 130°C for 1 hour, followed by stirring at 150°C for 1 hour. Trimethylboroxine (0.8 mL) was added thereto, and the mixture was reacted in a microwave reactor at 180°C for 5 minutes. The solvent was removed under reduced pressure, and then the residue was purified by silica gel column chromatography to afford the title compound (66 mg). MS (m/z): 289.2 [M+H]⁺

### [Step 2] Preparation of Intermediate RE-36B

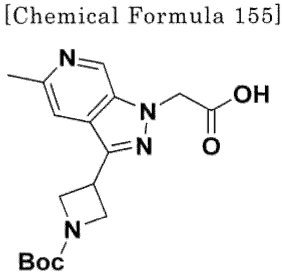

Intermediate RE-36B was prepared starting from Intermediate RE-36A, according to procedures similar to those described in Steps 4 to 5 of Reference Example 1 for the preparation of Intermediate RE-1E. MS(m/z):347.1[M+H]⁺

### [Step 3] Preparation of Compound RE-36

A mixture of Intermediate RE-36B (30 mg), (2R)-2-(trifluoromethyl)morpholine hydrochloride (20 mg), HBTU (39 mg), DMF (0.3 mL) and DIPEA (0.004 mL) was stirred at room temperature overnight. The reaction solution was purified by silica gel column chromatography to afford the title compound (40 mg). MS (m/z): 484.3 [M+H]⁺

### Reference Example 37 2-[3-(azetidin-3-yl)-5-methyl-1H-indazol-1-yl]-1-[(2S)-2-(trifluoromethyl)pyrrolidin-1-yl]ethan-1-one trifluoroacetate (Compound RE-37)

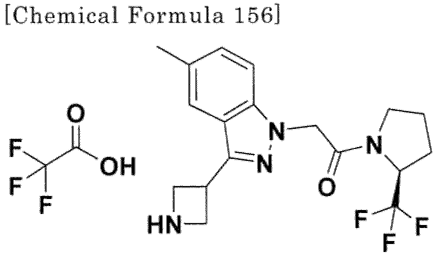

### [Step 1] Preparation of Intermediate RE-37A

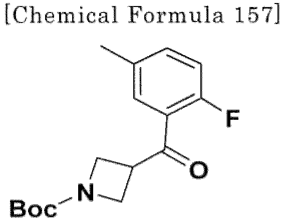

In the method according to Step 1 of Reference Example 5, 2-bromo-1-fluoro-4-methylbenzene was used instead of 2-bromo-4-chloro-1-fluorobenzene to afford the title compound. MS (m/z): 294.0 [M+H]⁺

### [Step 2] Preparation of Intermediate RE-37B

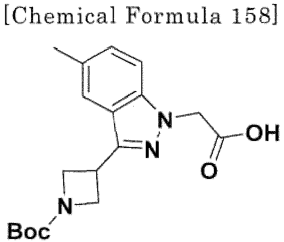

A mixture of Intermediate RE-37A (1.87 g), 1,4-dioxane (64 mL) and hydrazine monohydrate (1.55 mL) was stirred at 100°C for 3 days. To the reaction solution was added water, and the mixture was extracted with ethyl acetate. The organic layer was washed with a saturated saline solution and dried with anhydrous magnesium sulfate. The solvent was removed under reduced pressure, and then the residue was purified by silica gel column chromatography to afford tert-butyl 3-(5-methyl-1H-indazol-3-yl)azetidine-1-carboxylate. To a mixture of the resulting tert-butyl 3-(5-methyl-1H-indazol-3-yl)azetidine-1-carboxylate (473 mg) and DMF (8 mL) were added cesium carbonate (644 mg) and methyl bromoacetate (330 mg) under ice cooling, and then the mixture was stirred at room temperature for 1 hour. To the reaction solution was added water, and the mixture was extracted with ethyl acetate. The organic layer was washed with a saturated saline solution and dried over anhydrous magnesium sulfate. The solvent was removed under reduced pressure, and then the residue was purified by silica gel column chromatography to afford tert-butyl 3-[1-(2-ethoxy-2-oxoethyl)-5-methyl-1H-indazol-3-yl]azetidine-1-carboxylate. To a mixture of the resulting tert-butyl 3-[1-(2-ethoxy-2-oxoethyl)-5-methyl-1H-indazol-3-yl]azetidine-1-carboxylate (467 mg), methanol (2 mL) and THF (2 mL) was added 2 M aqueous sodium hydroxide solution (1.9 mL) at room temperature, and then the mixture was stirred overnight. The reaction solution was concentrated under reduced pressure, diluted with water, and acidified with 2 M hydrochloric acid under an ice bath condition. The resulting precipitate was obtained by filtration to afford the title compound (383 mg). MS (m/z): 344.2 [M-H])⁻

### [Step 3] Preparation of Intermediate RE-37C

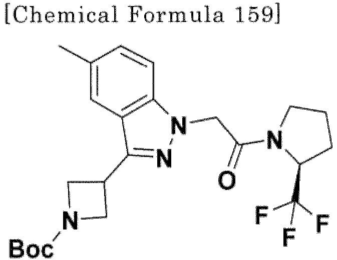

In the method according to Step 4 of Reference Example 4, Intermediate RE-37B was used instead of Intermediate RE-4C to afford the title compound. MS (m/z): 467.3 [M+H]

### [Step 4] Preparation of Compound RE-37

To a mixture of Intermediate RE-37C (171 mg) and dichloromethane (4 mL) was added trifluoroacetic acid (1 mL), and then the mixture was stirred at room temperature for 1 hour. The solvent was removed under reduced pressure, and to the resulting residue was added toluene. Then, the solvent was removed under reduced pressure. To the resulting residue were added ethyl acetate and hexane, and then the solvent was removed under reduced pressure to afford the title compound (145 mg). MS (m/z): 367.2 [M+H]⁺

### Reference Example 38 2-[3-(azetidin-3-yl)-5-fluoro-1H-indazol-1-yl]-1-[(2S)-2-(trifluoromethyl)pyrrolidin-1-yl]ethan-1-one trifluoroacetate (Compound RE-38)

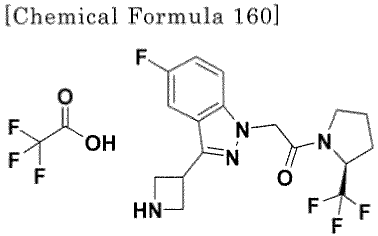

### [Step 1] Preparation of Intermediate RE-38A

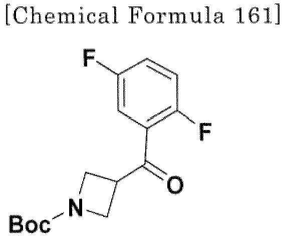

In the method according to Step 1 of Reference Example 5, 1,4-difluoro-2-iodobenzene was used instead of 2-bromo-4-chloro-1-fluorobenzene to afford the title compound. MS (m/z): 298.1 [M+H]⁺

### [Step 2] Preparation of Intermediate RE-38B

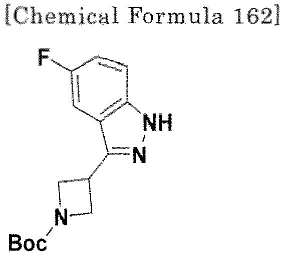

In the method according to Step 3 of Reference Example 1, Intermediate RE-38A was used instead of Intermediate RE-1B to afford the title compound. MS (m/z): 290.1 [M-H]⁻

### [Step 3] Preparation of Intermediate RE-38C

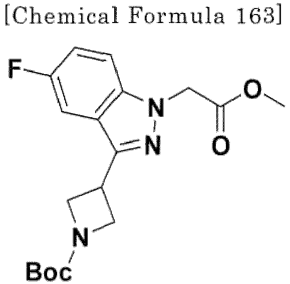

To a mixture of Intermediate RE-38B (176 mg) and DMF (3 mL) were added cesium carbonate (236 mg) and methyl bromoacetate (111 mg) under ice cooling, and then the mixture was stirred at room temperature for 1 hour. A saturated aqueous solution of ammonium chloride was added to the reaction solution, and the mixture was extracted with ethyl acetate. The organic layer was washed with a saturated saline solution and dried over anhydrous magnesium sulfate. The solvent was removed under reduced pressure, and then the residue was purified by silica gel column chromatography to afford the title compound (183 mg). ¹H-NMR (400 MHz, CDCl₃) δ 7.37 (d, 1H), 7.25-7.15 (m, 2H), 5.10 (s, 2H), 4.41 (t, 2H), 4.29 (t, 2H), 4.13-4.03 (m, 1H), 3.77 (s, 3H), 1.48 (s, 9H)

### [Step 4] Preparation of Intermediate RE-38D

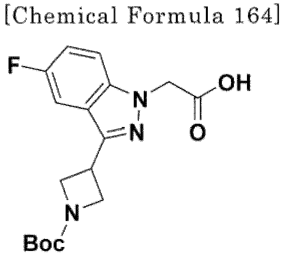

In the method according to Step 5 of Reference Example 1, Intermediate RE-38C was used instead of Intermediate RE-1D to afford the title compound. MS (m/z): 348.4 [M-H]⁻

### [Step 5] Preparation of Intermediate RE-38E

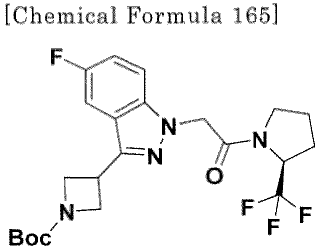

In the method according to Step 6 of Reference Example 1, Intermediate RE-38D and (2S)-2-(trifluoromethyl)pyrrolidine hydrochloride were used instead of Intermediate RE-1E and 2,2,2-trifluoroethaneamine hydrochloride, respectively, to afford the title compound. MS (m/z): 471.3 [M+H]⁺

### [Step 6] Preparation of Compound RE-38

In the method according to Step 4 of Reference Example 37, Intermediate RE-38E was used instead of Intermediate RE-37C to afford the title compound. MS (m/z): 371.2 [M+H]⁺

### Reference Example 39 tert-butyl 2-[3-(1-{2-[3-(azetidin-3-yl)-1H-indazol-1-yl]acetyl}azetidin-3-yl)-1H-indazol-1-yl]acetate (Compound RE-39)

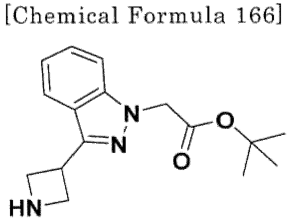

### [Step 1] Preparation of Intermediate RE-39A

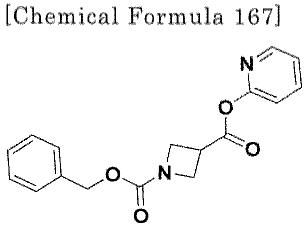

A mixture of 1-benzyloxycarbonylazetidine-3-carboxylic acid (120 mg), di(pyridin-2-yl)carbonate (100 mg), DMAP (6 mg) and dichloromethane (1.2 mL) was stirred at room temperature for 1 hour. The solvent was removed under reduced pressure, and then the residue was purified by silica gel column chromatography to afford the title compound (94 mg). MS (m/z): 313.1 [M+H]⁺

### [Step 2] Preparation of Intermediate RE-39B

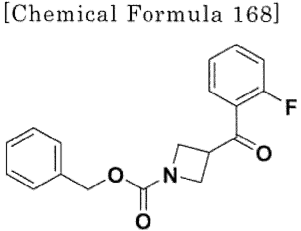

A mixture of Intermediate RE-39A (630 mg), (2-fluorophenyl)boronic acid (560 mg), triphenylphosphine (160 mg) and 1,4-dioxane (6.7 mL) was degassed, Pd(OAc)₂ (45 mg) was added thereto, and then the mixture was stirred at 50°C overnight under an argon atmosphere. The solvent was removed under reduced pressure, and then the residue was purified by silica gel column chromatography to afford the title compound (207 mg). MS (m/z): 314.0 [M+H]⁺

### [Step 3] Preparation of Intermediate RE-39C

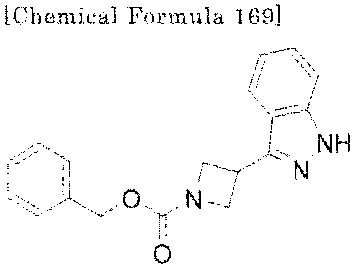

A mixture of Intermediate RE-39B (5.50 g), hydrazine monohydrate (4.3 mL) and NMP (35 mL) was stirred at 150°C overnight. Ice water was added to the reaction solution, and the mixture was extracted with ethyl acetate. The organic layer was washed with a saturated saline solution and dried over anhydrous magnesium sulfate. The solvent was removed under reduced pressure, and then the residue was purified by silica gel column chromatography to afford the title compound (3.26 g). MS (m/z): 308.1 [M+H]⁺

### [Step 4] Preparation of Intermediate RE-39D

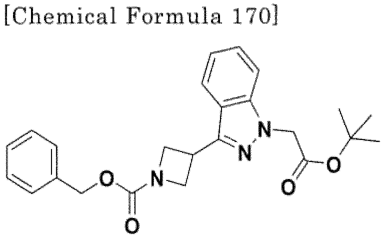

To a mixture of Intermediate RE-39C (1.00 g) and DMF (11 mL) were added cesium carbonate (1.27 g) and tert-butyl 2-bromoacetate (762 mg) under ice cooling, and then the mixture was stirred for 30 minutes. The mixture was then stirred at room temperature overnight. To the reaction solution was added water, and the mixture was extracted with ethyl acetate. The organic layer was washed with a saturated saline solution and dried with anhydrous magnesium sulfate. The solvent was removed under reduced pressure, and then the residue was purified by silica gel column chromatography to afford the title compound (875 mg). MS (m/z): 444.2 [M+Na]⁺

### [Step 5] Preparation of Compound RE-39

To a mixture of Intermediate RE-39D (875 mg) and methanol (21 mL) was added 5% Pd-C (440 mg), and then the mixture was stirred at room temperature overnight under hydrogen atmosphere (0.1 MPa). The insoluble material was filtered off by Celite^{®} filtration, and the filtrate was concentrated under reduced pressure to afford the title compound (473 mg). MS (m/z): 288.2 [M+H]⁺

### Reference Example 40 tert-butyl 4-(1-{2-oxo-2-[(2S)-2-(trifluoromethyl)pyrrolidin-1-yl]ethyl}-1H-indazol-3-yl)-1,2,3,6-tetrahydropyridine-1-carboxylate (Compound RE-40)

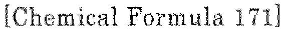

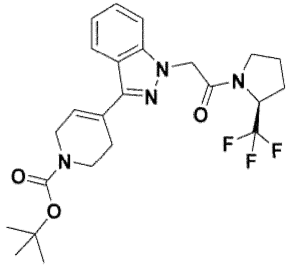

### [Step 1] Preparation of Intermediate RE-40A

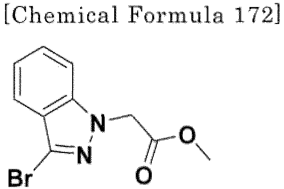

To a mixture of 3-bromo-1H-indazole (2 g) and DMF (34 mL) were added cesium carbonate (1.27 g) and methyl bromoacetate (1.86 g) under ice cooling, and the mixture was stirred for 30 minutes. The mixture was then stirred at room temperature for 30 minutes. A saturated aqueous solution of ammonium chloride was added to the reaction solution, and the mixture was extracted with ethyl acetate. The organic layer was washed with a saturated saline solution and dried with anhydrous magnesium sulfate. The solvent was removed under reduced pressure, and then the residue was purified by silica gel column chromatography to afford the title compound (2.88 g). ¹H-NMR (400 MHz, CDCl₃) δ 7.64 (d, 1H), 7.50-7.44 (m, 1H), 7.32 (d, 1H), 7.27 (t, 1H), 5.13 (s, 2H), 3.75 (s, 3H)

### [Step 2] Preparation of Intermediate RE-40B

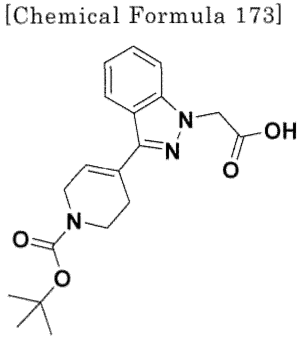

A mixture of Intermediate RE-40A (200 mg), tert-butyl 4-(4,4,5,5-tetramethyl-1,3,2-dioxaborolan-2-yl)-3,6-dihydro-2H-pyridine-1-carboxylate (345 mg), sodium carbonate (236 mg), Pd(dppf)Cl₂·CH₂Cl₂ (91 mg), 1,4-dioxane (1.5 mL) and water (0.5 mL) was degassed, and then the mixture was stirred at 95°C overnight under argon atmosphere. The insoluble material was filtered off by Celite^{®} filtration, the filtrate was concentrated under reduced pressure, and then the residue was purified by silica gel column chromatography to afford the title compound (255 mg). MS (m/z): 356.3 [M-H]⁻

### [Step 3] Preparation of Compound RE-40

To a mixture of Intermediate RE-40B (127 mg), DMF (1.2 mL), DIPEA (0.184 mL) and (2S)-2-(trifluoromethyl)pyrrolidine hydrochloride (75 mg) was added HBTU (162 mg), and then the mixture was stirred at room temperature for 4 hours. The reaction solution was purified by silica gel column chromatography to afford the title compound (105 mg). MS *(m*/*z):* 479.3 [M+H]⁺

### Reference Example 41 tert-butyl 4-(1-{[methyl(2,2,2-trifluoroethyl)carbamoyl]methyl}-1H-indazol-3-yl)-1,2,3,6-tetrahydropyridine-1-carboxylate (Compound RE-41)

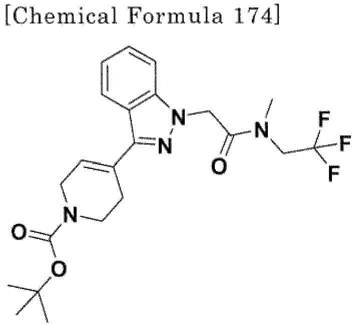

To a mixture of Intermediate RE-40B (127 mg), DMF (1.2 mL), DIPEA (0.184 mL) and 2,2,2-trifluoroethaneamine hydrochloride (64 mg) was added HBTU (162 mg), and then the mixture was stirred at room temperature overnight. The reaction solution was purified by silica gel column chromatography to afford the title compound (104 mg). MS (m/z): 475.2 [M+Na]⁺

### Reference Example 42 2-[3-(azetidin-3-yl)-5-fluoro-1H-indazol-1-yl]-N-methy]-N-(2,2,2-trifluoroethyl)acetamide trifluoroacetate (Compound RE-42)

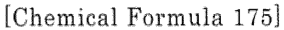

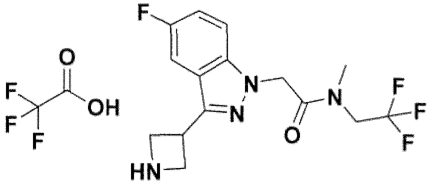

### [Step 1] Preparation of Intermediate RE-42A

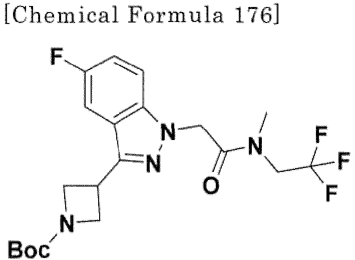

In the method according to Step 6 of Reference Example 1, Intermediate RE-38D and 2,2,2-trifluoro-N-methylethanamine hydrochloride were used instead of Intermediate RE-1E and 2,2,2-trifluoro-N-methylethanamine hydrochloride, respectively, to afford the title compound. MS (m/z): 467.2 [M+Na]⁺

### [Step 2] Preparation of Compound RE-42

In the method according to Step 4 of Reference Example 37, Intermediate RE-42A was used instead of Intermediate RE-37C to afford the title compound. MS (m/z): 345.1 [M+H]⁺

### Reference Example 43 2-[3-(azetidin-3-yl)-5,6-difluoro-1H-indazol-1-yl]-N-methyl-N-(2,2,2-trifluoroethyl)acetamide trifluoroacetate (Compound RE-43)

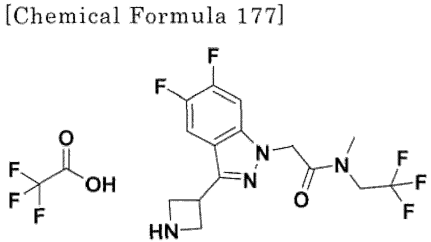

### [Step 1] Preparation of Intermediate RE-43A

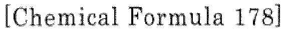

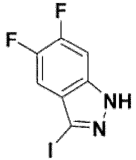

To a mixture of 5,6-difluoro-1H-indazole (513 mg), potassium hydroxide (374 mg) and DMF (1.3 mL) was added iodine (1.01 g) under ice cooling, and then the mixture was stirred at room temperature for 2 hours. A saturated aqueous solution of sodium thiosulfate was added to the reaction solution. Then, the precipitate produced was obtained by filtration, washed with water, and dried to afford the title compound (899 mg). MS (m/z): 281.0 [M+H]⁺

### [Step 2] Preparation of Intermediate RE-43B

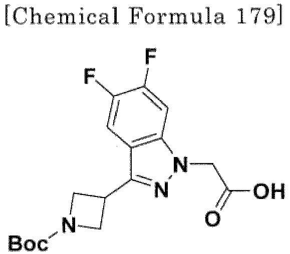

Intermediate RE-43B was prepared starting from Intermediate RE-43A, according to procedures similar to those described in Steps 1 through 3 of Reference Example 6 for the preparation of Compound RE-6C. MS (m/z): 366.2 [M-H]⁻

### [Step 3] Preparation of Intermediate RE-43C

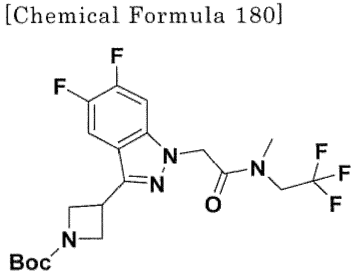

In the method according to Step 6 of Reference Example 1, Intermediate RE-43B and 2,2,2-trifluoro-N-methylethanamine hydrochloride were used instead of Intermediate RE-1E and 2,2,2-trifluoroethanamine hydrochloride, respectively, to afford the title compound. MS (m/z): 485.2 [M+Na]⁺

### [Step 4] Preparation of Compound RE-43

In the method according to Step 4 of Reference Example 37, Intermediate RE-43C was used instead of Intermediate RE-37C to afford the title compound. MS (m/z): 363.1 [M+H]⁺

### Reference Example 44 2-[3-(azetidin-3-yl)-5,6-difluoro-1H-indazol-1-yl]-1-[(2S)-2-(trifluoromethyl)pyrrolidin-1-yl]ethan-1-one trifluoroacetate (Compound RE-44)

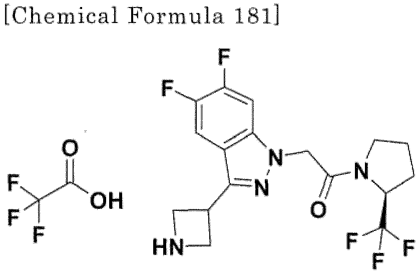

Compound RE-44 was prepared starting from Intermediate RE-43B, according to procedures similar to those described in Steps 5 to 6 of Reference Example 38 for the preparation of Compound RE-38. MS (m/z): 389.2 [M+H]⁺

### Reference Example 45 2-[3-(3-hydroxyazetidin-3-yl)-1H-indazol-1-yl]-1-(2S)-2-(trifluoromethyl)pyrrolidin-1-yl]ethan-1-one trifluoroacetate (Compound RE-45)

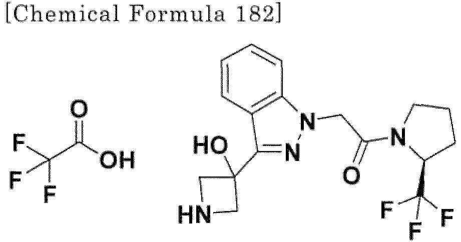

### [Step 1] Preparation of Intermediate RE-45A

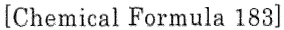

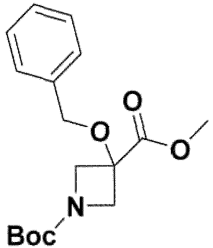

To a mixture of 1-(tert-butyl) 3-methyl 3-hydroxyazetidine-1,3-dicarboxylate (1.0 g) and DMF (14 mL) (1.0 g) was added 60% sodium hydride (260 mg) under ice cooling, and the mixture was stirred for 50 minutes. Then, benzyl bromide (1.10 g) was added thereto, and the mixture was stirred at room temperature for 5 hours. A saturated aqueous solution of ammonium chloride was added to the reaction solution, and the mixture was extracted with ethyl acetate. The organic layer was washed with a saturated saline solution and dried with anhydrous magnesium sulfate. The solvent was removed under reduced pressure, and then the residue was purified by silica gel column chromatography to afford the title compound (1.07 g). ¹H-NMR (400 MHz, CDCl₃) δ 7.40-7.30 (m, 5H), 4.50 (s, 2H), 4.26 (d, 2H), 4.06 (d, 2H), 3.84 (s, 3H), 1.56 (s, 9H)

### [Step 2] Preparation of Intermediate RE-45B

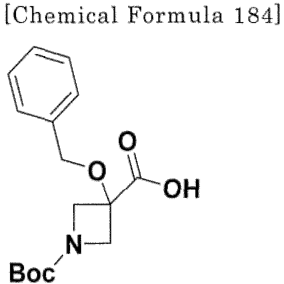

To a mixture of Intermediate RE-45A (1.07 g), methanol (8 mL) and THF (8 mL) was added 2 M aqueous sodium hydroxide solution, and the mixture was stirred at room temperature. The solvent was removed under reduced pressure, and then the mixture was neutralized with 2 M hydrochloric acid and extracted with ethyl acetate. The organic layer was dried with anhydrous magnesium sulfate. The solvent was removed under reduced pressure, and then the residue was washed with hexane and dried to afford the title compound (866 mg). MS (m/z): 306.1 [M-H]⁻

### [Step 3] Preparation of Intermediate RE-45C

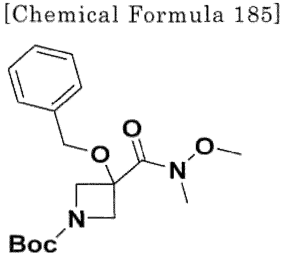

A mixture of Intermediate RE-45B (866 mg), N,O-dimethylhydroxylamine (412 mg), 1-hydroxybenzotriazole monohydrate (561 mg), EDCI·HCl (702 mg) and DIPEA (1.46 mL) was stirred at room temperature overnight. A saturated aqueous solution of sodium bicarbonate was added to the reaction solution, and the mixture was extracted with ethyl acetate. The organic layer was washed with a saturated saline solution and dried over anhydrous magnesium sulfate. The solvent was removed under reduced pressure, and then the residue was purified by silica gel column chromatography to afford the title compound (961 mg). MS (m/z): 373.0 [M+H]⁺

### [Step 4] Preparation of Intermediate RE-45D

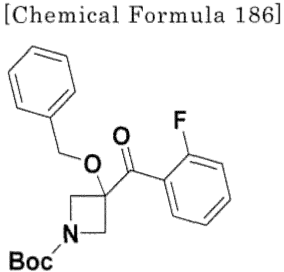

In the method according to Step 1 of Reference Example 5, 1-fluoro-2-iodobenzene and Intermediate RE-45C were used instead of 2-bromo-4-chloro-1-fluorobenzene and tert-butyl 3-[methoxy(methyl)carbamoyllazetidine-1-carboxylate, respectively, to afford the title compound. MS(m/z):286.2[M-Boc+2H]⁺, ¹H-NMR (400 MHz, DMSO-d6) δ 7.75-7.68 (m, 2H), 7.35 (dd, 2H), 7.22-7.20 (m, 3H), 6.93-6.90 (m, 2H), 4.36 (d, 2H), 4.23 (s, 2H), 4.13 (d, 2H), 1.41 (s, 9H)

### [Step 5] Preparation of Intermediate RE-45E

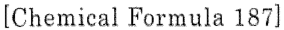

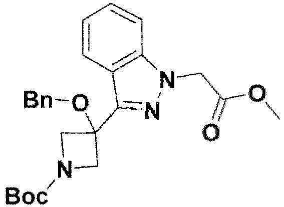

A mixture of Intermediate RE-45D (785 mg), hydrazine monohydrate (0.99 mL), potassium carbonate (844 mg) and DMA (15 mL) was reacted in a microwave reactor at 100°C for 1 hour. A saturated aqueous solution of ammonium chloride was added to the reaction solution, and the mixture was extracted with ethyl acetate. The organic layer was washed with a saturated saline solution and dried with anhydrous magnesium sulfate. The solvent was removed under reduced pressure, and to a mixture of the resulting residue, cesium carbonate (1.99 g) and DMA (7 mL) was added methyl bromoacetate (467 mg) under ice cooling, and then the mixture was stirred at room temperature overnight. A saturated aqueous solution of ammonium chloride was added to the reaction solution, and the mixture was extracted with a mixed solvent of ethyl acetate and hexane. The organic layer was washed with a saturated saline solution and dried over anhydrous magnesium sulfate. The solvent was removed under reduced pressure, and then the residue was purified by silica gel column chromatography to afford the title compound (709 mg). MS (m/z): 474.3 [M+Na]⁺

### [Step 6] Preparation of Intermediate RE-45F

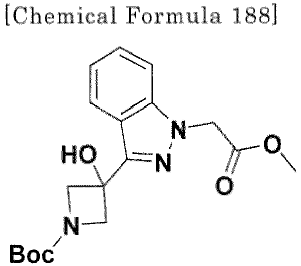

To a mixture of Intermediate RE-45E (709 mg) and THF (20 mL) was added 5% Pd-C (600 mg), and then the mixture was stirred at 40°C for 6 hours under hydrogen atmosphere (0.3 MPa). After stirring the mixture at 50°C for 11 hours, the insoluble material was filtered off by Celite^{®} filtration. The filtrate was concentrated under reduced pressure, and then the residue was purified by silica gel column chromatography to afford the title compound (425 mg). MS (m/z): 384.2 [M+Na]⁺

### [Step 7] Preparation of Intermediate RE-45G

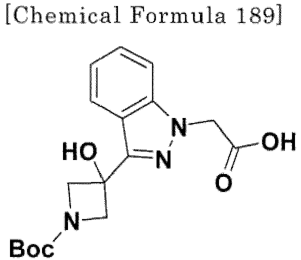

In the method according to Step 5 of Reference Example 1, Intermediate RE-45F was used instead of Intermediate RE-1D to afford the title compound. MS (m/z): 346.1 [M-H]⁻

### [Step 8] Preparation of Intermediate RE-45H

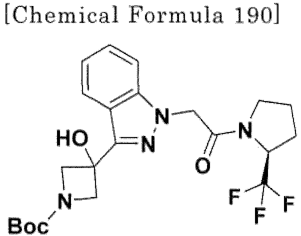

To a mixture of Intermediate RE-45G (159 mg), (2S)-2-(trifluoromethyl)pyrrolidine hydrochloride (121 mg), HOBt (93 mg), DIPEA (0.24 mL) and DMF (0.3 mL) was added EDCI·HCl (132 mg), and then the mixture was stirred at room temperature overnight. A saturated aqueous solution of sodium bicarbonate was added to the reaction solution, and the mixture was extracted with ethyl acetate. The organic layer was washed with a saturated saline solution and dried over anhydrous magnesium sulfate. The solvent was removed under reduced pressure, and then the residue was purified by silica gel column chromatography to afford the title compound (193 mg). MS (m/z): 491.3 [M+Na]⁺

### [Step 9] Preparation of Compound RE-45

In the method according to Step 4 of Reference Example 37, Intermediate RE-45H was used instead of Intermediate RE-37C to afford the title compound. MS (m/z): 369.2 [M+H]⁺

### Reference Example 46 tert-butyl 3-fluoro-3-(1-{2-oxo-2-[(2S)-2-(trifluoromethyl)pyrrolidin-1-yl]ethyl}-1H-indazol-3-yl)azetidine-1-carboxylate (Compound RE-46)

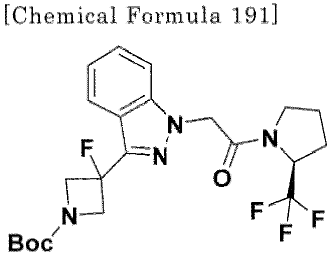

To a mixture of Intermediate RE-45H (96 mg) and dichloromethane (4 mL) was added DAST (50 mg) at -78°C, and then the mixture was stirred for 3 hours. The mixture was then stirred under ice cooling for 30 minutes. A saturated aqueous solution of sodium bicarbonate was added to the reaction solution, and the mixture was extracted with dichloromethane. The organic layer was washed with a saturated saline solution and dried with anhydrous magnesium sulfate. The solvent was removed under reduced pressure, and then the residue was purified by silica gel column chromatography to afford the title compound (47 mg). MS (m/z): 493.2 [M+Na]⁺

### Reference Example 47 2-[3-(azetidin-3-yl)-5-methyl-1H-indazol-1-yl]-N-methyl-N-(2,2,2-trifluoroethyl)acetamide trifluoroacetate (Compound RE-47)

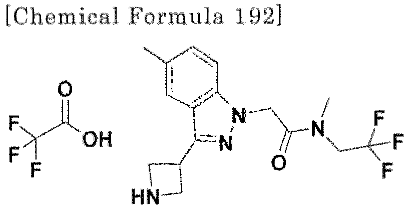

### [Step 1] Preparation of Intermediate RE-47A

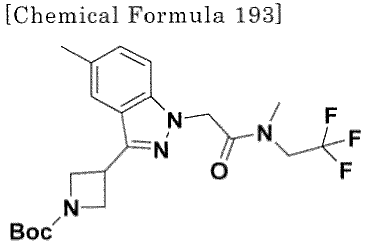

In the method according to Step 6 of Reference Example 1, Intermediate RE-37B and 2,2,2-trifluoro-N-methylethanamine hydrochloride were used instead of Intermediate RE-1E and 2,2,2-trifluoroethanamine hydrochloride, respectively, to afford the title compound. MS (m/z): 463.3 [M+Na]⁺

### [Step 2] Preparation of Compound RE-47

In the method according to Step 4 of Reference Example 37, Intermediate RE-47A was used instead of Intermediate RE-37C to afford the title compound. MS (m/z): 341.0 [M+H]⁺

### Reference Example 48 tert-butyl 3-(5-methoxy-1-{2-oxo-2-[(2S)-2-(trifluoromethyl)pyrrolidin-1-yl]ethyl}-1H-indazol-3-yl)azetidine-1-carboxylate (Compound RE-48)

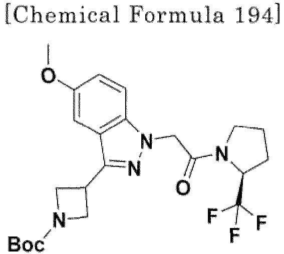

### [Step 1] Preparation of Intermediate RE-48A

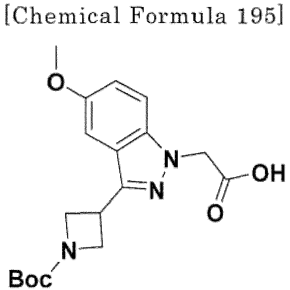

Intermediate RE-48A was prepared starting from 3-iodo-5-methoxy-1H-indazole, according to procedures similar to those described in Steps 1 through 3 of Reference Example 6 for the preparation of Intermediate RE-6C. MS (m/z): 360.2 [M-H]⁻

### [Step 2] Preparation of Compound RE-48

In the method according to Step 6 of Reference Example 1, Intermediate RE-48A and (2S)-2-(trifluoromethyl)pyrrolidine hydrochloride were used instead of Intermediate RE-1E and 2,2,2-trifluoroethaneamine hydrochloride, respectively, to afford the title compound. MS (m/z): 505.2 [M+Na]⁺

### Reference Example 49 2-[3-(azetidin-3-yl)-5,5-dimethyl-4,5,6,7-tetrahydro-1H-indazol-1-yl]-1-[(2S)-2-(trifluoromethyl)pyrrolidin-1-yl]ethan-1-one trifluoroacetate (Compound RE-49)

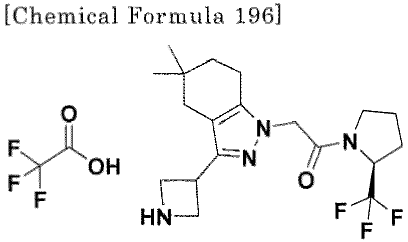

### [Step 1] Preparation of Intermediate RE-49A

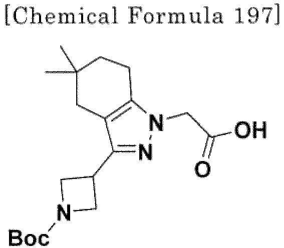

Intermediate RE-49A was prepared starting from 3-iodo-5,5-dimethyl-1,4,6,7-tetrahydroindazole (for example, synthesized according to a method described in Journal of Medicinal Chemistry 2013, 56(4), 1677-1692), according to procedures similar to those described in Steps 1 through 3 of Reference Example 6 for the preparation of Intermediate RE-6C. MS (m/z): 362.3 [M-H]⁻

### [Step 2] Preparation of Intermediate RE-49B

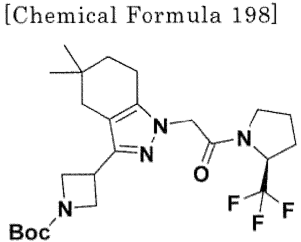

In the method according to Step 6 of Reference Example 1, Intermediate RE-49A and (2S)-2-(trifluoromethyl)pyrrolidine hydrochloride were used instead of Intermediate RE-1E and 2,2,2-trifluoroethaneamine hydrochloride, respectively, to afford the title compound. MS (m/z): 485.4 [M+H]⁺

### [Step 3] Preparation of Compound RE-49

In the method according to Step 4 of Reference Example 37, Intermediate RE-49B was used instead of Intermediate RE-37C to afford the title compound. MS (m/z): 385.4 [M+H]⁺

### Reference Example 50 2-[3-(azetidin-3-yl)-4-chloro-5-fluoro-1H-indazol-1-yl]-1-[(2S)-2-(trifluoromethyl)pyrrolidin-1-yl]ethan-1-one trifluoroacetate (Compound RE-50)

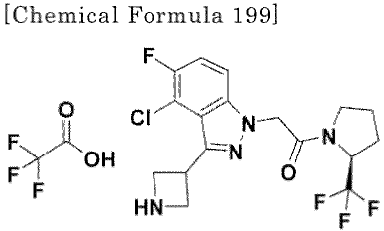

To a mixture of Intermediate RE-1E (200 mg), HATU (258 mg), (2S)-2-(trifluoromethyl)pyrrolidine hydrochloride (119 mg) and DMF (1.7 mL) was added DIPEA (0.3 mL), and then the mixture was stirred at room temperature overnight. The reaction solution was purified by silica gel column chromatography to afford tert-butyl 3-(4-chloro-5-fluoro-1-(2-oxo-2-[(2S)-2-(trifluoromethyl)pyrrolidin-1-yl]ethyl}-1H-indazol-3-yl)azetidine-1-carboxylate (250 mg). To a mixture of this tert-butyl 3-(4-chloro-5-fluoro-1-{2-oxo-2-[(2S)-2-(trifluoromethyl)pyrrolidin-1-yllethyl}-1H-indazol-3-yl)azetidine-1-carboxylate (250 mg) and dichloromethane (0.5 mL) was added trifluoroacetic acid (1 mL), and then the mixture was stirred at room temperature for 4 hours. The solvent was removed under reduced pressure, and to the residue obtained was added toluene, followed by ethyl acetate and hexane. Then, the solvent was removed under reduced pressure to afford the title compound (260 mg). MS (m/z): 405.1 [M+H]⁺

### Reference Example 51 2-[3-(azetidin-3-yl)-4-chloro-5-fluoro-1H-indazol-1-yl]-N-methyl-N-(2,2,2-trifluoroethyl)acetamide trifluoroacetate (Compound RE-51)

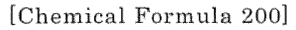

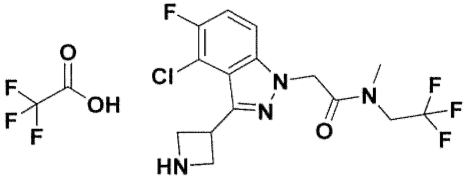

### [Step 1] Preparation of Intermediate RE-51A

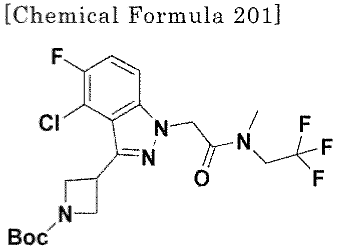

In the method according to Step 6 of Reference Example 1, 2,2,2-trifluoro-N-methylethanamine hydrochloride was used instead of 2,2,2-trifluoroethanamine hydrochloride to afford the title compound. MS (m/z): 501.2 [M+Na]⁺

### [Step 2] Preparation of Compound RE-51

In the method according to Step 4 of Reference Example 37, Intermediate RE-51A was used instead of Intermediate RE-37C to afford the title compound. MS (m/z): 378.9 [M+H]⁺

### Reference Example 52 tert-butyl 3-(4,6-difluoro-1-([(2,2,2-trifluoroethyl)carbamoyl]methyl}-1H-indazol-3-yl)azetidine-1-carboxylate (Compound RE-52)

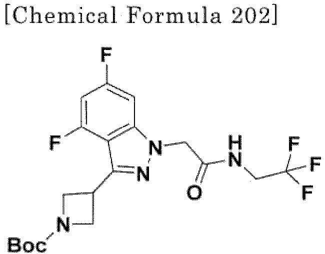

In the method according to Step 6 of Reference Example 1, Intermediate RE-2E was used instead of Intermediate RE-1E to afford the title compound. ¹H-NMR (400 MHz, CDCl₂) δ 6.85 (dd, 1H), 6.71-6.65 (m, 1H), 6.50 (brs, 1H), 4.97 (s, 2H), 4.41-4.35 (m, 2H), 4.30-4.16 (m, 3H), 3.96-3.88 (m, 2H)1.46 (s, 9H)

### Reference Example 53 2-[3-(azetidin-3-yl)-4,6-difluoro-1H-indazol-1-yl]-1-[(2S)-2-(trifluoromethyl)pyrrolidin-1-yl]propane-1-one hydrochloride (Compound RE-53)

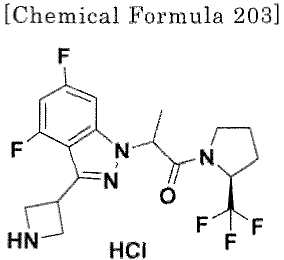

Compound RE-53 was prepared starting from Intermediate RE-2C, according to procedures similar to those described in Steps 4 through 7 of Reference Example 2 for the preparation of Compound RE-2, using methyl 2-chloropropionate instead of ethyl 2-bromoacetate. MS (m/z): 403.2 [M+H]⁺

### Reference Example 54 tert-butyl 3-(4,6-difluoro-1-{1-fluoro-2-oxo-2-[(2S)-2-(trifluoromethyl)pyrrolidin-1-yl]ethyl-1H-indazol-3-yl)azetidine-1-carboxylate (Compound RE-54)

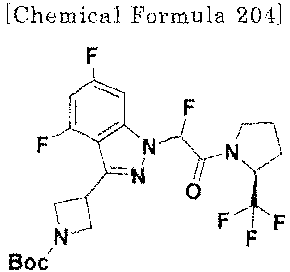

Compound RE-54 was prepared starting from Intermediate RE-2C, according to procedures similar to those described in Steps 4 through 6 of Reference Example 2 for the preparation of Intermediate RE-2F, using ethyl bromofluoroacetate instead of ethyl 2-bromoacetate. MS (m/z): 529.2 [M+Na]⁺

### Reference Example 55 tert-butyl 3-{1-[(4,4-difluorocyclohexyl)methyl]-4,6-difluoro-1H-indazol-3-yl]azetidine-1-carboxylate (Compound RE-55)

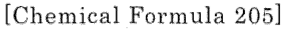

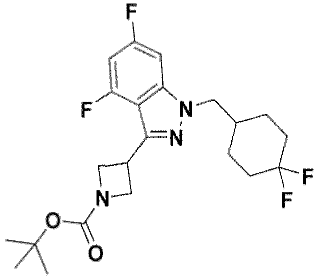

A mixture of Intermediate RE-2C (50 mg), (4,4-difluorocyclohexyl)methyl 4-methylbenzenesulfonate (59 mg), cesium carbonate (79 mg) and DMF (0.8 mL) was stirred at room temperature overnight. A saturated aqueous solution of ammonium chloride was added to the reaction solution, and the mixture was extracted with ethyl acetate. The organic layer was washed with a saturated saline solution and dried over anhydrous magnesium sulfate. The solvent was removed under reduced pressure, and then the residue was purified by silica gel column chromatography to afford the title compound (80 mg). MS (m/z): 342.1 [M-Boc+2H]⁺

### Reference Example 56 tert-butyl 3-[4,6-difluoro-1-(3-phenylprop-2-yl-1-yl)-1H-indazol-3-yl]azetidine-1-carboxylate (Compound RE-56)

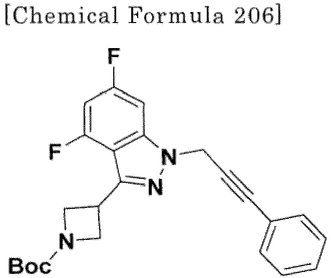

In the method according to Reference Example 55, 3-phenylprop-2-yn-1-yl 4-methylbenzenesulfonate was used instead of (4,4-difluorocyclohexyl)methyl 4-methylbenzenesulfonate to afford the title compound. MS (m/z): 342.1 (M-Boc+2H]⁺

### Reference Example 57 tert-butyl 3-[1-(3-cyclopropylprop-2-yn-1-yl)-4,6-difluoro-1H-indazol-3-yl]azetidine-1-carboxylate (Compound RE-57)

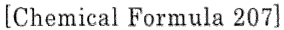

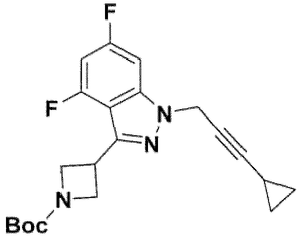

In the method according to Reference Example 55, 3-cycloprop-2-yn-1-yl 4-methylbenzenesulfonate was used instead of (4,4-difluorocyclohexyl)methyl 4-methylbenzenesulfonate to afford the title compound. MS (m/z): 288.2 [M-Boc+2H]⁺

### Reference Example 58 tert-butyl 3-(4,6-difluoro-1-[(1-phenylazetidin-3-yl)methyl]-1H-indazol-3-yl}azetidine-1-carboxylate (Compound RE-58)

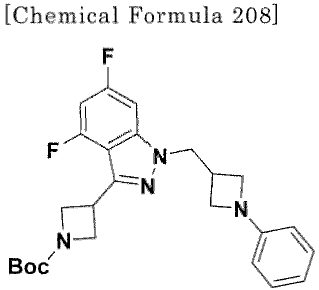

### [Step 1] Preparation of Intermediate RE-58A

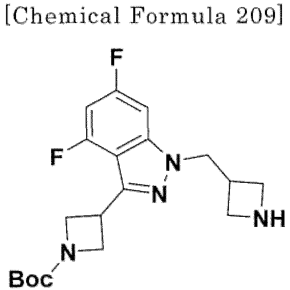

A mixture of Intermediate RE-2C (300 mg), benzyl 3-(p-toluenesulfonyloxymethyl)azetidine-1-carboxylate (437 mg), cesium carbonate (474 mg) and DMF (3.9 mL) was stirred at room temperature for 4 hours. To the reaction solution was added water, and the mixture was extracted with ethyl acetate. The organic layer was dried over anhydrous sodium sulfate. The solvent was removed under reduced pressure, and then the residue was purified by silica gel column chromatography to afford tert-butyl 3-[1-({1-[(benzyloxy)carbonyl]azetidin-3-yl}methyl)-4,6-difluoro-1H-indazol-3-yl]azetidine-1-carboxylate (200 mg). A mixture of this tert-butyl 3-[1-({1-[(benzyloxy)carbonyl]azetidin-3-yl}methyl)-4,6-difluoro-1H-indazol-3-yl]azetidine-1-carboxylate (200 mg), 10% Pd-C (200 mg), hydrogen chloride (2 M methanol solution, 0.04 mL) and methanol (3.9 mL) was stirred at room temperature for 4 hours under a hydrogen atmosphere. The insoluble material was then filtered off by Celite^{®} filtration, the filtrate was concentrated under reduced pressure, and then the residue was purified by silica gel column chromatography to afford the title compound (270 mg). MS (m/z): 379.2 [M+H]⁺

### [Step 2] Preparation of Compound RE-58

A mixture of Intermediate RE-58A (30 mg), bromobenzene (15 mg), Pd(OAc)₂ (2 mg), Xantphos (9 mg), cesium carbonate (52 mg) and 1,4-dioxane (0.3 mL) was stirred at 90°C for 3 hours. The reaction solution was purified by silica gel column chromatography to afford the title compound (6 mg). MS (m/z): 455.3 [M+H]⁺

### Reference Example 59 tert-butyl 3-(4,6-difluoro-1-{[1-(2,2,2-trifluoroethyl)azetidin-3-yl]methyl]-1H-indazol-3-yl)azetidine-1-carboxylate (Compound RE-59)

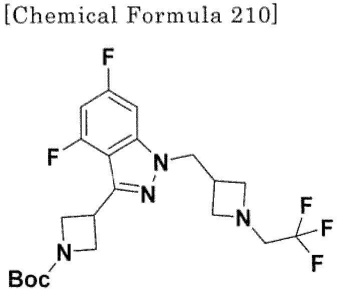

To a mixture of Intermediate RE-58A (30 mg), DIPEA (0.04 mL) and THF (0.3 mL) was added (2,2,2-trifluoroethyl)trifluoromethanesulfonate (37 mg), and then the mixture was stirred at room temperature for 3 hours. The reaction solution was purified by silica gel column chromatography to afford the title compound (10 mg). MS (m/z): 461.2 [M+H]⁺

### Reference Example 60 tert-butyl 3-{1-[(2,3-dihydro-1H-inden-2-yl)methyl]-4,6-difluoro-1H-indazol-3-yl}azetidine-1-carboxylate (Compound RE-60)

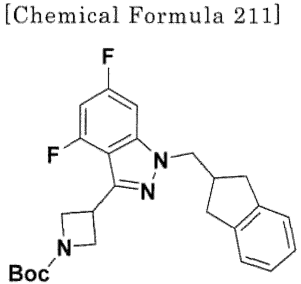

In the method according to Reference Example 55, indan-2-ylmethyl 4-methylbenzenesulfonate was used instead of (4,4-difluorocyclohexyl)methyl 4-methylbenzenesulfonate to afford the title compound. MS (m/z): 340.1 [M-Boc+2H]⁺

### Reference Example 61 2-[3-(azetidin-3-yl)-4,6-difluoro-1H-indazol-1-yl]-1-[(2R)-2-(trifluoromethyl)morpholin-4-yl]ethan-1-one trifluoroacetate (Compound RE-61)

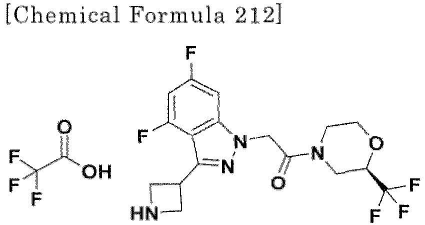

### [Step 1] Preparation of Intermediate RE-61A

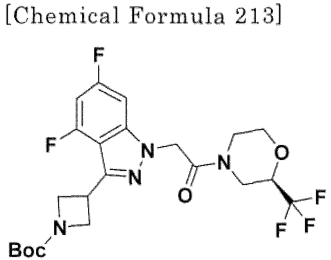

To a mixture of Intermediate RE-2E (100 mg), DMF (0.9 mL), DIPEA (0.14 mL) and (2R)-2-(trifluoromethyl)morpholine hydrochloride (63 mg) was added HBTU (124 mg), and the mixture was stirred at room temperature for 2 hours. The reaction solution was purified by silica gel column chromatography to afford the title compound (131 mg). MS (m/z): 405.1 [M-Boc+2H]⁺

### [Step 2] Preparation of Compound RE-61

In the method according to Step 4 of Reference Example 37, Intermediate RE-61A was used instead of Intermediate RE-37C to afford the title compound. MS (m/z): 405.1 [M+H]⁺

### Reference Example 62 2-[3-(azetidin-3-yl)-4,6-difluoro-1H-indazol-1-yl]-N-methyl-N-(2,2,2-trifluoroethyl)acetamide trifluoroacetate (Compound RE-62)

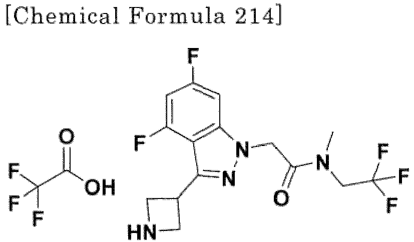

Compound RE-62 was prepared starting from Intermediate RE-2E, according to procedures similar to those described in Steps 1 to 2 of Reference Example 51 for the preparation of Compound RE-51. MS(m/z): 363.1 [M+H]⁺

### Reference Example 63 2-[3-(azetidin-3-yl)-1H-indazol-1-yl]-N-tert-butylacetamide trifluoroacetate (Compound RE-63)

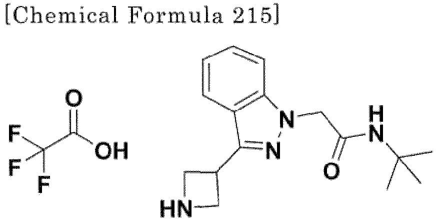

Compound RE-63 was prepared starting from Intermediate RE-4C, according to procedures similar to those described in Steps 1 to 2 of Reference Example 61, for the preparation of Compound RE-61, using 2-methylpropan-2-amine instead of (2R)-2-(trifluoromethyl)morpholine hydrochloride. MS (m/z): 287.2 [M+H]⁺

### Reference Example 64 2-(3-(azetidin-3-yl)-1H-indazol-1-yl]-N,N-bis(propan-2-yl)acetamide trifluoroacetate (Compound RE-64)

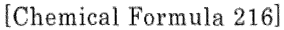

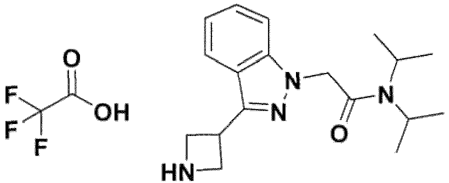

Compound RE-64 was prepared starting from Intermediate RE-4C, according to procedures similar to those described in Steps 1 to 2 of Reference Example 51 for the preparation of Compound RE-51, using diisopropylamine instead of 2,2,2-trifluoro-N-methylethanamine hydrochloride. MS (m/z): 315.3 [M+H]⁺

### Reference Example 65 2-[3-(azetidin-3-yl)-1H-indazol-1-yl]-N-tert-butyl-N-methylacetamide trifluoroacetate (Compound RE-65)

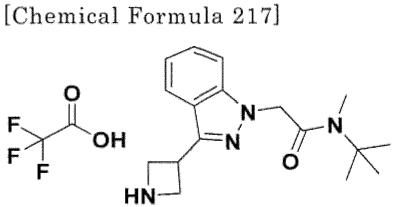

Compound RE-65 was prepared starting from Intermediate RE-4C, according to procedures similar to those described in Steps 1 to 2 of Reference Example 51 for the preparation of Compound RE-51, using N,2-dimethylpropan-2-amine instead of 2,2,2-trifluoro-N-methylethanamine hydrochloride. MS(m/z): 301.2 [M+H]⁺

### Reference Example 66 2-[3-(azetidin-3-yl)-1H-indazol-1-yl]-N-methyl-N-(2,2,2-trifluoroethyl)acetamide trifluoroacetate (Compound RE-66)

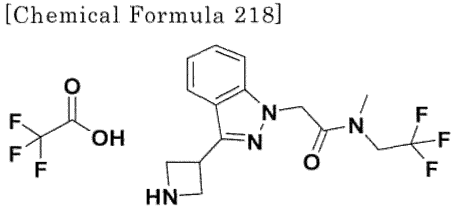

### [Step 1] Preparation of Intermediate RE-66A

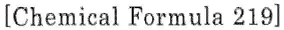

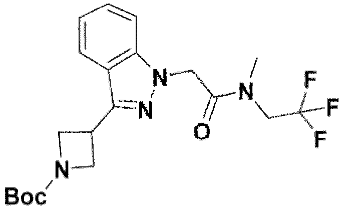

To a mixture of Intermediate RE-4C (350 mg), 2,2,2-trifluoro-N-methylethanamine hydrochloride (190 mg), 1-hydroxybenzotriazole monohydrate (194 mg), NMM (0.35 mL) and acetonitrile (3 mL) was added EDCI·HCl (243 mg), and then the mixture was stirred at room temperature overnight. A saturated aqueous solution of sodium bicarbonate was added to the reaction solution, and the mixture was extracted with ethyl acetate. The organic layer was washed with a saturated saline solution and dried with anhydrous magnesium sulfate. The solvent was removed under reduced pressure, and then the residue was purified by silica gel column chromatography to afford the title compound (280 mg). MS (m/z): 449.3 [M+Na]⁺

### [Step 2] Preparation of Compound RE-66

In the method according to Step 4 of Reference Example 37, Intermediate RE-66A was used instead of Intermediate RE-37C to afford the title compound. MS (m/z): 327.2 [M+H]⁺

### Reference Example 67 tert-butyl 3-(5-chloro-1-{2-oxo-2-[(2R)-2-(trifluoromethyl)morpholin-4-yl}ethyl}-1H-indazol-3-yl)azetidine-1-carboxylate (Compound RE-67)

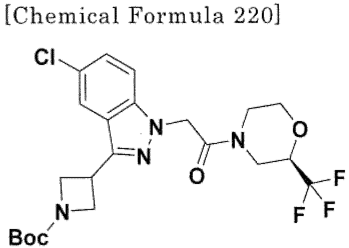

To a mixture of Intermediate RE-5D (115 mg), DMF (1 mL), DIPEA (0.16 mL) and (2R)-2-(trifluoromethyl)morpholine hydrochloride (72 mg) was added HBTU (143 mg) at room temperature, and then the mixture was stirred for 2 hours at room temperature. The reaction solution was purified by silica gel column chromatography to afford the title compound (86 mg). MS (m/z): 503.2 [M+H]⁺

### Reference Example 68 2-[3-(azetidin-3-yl)-5-chloro-1H-indazol-1-yl]-1-[(2S)-2-(trifluoromethyl)pyrrolidin-1-yl]ethan-1-one trifluoroacetate (Compound RE-68)

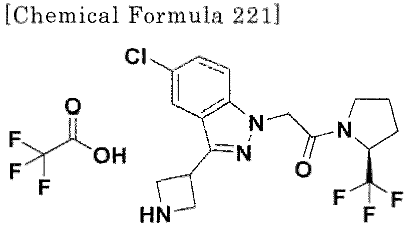

To a mixture of Intermediate RE-5D (115 mg), DMF (0.6 mL), DIPEA (0.16 mL) and (2S)-2-(trifluoromethyl)pyrrolidine hydrochloride (66 mg) was added HATU (143 mg), and then the mixture was stirred at room temperature overnight. The reaction solution was purified by silica gel column chromatography to afford the compound (116 mg). To a mixture of the obtained compound (116 mg) and dichloromethane (2 mL) was added trifluoroacetic acid (0.5 mL), and then the mixture was stirred at room temperature. The solvent was removed under reduced pressure to afford the title compound (119 mg). MS (m/z): 387.1 [M+H]⁺

### Reference Example 69 2-[3-(azetidin-3-yl)-6-fluoro-1H-indazol-1-yl]-1-[(2S)-2-(trifluoromethyl)pyrrolidin-1-yl]ethan-1-one trifluoroacetate (Compound RE-69)

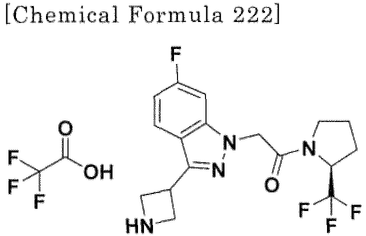

Compound RE-69 was prepared starting from Intermediate RE-6C, according to procedures similar to those described in Steps 1 to 2 of Reference Example 66 for the preparation of Compound RE-66, using (2S)-2-(trifluoromethyl)pyrrolidine hydrochloride instead of 2,2,2-trifluoro-N-methylethaneamine hydrochloride. MS (m/z): 371.3 [M+H]⁺

### Reference Example 70 tert-butyl 3-methyl-3-(1-{[methyl(2,2,2-trifluoroethyl)carbamoyl]methyl}-1H-indazol-3-yl)azetidine-1-carboxylate (Compound RE-70)

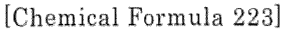

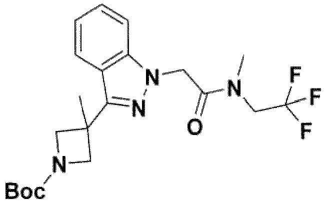

To a mixture of Intermediate RE-7C (100 mg), DMF (1.0 mL), HBTU (132 mg) and 2,2,2-trifluoro-N-methylethanamine hydrochloride (52 mg) was added DIPEA (0.15 mL), and then the mixture was stirred at room temperature overnight. The reaction solution was purified by silica gel column chromatography to afford the title compound (109 mg). MS (m/z): 463.4 [M+Na]⁺

### Reference Example 71 2-[4-fluoro-3-(piperidin-4-yl)-1H-indazol-1-yl]-1-[(2S)-2-(trifluoromethyl)pyrrolidin-1-yl]ethan-1-one (Compound RE-71)

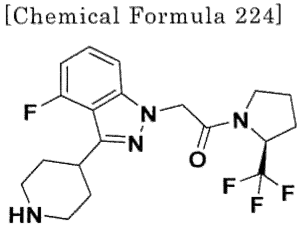

### [Step 1] Preparation of Intermediate RE-71A

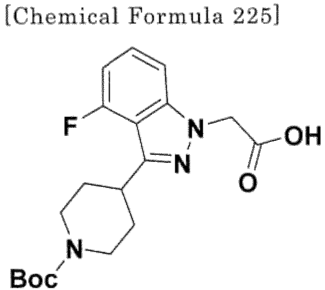

Compound RE-71A was prepared starting from Intermediate RE-10C, according to procedures similar to those described in Steps 4 to 5 of Reference Example 2 for the preparation of Intermediate RE-2E. MS (m/z): 376.4 [M-H]⁻

### [Step 2] Preparation of Compound RE-71

A mixture of Intermediate RE-71A (800 mg), HATU (967 mg), DIPEA (1.1 mL) and DMF (4.2 mL) was stirred at room temperature for 15 minutes. (2S)-2-(Trifluoromethyl)pyrrolidine hydrochloride (447 mg) was added thereto, and the mixture was stirred at room temperature overnight. A saturated aqueous solution of sodium bicarbonate was added to the reaction solution, and the mixture was extracted with ethyl acetate. The organic layer was dried over anhydrous sodium sulfate. The solvent was removed under reduced pressure, and to the residue were added ethyl acetate (5 mL) and hydrogen chloride (4 M ethyl acetate solution, 0.4 mL), and then the mixture was stirred at room temperature for 2 hours. The solvent was removed under reduced pressure, and then the residue was purified by silica gel column chromatography to afford the title compound (280 mg). MS (m/z): 399.2 [M+H]⁺

### Reference Example 72 tert-butyl 4-(4-fluoro-1-{2-oxo-2-[(2S)-2-(trifluoromethyl)morpholin-4-yl]ethyl}-1H-indazol-3-yl)piperidine-1-carboxylate (Compound RE-72)

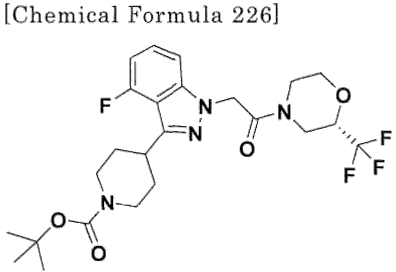

A mixture of Intermediate RE-10C (80 mg), HATU (97 mg), DIPEA (0.1 mL) and DMF (0.4 mL) was stirred at room temperature for 15 minutes. (2S)-2-(Trifluoromethyl)morpholine hydrochloride (49 mg) was added thereto, and the mixture was stirred at room temperature for 2 hours 30 minutes. The reaction solution was purified by silica gel column chromatography to afford the title compound (70 mg). MS (m/z): 415.2 [M-Boc+2H]⁺

### Reference Example 73 tert-butyl 4-(4-fluoro-1-{2-oxo-2-[(2R)-2-(trifluoromethyl)morpholin-4-yl}ethyl)-1H-indazol-3-yl)piperidine-1-carboxylate (Compound RE-73)

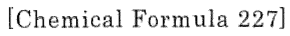

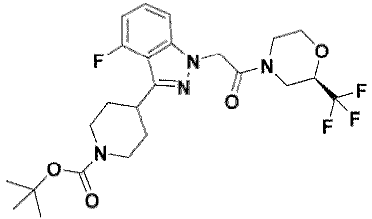

In the method according to Reference Example 72, (2R)-2-(trifluoromethyl)morpholine hydrochloride was used instead of (2S)-2-(trifluoromethyl)morpholine hydrochloride to afford the title compound. MS (m/z): 537.1 [M+Na]⁺

### Reference Example 74 tert-butyl 4-(4-fluoro-1-{[6-(trifluoromethyl)pyridin-3-yl]methyl}-1H-indazol-3-yl)piperidine-1-carboxylate (Compound RE-74)

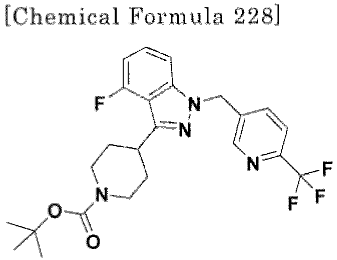

In the method according to Step 4 of Reference Example 10, 5-(chloromethyl)-2 -(trifluoromethyl)pyridine was used instead of 2-(bromomethyl)-5-(trifluoromethyl)pyridine to afford the title compound. MS (m/z): 501.2 [M+Na]⁺

### Reference Example 75 tert-butyl 4-(4-fluoro-1-{2-oxo-2-[(2S)-2-(trifluoromethyl)pyrrolidin-1-yl]ethyl]-1H-indol-3-yl)-1,2,3,6-tetrahydropyridine-1-carboxylate (Compound RE-75)

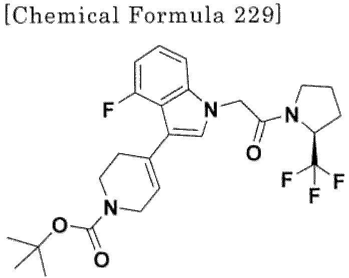

Compound RE-75 was prepared starting from Intermediate RE-11A, according to procedures similar to those described in Steps 3 through 5 of Reference Example 11 for the preparation of Intermediate RE-11E. MS (m/z): 396.2 [M-Boc+2H]⁺

### Reference Example 76 tert-butyl 4-(4-fluoro-1-{2-oxo-2-[(2R)-2-(trifluoromethyl)morpholin-4-yl]ethyl}-1H-indol-3-yl)piperidine-1-carboxylate (Compound RE-76)

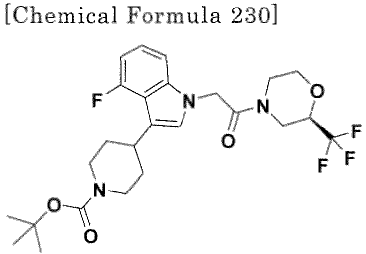

Compound RE-76 was prepared starting from Intermediate RE-11B, according to procedures similar to those described in Steps 3 through 5 of Reference Example 11 for the preparation of Intermediate RE-11E, using (2R)-2-(trifluoromethyl)morpholine hydrochloride instead of (2S)-2-(trifluoromethyl)pyrrolidine hydrochloride. MS (m/z): 536.2 [M+Na]⁺

### Reference Example 77 tert-butyl 4-(4-fluoro-1-{2-oxo-2-[(2S)-2-(trifluoromethyl)morpholin-4-yl]ethyl}-1H-indol-3-yl)piperidine-1-carboxylate (Compound RE-77)

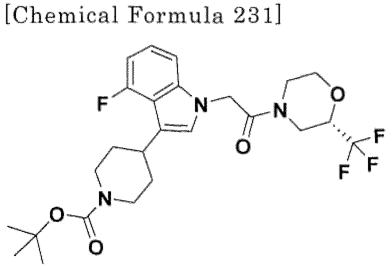

Compound RE-77 was prepared starting from Intermediate RE-11B, according to procedures similar to those described in Steps 3 through 5 of Reference Example 11 for the preparation of Intermediate RE-11E, using (2S)-2-(trifluoromethyl)morpholine hydrochloride instead of (2S)-2-(trifluoromethyl)pyrrolidine hydrochloride. MS (m/z): 536.2 [M+Na]⁺

### Reference Example 78 3-[3-(azetidin-3-yl)-5-methyl-1H-pyrazolo[4,3-b]pyridin-1-yl]-1-[(2R)-2-(trifluoromethyl)morpholin-4-yl]ethan-1-one dihydrochloride (Compound RE-78)

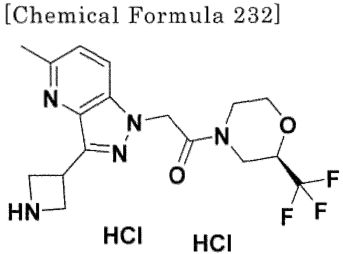

### [Step 1] Preparation of Intermediate RE-78A

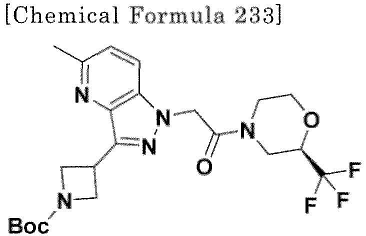

In the method according to Reference Example 67, Intermediate RE-13D was used instead of Intermediate RE-5D to afford the title compound. MS (m/z): 484.2 [M+H]⁺

### [Step 2] Preparation of Compound RE-78

In the method according to Step 7 of Reference Example 1, Intermediate RE-78A was used instead of Intermediate RE-1F to afford the title compound. MS (m/z): 384.2 [M+H]⁺

### Reference Example 79 2-[3-(azetidin-3-yl)-5-methyl-1H-pyrazolo[4,3-b]pyridin-1-yl]-1-(3,3-dimethylmorpholin-4-yl)ethan-1-one dihydrochloride (Compound RE-79)

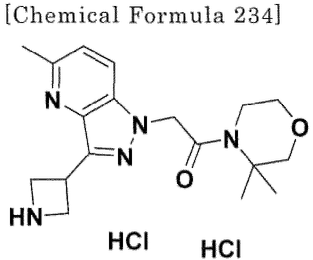

### [Step 1] Preparation of Intermediate RE-79A

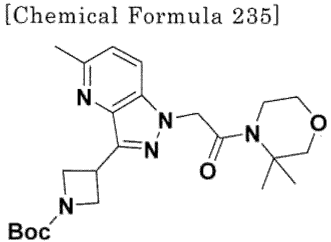

To a mixture of Intermediate RE-13D (52 mg), DMF (1.0 mL), HBTU (74 mg), and 3,3-dimethylmorpholine (22 mg) was added DIPEA (0.08 mL) and then the mixture was stirred at room temperature overnight. The reaction solution was purified by silica gel column chromatography to afford the title compound (50 mg). MS (m/z): 444.3 [M+H]⁺

### [Step 2] Preparation of Compound RE-79

In the method according to Step 7 of Reference Example 1, Intermediate RE-79A was used instead of Intermediate RE-1F to afford the title compound. MS (m/z): 344.2 [M+H]⁺.

### Reference Example 80 tert-butyl 3-(4-fluoro-1-{2-oxo-2-[(2R)-2-(trifluoromethyl)morpholin-4-yl]ethyl}-1H-pyrazolo[3,4-c]pyridin-3-yl)azetidine-1-carboxylate (Compound RE-80)

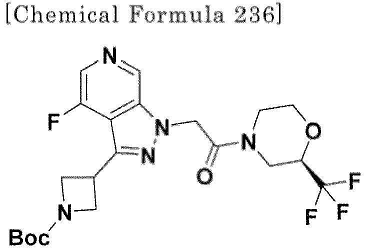

### [Step 1] Preparation of Intermediate RE-80A

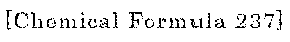

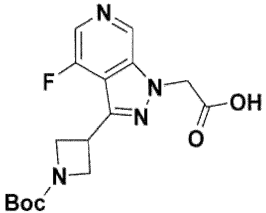

Intermediate RE-80A was prepared starting from Intermediate RE-15C, according to procedures similar to those described in Steps 4 through 5 of Reference Example 1 for the preparation of Intermediate RE-1E. MS(m/z): 351.0 [M+H]⁺

### [Step 2] Preparation of Compound RE-80

In the method according to Step 6 of Reference Example 1, Intermediate RE-80A and (2R)-2-(trifluoromethyl) morpholine hydrochloride were used instead of Intermediate RE-1E and 2,2,2-trifluoroethaneamine hydrochloride, respectively, to afford the title compound. MS (m/z): 488.2 [M+H]⁺

### Reference Example 81 tert-butyl 3-(4-fluoro-1-{2-oxo-2-[(2S)-2-(trifluoromethyl)pyrrolidin-1-yl]ethyl}-1H-pyrazolo[3,4-c]pyridin-3-yl)azetidine-1-carboxylate (Compound RE-81)

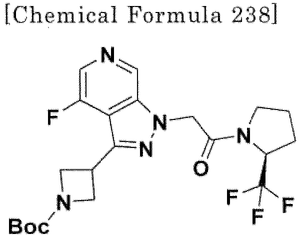

In the method according to Step 6 of Reference Example 1, Intermediate RE-80A and (2S)-2-(trifluoromethyl)pyrrolidine hydrochloride were used instead of Intermediate RE-1E and 2,2,2-trifluoroethaneamine hydrochloride, respectively, to afford the title compound. MS (m/z): 472.2 [M+H]⁺

### Reference Example 82 tert-butyl 3-(4-fluoro-1-{[5-(trifluoromethyl)pyridin-2-yl]methyl}-1H-pyrazolo[3,4-c]pyridin-3-yl)azetidine-1-carboxylate (Compound RE-82)

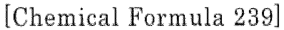

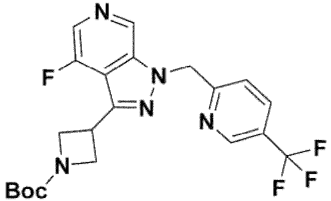

In the method according to Step 4 of Reference Example 10, Intermediate RE-15C was used instead of Intermediate RE-10C to afford the title compound. MS (m/z): 452.2 [M+H]⁺

### Reference Example 83 tert-butyl 3-(4-fluoro-1-{[4-(trifluoromethyl)phenyl]methyl]-1H-pyrazolo[3,4-c]pyridin-3-yl)azetidine-1-carboxylate (Compound RE-83)

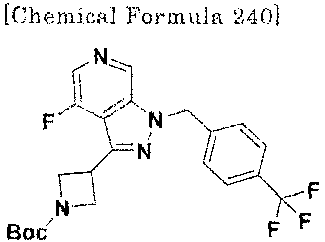

In the method according to Step 4 of Reference Example 10, Intermediate RE-15C and 1-(bromomethyl)-4-(trifluoromethyl)benzene were used instead of Intermediate RE-10C and 2-(bromomethyl)-5-(trifluoromethyl)pyridine, respectively, to afford the title compound. MS (m/z): 451.2 [M+H]⁺

### Reference Example 84 tert-butyl 3-(4-fluoro-1-{[5-(2-fluorophenyl)-1,2,4-oxadiazol-3-yl]methyl}-1H-pyrazolo[3,4-c]pyridin-yl)azetidine-1-carboxylate (Compound RE-84)

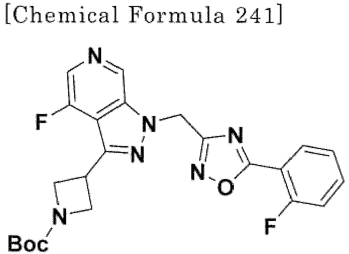

In the method according to Step 6 of Reference Example 15, 2-fluorobenzoic acid was used instead of 1-(trifluoromethyl)cyclopropane-1-carboxylic acid to afford the title compound. MS (m/z ): 469.2 [M+H]⁺

### Reference Example 85 tert-butyl 3-(4-fluoro-1-{[3-(3-fluoropyridin-4-yl)-1,2,4-oxadiazol-5-yl]methyl}-1H-pyrazolo[3,4-pyridin-3-yl)azetidine-1-carboxylate (Compound RE-85)

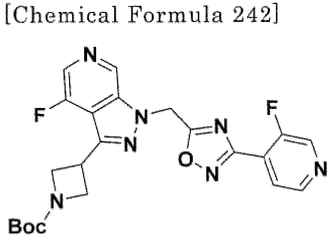

To a mixture of Intermediate RE-80A (40 mg), 3-fluoro-N'-hydroxy-pyridine-4-carboxamide (for example, synthesized according to a method described in European Journal of Medicinal Chemistry, 2018, vol. 157, p. 1376 - 1394) (19 mg), DIPEA (0.02 m L) and DMF (0.4 mL) was added HATU (48 mg), and the mixture was stirred at room temperature overnight. The mixture was then stirred at 120°C for 2 hours. After leaving the mixture to cool, the reaction solution was purified by silica gel column chromatography to afford the title compound (15 mg). MS (m/z): 470.2 [M+H]⁺

### Reference Example 86 2-[3-(azetidin-3-yl)-4-fluoro-1H-pyrazolo[3,4-c]pyridin-1-yl-1-[(2R)-2-(trifluoromethyl)morpholin-4-yl]ethan-1-one dihydrochloride (Compound RE-86)

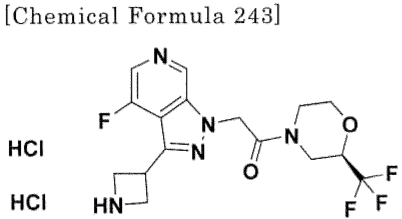

In the method according to Step 7 of Reference Example 1, Compound RE-80 was used instead of Intermediate RE-1F to afford the title compound. MS (m/z): 388.1 [M+H]⁺

### Reference Example 87 2-[3-(azetidin-3-yl)-4-fluoro-1H-pyrazolo[3,4-c]pyridin-1-yl-1-[(2S)-2-[(trifluoromethoxy)methyl]pyrrolidin-1-yl]ethan-1-one dihydrochloride (Compound RE-87)

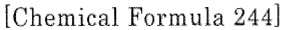

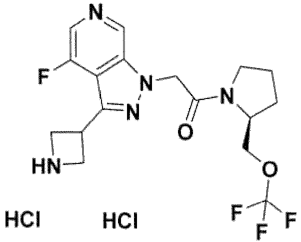

### [Step 1] Preparation of Intermediate RE-87A

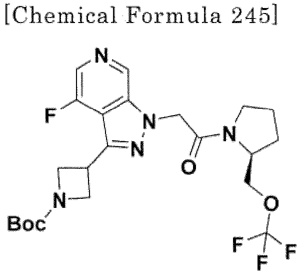

To a mixture of Intermediate RE-80A (80 mg), DMF (0.8 mL), HBTU (130 mg) and (2S)-2-(trifluoromethoxymethyl)pyrrolidine hydrochloride (56 mg) was added DIPEA (0.2 mL), and the mixture was stirred at room temperature overnight. The reaction solution was purified by silica gel column chromatography to afford the title compound (60 mg). MS (m/z): 502.3 [M+H]⁺

### [Step 2] Preparation of Compound RE-87

In the method according to Step 7 of Reference Example 1, Intermediate RE-87A was used instead of Intermediate RE-1F to afford the title compound. MS (m/z): 402.2 [M+H]⁺

### Reference Example 88 3-(azetidin-3-yl)-1-{[5-(trifluoromethyl)-1,2,4-oxadiazol-3-yl]methyl}-1H-indazole (Compound RE-88)

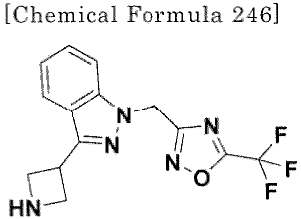

### [Step 1] Preparation of Intermediate RE-88A

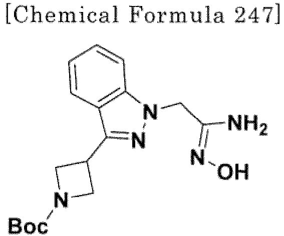

Compound RE-88A was prepared starting from Intermediate RE-17B, according to procedures similar to those described in Steps 4 to 5 of Reference Example 15 for the preparation of Intermediate RE-15E. MS(m/z): 346.2 [M+H]⁺

### [Step 2] Preparation of Compound RE-88

A mixture of Intermediate RE-88A (99 mg), toluene (2 mL) and trifluoroacetic anhydride (66 mg) was stirred at 60°C overnight. The reaction solution was purified by silica gel column chromatography to afford the title compound (49 mg). MS (m/z): 324.0 [M+H]⁺

### Reference Example 89 tert-butyl 3-{4-fluoro-1-[(1-phenyl-1H-1,2,3-triazol-4-yl)methyl]-1H-indazol-3-yl)azetidine-1-carboxylate (Compound RE-89)

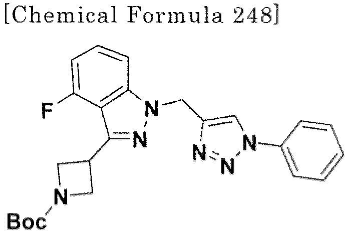

To a mixture of Intermediate RE-18D (74 mg), azidobenzene (54 mg), DMF (2 mL) and methanol (0.2 mL) was added copper(I) iodide (4 mg), and then the mixture was stirred at 60°C for 3 hours under argon atmosphere. The reaction solution was diluted with ethyl acetate and washed with a saturated saline solution. The solvent was removed under reduced pressure, and then the residue was purified by silica gel column chromatography to afford the title compound (62 mg). ¹H-NMR (400 MHz, CDCl₃) δ 7.88 (s, 1H), 7.68 (d, 2H), 7.51-7.46 (m, 2H), 7.45-7.29 (m, 3H), 6.77 (dd, 1H), 5.73 (s, 2H), 4.40-4.30 (m, 4H), 4.29-4.22 (m, 1H), 1.46 (s, 9H)

### Reference Example 90 tert-butyl 3-{1-[(1-benzyl-1H-1,2,3-triazol-4-yl)methyl]-4-fluoro-1H-indazol-3-yl]azetidine-1-carboxylate (Compound RE-90)

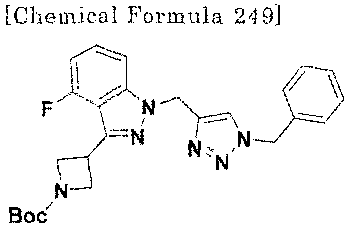

In the method according to Reference Example 89, azidomethylbenzene was used instead of azidobenzene to afford the title compound. ¹H-NMR (400 MHz, CDCl₃) δ 7.39-7.22 (m, 8H), 6.75 (t, 1H), 5.61 (s, 2H), 5.47 (s, 2H), 4.38-4.18 (m, 4H), 4.17-4.10 (m, 1H), 1.47 (s, 9H)

### Reference Example 91 tert-butyl 3-(4-fluoro-1-{[1-(2,2,2-trifluoroethyl)-1H-1,2,3-triazol-4-yl]methyl}-1H-indazol-3-yl)azetidine-1-carboxylate (Compound RE-91)

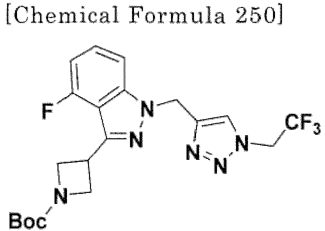

In the method according to Reference Example 89, 2-azido-1,1,1-trifluoroethane was used instead of azidobenzene to afford the title compound. MS(m/z): 355.0 [M-Boc+2H]⁺

### Reference Example 92 tert-butyl 3-(1-{[1-(cyclobutylmethyl)-1H-1,2,3-triazol-4-yl]methyl}-4-fluoro-1H-indazol-3-yl)azetidine-1-carboxylate (Compound RE-92)

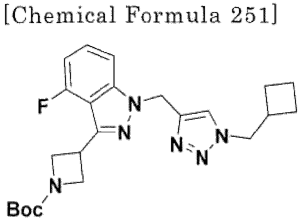

In the method according to Reference Example 89, azidomethylcyclobutane was used instead of azidobenzene to afford the title compound. MS (m/z): 341.1 [M-Boc+2H]⁺

### Reference Example 93 tert-butyl 3-{4-fluoro-1-[(3-methyl-1,2,4-oxadiazol-5-yl)methyl]-1H-indazol-3-yl]azetidine-1-carboxylate (Compound RE-93)

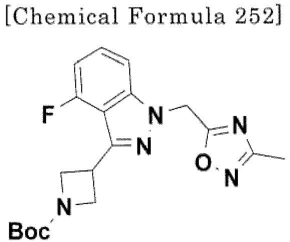

To a mixture of Intermediate RE-18C (50 mg) and THF (0.6 mL) was added 60% sodium hydride (5 mg) under ice cooling, and then the mixture was stirred for 5 minutes. Then, 5-(chloromethyl)-3-methyl-1,2,4-oxadiazole (30 mg) was added thereto, and the mixture was stirred for 2 hours. A saturated aqueous solution of sodium bicarbonate was added to the reaction solution, and the mixture was extracted with ethyl acetate. The organic layer was dried over anhydrous magnesium sulfate. The solvent was removed under reduced pressure, and then the residue was purified by silica gel column chromatography to afford the title compound (41 mg). MS (m/z): 288.0 [M-Boc+2H]⁺

### Reference Example 94 tert-butyl 3-(4-fluoro-1-{[4-(trifluoromethyl)phenyl]methyl}-1H-indazol-8-yl)azetidine-1-carboxylate (Compound RE-94)

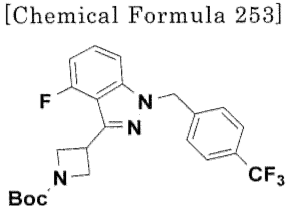

In the method according to Step 4 of Reference Example 10, Intermediate RE-18C and 1-(bromomethyl)-4-(trifluoromethyl)benzene were used instead of Intermediate RE-10C and 2-(bromomethyl)-5-(trifluoromethyl)pyridine, respectively, to afford the title compound. MS (m/z): 350.1 [M-Boc+2H]⁺

### Reference Example 95 tert-butyl 3-(4-fluoro-1-{[3-(trifluoromethyl)phenyl]methyl}-1H-indazol-3-yl)azetidine-1-carboxylate (Compound RE-95)

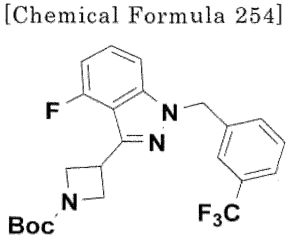

In the method according to Step 4 of Reference Example 10, Intermediate RE-18C and 1-(bromomethyl)-3-(trifluoromethyl)benzene were used instead of Intermediate RE-10C and 2-(bromomethyl)-5-(trifluoromethyl)pyridine, respectively, to afford the title compound. MS (m/z): 350.1 [M-Boc+2H]⁺

### Reference Example 96 tert-butyl 3-(4-fluoro-1-{[2-(trifluoromethyl)phenyl]methyl}-1H-indazol-3-yl)azetidine-1-carboxylate (Compound RE-96)

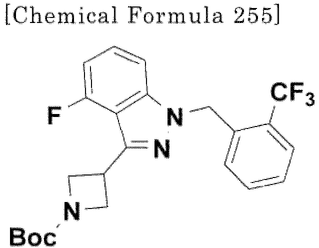

In the method according to Step 4 of Reference Example 10, Intermediate RE-18C and 1-(bromomethyl)-2-(trifluoromethyl)benzene were used instead of Intermediate RE-10C and 2-(bromomethyl)-5-(trifluoromethyl)pyridine, respectively, to afford the title compound. MS (m/z): 350.1 [M-Boc+2H]⁺

### Reference Example 97 tert-butyl 3-(4-fluoro-1-{[3-(trifluoromethyl)-1,2,4-oxadiazol-5-yl]methyl}-1H-indazol-3-yl)azetidine-1-carboxylate (Compound RE-97)

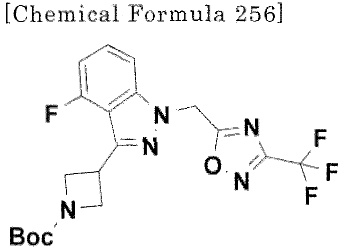

In the method according to Step 4 of Reference Example 10, Intermediate RE-18C and 5-(chloromethyl)-3-trifluoromethyl-1,2,4-oxadiazole were used instead of Intermediate RE-10C and 2-(bromomethyl)-5-(trifluoromethyl)pyridine to afford the title compound. MS (m/z): 342.1 [m-boc+2H]⁺

### Reference Example 98 tert-butyl 3-(4-fluoro-1-{[5-(4-fluorophenyl)-1,2,4-oxadiazol-3-yl]methyl}-1H-indazol-3-yl)azetidine-1-carboxylate (Compound RE-98)

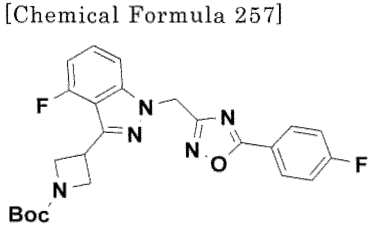

### [Step 1] Preparation of Intermediate RE-98A

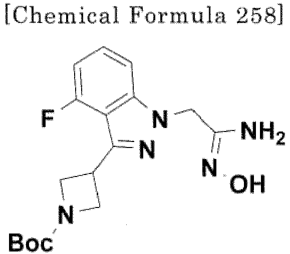

Intermediate RE-98A was prepared starting from Intermediate RE-18C, according to procedures similar to those described in Steps 4 to 5 of Reference Example 15 for the preparation of Intermediate RE-15E. MS (m/z): 364.2 [M+H]+

### [Step 2] Preparation of Compound RE-98

A mixture of Intermediate RE-98A (51 mg), 4-fluorobenzoic acid (18 mg), DIPEA (0.02 mL), HATU (54 mg) and DMF (0.4 mL) was stirred at room temperature overnight. The mixture was then stirred at 120°C for 2 hours. After cooling, the reaction solution was purified by silica gel column chromatography to afford the title compound (13 mg). MS (m/z): 368.1 [M-Boc+2H]⁺

### Reference Example 99 tert-butyl 3-[4-fluoro-1-({5-[3-(trifiuoromethyl)phenyl]-1,2,4-oxadiazol-3-yl}methyl)-1H-indazol-3-yl]azetidine-1-carboxylate (Compound RE-99)

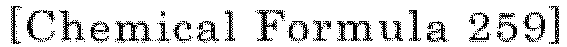

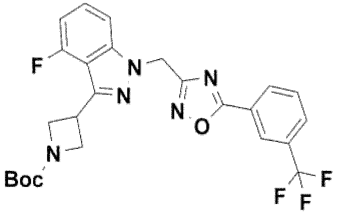

In the method according to Reference Example 98, 3-trifluoromethylbenzoic acid was used instead of 4-fluorobenzoic acid to afford the title compound. MS (m/z): 418.1 [M-Boc+2H]⁺

### Reference Example 100 tert-butyl 3-(4-fluoro-1-{[5-(trifluoromethyl)-1,3,4-oxadiazol-2-yl]methyl]-1H-indazol-3-yl)azetidine-1-carboxylate (Compound RE-100)

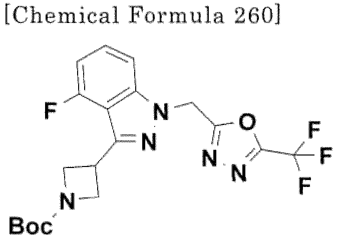

In the method according to Step 4 of Reference Example 10, Intermediate RE-18C and 2-(chloromethyl)-5-trifluoromethyl-1,3,4-oxadiazole were used instead of Intermediate RE-10C and 2-(bromomethyl)-5-(trifluoromethyl)pyridine, respectively, to afford the title compound. MS (m/z): 440.2 [M-H]⁻

### Reference Example 101 tert-butyl 3-(4-fluoro-1-{[5-(oxan-4-yl)-1,2,4-oxadiazol-3-yl]methyl}-1H-indazol-3-yl)azetidine-1-carboxylate (Compound RE-101)

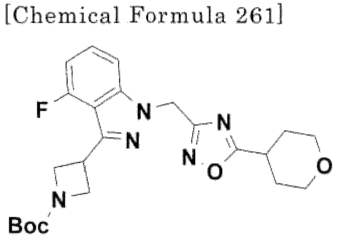

In the method according to Reference Example 98, tetrahydropyrane-4-carboxylic acid was used instead of 4-fluorobenzoic acid to afford the title compound. MS (m/z): 358.2 [M-Boc+2H]⁺

### Reference Example 102 tert-butyl 3-[4-fluoro-1-({5-[1-(trifluoromethyl)cyclobutyl]-1,2,4-oxadiazol-3-yl}methyl)-1H-indazol-3-yl]azetidine-1-carboxylate (Compound RE-103) (Compound RE-102)

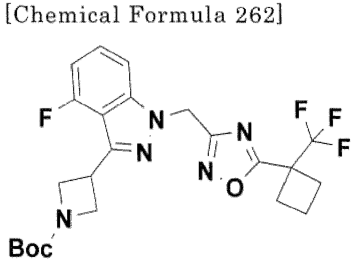

In the method according to Reference Example 98, 1-(trifluoromethyl)cyclobutanecarboxylic acid was used instead of 4-fluorobenzoic acid to afford the title compound. MS (m/z): 518.2 [M+Na]⁺

### Reference Example 103 tert-butyl 3-[4-fluoro-1-({5-[1-(trifluoromethyl)cyclopropyl]-1,2,4-oxadiazol-3-yl)methyl)-1H-indazol-3-yl]azetidine-1-carboxylate (Compound RE-103)

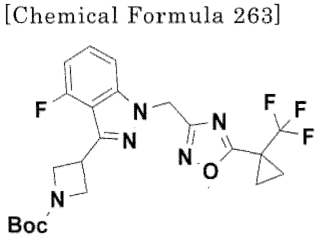

In the method according to Reference Example 98, 1-(trifluoromethyl)cyclopropanecarboxylic acid was used instead of 4-fluorobenzoic acid to afford the title compound. MS (m/z): 504.2 [M+Na]⁺

### Reference Example 104 tert-butyl 3-(4-fluoro-1-{[5-(2-fluorophenyl)-1,2,4-oxadiazol-3-yl]methyl}-1H-indazol-3-yl)azetidine-1-carboxylate (Compound RE-104)

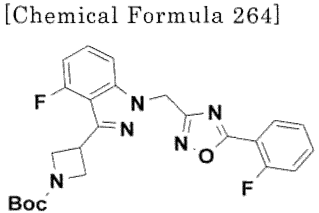

In the method according to Reference Example 98, 2-fluorobenzoic acid was used instead of 4-fluorobenzoic acid to afford the title compound. MS (m/z): 368.1 [M-Boc+2H]⁺

### Reference Example 105 tert-butyl 3-(4-fluoro-1-{[5-(5-fluoropyridin-2-yl)-1,2,4-oxadiazol-3-yl]methyl}-1H-indazol-3-yl)azetidine-1-carboxylate (Compound RE-105)

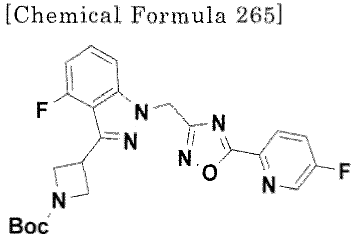

In the method according to Reference Example 98, 5-fluoropyridine-2-carboxylic acid was used instead of 4-fluorobenzoic acid to afford the title compound. MS (m/z): 369.1 [M-Boc+2H]⁺

### Reference Example 106 tert-butyl 3-(4-fluoro-1-{[5-(6-methylpyridazin-3-yl)-1,2,4-oxadiazol-3-yl]methyl}-1H-indazol-3-yl)azetidine-1-carboxylate (Compound RE-106)

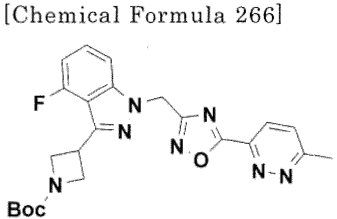

In the method according to Reference Example 98, 6-methylpyridazine-3-carboxylic acid was used instead of 4-fluorobenzoic acid to afford the title compound. MS (m/z): 366.1 [M-Boc+2H]⁺

### Reference Example 107 3-(azetidin-3-yl)-4-fluoro-1-{[5-(trifluoromethyl)-1,2,4-oxadiazol-3-yl]methyl)-1H-indazole (Compound RE-107)

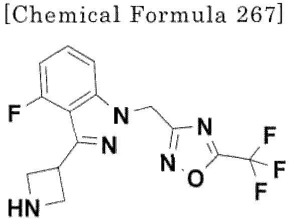

A mixture of Intermediate RE-98A (70 mg), toluene (1 mL) and trifluoroacetic anhydride (45 mg) was stirred at 60°C overnight. Trifluoroacetic anhydride (81 mg) was added thereto and then the mixture was stirred at 60°C for 2 hours. The reaction solution was purified by silica gel column chromatography to afford the title compound (13 mg). MS (m/z): 342.1 [M+H]⁺

### Reference Example 108 2-[3-(azetidin-3-yl)-4-fluoro-1H-indazol-1-yl]-N-(2,2,2-trifluoroethyl)acetamide hydrochloride (Compound RE-108)

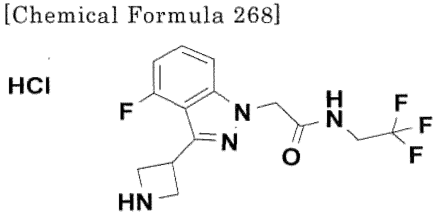

Compound RE-108 was prepared starting from Intermediate RE-20B, according to procedures similar to those described in Steps 6 to 7 of Reference Example 1 for the preparation of Compound RE-1. MS(m/z): 331.5 [M+H]⁺

### Reference Example 109 tert-butyl 3-[4-fluoro-1-({3-[3-(trifluoromethyl)phenyl]-1,2,4-oxadiazol-5-yl}methyl)-1H-indazol-3-yl]azetidine-1-carboxylate (Compound RE-109)

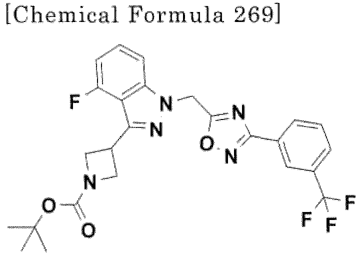

A mixture of Intermediate RE-20B (47 mg), N'-hydroxy-3-(trifluoromethyl)benzamidine (30 mg), DIPEA (0.03 mL), HATU (56 mg) and DMF (0.5 mL) was stirred at room temperature overnight. The mixture was then stirred at 120°C for 2 hours. After leaving the mixture to cool, the reaction solution was purified by silica gel column chromatography to afford the title compound (45 mg). MS (m/z): 418.1 [M-Boc+2H]⁺

### Reference Example 110 tert-butyl 3-(4-fluoro-1-{[3-(4-fluorophenyl)-1,2,4-oxadiazol-5-yl]methyl}-1H-indazol-3-yl)azetidine-1-carboxylate (Compound RE-110)

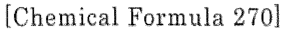

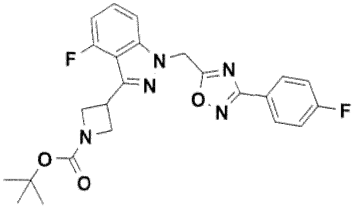

In the method according to Reference Example 109, 4-fluoro-N'-hydroxybenzamidine was used instead of N'-hydroxy-3-(trifluoromethyl)benzamidine to afford the title compound. MS (m/z): 368.1 [M-Boc+2H]⁺

### Reference Example 111 tert-butyl 3-(4-fluoro-1-{[3-pyridin-4-yl)-1,2,4-oxadiazol-5-yl]methyl}-1H-indazol-3-yl)azetidine-1-carboxylate (Compound RE-111)

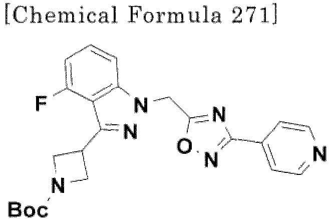

In the method according to Reference Example 109, N'-hydroxypyridine-4-carboxamidine was used instead of N'-hydroxy-3-(trifluoromethyl)benzamidine to afford the title compound. ¹H-NMR (400 MHz, CDCl₃) δ 8.76 (d, 2H), 7.90 (d, 2H), 7.42-7.36 (m, 1H), 7.25 (d, 1H), 6.85 (t, 1H), 5.84 (s, 2H), 4.40-4.30 (m, 4H), 4.29-4.21 (m, 1H), 1.46 (s, 9H)

### Reference Example 112 tert-butyl 3-{1-[({bicyclo[2.2.1]heptan-2-yl}carbamoyl)methyl]-4-fluoro-1H-indazol-3-yl}azetidine-1-carboxylate (Compound RE-112)

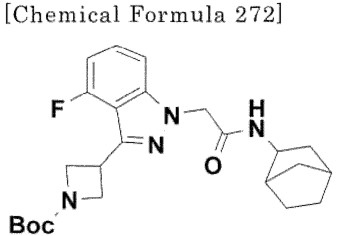

In the method according to Step 1 of Reference Example 87, Intermediate RE-20B was used instead of Intermediate RE-80A, and norbornan-2-amine hydrochloride was used instead of (2S)-2-(trifluoromethoxymethyl)pyrrolidine hydrochloride to afford the title compound. MS (m/z): 343.2 [M+H]⁺

### Reference Example 113 tert-butyl 3-{1-[({bicyclo[1.1.1]pentan-1-yl}carbamoyl)methyl]-4-fluoro-1H-indazol-3-yl}azetidine-1-carboxylate (Compound RE-113)

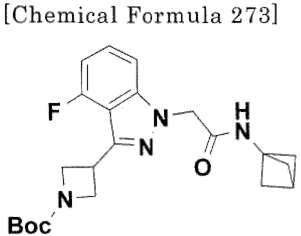

In the method according to Step 1 of Reference Example 87, Intermediate RE-20B and bicyclo[1.1.1]pentan-1-amine were used instead of Intermediate RE-80A and (2S)-2-(trifluoromethoxymethyl)pyrrolidine hydrochloride, respectively, to afford the title compound. MS(m/z):413.3[M-H]⁻

### Reference Example 114 tert-butyl 3-(1-{[(2,2-difluoroethyl)carbamoyl]methyl}-4-fluoro-1H-indazol-3-yl)azetidine-1-carboxylate (Compound RE-114)

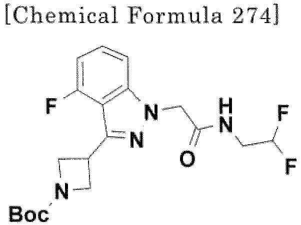

In the method according to Step 1 of Reference Example 87, Intermediate RE-20B and 2,2-difluoroethaneamine were used instead of Intermediate RE-80A and (2S)-2-(trifluoromethoxymethyl)pyrrolidine hydrochloride, respectively, to afford the title compound. MS (m/z): 313.1 [M-Boc+2H]⁺

### Reference Example 115 4-{4-[4-fluoro-1-(prop-2-yn-1-yl)-1H-indazol-3-yl]piperidine-1-carbonyl)pyridin-2-amine (Compound RE-115)

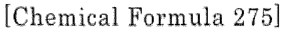

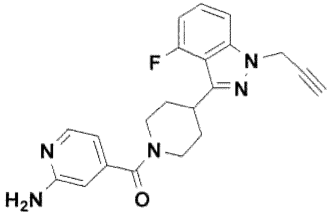

To a mixture of Intermediate RE-10C (70 mg) and DMF (0.7 mL) were added potassium carbonate (45 mg) and propagyl bromide (29 mg), and then the mixture was stirred at room temperature overnight. A saturated aqueous solution of ammonium chloride was added to the reaction solution, and the mixture was extracted with ethyl acetate. The organic layer was dried over anhydrous magnesium sulfate, and the solvent was removed under reduced pressure. To a mixture of the resulting residue (78 mg) and dichloromethane (2 mL) was added trifluoroacetic acid (0.5 mL), and then the mixture was stirred at room temperature for 3 hours. The solvent was removed under reduced pressure and the resulting residue (81 mg) was dissolved in a small amount of DMF. In a separate vessel, a mixture of 2-aminopyridine-4-carboxylic acid (24 mg), DMF (1.7 mL), DIPEA (0.2 mL) and HBTU (86 mg) was stirred at room temperature overnight, and then the separately prepared DMF solution was added thereto, and then the mixture was stirred at room temperature overnight. The reaction solution was purified by silica gel column chromatography to afford the title compound (65 mg). MS (m/z): 378.2 [M+H]⁺

### Reference Example 116 2-{3-[1-(2-aminopyridine-4-carbonyl)azetidin-3-yl]-4,6-difluoro-1H-indazol-1-yl)acetic acid (Compound RE-116)

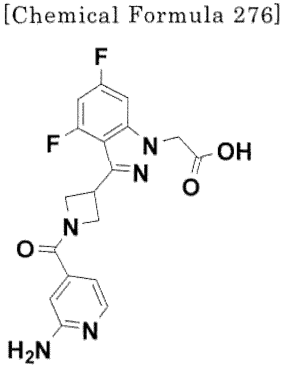

### [Step 1] Preparation of Intermediate RE-116A

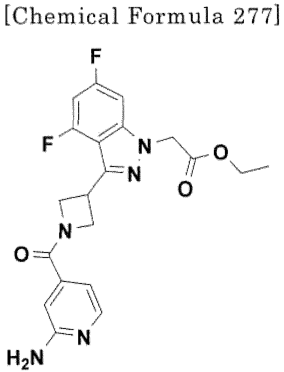

A mixture of Intermediate RE-2D (280 mg), ethanol (2 mL) and hydrogen chloride (2 M ethanol solution, 1.8 mL) was stirred at room temperature overnight. The solvent was removed under reduced pressure. Then, a mixture of the residue, ethanol (2 mL) and hydrogen chloride (2 M ethanol solution, 1.8 mL) was stirred at room temperature overnight. The solvent was removed under reduced pressure, and the resulting residue (220 mg) was dissolved in DMF (2 mL). In a separate vessel, HBTU (327 mg) was added to a mixture of 2-aminopyridine-4-carboxylic acid (119 mg), DMF (2.2 mL) and DIPEA (0.92 mL), and then the mixture was stirred at room temperature for 15 minutes. The DMF solution of the residue prepared separately was added thereto under ice cooling, and then the mixture was stirred at room temperature overnight. The reaction solution was purified by silica gel column chromatography to afford the title compound (130 mg). MS (m/z): 416.1 [M+H]⁺

### [Step 2] Preparation of Compound RE-116

To a mixture of Intermediate RE-116A (343 mg) and methanol (4.1 mL) was added 2 M aqueous sodium hydroxide solution (0.5 mL) under ice cooling, and then the mixture was stirred at room temperature overnight. 2 M Hydrochloric acid was added to neutralize the mixture, and the solvent was removed under reduced pressure. Then, the residue was purified by silica gel column chromatography to afford the title compound (183 mg). MS (m/z): 388.1 [M+H]⁺

### Reference Example 117 2-{3-[1-(2-amino-5-fluoropyridine-4-carbonyl)azetidin-3-yl]-1H-indazol-1-yl}acetic acid trifluoroacetate (Compound RE-117)

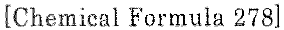

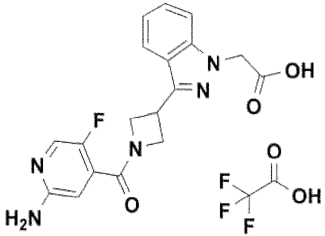

### [Step 1] Preparation of Intermediate RE-117A

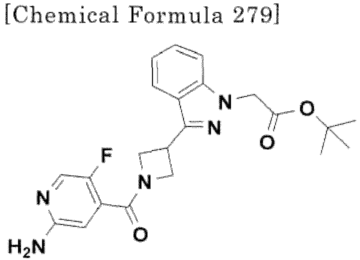

To a mixture of Compound RE-39 (473 mg), Compound RE-24 (349 mg), HOBt (289 mg), EDCI ·HCl (410 mg) and DMF (5.5 mL) was added DIPEA (0.85 mL), and the mixture was stirred at room temperature for 2 hours. A saturated aqueous solution of sodium bicarbonate was added to the reaction solution, and the mixture was extracted with ethyl acetate. The organic layer was washed with a saturated saline solution and dried with anhydrous magnesium sulfate. The solvent was removed under reduced pressure, and then the residue was purified by silica gel column chromatography to afford the title compound (409 mg). MS (m/z): 426.2 [M+H]⁺

### [Step 2] Preparation of Compound RE-117

To a mixture of Intermediate RE-117A (389 mg) and dichloromethane (2 mL) was added trifluoroacetic acid (0.5 mL), and the mixture was stirred at room temperature for 1 hour. Trifluoroacetic acid (0.5 mL) was added thereto, and the mixture was stirred at room temperature overnight. The solvent was removed under reduced pressure, and to the residue obtained was added toluene, followed by ethyl acetate and hexane, and then the solvent was removed under reduced pressure. The residue was washed with diethyl ether and dried to afford the title compound (396 mg). MS (m/z): 370.2 [M+H]⁺

### Reference Example 118 2-{3-[1-(2-aminopyridine-4-carbonyl)azetidin-3-yl]-4-fluoro-1H-indazol-1-yl}acetic acid (Compound RE-118)

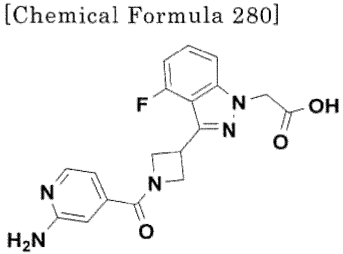

### [Step 1] Preparation of Intermediate RE-118A

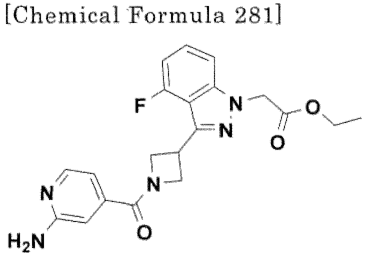

A mixture of Intermediate RE-20A (300 mg) and hydrogen chloride (2 M ethanol solution, 2 mL) was stirred at room temperature overnight. The solvent was removed under reduced pressure and the resulting residue (232 mg) was dissolved in a small amount of DMF. In a separate vessel, a mixture of 2-aminopyridine-4-carboxylic acid (80 mg), DMF (5.8 mL), DIPEA (0.8 mL) and HBTU (286 mg) was stirred at room temperature for 2 hours. Then, the separately prepared DMF solution of the residue was added, and the mixture was stirred at room temperature overnight. A saturated aqueous solution of sodium bicarbonate was added to the reaction solution, and the mixture was extracted with a mixed solvent of ethyl acetate and hexane. The organic layer was washed with water and dried over anhydrous magnesium sulfate. The solvent was removed under reduced pressure, and then the residue was purified by silica gel column chromatography to afford the title compound (83 mg). MS (m/z): 397.8 [M+H]⁺

### [Step 2] Preparation of Compound RE-118

To a mixture of Intermediate RE-118A (83 mg) and methanol (2 mL) was added 2 M aqueous sodium hydroxide solution (0.1 mL) under ice cooling, and the mixture was stirred at room temperature overnight. 2 M Hydrochloric acid was added to neutralize the mixture, and then the solvent was removed under reduced pressure to afford the title compound (77 mg) MS (m/z): 370.0 [M+H]⁺

### Reference Example 119 2-{3-[1-(2-amino-3-methylpyridine-4-carbonyl)azetidin-3-yl]-4,6-difluoro-1H-indazol-1-yl}acetic acid (Compound RE-119)

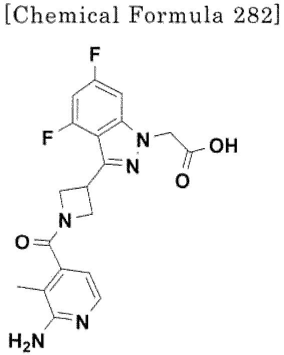

Compound RE-119 was prepared starting from Intermediate RE-2D, according to procedures similar to those described in Steps 1 to 2 of Reference Example 116 for the preparation of Compound RE-116, using 2-amino-3-methylpyridine-4-carboxylic acid instead of 2-aminopyridine-4-carboxylic acid. MS (m/z): 402.1 [M+H]⁺

### Reference Example 120 2-{3-[1-(2-amino-5-fluoropyridine-4-carbonyl)azetidin-3-yl]-4,6-difluoro-1H-indazol-1-yl]acetic acid (Compound RE-120)

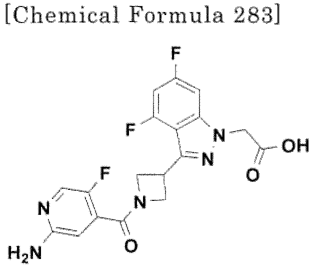

Compound RE-120 was prepared starting from Intermediate RE-2D, according to procedures similar to those described in Steps 1 to 2 of Reference Example 116 for the preparation of Compound RE-116, using Compound RE-24 instead of 2-aminopyridine-4-carboxylic acid. MS (m/z): 406.1 [M+H]⁺

### Reference Example 121 2-{3-[1-(2-aminopyridine-4-carbonyl)azetidin-3-yl]-1H-indazol-1-yl}acetic acid (Compound RE-121)

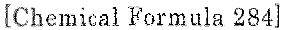

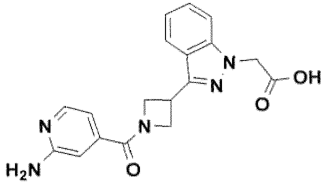

Compound RE-121 was prepared starting from Intermediate RE-4B, according to procedures similar to those described in Steps 1 to 2 of Reference Example 116 for the preparation of Compound RE-116. MS(m/z): 352.1 [M+H]⁺

### Reference Example 122 4-{3-[4-fluoro-1-(prop-2-yn-1-yl)-1H-indazol-3-yl]azetidine-1-carbonyl}pyridin-2-amine (Compound RE-122)

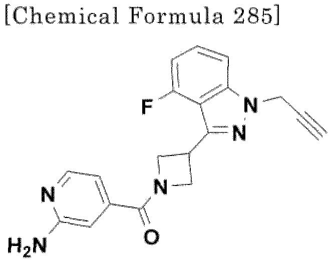

A mixture of Intermediate RE-18D (100 mg) and hydrogen chloride (4 M 1,4-dioxane solution, 2 mL) was stirred at room temperature for 3 hours. The solvent was removed under reduced pressure and the resulting residue (80 mg) was dissolved in a small amount of DMF. In a separate vessel, a mixture of 2-aminopyridine-4-carboxylic acid (32 mg), DMF (2.3 mL), DIPEA (0.4 mL) and HBTU (114 mg) was stirred at room temperature for 2 hours, and then the separately prepared DMF solution of the residue was added thereto, and then the mixture was stirred at room temperature for 4 hours. The reaction solution was purified by silica gel column chromatography to afford the title compound (34 mg). MS (m/z): 350.1 [M+H]⁺

### Example 1 2-(4-chloro-3-{1-[2-(cycloropylamino)pyridine-4-carbonyl]azetidin-3-yl}-5-fluoro-1H-indazol-1-yl)-N-(2,2,2-trifluoroethyl)acetamide hydrochloride (Compound E-1)

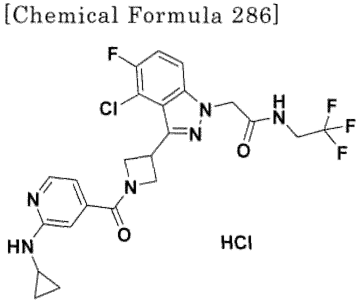

### [Step 1] Preparation of 2-(4-chloro-3-{1-[2-(cyclopropylamino)pyridine-4-carbonyl]azetidin-3-yl}-5-fluoro-1H-indazol-1-yl)-N-(2,2,2-trifluoroethyl)acetamide (Compound E-1A)

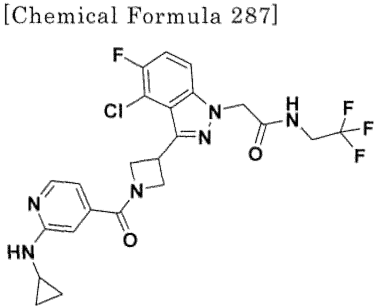

To a mixture of Compound RE-1 (30 mg), obtained in Step 7 of Reference Example 1, Compound RE-23 (16 mg), obtained in Step 2 of Reference Example 23, DMF (1 mL) and DIPEA (0.091 mL) was added HATU (37 mg), and the mixture was stirred for 1.5 hours. The reaction solution was purified by silica gel column chromatography to afford the title compound (15 mg).

### [Step 2] Preparation of Compound E-1

Compound E-1A (15 mg) was dissolved in methanol (2 mL), and 1 M hydrochloric acid (0.032 mL) was added thereto. Then, the mixture was stirred at room temperature for 5 minutes. The solvent was removed under reduced pressure to afford the title compound (13 mg). MS (m/z): 525.5 [M+H]⁺
Elemental analysis value (as C₂₃H₂₁ClF₄N₆O₂·HCl+1.3H₂O)
Calculated value (%) C: 47.24, H: 4.24, N14.37
Measured value (%) C: 47.37, H: 4.58, N13.98

### Example 2 2-{3-[1-(2-aminopyridine-4-carbonyl)azetidin-3-yl]-4,6-difluoro-1H-indazol-1-yl}-1-[(2S)-2-(trifluoromethyl)pyrrolidin-1-yl]ethan-1-one hydrochloride (Compound E-2)

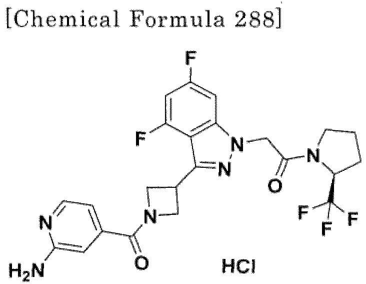

### [Step 1] Preparation of 2-{3-[1-(2-aminopyridine-4-carbonyl)azetidin-3-yl]-4,6-difluoro-1H-indazol-1-yl}-1-[(2S)-2-(trifluoromethyl)pyrrolidin-1-yl]ethan-1-one (Compound E-2A)

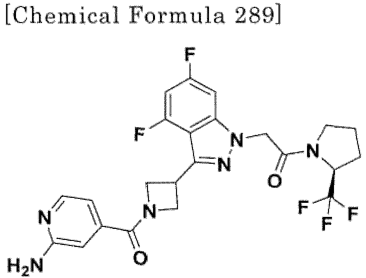

To a mixture of 2-aminopyridine-4-carboxylic acid (13 mg), HBTU (35 mg) and DMF (1 mL) was added DIPEA (0.098 mL), and the mixture was stirred at room temperature for 15 minutes. Compound RE-2 (30 mg), obtained in Step 7 of Reference Example 2, was added thereto, and then the mixture was stirred at room temperature for 3 hours. The reaction solution was purified by silica gel column chromatography to afford the title compound (34 mg).

### [Step 2] Preparation of Compound E-2

Compound E-2A (34 mg) was dissolved in methanol (2 mL), and 1 M hydrochloric acid (0.074 mL) was added thereto. Then, the mixture was stirred at room temperature for 5 minutes. The solvent was removed under reduced pressure to afford the title compound (28 mg). MS (m/z): 509.1 [M+H]⁺
Optical rotation
[α]₅₈₉²⁵=-4.7° (0.01 g, methanol, 1 mL, 50 mm)
Elemental analysis value (as C₂₃H₂₁F₅N₆O₂·HCl+1.2H₂O)
Calculated value (%) C: 48.76, H: 4.34, N: 14.83
Measured value (%) C: 48.50, H: 4.73, N: 14.68

### Example 3 2-{3-[1-(2-amino-5-fluoropyridine-4-carbonyl)azetidin-3-yl]-1H-indazol-1-yl}-1-[(2S)-2-(trifluoromethyl)pyrrolidin-1-yl]ethan-1-one (Compound E-3)

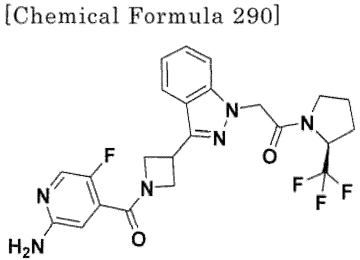

To a mixture of Compound RE-4 (20 mg), obtained in Step 5 of Reference Example 4, Compound RE-24 (9.1 mg), obtained in Reference Example 24. HOBt (7.5 mg), EDCI·HCl (10.7 mg) and DMF (0.5 mL) was added DIPEA (0.022 mL), and then the mixture was stirred at room temperature overnight. The reaction solution was purified by silica gel column chromatography to afford the title compound (12 mg). MS (m/z): 491.1 [M+H]⁺

### Example 4 2-{3-[1-(2-aminopyrimidine-4-carbonyl)azetidin-3-yl]-5-chloro-1H-indazol-1-yl}-N-methyl-N-(2,2,2-trifluoroethyl)acetamide (Compound E-4)

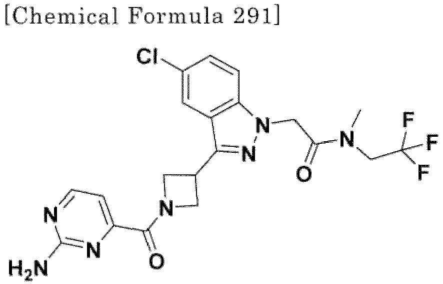

In the method according to Example 3, Compound RE-5 (43 mg), obtained in Step 6 of Reference Example 5, and 2-aminopyrimidine-4-carboxylic acid (15 mg) were used instead of Compound RE-4 and Compound RE-24, respectively, to afford the title compound (24 mg). MS (m/z): 482.3 [M+H]⁺

### Example 5 2-{3-[1-(2-amino-5-fluoropyridine-4-carbonyl)azetidin-3-yl]-6-fluoro-1H-indazol-1-yl}-N-methyl-N-(2,2,2-trifluoroethyl)acetamide (Compound E-5)

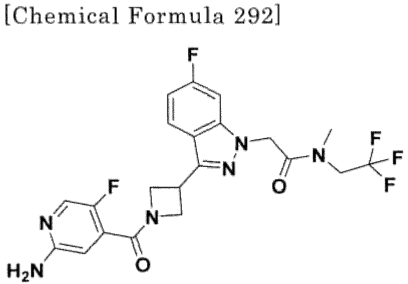

In the method according to Example 3, Compound RE-6 (40 mg), obtained in Step 5 of Reference Example 6, was used instead of Compound RE-4 to afford the title compound (32 mg). MS (m/z): 483.3 [M+H]⁺

### Example 6 2-{3-[1-(2-aminopyridine-4-carbonyl)-3-methylazetidin-3-yl]-1H-indazol-1-yl}-1-[(2S)-2-(trifluoromethyl)pyrrolidin-1-yl]ethan-1-one (Compound E-6)

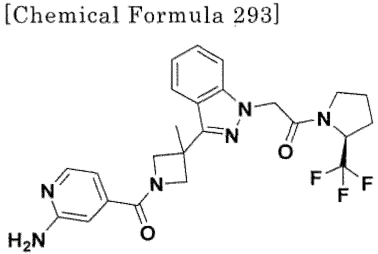

In the method according to Step 1 of Example 2, Compound RE-7 (55 mg), obtained in Step 5 of Reference Example 7, was used instead of Compound RE-2 to afford the title compound (7 mg). MS (m/z): 487.4 [M+H]⁺

### Example 7 2-{3-[1-(2-amino-4-methyl-1,3-thiazole-5-carbonyl)azetidin-3-yl]-1H-indazol-1-yl}-1-(3,3-dimethylmorpholin-4-yl)ethan-1-one (Compound E-7)

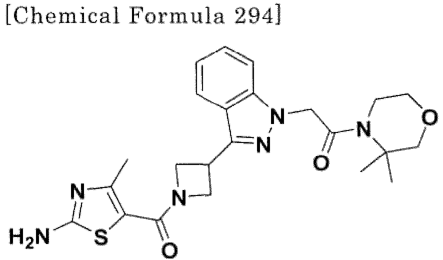

In the method according to Example 3, Compound RE-8 (15 mg), obtained in Step 2 of Reference Example 8, and 2-amino 4-methyl-1,3-thiazole-5-carboxylic acid (5.9 mg) were used instead of Compound RE-4 and Compound RE-24, respectively, to afford the title compound (12 mg) MS (m/z): 469.2 [M+H]⁺

### Example 8 2-{3-[1-(2-amino-3-methylpyridine-4-carbonyl)azetidin-3-yl]-1H-indazol-1-yl}-1-[(2R)-2-(trifluoromethyl)morpholin-4-yl]ethan-1-one (Compound E-8)

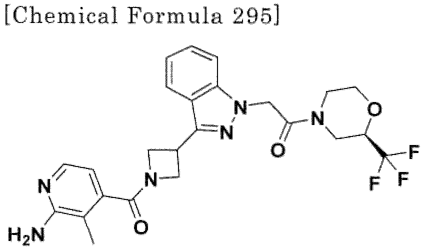

In the method according to Step 1 of Example 2, Compound RE-9 (20 mg), obtained in Step 2 of Reference Example 9, and Compound RE-25 (14 mg), obtained in Step 3 of Reference Example 25 were used instead of Compound RE-2 and 2-aminopyridine-4-carboxylic acid, respectively, to afford the title compound (15 mg). -MS (m/z): 503.2 [M+H]⁺

### Example 9 4-[4-(4-fluoro-1-{[5-(trifluoromethy)pyridin-2-yl]methyl}-1H-indazol-3-yl)piperidine-1-carbonyl]pyridin-2-amine (Compound E-9)

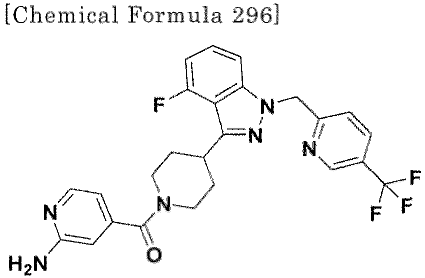

In the method according to Step 1 of Example 2, Compound RE-10 (35 mg), obtained in Step 5 of Reference Example 10, was used instead of Compound RE-2 to afford the title compound (25 mg). MS (m/z): 499.3 [M+H]⁺

### Example 10 2-{3-[1-(2-amino-5-fluoropyridine-4-carbonyl)piperidin-4-yl]-4-fluoro-1H-indol-1-yl}-1-[(2S)-2-(trifluoromethyl)pyrrolidin-1-yl]ethan-1-one (Compound E-10)

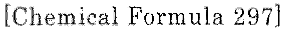

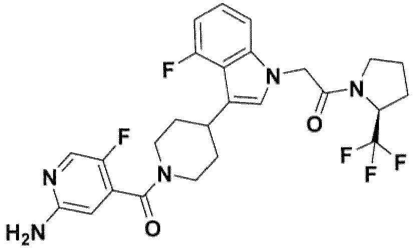

In the method according to Example 3, Compound RE-11 (46 mg), obtained in Step 6 of Reference Example 11, was used instead of Compound RE-4 to afford the title compound (17 mg). MS (m/z): 536.3 [M+H]⁺

### Example 11 2-{3-[1-(2-aminopyridine-4-carbonyl)azetidin-3-yl]-1H-indol-1-yl}-1-[(2S)-2-(trifluoromethyl)pyrrolidin-1-yl]ethan-1-one (Compound E-11)

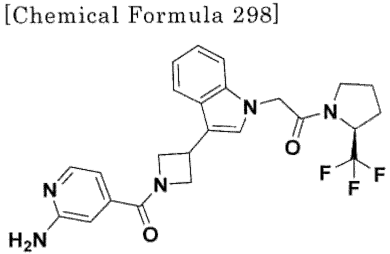

To a mixture of Compound RE-12 (50 mg), obtained in Step 5 of Reference Example 12, and ethyl acetate (0.4 mL) was added hydrogen chloride (4 M ethyl acetate solution, 0.14 mL), and the mixture was stirred at room temperature for 2 hours. The solvent was removed under reduced pressure to afford 2-[3-(azetidin-3-yl)-1H-indol-1-yl]-1-[(2S)-2-(trifluoromethyl)pyrrolidin-1-yl]ethan-1-one hydrochloride.

Then, in the method according to Step 1 of Example 2, 2-[3-(azetidin-3-yl)-1H-indol-1-yl]-1-[(2S)-2-(trifluoromethyl)pyrrolidin-1-yl]ethan-1-one hydrochloride was used instead of Compound RE-2 to afford the title compound (14 mg). MS (m/z): 472.3 [M+H]⁺

### Example 12 2-{3-[1-(2-aminopyridine-4-carbonyl)azetidin-3-yl]-5-methyl-1H-pyrazolo[4,3-b]pyridin-1-yl}-1-[(2S)-2-(trifluoromethyl)pyrrolidin-1-yl]ethan-1-one (Compound E-12)

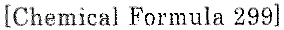

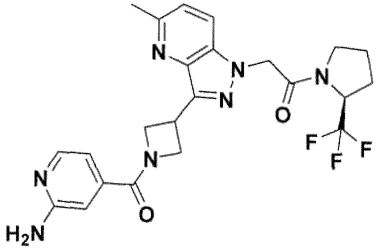

To a mixture of Compound RE-13 (100 mg), obtained in Step 6 of Reference Example 13, and methanol (1 mL) was added hydrogen chloride (2 M methanol solution, 0.53 mL), and the mixture was stirred at room temperature overnight. Hydrogen chloride (2 M methanol solution, 0.53 mL) was added for the remainder of the material, and then the mixture was stirred at room temperature for 7 hours. Hydrogen chloride (2 M methanol solution, 0.53 mL) was further added thereto, and then the mixture was stirred at room temperature overnight. The solvent was removed under reduced pressure to afford 2-[3-(azetidin-3-yl)-5-methyl-1H-pyrazolo[4,3-b]pyridin-1-yl]-1-[(2S)-2-(trifluoromethyl)pyrrolidin-1-yl]ethan-1-one hydrochloride.

Then, in the method according to Step 1 of Example 2, the obtained 2-[3-(azetidin-3-yl)-5-methyl-1H-pyrazolo[4,3-b]pyridin-1-yl]-1-[(2S)-2-(trifluoromethyl)pyrrolidin-1-yl]ethan-1-one hydrochloride (33 mg) was used instead of Compound RE-2 to afford the title compound (12 mg). MS (m/z): 488.2 [M+H]⁺

### Example 13 2-{3-[1-(2-aminopyridine-4-carbonyl)azetidin-3-yl]-1H-pyrazolo[4,3-c]pyridin-1-yl]-1-[(2S)-2-(trifluoromethyl)pyrrolidin-1-yl]ethan-1-one hydrochloride (Compound E-13)

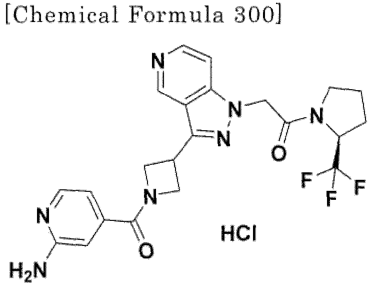

### [Step 1] Preparation of 2-{3-[1-(2-aminopyridine-4-carbonyl)azetidin-3-yl]-1H-pyrazolo[4,3-c]pyridin-1-yl}-1-[(2S)-2-(trifluoromethyl)pyrrolidin-1-yl]ethan-1-one (Compound E-13A)

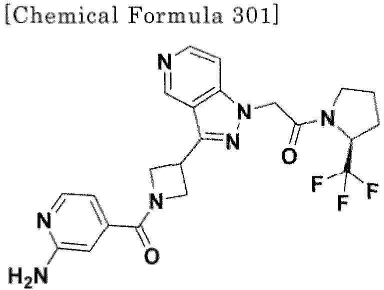

In the method according to Step 1 of Example 2, Compound RE-14 (30 mg), obtained in Step 8 of Reference Example 14, was used instead of Compound RE-2 to afford the title compound (22 mg).

### [Step 2] Preparation of Compound E-13

In the method according to Step 2 of Example 2, Compound E-13A (22 mg) was used instead of Compound E-2A to afford the title compound (18 mg). MS (m/z): 474.2 [M+H]⁺

### Example 14 4-{3-[4-fluoro-1-({5-[1-(trifluoromethyl)cyclopropyl]-1,2,4-oxadiazol-3-yl)methyl)-1H-pyrazolo[3,4-c]pyridin-3-yl]azetidine-1-carbonyl}pyridin-2-amine (Compound E-14)

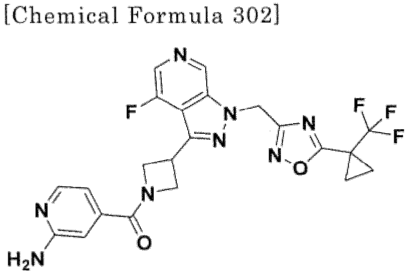

In the method according to Step 1 of Example 2, Compound RE-15 (27.5 mg), obtained in Step 7 of Reference Example 15, was used instead of Compound RE-2 to afford the title compound (22.3 mg). MS (m/z): 503.2 [M+H]⁺

### Example 15 2-{3-[1-(2-aminopyridine-4-carbonyl)azetidin-3-yl]-4-methyl-1H-pyrazolo[3,4-b]pyridin-1-yl}-1-[(2S)-2-(trifluoromethyl)pyrrolidin-1-yl]ethan-1-one (Compound E-15)

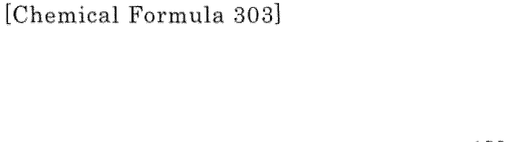

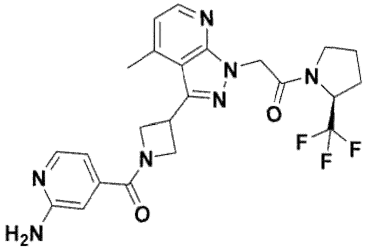

In the method according to Step 1 of Example 2, Compound RE-16 (43 mg), obtained in Step 7 of Reference Example 16, was used instead of Compound RE-2 to afford the title compound (27 mg). MS (m/z): 488.1 [M+H]⁺

### Example 16 2-{3-[1-(6-aminopyridazine-4-carbonyl)azetidin-3-yl]-4,6-difluoro-1H-indazol-1-yl)-1-[(2S)-2-(trifluoromethyl)pyrrolidin-1-yl]ethan-1-one (Compound E-16)

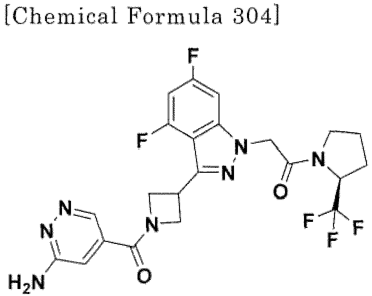

In the method according to Step 1 of Example 2, Compound RE-3 (20 mg), obtained in Reference Example 3, and 6-aminopyridazine-4-carboxylic acid (8.3 mg) were used instead of Compound RE-2 and 2-aminopyridine-4-carboxylic acid, respectively, to afford the title compound (5 mg). MS (m/z): 510.3 [M+H]⁺

### Example 17 2-[4,6-difluoro-3-(1-{1H-pyrazolo[3,4-b]pyridine-4-carbonyl}azetidin-3-yl)-1H-indazol-1-yl]-1-[(S)-2-(trifluoromethyl)pyrrolidin-1-vilethan-1-one (Compound E-17)

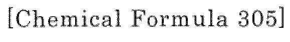

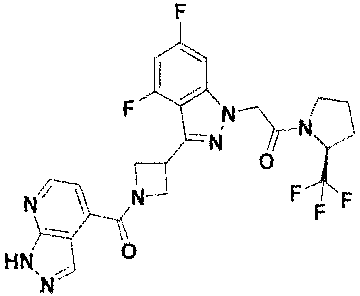

To a mixture of 1H-pyrazolo[3,4-b]pyridine-4-carboxylic acid (7.8 mg), EDCI·HCl (9.2 mg), HOBt (6.5 mg) and DMF (0.5 mL) was added DIPEA (0.021 mL), and the mixture was stirred at room temperature for 10 minutes. Compound RE-3 (20 mg), obtained in Reference Example 3, was added thereto, and the mixture was stirred at room temperature for 3 hours. The reaction solution was purified by silica gel column chromatography to afford the title compound (8 mg). MS (m/z): 534.3 [M+H]⁺

### Example 18 5-fluoro-4-[3-(1-{[3-(trifluoromethyl)-1,2,4-oxadiazol-5-yl]methyl}-1H-indazol-3-yl)azetidine-1-carbonyl]pyridin-2-amine (Compound E-18)

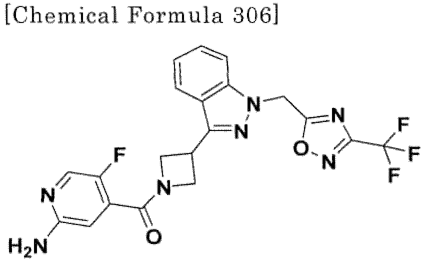

In the method according to Step 1 of Example 2, Compound RE-17 (49 mg), obtained in Step 4 of Reference Example 17, and Compound RE-24 (32 mg), obtained in Reference Example 24, were used instead of Compound RE-2 and 2-aminopyridine-4-carboxylic acid, respectively, to afford the title compound (12 mg). -MS (m/z): 462.2 [M+H]⁺

### Example 19 4-{3-[1-({1-[(3,3-difluorocyclobutyl)methyl]-1H-1,2,3-triazol-4-yl}methyl)-4-fluoro-1H-indazol-3-yl]azetidine-1-carbonyl}pyridin-2-amine hydrochloride (Compound E-19)

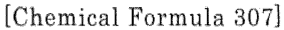

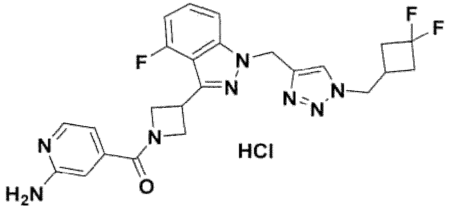

### [Step 1] Preparation of 4-{3-[1-({1-[(3,3-difluorocyclobutyl)methyl]-1H-1,2,3-triazol-4-yl}methyl)-4-fluoro-1H-indazol-3-yl]azetidine-1-carbonyl}pyridin-2-amine (Compound E-19A)

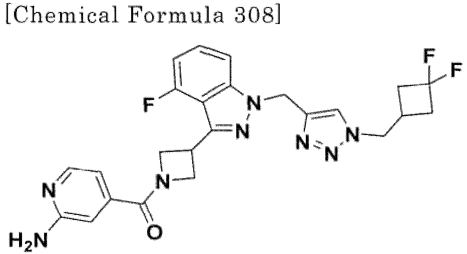

In the method according to Step 7 of Reference Example 1, Compound RE-18 (56 mg) was used instead of Intermediate RE-1F to afford 3-(azetidin-3-yl)-1-({1-((3,3-difluorocyclobutyl)methyl]-1H-1,2,3-triazol-4-yl}methyl)-4-fluoro-1H-indazole hydrochloride.

Then, in the method according to Step 1 of Example 2, the obtained 3-(azetidin-3-yl)-1-({1-[(3,3-difluorocyclobutyl)methyl]-1H-1,2,3-triazol-4-yl]methyl)-4-fluoro-1H-indazole hydrochloride was used instead of Compound RE-2 to afford the title compound (14 mg).

### [Step 2] Preparation of Compound E-19

In the method according to Step 2 of Example 2, Compound E-19A (14 mg) was used instead of Compound E-2A to afford the title compound (5.4 mg). MS (m/z): 497.1 [M+H]⁺

### Example 20 4-[3-(4-fluoro-1-{[5-(4-fluorophenyl)-1,3,4-oxadiazol-2-yl]methyl}-1H-indazol-3-yl)azetidine-1-carbonyl]pyridin-2-amine (Compound E-20)

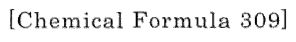

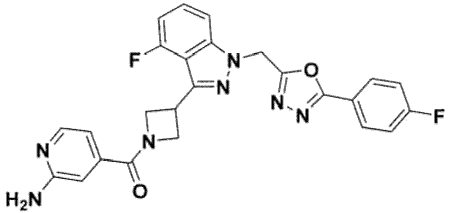

In the method according to Step 1 of Example 2, Compound RE-19 (67 mg), obtained in Step 2 of Reference Example 19, was used instead of Compound RE-2 to afford the title compound (53 mg). MS (m/z): 488.2 [M+H]⁺

### Example 21 2-{3-[1-(2-amino-5-fluoropyrimidine-4-carbonyl)azetidin-3-yl]-5-chloro-1H-indazol-1-yl}-N-methyl-N-(2,2,2-trifluoroethyl)acetamide (Compound E-21)

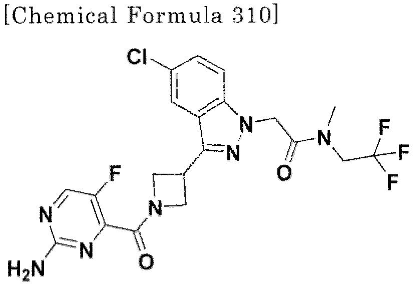

In the method according to Example 3, Compound RE-5 (43 mg), obtained in Step 6 of Reference Example 5, and Compound RE-26 (17 mg), obtained in Step 3 of Reference Example 26, were used instead of Compound RE-4 and Compound RE-24, respectively, to afford the title compound (6 mg). MS (m/z): 500.3 [M+H]⁺

### Example 22 2-{3-[1-(2-amino-5-fluoropyrimidine-4-carbonyl)azetidin-3-yl]-1H-indazol-1-yl}-1-[(2S)-2-(trifluoromethyl)pyrrolidin-1-yl]ethan-1-one (Compound E-22)

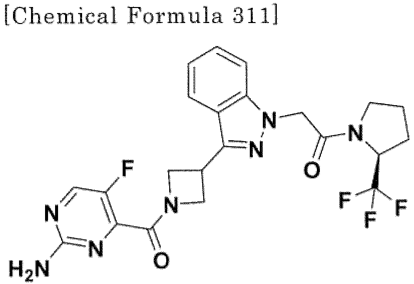

In the method according to Example 3, Compound RE-26 (16 mg), obtained in Step 3 of Reference Example 26, was used instead of Compound RE-24 to afford the title compound (22 mg). MS (m/z): 492.2 [M+H]⁺

### Example 23 2-{3-[1-(2-amino-5-fluoropyrimidine-4-carbonyl)azetidin-3-yl]-4-fluoro-1H-indazol-1-yl}-N-methyl-N-(2,2,2-trifluoroethyl)acetamide (Compound E-23)

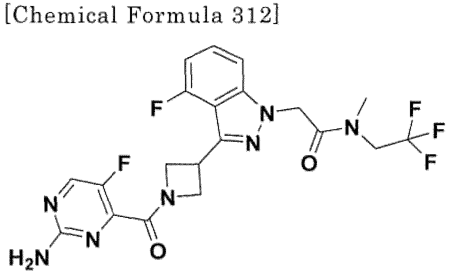

In the method according to Example 3, Compound RE-20 (41 mg), obtained in Step 4 of Reference Example 20, and Compound RE-26 (17 mg), obtained in Step 3 of Reference Example 26, were used instead of Compound RE-4 and Compound RE-24 to afford the title compound (19 mg). MS (m/z): 484.3 [M+H]⁺

### Example 24 2-{3-[1-(2-amino-5-fluoropyridine-4-carbonyl)azetidin-3-yl]-4,5,6,7-tetrahydro-1H-indazol-1-yl}-1-[(2S)-2-(trifluoromethyl)pyrrolidin-1-yl]ethan-1-one (Compound E-24)

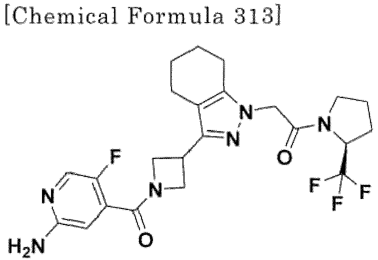

In the method according to Example 3, Compound RE-21 (16 mg), obtained in Step 6 of Reference Example 21, was used instead of Compound RE-4 to afford the title compound (9 mg). MS (m/z): 495.3 [M+H]⁺

### Example 25 2-{3-[1-(2-amino-5-fluoropyridine-4-carbonyl)azetidin-3-yl]-5,5-difluoro-4,5,6,7-tetrahydro-1H-indazol-1-yl}-1-[(2S)-2-(trifluoromethyl)pyrrolidin-1-vllethan-1-one (Compound E-25)

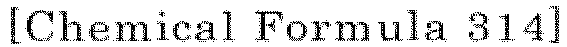

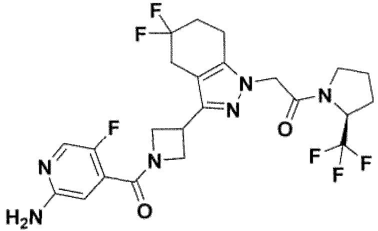

In the method according to Example 3, Compound RE-22 (62 mg), obtained in Step 8 of Reference Example 22, was used instead of Compound RE-4 to afford the title compound (40 mg). MS (m/z): 531.3 [M+H]⁺

### Example 26 2-(4-chloro-3-{1-[2-(cyclopropylamino)pyridine-4-carbonyl]azetidin-3-yl}-5-fluoro-1H-indazol-1-yl)-N-(2,2,2-trifluoroethyl)acetamide p-toluenesulfonate (Compound E-26)

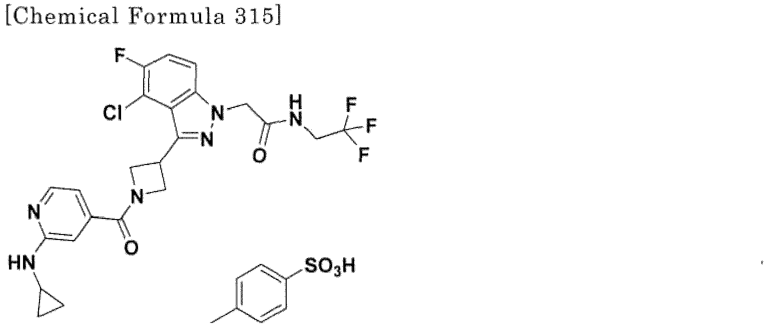

Compound E-1A (145 mg) was dissolved in THF (10 mL) under heating, p-toluenesulfonic acid monohydrate (58 mg) was added thereto, and the mixture was stirred at room temperature for 5 minutes. The solvent was removed under reduced pressure, and then the residue was washed with a mixed solvent of methanol and diethyl ether, and dried to afford the title compound (150 mg). MS (m/z): 525.4 [M+H]⁺
Elemental analysis value (as C₂₃H₂₁ClF₄N₆O₂ ·C₇H₈O₂S+2.7H₂O)
Calculated value (%) C: 48.32, H: 4.65, N11.27
Measured value (%) C: 48.35, H: 4.44, N10.95

### Example 27 2-{3-[1-(2-aminopyridine-4-carbonyl)azetidin-3-yl]-4,6-difluoro-1H-indazol-1-yl}-1-[(2S)-2-(trifluoromethyl)pyrrolidin-1-yl]ethan-1-one methanesulfonate (Compound E-27)

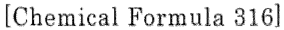

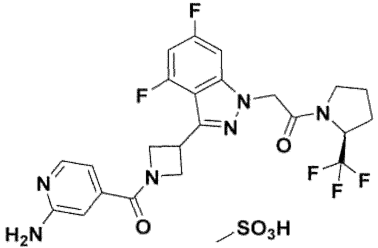

Compound E-2A (1.80 g) was dissolved in THF (36 mL) under heating, methanesulfonic acid (344 mg) was added thereto at room temperature, and then the mixture was stirred at room temperature for 1 hour. The solvent was removed under reduced pressure, and the residue was dissolved in acetonitrile under heating. After leaving the mixture to cool, the precipitated solid was obtained by filtration and dried to afford the title compound (1.45 g). MS (m/z): 509.3 [M+H]^{+ 1}H-NMR (400 MHz, DMSO-D₆) 6 8.01 (d, 1H), 8.00 (brs, 1H), 7.38 (d, 1H), 7.12 (s, 1H), 7.04 (t, 1H), 6.95 (d, 1H), 5.44 (s, 2H), 4.81-4.69 (m, 2H), 4.60-4.45 (m, 2H). 4.43-4.30 (m, 2H), 3.80-3.70 (m, 1H), 2.32 (s, 3H), 2.11-2.00 (m, 4H)
Optical rotation
[α]₅₈₉²⁵=-4.4° (0.01 g, methanol, 1 mL, 50 mm)
Elemental analysis value (as C₂₃H₂₁F₅N₆O₂·CH₄O₃S+0.5H₂O)
Calculated value (%) C: 46.98, H: 4.27, N13.70
Measured value (%) C: 46.73, H: 3.89, N13.62

### Example 28 2-{3-[1-(2-amino-5-fluoropyridine-4-carbonyl)azetidin-3-yl]-1H-indazol-1-yl}-[(2S)-2-(trifluoromethyl)pyrrolidin-1-yl]ethan-1-one hydrochloride (Compound E-28)

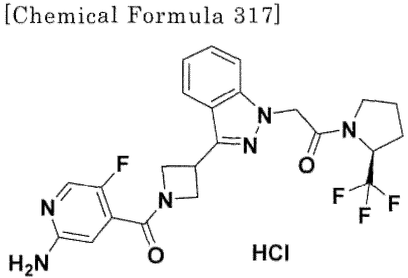

In the method according to Step 2 in Example 2, Compound E-3 was used instead of Compound E-2A to afford the title compound. MS (m/z): 474.2 [M+H]⁺
Optical rotation
[α]₅₈₉²⁵=-2.78° (0.01 g. methanol, 1 mL, 50 mm)
Elemental analysis value (as C₂₃H₂₂F₄N₈O₂ ·HCl+0.9H₂O)
Calculated value (%) C: 50.86, H:4.60, N15.47
Measured value (%) C: 51.13, H:4.75, N15.14

### Example 29 2-{3-[1-(2-amino-5-fluoropyridine-4-carbonyl)azetidin-3-yl]-1H-indazol-1-yl}-1-[(2S)-2-(trifluoromethyl)pyrrolidin-1-yl]ethan-1-one methanesulfonate (Compound E-29)

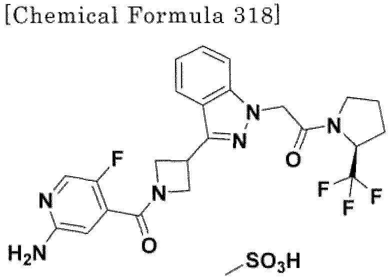

Compound E-3 (50 mg) was dissolved in THF (2 mL), and methanesulfonic acid (11 mg) was added thereto. Then, the mixture was stirred at room temperature for 30 minutes. The solvent was removed under reduced pressure, and the residue was washed with a mixed solvent of methanol and diethyl ether. The resulting product was dissolved in methanol. The solvent was removed under reduced pressure, and the residue was washed with a mixed solvent of 2-propanol and hexane and dried to afford the title compound (22 mg). MS (m/z): 491.2 [M+H]⁺
Optical rotation
[α]₅₈₉²⁰: -2.2° (0.3 g, methanol, 10 mL, 100 mm)
Elemental analysis value (as C₂₃H₂₂F₄N₆O₂·CH₄O₃S)
Calculated value (%) C: 49.14, H: 4.47, N14.33
Measured value (%) C: 49.10, H: 4.50, N14.36

### Example 30 2-(3-{1-[2-(cyclopropylamino)pyridine-4-carbonyl]azetidin-3-yl}-4,6-difluoro-1H-indazol-1-yl)-N-(2,2,2-trifluoroethyl)acetamide hydrochloride (Compound E-30)

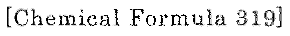

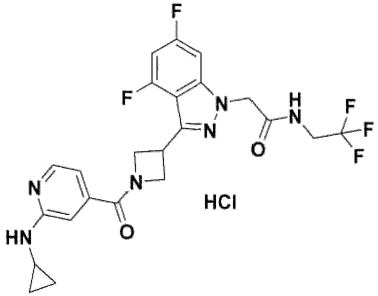

### [Step 1] Preparation of 2-(3-{1-[2-(cyclopropylamino)pyridine-4-carbonyl]azetidin-3-yl}-4,6-difluoro-1H-indazol-1-yl)-N-(2,2,2-trifluoroethyl)acetamide (Compound E-30A)

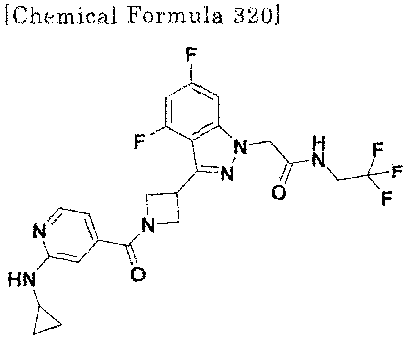

Compound RE-52 (125 mg) was dissolved in methanol (1 mL), and hydrogen chloride (2 M methanol solution, 1 mL) was added thereto. Then, the mixture was stirred at room temperature overnight. The solvent was removed under reduced pressure, and to a mixture of the resulting residue (50 mg), Compound RE-23 (28 mg), DIPEA (0.16 mL) and DMF (1 mL) was added HATU (59 mg) at room temperature, and then the mixture was stirred at room temperature overnight. The reaction solution was purified by silica gel column chromatography to afford the title compound (32 mg).

### [Step 2] Preparation of Compound E-30

In the method according to Step 2 in Example 2, Compound E-30A was used instead of Compound E-2A to afford the title compound. MS (m/z): 509.5 [M+H]⁺

### Example 31 2-(3-{1-[6-(cyclopropylamino)pyrimidine-4-carbonyl] azetidin-3-yl}-4,6-difluoro-1H-indazol-1-yl)-N-(2,2,2-trifluoroethyl)acetamide (Compound E-31)

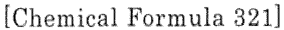

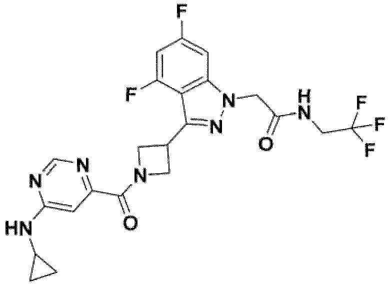

### [Step 1] Preparation of 6-(cyclopropylamino)pyrimidine-4-carboxylic acid trifluoroacetate

A mixture of Compound RE-27 (46 mg), dichloromethane (2 mL) and trifluoroacetic acid (1 mL) was stirred at room temperature overnight. The solvent was removed under reduced pressure to afford the title compound (56 mg).

### [Step 2] Preparation of Compound E-31

Compound RE-52 (125 mg) was dissolved in methanol (1 mL), and hydrogen chloride (2 M methanol solution, 1 mL) was added thereto. Then, the mixture was stirred at room temperature overnight. The solvent was removed under reduced pressure, and to a mixture of the resulting residue (20 mg), 6-(cyclopropylamino)pyrimidine-4-carboxylic acid trifluoroacetate (23 mg), DIPEA (0.04 mL) and DMF (1 mL), HBTU (30 mg) was added at room temperature, and then the mixture was stirred for 3 hours at room temperature. The reaction solution was purified by silica gel column chromatography to afford the title compound (16 mg). MS (m/z): 510.1 [M+H]⁺

### Example 32 2-[4,6-difluoro-3-(1-{2-[(oxetan-3-yl)amino]pyridine-4-carbonyl}azetidin-3-yl)-1H-indazol-1-yl]-N-(2,2,2-trifluoroethyl)acetamide (Compound E-32)

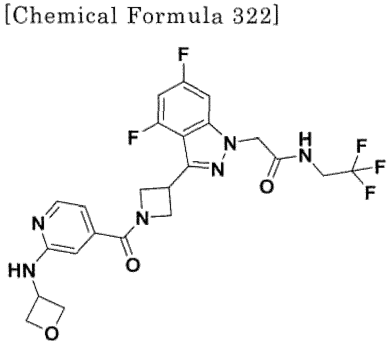

### [Step 1] Preparation of 2-{3-[1-(2-bromopyridine-4-carbonyl)azetidin-3-yl}-4,6-difluoro-1H-indazol-1-yl}-N-(2,2,2-trifluoroethyl)acetamide (Compound E-32A)

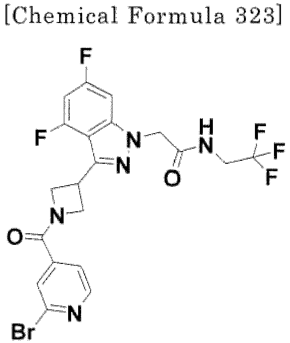

Compound RE-52 (125 mg) was dissolved in methanol (1 mL), and hydrogen chloride (2 M methanol solution, 1 mL) was added thereto. Then, the mixture was stirred at room temperature overnight. The solvent was removed under reduced pressure and the resulting residue (84 mg) was dissolved in DMF (2 mL). In a separate vessel, to a mixture of 2-bromopyridine-4-carboxylic acid (57 mg), HBTU (108 mg), and DMF (3 mL) was added DIPEA (0.1 mL), and the mixture was stirred at room temperature for 15 minutes. The separately prepared DMF solution of the residue was added thereto, and then the mixture was stirred at room temperature overnight. The reaction solution was purified by silica gel column chromatography to afford the title compound (72 mg). MS (m/z): 532.0 [M+H]⁺

### [Step 2] Preparation of Compound E-32

A mixture of Compound E-32A (25 mg), rac-BINAP (18 mg), cesium carbonate (46 mg), 3-oxetanamine (10 mg) and 1,4-dioxane (2 mL) was degassed with argon, Pd(OAc)₂ (2 mg) was added thereto, and the mixture was stirred at 100°C for 3 hours. The insoluble material was filtered off with Celite^{®}, the solvent was removed under reduced pressure, and then the residue was purified by silica gel column chromatography to afford the title compound (3 mg). MS (m/z): 525.1 [M+H]⁺

### Example 33 2-[4,6-difluoro-3-(1-{1H-pyrrolo[2,3-b]pyridine-4-carbonyl}azetidin-3-yl)-1H-indazol-1-yl]-1-[(2S)-2-(trifluoromethyl)pyrrolidin-1-yl]ethan-1-one hydrochloride (Compound E-33)

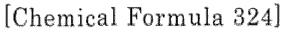

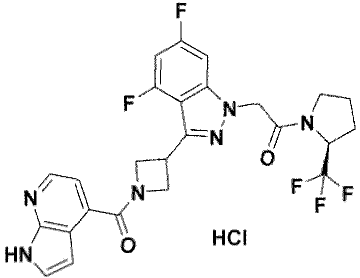

### [Step 1] Preparation of 2-[4,6-difluoro-3-(1-(1H-pyrrolo[2,3-b]pyridine-4-carbonyl}azetidin-3-yl)-1H-indazol-1-yl]-1-[(2S)-2-(trifluoromethyl)pyrrolidin-1-yl]ethan-1-one (Compound E-33A)

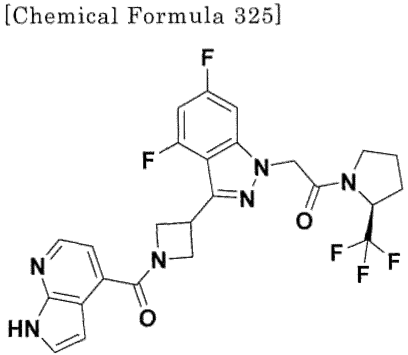

A mixture of 1H-pyrrolo[2,3-b]pyridine-4-carboxylic acid (17 mg), HOBt (14 mg), DIPEA (0.05 mL), DMF (0.3 mL) and EDCI ·HCl (20 mg) was stirred at room temperature for 10 minutes, Compound RE-2 (30 mg) was added thereto, and then the mixture was stirred at room temperature for 1 hour. The reaction solution was purified by silica gel column chromatography to afford the title compound (37 mg).

### [Step 2] Preparation of Compound E-33

In the method according to Step 2 of Example 2, Compound E-33A was used instead of Compound E-2A to afford the title compound. MS (m/z): 533.2 [M+H]⁺

### Example 34 4-(3-{4-fluoro-1-[(1-phenyl-1H-1,2,3-triazol-4-yl)methyl]-1H-indazol-3-yl}azetidine-1-carbonyl)pyridin-2-amine (Compound E-34)

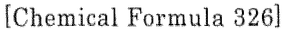

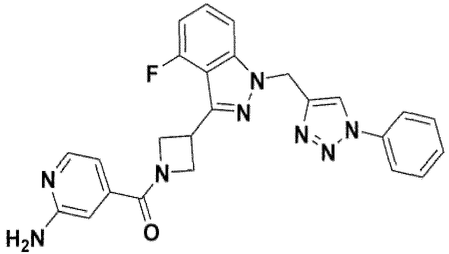

### [Step 1] Preparation of tert-butyl N-[4-(3-14-fluoro-1-[(1-phenyl-1H-1,2,3-triazol-4-yl)methyl]-1H-indazol-3-yl}azetidine-1-carbonyl)pyridin-2-yl]carbamate (Compound E-34A)

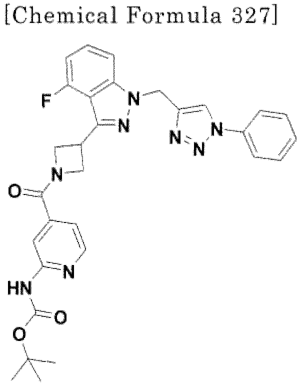

A mixture of Compound RE-89 (62 mg) and hydrogen chloride (2 M ethanol solution, 2 mL) was stirred at room temperature overnight. The solvent was removed under reduced pressure and the resulting residue (49 mg) was dissolved in a small amount of DMF. In a separate vessel, to a mixture of 2-(tert-butoxycarbonylamino)pyridine-4-carboxylic acid (39 mg), DMF (5.4 mL), DIPEA (0.2 mL) and HATU (62 mg) was added the separately prepared DMF solution of the residue, and then the mixture was stirred at room temperature overnight. The reaction solution was purified by silica gel column chromatography to afford the title compound (48 mg). MS (m/z): 569.2 [M+H]⁺

### [Step 2] Preparation of Compound E-34

A mixture of Compound E-34A (48 mg) and hydrogen chloride (2 M 1,4-dioxane solution, 2 mL) was stirred at room temperature for 6 hours. The solvent was removed under reduced pressure, and then the residue was purified by silica gel column chromatography to afford the title compound (14 mg). MS (m/z): 469.2 [M+H]⁺

### Example 35 4-(3-{1-[(1-benzyl-1H-1,2,3-triazol-4-yl)methyl]-4-fluoro-1H-indazol-3-yl)azetidine-1-carbonyl)pyridin-2-amine hydrochloride (Compound E-35)

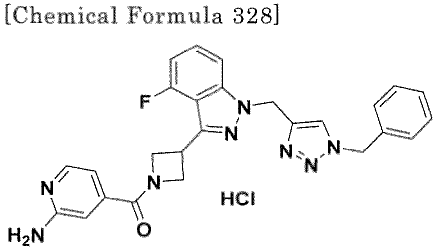

### [Step 1] Preparation of tert-butyl N-[4-(3-{1-[(1-benzyl-1H-1,2,3-triazol-4-yl)methyl]-4-fluoro-1H-indazol-3-yl}azetidine-1-carbonyl)pyridin-2-yl]carbamate (Compound E-35A)

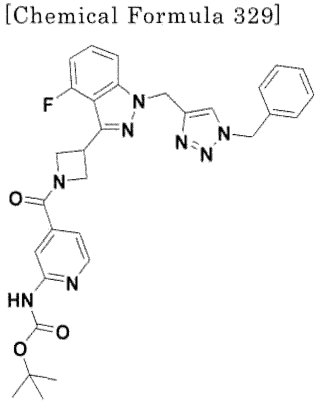

In the method according to Step 1 of Example 34, Compound RE-90 was used instead of Compound RE-89 to afford the title compound. MS (m/z): 583.3 [M+H]⁺

### [Step 2] Preparation of Compound E-35

A mixture of Compound E-35A (141 mg), hydrogen chloride (4 M 1,4-dioxane solution, 2 mL) and 1,4-dioxane (2 mL) was stirred at room temperature overnight. Then, 4 M hydrochloric acid (3 mL) was added and stirred. The solvent was removed under reduced pressure, and then the residue was purified by silica gel column chromatography to afford the title compound (64 mg). MS (m/z): 483.2 [M+H]⁺

### Example 36 2-{3-[1-(2-aminopyridine-4-carbonyl)azetidin-3-yl]-4.6-difluoro-1H-indazol-1-yl-1-[(2R)-2-(hydroxymethyl)pyrrolidin-1-yl]ethan-1-one (Compound E-36)

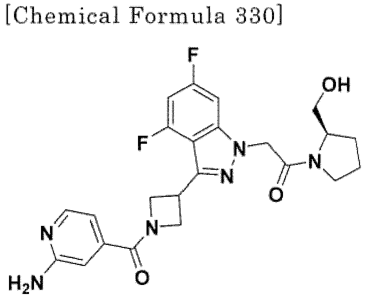

To a mixture of Compound RE-116 (20 mg), D-prolinol (8 mg), DIPEA (0.03 mL), and methanol (0.5 mL) was added DMTMM (21 mg), and the mixture was stirred at room temperature overnight. The reaction solution was purified by silica gel column chromatography to afford the title compound (5 mg). MS (m/z): 471.2 [M+H]⁺

### Example 37 4-[3-(1-{[1-(cyclopropylmethyl)-1H-1,2,3-triazol-4-yl]methyl}-4-fluoro-1H-indazol-3-yl)azetidine-1-carbonyl]pyridin-2-amine hydrochloride (Compound E-37)

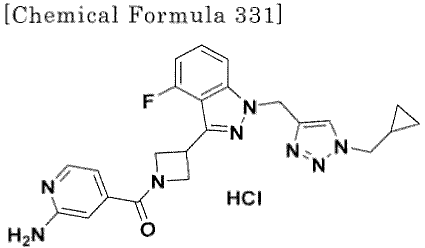

### [Step 1] Preparation of 4-[3-(1-{[1-(cyclopropylmethyl)-1H-1,2,3-triazol-4-yl]methyl}-4-fluoro-1H-indazol-3-yl)azetidine-1-carbonyl]pyridin-2-amine (Compound E-37A)

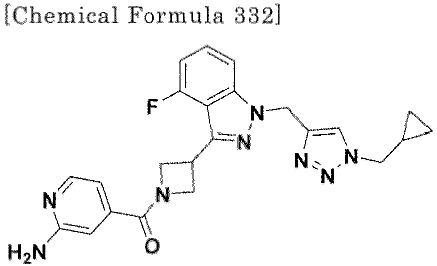

To a mixture of Compound RE-122 (120 mg), azidomethylcyclopropane (83 mg), DMF (2 mL) and methanol (0.2 mL) was added copper(I) iodide (7 mg), and the mixture was stirred at 60°C overnight under argon atmosphere. The reaction solution was purified by silica gel column chromatography to afford the title compound (28 mg). MS (m/z): 447.2 [M+H]⁺

### [Step 2] Preparation of Compound E-37

A mixture of Compound E-37A (28 mg), 4 M HCl (0.02 mL) and 1,4-dioxane (2 mL) was stirred at room temperature for 20 minutes. The solvent was removed under reduced pressure and dried to afford the title compound (14 mg). MS (m/z): 447.2 [M+H]⁺

### Example 38 4-{3-[4-fluoro-1-({1-(oxetan-3-yl)methyl]-1H-1,2,3-triazol-4-yl}methyl)-1H-indazol-3-yl]azetidine-1-carbonyl}pyridin-2-amine (Compound E-38)

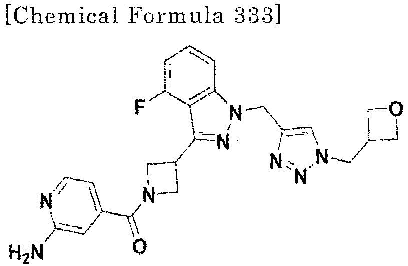

In the method according to Step 1 of Example 37, 3-(azidomethyl)oxetane was used instead of azidomethylcyclopropane to afford the title compound. MS (m/z): 463.2 [M+H]⁺

### Example 39 4-{4-[1-({1-[(3,3-difluorocyclobutyl)methyl-1H-1,2,3-triazol-4-yl}methyl)-4-fluoro-1H-indazol-3-yl]piperidine-1-carbonyl}pyridin-2-amine (Compound E-39)

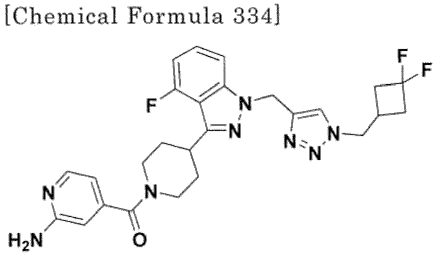

In the method according to Step 1 of Example 37, Compound RE-115 and 3-(azidomethyl)-1,1-difluorocyclobutane were used instead of Compound RE-122 and azidomethylcyclopropane, respectively, to afford the title compound. MS (m/z): 525.3 [M+H]⁺

### Example 40 2-{3-[1-(2-aminopyridine-4-carbonyl)azetidin-3-yl]-1H-indazol-1-yl}-1-[(2S)-2-(trifluoromethyl)pyrrolidin-1-yl]ethan-1-one (Compound E-40)

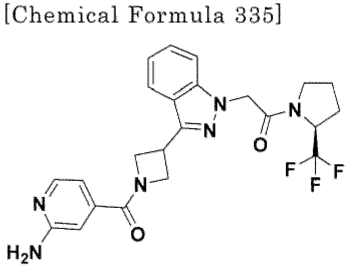

In the method according to Step 1 of Example 2, Compound RE-4 was used instead of Compound RE-2 to afford the title compound. MS (m/z): 473.2 [M+H]⁺

### Example 41 2-{3-[1-(2-aminopyridine-4-carbonyl)azetidin-3-yl]-1H-indazol-1-yl}-1-[(2S)-2-(trifluoromethyl)pyrrolidin-1-yl]ethan-1-one hydrochloride (Compound E-41)

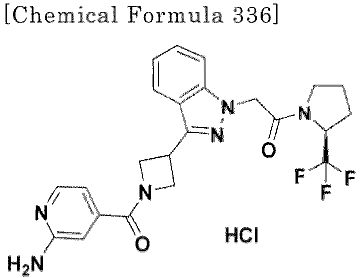

Compound E-40 (3.2 g) was dissolved in methanol (23 mL), and 2 M hydrochloric acid (3.6 mL) was added thereto. Then, the mixture was stirred at room temperature for 10 minutes. The solvent was removed under reduced pressure. The residue was dissolved in 2-propanol. and hexane was added thereto. Then, the precipitated solid was obtained by filtration and dried under reduced pressure to afford the title compound (3.47 g). MS (m/z): 473.2 [M+H]⁺
[α]₅₈₉²⁵: -3.93° (0.02 g, methanol, 5 mL, 100 mm)
Elemental analysis value (as C₂₃H₂₃F₃N₆O₂·HCl+3H₂O)
Calculated value (%) C: 49.07. H: 5.37, N14.93
Measured value (%) C: 49.54, H: 5.81, N14.51

### Example 42 2-{3-[1-(2-amino-5-fluoropyridine-4-carbonyl)azetidin-3-yl]-4,6-difluoro-1H-indazol-1-yl)-1-[(2R)-2-(trifluoromethyl)morpholin-4-yl]ethan-1-one (Compound E-42)

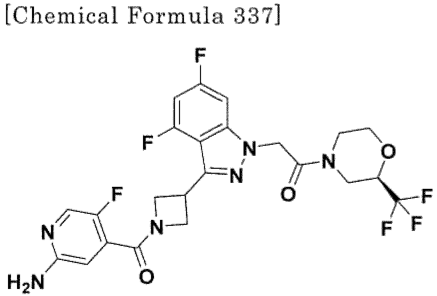

In the method according to Example 3, Compound RE-61 was used instead of Compound RE-4 to afford the title compound. MS (m/z): 543.2 [M+H]⁺

### Example 43 2-{3-[1-(2-amino-5-fluoropyridine-4-carbonyl)azetidin-3-yl-4,6-difluoro-1H-indazol-1-yl}-1-[(2R)-2-(trifluoromethyl)morpholin-4-yl]ethan-1-one p-toluenesulfonate (Compound E-43)

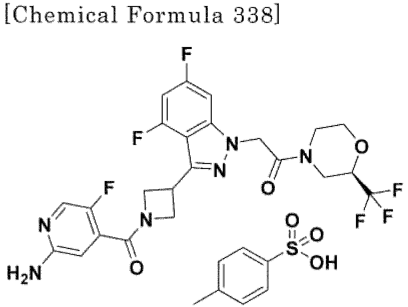

Compound E-42 (32 mg) was dissolved in THF (2 mL), and p-toluenesulfonic acid monohydrate (13 mg) was added thereto. Then, the mixture was stirred at room temperature for 1 hour. The precipitated solid was obtained by filtration and dried under reduced pressure to afford the title compound (36 mg). MS (m/z): 543.2 [M+H]⁺
[α]₅₈₉²⁵=+7.42° (0.001 g, methanol, 1 mL, 100 mm)
Elemental analysis value (as C₂₃H₂₀F₆N₆O₃·C₇H₈O₃S+1.4H₂O)
Calculated value (%) C: 48.70, H: 4.20. N11.36
Measured value (%) C: 49.01, H: 4.05, N10.89

### Example 44 2-{3-[1-(2-amino-3-methylpyridine-4-carbonyl)azetidin-3-yl]-4-fluoro-1H-pyrazolo[3,4-c]pyridin-1-yl]-1-[(2S)-2-[(trifluoromethoxy)methyl]pyrrolidin-1-yl]ethan-1-one di-p-toluenesulfonate (Compound E-44)

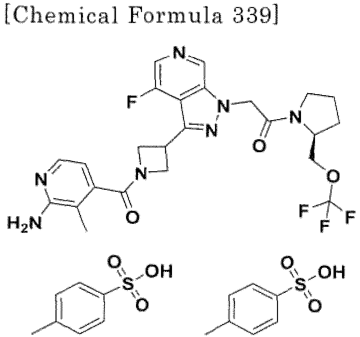

### [Step 1] Preparation of 2-{3-[1-(2-amino-3-methylpyridine-4-carbonyl)azetidin-3-yl]-4-fluoro-1H-pyrazolo[3,4-c]pyridin-1-yl}-1-[(2S)-2-[(trifluoromethoxy)methyl]pyrrolidin-1-yl]ethan-1-one (Compound E-44A)

A mixture of Compound RE-25 (14 mg), HBTU (20 mg), DIPEA (0.07 mL) and DMF (1 mL) was stirred at room temperature for 10 minutes, and Compound RE-87 (19 mg) was added thereto. Then, the mixture was stirred at room temperature for 4 hours. The reaction solution was purified by silica gel column chromatography to afford the title compound (17 mg).

### [Step 2] Preparation of Compound E-44

Compound E-44A (17 mg) was dissolved in ethyl acetate (2 mL), and p-toluenesulfonic acid monohydrate (13 mg) was added thereto. Then, the mixture was stirred at room temperature. The precipitated solid was obtained by filtration and dried under reduced pressure to afford the title compound (19 mg). MS (m/z): 536.2 [M+H]⁺

### Example 45 2-{3-[1-(2-aminopyridine-4-carbonyl)azetidin-3-yl]-1H-indazol-1-yl}-N-methyl-N-(2,2,2-trifluoroethyl)acetamide (Compound E-45)

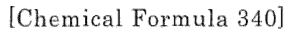

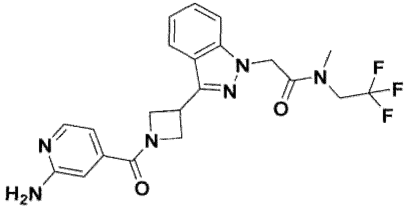

To a mixture of Compound RE-121 (20 mg), 2,2,2-trifluoro-N-methylethanamine hydrochloride (13 mg), HBTU (33 mg) and DMF (0.3 mL) was added DIPEA (0.05 mL), and the mixture was stirred at room temperature overnight. The reaction solution was purified by silica gel column chromatography to afford the title compound (16 mg). MS (m/z) 447.2 [M+H]⁺

### Example 46 2-{3-[1-(2-aminopyridine-4-carbonyl)azetidin-3-yl]-1H-indazol-1-yl}-N-methyl-N-(2,2,2-trifluoroethyl)acetamide p-toluenesulfonate (Compound E-46)

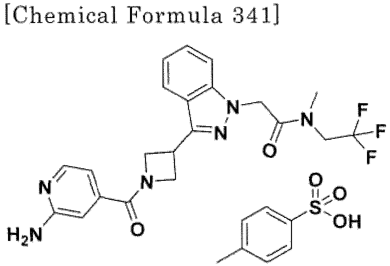

Compound E-45 (50 mg) was dissolved in THF (1.1 mL), and p-toluenesulfonic acid monohydrate (23 mg) was added thereto. Then, the mixture was stirred at room temperature for 10 minutes. The solvent was removed under reduced pressure, and the residue was dissolved in methanol. Diethyl ether was added thereto to precipitate a solid. The solid was obtained by filtration and dried under reduced pressure to afford the title compound (69 mg). MS (m/z): 447.2 [M+H]⁺ Elemental analysis value (as C₂₁H₂₁F₃N₆O₂·C7H8O3S+3.5H2O)
Calculated value (%) C: 49.33, H: 5.32,N12.33
Measured value (%) C: 49.34, H: 5.01,N11.93

### Example 47 2-{3-[1-(2-amino-5-fluoropyridine-4-carbonyl)-1,2,3,6-tetrahydropyridin-4-yl)-1H-indazol-1-yl)-1-[(2S)-2-(trifluoromethyl)pyrrolidin-1-yl]ethan-1-one (Compound E-47)

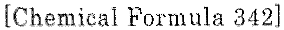

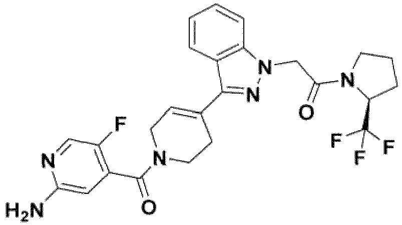

A mixture of Compound RE-40 (105 mg), dichloromethane (2 mL) and trifluoroacetic acid (0.5 mL) was stirred at room temperature. The solvent was removed under reduced pressure, and to the residue was added toluene, followed by ethyl acetate and hexane. Then, the solvent was removed under reduced pressure. In a separate vessel, to a mixture of Compound RE-24 (51 mg), DIPEA (0.1 mL), HOBt (35 mg) and DMF (0.5 mL) was added EDCI-HCl (50 mg), and the mixture was stirred at room temperature for 30 minutes. The mixture was added to the residue obtained previously, and then the mixture was stirred at room temperature overnight. The reaction solution was purified by silica gel column chromatography to afford the title compound (12 mg). MS (m/z): 517.3 [M+H]

### Example 48 2-{3-[1-(2-amino-5-fluoropyridine-4-carbanyl)piperidin-4-yl]-1H-indazol-1-yl}-N-methyl-N-(2,2,2-trifluoroethyl)acetamide (Compound E-48)

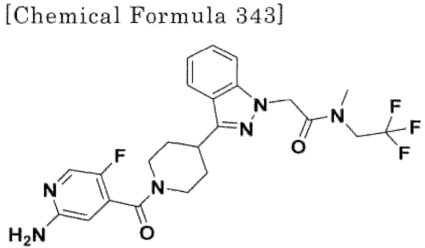

### [Step 1] Preparation of 2-{3-[1-(2-amino-5-fluoropyridine-4-carbonyl)-1,2,3,6-tetrahydropyridin-4-yl]-1H-indazol-1-y1}-N-methyl-N-(2,2,2-trifluoroethyl)acetamide (Compound E-48A)

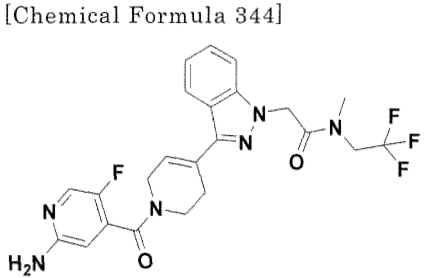

In the method according to Example 47, Compound RE-41 was used instead of Compound RE-40 to afford the title compound. MS (m/z): 491.3 [M+H]⁺

### [Step 2] Preparation of Compound E-48

To a mixture of Compound E-48A (47 mg), methanol (3 mL) and ammonium formate (79 mg) was added 10% Pd-C (13 mg), and the mixture was stirred at 80°C overnight. The insoluble material was filtered off, the solvent was removed under reduced pressure, and then the residue was purified by silica gel column chromatography to afford the title compound (24 mg). MS (m/z): 493.3 [M+H]⁺

### Example 49 2-{3-[1-(2-amino-5-fluoropyridine-4-carbonyl)piperidin-4-yl]-1H-indazol-1-yl}-1-[(2S)-2-(trifluoromethyl)pyrrolidin-1-yl]ethan-1-one (Compound E-49)

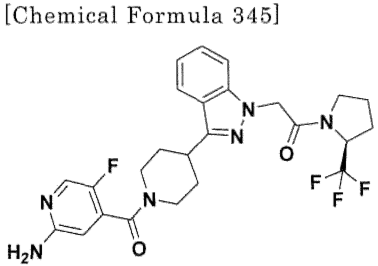

In the method according to Step 2 of Example 48, Compound E-47 was used instead of Compound E-48A to afford the title compound. MS (m/z): 519.3 [M+H]⁺

### Example 50 2-{3-[1-(2-aminoryridine-4-carbonyl)azetidin-3-yl]-4,6-difluoro-1H-indazol-1-yl}-1-[(2R)-2-(trifluoromethyl)pyrrolidin-1-yl]ethan-1-one (Compound E-50)

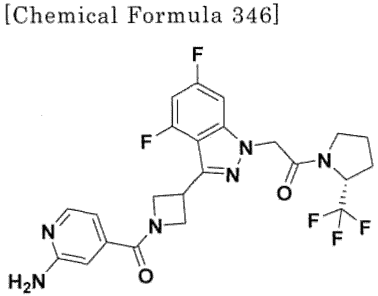

A mixture of Compound RE-116 (20 mg), (2R)-2-(trifluoromethyl)pyrrolidine hydrochloride (14 mg), HATU (29 mg), DIPEA (0.03 mL) and DMF (0.3 mL) was stirred at room temperature for 3 hours. The reaction solution was purified by silica gel column chromatography to afford the title compound (21 mg). MS (m/z): 509.1 [M+H]⁺

### Example 51 2-{3-[1-(2-amino-3-methylpyridine-4-carbonyl)azetidin-3-yl]-4,6-difluoro-1H-indazol-1-yl}-1-[(2R)-2-(trifluoromethyl)morpholin-4-yl]ethan-1-one hydrochloride (Compound E-51)

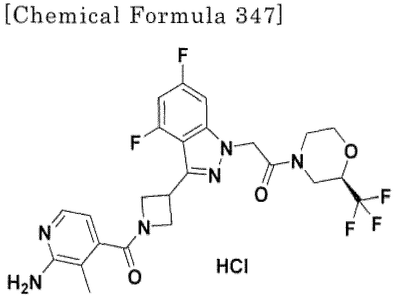

### [Step 1] Preparation of 2-{3-[1-(2-amino-3-methylpyridine-4-carbonyl)azetidin-3-yl]-4,6-difluoro-1H-indazol-1-yl}-1-[(2R)-2-(trifluoromethyl)morpholin-4-yl]ethan-1-one (Compound E-51A)

A mixture of Compound RE-119 (19 mg), (2R)-2-(trifluoromethyl)morpholine hydrochloride (46 mg), HATU (27 mg), DIPEA (0.04 mL) and DMF (1 mL) was stirred at room temperature overnight. The reaction solution was purified by silica gel column chromatography to afford the title compound (31 mg).

### [Step 2] Preparation of (Compound E-51)

Compound E-51A (31 mg) was dissolved in methanol (2 mL), and 1 M hydrochloric acid (0.06 mL) was added thereto. Then, the mixture was stirred at room temperature for 5 minutes. The solvent was removed under reduced pressure. Then, the residue was washed with a mixed solvent of ethanol and diethyl ether and dried to afford the title compound (18 mg). MS (m/z): 539.2 [M+H]⁺

### Example 52 4-[3-(1-{[1-(2,2,2-trifluoroethyl)-1H-1,2,3-triazol-4-yl]methyl]-4-fluoro-1H-indazol-3-yl)azetidine-1-carbonyl]pyridin-2-amine hydrochloride (Compound E-53)

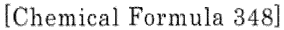

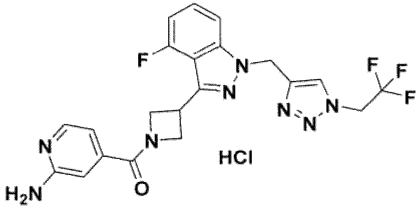

### [Step 1] Preparation of 4-[3-(1-{[1-(2,2,2-trifluoroethyl)-1H-1,2,3-triazol-4-yl]methyl}-4-fluoro-1H-indazol-3-yl)azetidine-1-carbonyl]pyridin-2-amine (Compound E-52A)

Compound RE-91 (42 mg) was dissolved in 1,4-dioxane (1 mL), and 4 M hydrochloric acid (1 mL) was added thereto. Then, the mixture was stirred at 50°C for 3 hours. The solvent was removed under reduced pressure and the resulting residue was dissolved in DMF. In a separate vessel, a mixture of 2-aminopyridine-4-carboxylic acid (10 mg), HBTU (36 mg), DIPEA (0.1 mL) and DMF (0.7 mL) was stirred at room temperature for 2 hours. The separately prepared DMF solution of the residue was added thereto, and the mixture was stirred at room temperature overnight. The reaction solution was purified by silica gel column chromatography to afford the title compound (17 mg).

### [Step 2] Preparation of Compound E-68

A mixture of Compound E-52A (17 mg) and hydrogen chloride (4 M 1,4-dioxane solution, 0.02 mL) was stirred at room temperature. The solvent was removed under reduced pressure. Then, the residue was washed with a mixed solvent of methanol and diethyl ether and dried to afford the title compound (8 mg). MS (m/z): 475.1 [M+H]⁺

### Example 53 2-{3-[1-(2-aminopyridine-4-carbonyl)azetidin-3-yl]-4-methyl-1H-pyrazolo[4,3-c]pyridin-1-yl}-1-[(2S)-2-(trifluoromethyl)pyrrolidin-1-yl]ethan-1-one (Compound E-53)

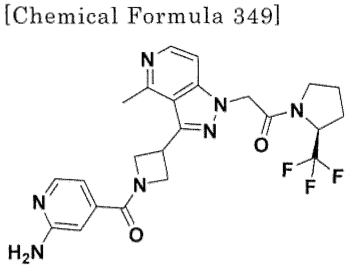

A mixture of Compound RE-33 (97 mg), dichloromethane (2 mL) and trifluoroacetic acid (0.2 mL) was stirred at room temperature for 8 hours. The solvent was removed under reduced pressure, and to the residue obtained was added toluene, followed by ethyl acetate and hexane, and the solvent was removed under reduced pressure to afford a solid (132 mg). In a separate vessel, a mixture of 2-aminopyridine-4-carboxylic acid (13 mg), DIPEA (0.13 mL), HBTU (36 mg) and DMF (1 mL) was stirred at room temperature for 30 minutes, and then the residue obtained previously (44 mg) was added thereto, and the mixture was stirred at room temperature overnight. The reaction solution was purified by silica gel column chromatography to afford the title compound (25 mg). MS (m/z): 488.1 [M+H]⁺

### Example 54 2-{3-[1-(2-aminopyridine-4-carbonyl)azetidin-3-yl]-4-fluoro-1H-pyrazolo[3,4-c]pyridin-1-yl}-1-[(2S)-2-(trifluoromethyl)pyrrolidin-1-yl]ethan-1-one hydrochloride (Compound E-54)

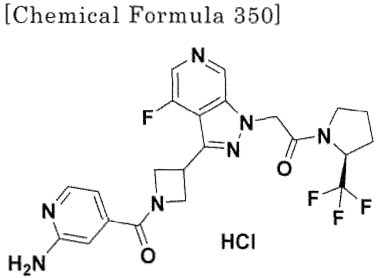

### [Step 1] Preparation of 2-{3-[1-(2-aminopyridine-4-carbonyl)azetidin-3-yl]-4-fluoro-1H-pyrazolo[3,4-c]pyridin-1-yl}-1-[(2S)-2-(trifluoromethyl)pyrrolidin-1-yl]ethan-1-one (Compound E-54A)

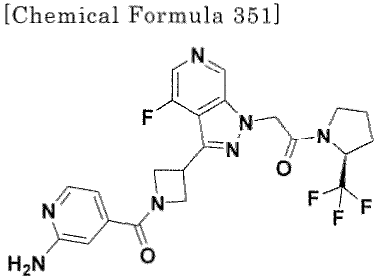

Compound RE-81 (51 mg) was dissolved in dichloromethane (2 mL), and trifluoroacetic acid (0.2 mL) was added thereto. Then, the mixture was stirred at room temperature overnight. The solvent was removed under reduced pressure and the resulting residue was dissolved in DMF. In a separate vessel, a mixture of 2-aminopyridine-4-carboxylic acid (30 mg), HBTU (82 mg), DIPEA (0.19 mL) and DMF (0.5 mL) was stirred at room temperature for 2 hours. The separately prepared DMF solution of the residue was added thereto, and the mixture was stirred at room temperature overnight. The reaction solution was purified by silica gel column chromatography to afford the title compound (30 mg).

### [Step 2] Preparation of Compound E-54

A mixture of Compound E-54A (30 mg) and hydrogen chloride (4 M 1,4-dioxane solution, 2 mL) was stirred at room temperature for 30 minutes. The solvent was removed under reduced pressure. Then, the residue was washed with a mixed solvent of methanol and diethyl ether and dried to afford the title compound (28 mg). MS (m/z): 492.2 [M+H]⁺

### Example 55 4-{3-[4-fluoro-1-({3-[3-(trifluoromethyl)phenyl]-1,2,4-oxadiazol-5-yl}methyl)-1H-indazol-3-yl]azetidine-1-carbonyl}pyridin-2-amine (Compound E-55)

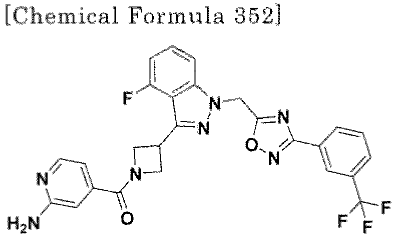

Compound RE-109 (45 mg) was dissolved in dichloromethane (2 mL), and trifluoroacetic acid (0.2 mL) was added thereto. Then, the mixture was stirred at room temperature for 2 hours. The solvent was removed under reduced pressure and the resulting residue was dissolved in DMF. In a separate vessel, a mixture of 2-aminopyridine-4-carboxylic acid (16 mg), HBTU (43 mg), DIPEA (0.15 mL) and DMF (0.3 mL) was stirred at room temperature for 2 hours. The separately prepared DMF solution of the residue was added thereto, and the mixture was stirred at room temperature overnight. The reaction solution was purified by silica gel column chromatography to afford the title compound (18 mg). MS (m/z): 538.2 [M+H]⁺

### Example 56 2-{3-[1-(2-aminopyridine-4-carbonyl)azetidin-3-yl]-4,6-difluoro-1H-indazol-1-yl}-1-[(2S)-2-[(trifluoromethoxy)methyl]pyrrolidin-1-yl]ethan-1-one (Compound E-56)

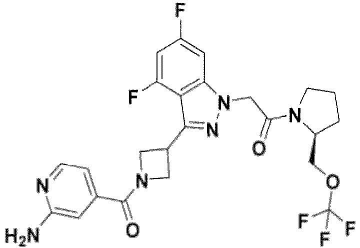

To a mixture of Compound RE-116 (30 mg), HBTU (38 mg) and DMF (0.5 mL) was added DIPEA (0.1 mL), and the mixture was stirred at room temperature for 5 minutes. (2S)-2-(Trifluoromethoxymethyl)pyrrolidine hydrochloride (19 mg) was added thereto, and the mixture was stirred at room temperature for 2 hours. The reaction solution was purified by silica gel column chromatography to afford the title compound (19 mg). MS (m/z): 539.2 [M+H]⁺

### Example 57 4-(3-{1-[(4,4-difluorocyclohexyl)methyl]-4,6-difluoro-1H-indazol-3-yl}azetidine-1-carbonvl)pyridin-2-amine (Compound E-57)

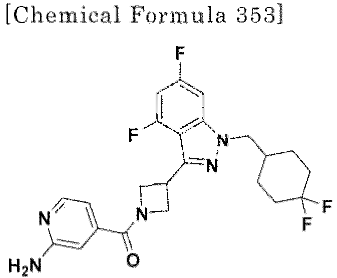

Compound RE-55 (80 mg) was dissolved in methanol (0.7 mL), and hydrogen chloride (4 M ethyl acetate solution, 0.03 mL) was added thereto. Then, the mixture was stirred at room temperature for 4 hours. The solvent was removed under reduced pressure and the resulting residue was dissolved in DMF (0.5 mL). In a separate vessel, a mixture of 2-aminopyridine-4-carboxylic acid (30 mg), HBTU (103 mg), DIPEA (0.25 mL) and DMF (1.2 mL) was stirred at room temperature for 1.5 hours. The separately prepared DMF solution of the residue was added thereto, and the mixture was stirred at room temperature overnight. The reaction solution was purified by silica gel column chromatography to afford the title compound (48 mg). MS (m/z): 462.3 [M+H]⁺

### Example 58 2-{3-11-(2-amino-5-fluoropyridine-4-carbonyl)azetidin-3-yl]-4-fluoro-1H-pyrazolo[3.4-c]pyridin-1-yl}-1-[(2S)-2-[(trifluoromethoxy)methyl]pyrrolidin-1-yl]ethan-1-one di-n-toluenesulfonate (Compound E-58)

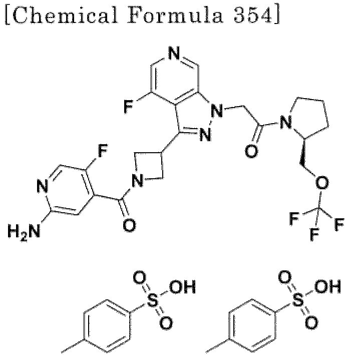

### [Step 1] Preparation of 2-{S-[1-(2-amino-5-fluoropyridine-4-carbonyl)azetidin-3-yl]-4-fluoro-1H-pyrazolo[3,4-c]pyridin-1-yl}-1-[(2S)-2-[(trifluoromethoxy)methyl]pyrrolidin-1-yl]ethan-1-one (Compound E-58A)

To a mixture of Compound RE-87 (19 mg), Compound RE-24 (8 mg), HOBt (8 mg), DIPEA (0.02 mL) and DMF (0.5 mL) was added EDCI ·HCl (12 mg), and the mixture was stirred at room temperature for 4 hours. The reaction solution was purified by silica gel column chromatography to afford the title compound (11 mg).

### [Step 2] Preparation of Compound E-58

Compound E-58A (11 mg) was dissolved in ethyl acetate (2 mL), and p-toluenesulfonic acid monohydrate (8 mg) was added thereto. Then, the mixture was stirred at room temperature. The precipitated solid was obtained by filtration and dried under reduced pressure to afford the title compound (19 mg). MS (m/z): 540.2 [M+H]⁺
¹H-NMR (400 MHz, DMSO-De) δ 8.92 (d, 1H), 8.22 (d, 1H), 8.09 (d, 1H), 7.47 (d, 4H), 7.11 (s, 4H), 5.55 (d, 2H), 4.61-4.52 (m, 2H), 4.50-4.40 (m, 1H), 4.37-4.28 (m, 2H), 4.20-4.07 (m, 2H), 2.29 (s, 6H), 2.11-1.84 (m, 5H)
Elemental analysis value (as C₂₃H₂₂F₅N₇O₃·C₁₄H₁₆O₆S₂+3.0H₂O)
Calculated value (%) C: 47.38, H: 4.73, N10.45
Measured value (%) C: 47.14, H: 4.72, N10.36

### Example 59 2-{3-[1-(2-amino-5-fluorogyridine-4-carbonyl)azetidin-3-yl]-4-fluoro-1H-indazol-1-yl}-N-methyl-N-(2,2,2-trifluoroethyl)acetamide (Compound E-59)

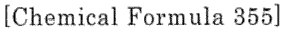

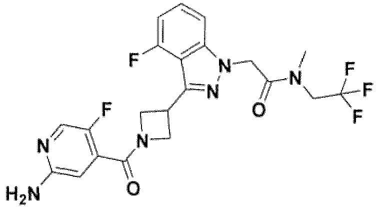

A mixture of Intermediate RE-20C (81 mg), dichloromethane (2 mL) and trifluoroacetic acid (0.7 mL) was stirred at room temperature for 2.5 hours. The solvent was removed under reduced pressure, and to the residue obtained was added toluene, followed by ethyl acetate and hexane. The solvent was removed under reduced pressure. A mixture of a portion of the residue obtained (41 mg), Compound RE-24 (21 mg), HOBt (16 mg), DIPEA (0.08 mL), DMF (1 mL) and EDCI·HCl (22 mg) was stirred at room temperature for 2.5 hours. The reaction solution was purified by silica gel column chromatography to afford the title compound (18 mg). MS (m/z): 483.2 [M+H]⁺

### Example 60 2-{3-[1-(2-amino-5-fluoropyridine-4-carbonyl)azetidin-3-yl]-1H-indazol-1-yl}-1-[(2R)-2-(trifluoromethyl)pyrrolidin-1-yl]ethan-1-one (Compound E-60)

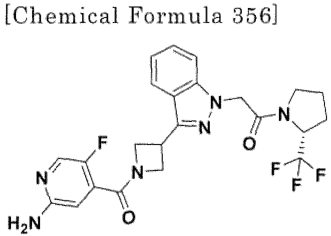

A mixture of Compound RE-117 (50 mg), (2R)-2-(trifluoromethyl)pyrrolidine hydrochloride (24 mg), HOBt (18 mg), DIPEA (0.07 mL), DMF (0.5 mL) and EDCI·HCl (26 mg) was stirred at room temperature overnight. The reaction solution was purified by silica gel column chromatography to afford the title compound (20 mg). MS (m/z): 491.0 [M+H]⁺

Each of the Example compounds listed in Tables 1 through 32 below (in the Tables, "XXX" in "E-XXX" denotes the Example number) was prepared by combining a Reference Example compound and a reaction fragment through condensation reaction, and if necessary, deprotection, and acid-base reaction, according to procedures similar to those described in the Examples above.

In the Tables, Example referred-to means that the compound in question was prepared by the method described in the Example number or by a method according to the preparation method thereof using the corresponding starting materials; for example, an Example compound whose Example number referred-to is 1 means that it was prepared by a method according to Example 1.

In the Tables, data refers to instrumental analysis data of such compounds, for example, mass spectrometry data (m/z values).

**[Table 1]**

| Compound number | Structural formula of compound (upper row) | Reaction fragment | Data |
|---|---|---|---|
| | Example referred-to (middle row) | | |
| | Compound of Reference Example used (lower row) | | |
| E-61 | 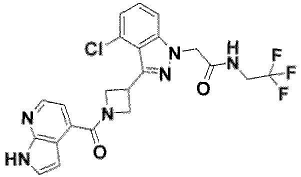 Example 17 | 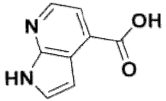 | MS(m/z) 491.1 [M+H]⁺ |
| | RE-30 | | |
| E-62 | 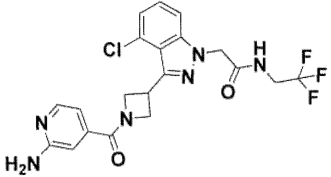 Example 2 step 1 | 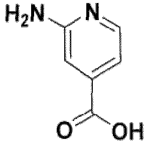 | MS(m/z) 467.5 [M+H]⁺ |
| | RE-30 | | |
| E-63 | 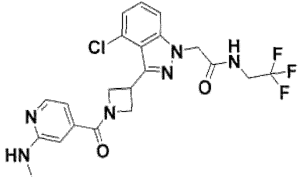 Example 17 | 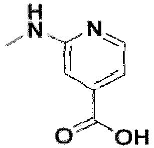 | MS(m/z) 481.3 [M+H]⁺ |
| | RE-30 | | |
| E-64 | 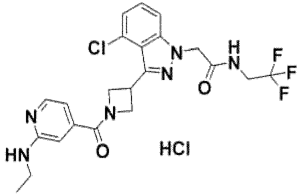 Example 1 | 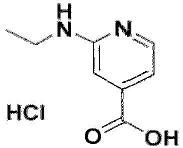 | MS(m/z) 495.5 [M+H]⁺ |
| | RE-30 | | |
| E-65 | 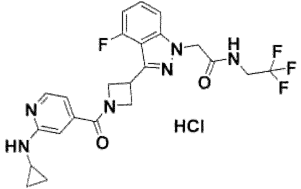 Example 1 | RE-23 | MS(m/z) 491.6 [M+H]⁺ |
| | RE-108 | | |

**[Table 2]**

| Compound number | Structural formula of compound (upper row) | Reaction fragment | Data |
|---|---|---|---|
| | Example referred-to (middle row) | | |
| | Compound of Reference Example used (lower row) | | |
| E-66 | 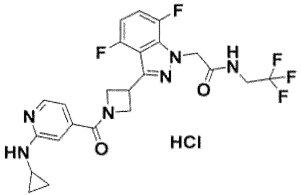 Example 1 | RE-23 | MS(m/z) 509.5 [M+H]⁺ |
| | RE-31 | | |
| E-67 | 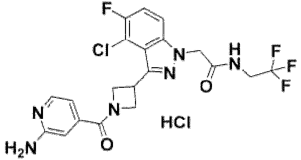 Example 2 | 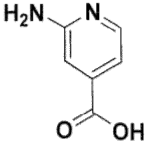 | MS(m/z) 485.4 [M+H]⁺ |
| | RE-1 | | |
| E-68 | 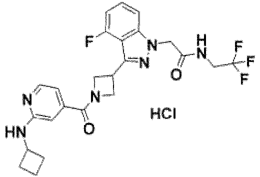 Example 1 | 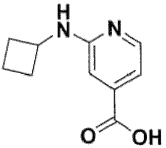 | MS(m/z) 505.5 [M+H]⁺ |
| | RE-108 | | |
| E-69 | 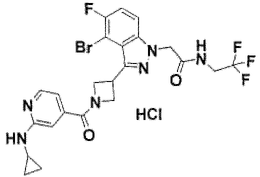 Example 1 | 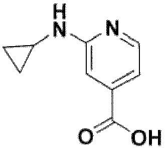 | MS(m/z) 569.4 [M+H]⁺ |
| | RE-32 | | |
| E-70 | 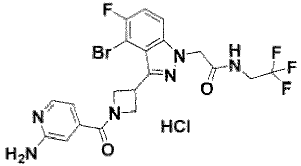 Example 2 | 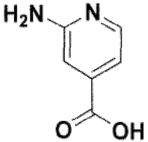 | MS(m/z) 529.4 [M+H]⁺ |
| | RE-32 | | |

**[Table 3]**

| Compound number | Structural formula of compound (upper row) | Reaction fragment | Data |
|---|---|---|---|
| | Example referred-to (middle row) | | |
| | Compound of Reference Example used (lower row) | | |
| E-71 | 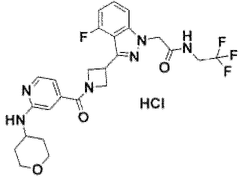 Example 2 | 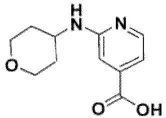 | MS(m/z) 535.2 [M+H]⁺ |
| | RE-108 | | |
| E-72 | 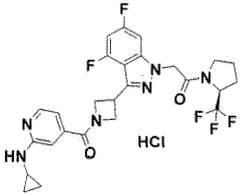 Example 2 | RE-23 | MS(m/z) 549.1 [M+H]⁺ |
| | RE-2 | | |
| E-74 | 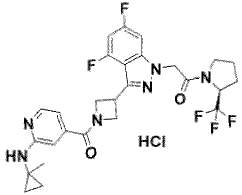 Example 2 | RE-28 | MS(m/z) 563.2 [M+H]⁺ |
| | RE-2 | | |
| E-75 | 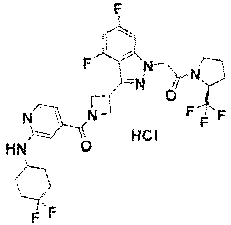 Example 2 | RE-29 | MS(m/z) 627.2 [M+H]⁺ |
| | RE-2 | | |
| E-77 | 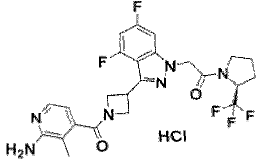 Example 2 | 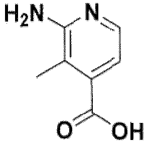 | MS(m/z) 523.2 [M+H]⁺ |
| | RE-2 | | |

**[Table 4]**

| Compound number | Structural formula of compound (upper row) Example referred-to (middle row) Compound of Reference Example used (lower row) | Reaction fragment | Data |
|---|---|---|---|
| E-78 | 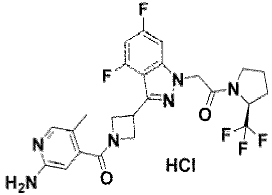 Example 2 | 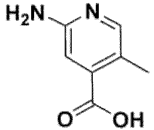 | MS(m/z) 523.2 [M+H]⁺ |
| | RE-2 | | |
| E-81 | 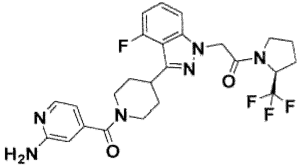 Example 2 step 1 | 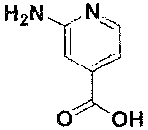 | MS(m/z) 519.2 [M+H]⁺ |
| | RE-71 | | |
| E-85 | 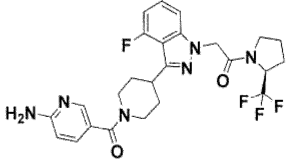 Example 2 step 1 | 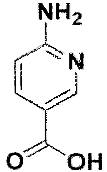 | MS(m/z) 519.2 [M+H]⁺ |
| | RE-71 | | |
| E-86 | 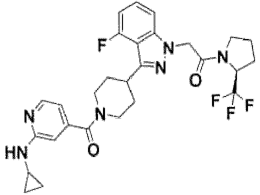 Example 2 step 1 | RE-23 | MS(m/z) 559.2 [M+H]⁺ |
| | RE-71 | | |
| E-87 | 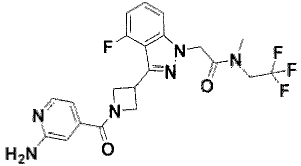 Example 1 step 1 | 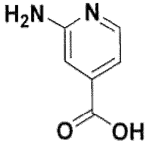 | MS(m/z) 465.1 [M+H]⁺ |
| | RE-20 | | |

**[Table 5]**

| Compound number | Structural formula of compound (upper row) | Reaction fragment | Data |
|---|---|---|---|
| | Example referred-to (middle row) | | |
| | Compound of Reference Example used (lower row) | | |
| E-88 | 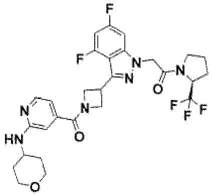 Example 2 step 1 | 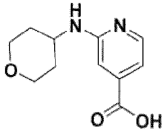 | MS(m/z) 593.2 [M+H]⁺ |
| | RE-2 | | |
| E-90 | 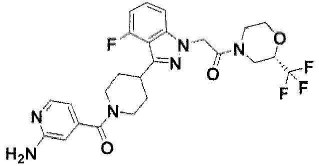 Example 11 | 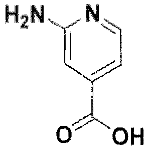 | MS(m/z) 535.2 [M+H]⁺ |
| | RE-72 | | |
| E-91 | 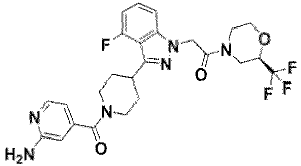 Example 11 | 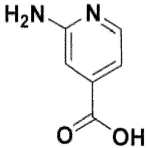 | MS(m/z) 535.2 [M+H]⁺ |
| | RE-73 | | |
| E-92 | 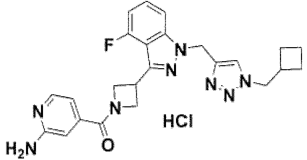 Example 52 | 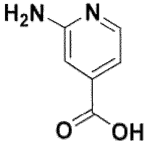 | MS(m/z) 461.1 [M+H]⁺ |
| | RE-92 | | |
| E-93 | 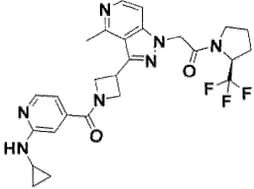 Example 53 | RE-23 | MS(m/z) 528.2 [M+H]⁺ |
| | RE-33 | | |

**[Table 6]**

| Compound number | Structural formula of compound (upper row) | Reaction fragment | Data |
|---|---|---|---|
| | Example referred-to. (middle row) Compound of Reference Example used (lower row) | | |
| E-94 | 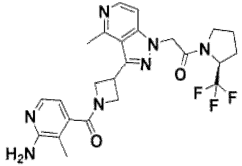 Example 53 RE-33 | RE-25 | MS(m/z) 502.1 [M+H]⁺ |
| E-97 | 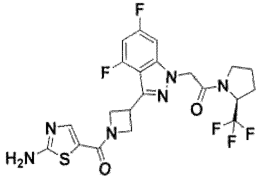 Example 1 step 1 RE-2 | 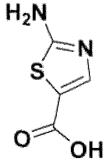 | MS(m/z) 515.1 [M+H]⁺ |
| E-98 | 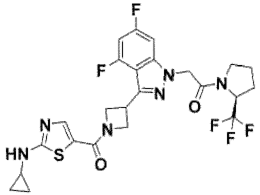 Example 1 step 1 RE-2 | 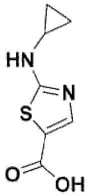 | MS(m/z) 555.1 [M+H]⁺ |
| E-99 | 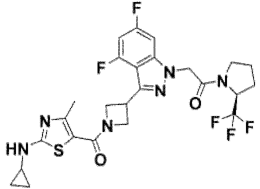 Example 1 step 1 RE-2 | 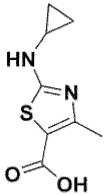 | MS(m/z) 569.1 [M+H]⁺ |
| E-100 | 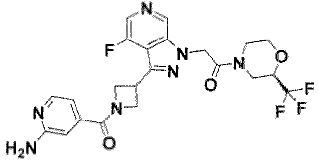 Example 55 RE-80 | 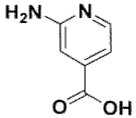 | MS(m/z) 508.1 [M+H]⁺ |

**[Table 7]**

| Compound number | Structural formula of compound (upper row) | Reaction fragment | Data |
|---|---|---|---|
| | Example referred-to (middle row) Compound of Reference Example used (lower row) | | |
| E-101 | 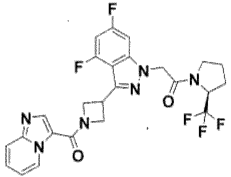 Example 2 step 1 RE-2 | 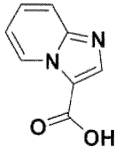 | MS(m/z) 533.1 [M+H]⁺ |
| E-102 | 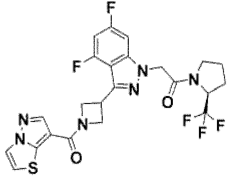 Example 2 step 1 RE-2 | 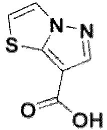 | MS(m/z) 539.0 [M+H]⁺ |
| E-103 | 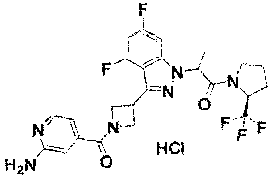 Example 2 RE-53 | 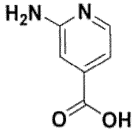 | MS(m/z) 523.3 [M+H]⁺ |
| E-104 | 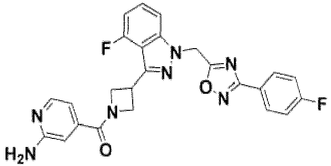 Example 55 RE-110 | 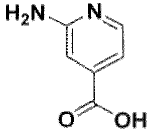 | MS(m/z) 488.2 [M+H]⁺ |
| E-105 | 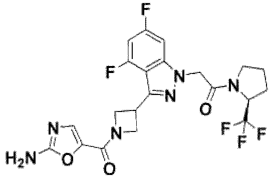 Example 2 step 1 RE-2 | 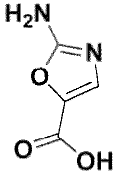 | MS(m/z) 499.2 [M+H]⁺ |

**[Table 8]**

| Compound number | Structural formula of compound (upper row) | Reaction fragment | Data |
|---|---|---|---|
| | Example referred-to (middle row) Compound of Reference Example used (lower row) | | |
| E-106 | 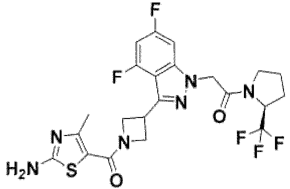 Example 2 step 1 RE-2 | 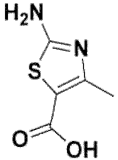 | MS(m/z) 529.3 [M+H]⁺ |
| E-107 | 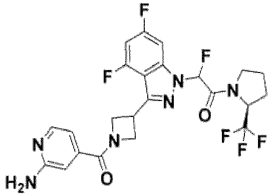 Example 52 step 1 RE-54 | 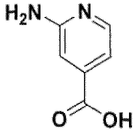 | MS(m/z) 527.2 [M+H]⁺ |
| E-108 | 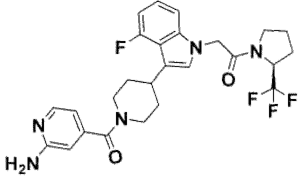 Example 2 step 1 RE-11 | 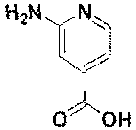 | MS(m/z) 518.3 [M+H]⁺ |
| E-109 | 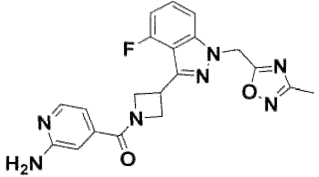 Example 55 RE-93 | 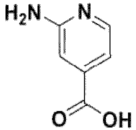 | MS(m/z) 408.2 [M+H]⁺ |
| E-110 | 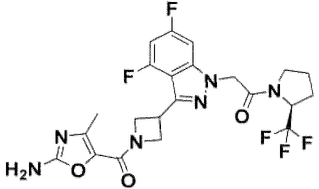 Example 2 RE-2 | 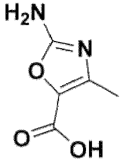 | MS(m/z) 513.2 [M+H]⁺ |

**[Table 9]**

| Compound number | Structural formula of compound (upper row) | Reaction fragment | Data |
|---|---|---|---|
| | Example referred-to (middle row) Compound of Reference Example used (lower row) | | |
| E-112 | 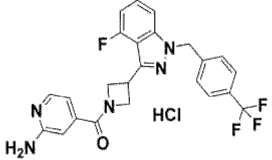 Example 54 RE-94 | 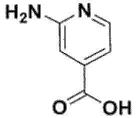 | MS(m/z) 470.2 [M+H]⁺ |
| E-113 | 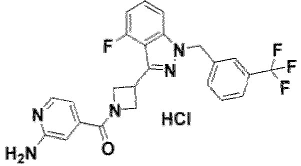 Example 54 RE-95 | 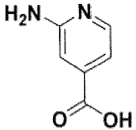 | MS(m/z) 470.2 [M+H]⁺ |
| E-114 | 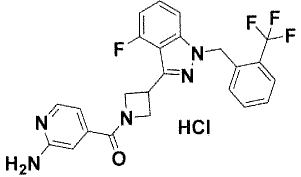 Example 54 RE-96 | 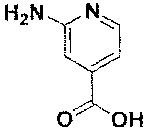 | MS(m/z) 470.2 [M+H]⁺ |
| E-115 | 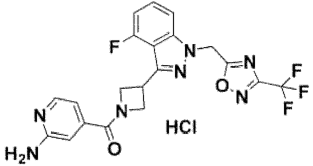 Example 54 RE-97 | 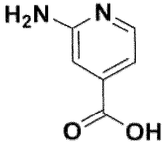 | MS(m/z) 462.2 [M+H]⁺ |
| E-116 | 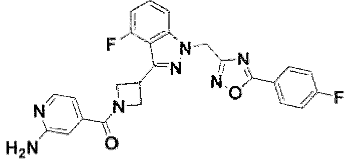 Example 55 RE-98 | 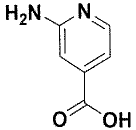 | MS(m/z) 488.3 [M+H]⁺ |

**(Table 10]**

| Compound number | Structural formula of compound (upper row) | Reaction fragment | Data |
|---|---|---|---|
| | Example referred-to (middle row) Compound of Reference Example used (lower row) | | |
| E-117 | 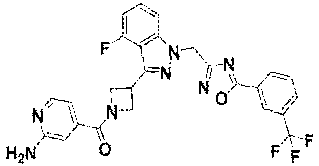 Example 55 RE-99 | 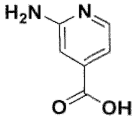 | MS(m/z) 538.2 [M+H]⁺ |
| E-118 | 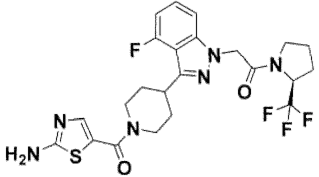 Example 2 step 1 RE-71 | 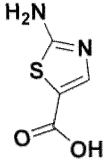 | MS(m/z) 525.2 [M+H]⁺ |
| E-119 | 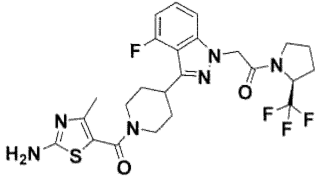 Example 2 step 1 RE-71 | 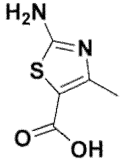 | MS(m/z) 539.2 [M+H]⁺ |
| E-120 | 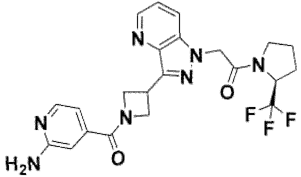 Example 53 RE-34 | 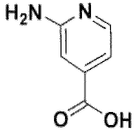 | MS(m/z) 474.2 [M+H]⁺ |
| E-121 | 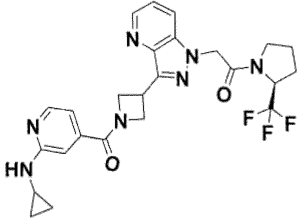 Example 53 RE-34 | RE-23 | MS(m/z) 514.2 [M+H]⁺ |

**[Table 11]**

| Compound number | Structural formula of compound (upper row) | Reaction fragment | Data |
|---|---|---|---|
| | Example referred-to (middle row) Compound of Reference Example used (lower row) | | |
| E-122 | 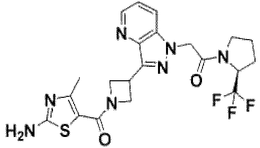 Example 53 RE-34 | 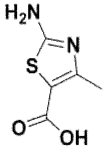 | MS(m/z) 494.1 [M+H]⁺ |
| E-124 | 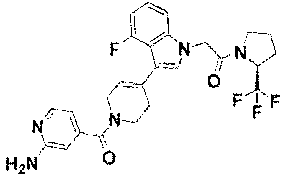 Example 11 RE-75 | 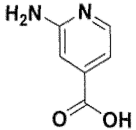 | MS(m/z) 516.3 [M+H]⁺ |
| E-128 | 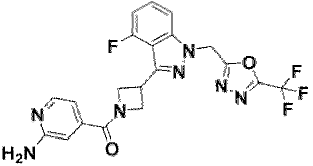 Example 55 RE-100 | 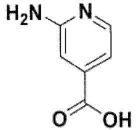 | MS(m/z) 462.1 [M+H]⁺ |
| E-129 | 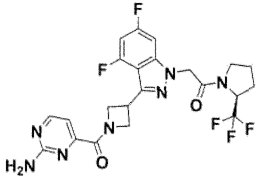 Example 2 step 1 RE-2 | 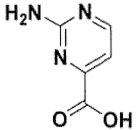 | MS(m/z) 510.2 [M+H]⁺ |
| E-130 | 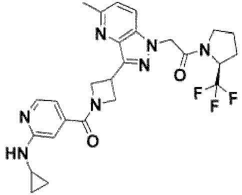 Example 2 step 1 RE-13 | RE-23 | MS(m/z) 528.3 [M+H]⁺ |

**[Table 12]**

| Compound number | Structural formula of compound (upper row) | Reaction fragment | Data |
|---|---|---|---|
| | Example referred-to (middle row) Compound of Reference Example used (lower row) | | |
| E-131 | 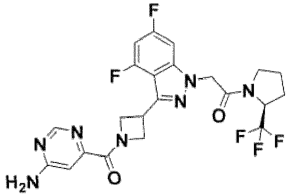 Example 2 step 1 RE-2 | 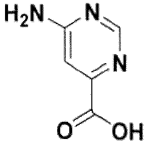 | MS(m/z) 510.2 [M+H]⁺ |
| E-132 | 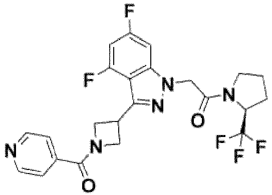 Example 2 step 1 RE-2 | 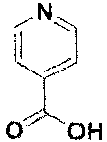 | MS(m/z) 494.2 [M+H]⁺ |
| E-133 | 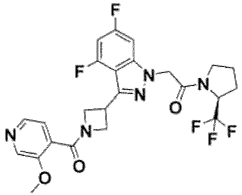 Example 2 step 1 RE-2 | 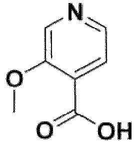 | MS(m/z) 524.2 [M+H]⁺ |
| E-134 | 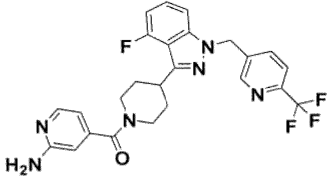 Example 55 RE-74 | 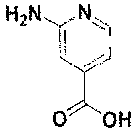 | MS(m/z) 499.2 [M+H]⁺ |
| E-135 | 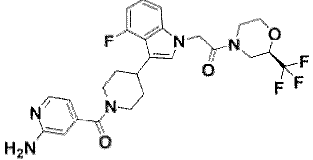 Example 11 RE-76 | 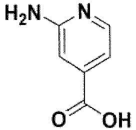 | MS(m/z) 534.3 [M+H]⁺ |

**[Table 13]**

| Compound number | Structural formula of compound (upper row) | Reaction fragment | Data |
|---|---|---|---|
| | Example referred-to (middle row) Compound of Reference Example used (lower row) | | |
| E-136 | 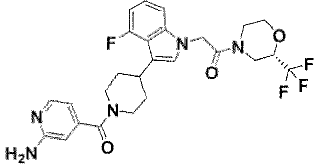 Example 11 RE-77 | 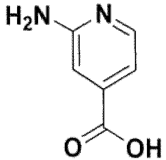 | MS(m/z) 534.3 [M+H]⁺ |
| E-137 | 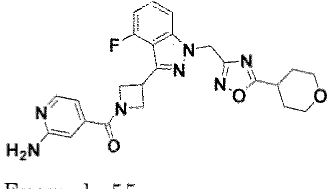 Example 55 RE-101 | 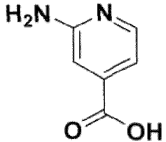 | MS(m/z) 478.3 [M+H]⁺ |
| E-138 | 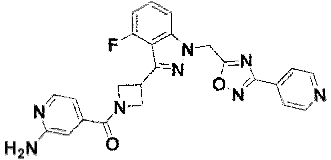 Example 55 RE-111 | 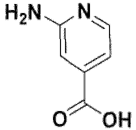 | MS(m/z) 471.2 [M+H]⁺ |
| E-139 | 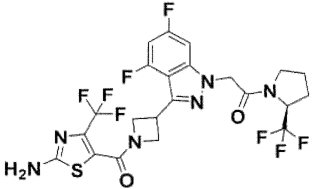 Example 3 RE-3 | 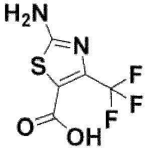 | MS(m/z) 583.1 [M+H]⁺ |
| E-140 | 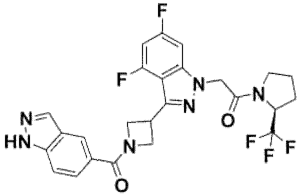 Example 17 RE-3 | 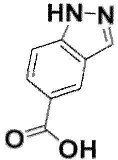 | MS(m/z) 533.2 [M+H]⁺ |

**[Table 14]**

| Compound number | Example referred-to (middle row) Compound of Reference Example used (lower row) | Reaction fragment | Data |
|---|---|---|---|
| | Structural formula of compound (upper row) | | |
| E-141 | 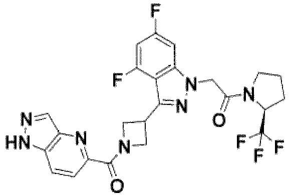 Example 17 RE-3 | 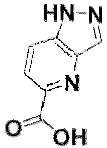 | MS(m/z) 534.3 [M+H]⁺ |
| E-142 | 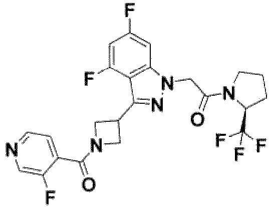 Example 3 RE-2 | 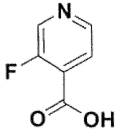 | MS(m/z) 512.2 [M+H]⁺ |
| E-143 | 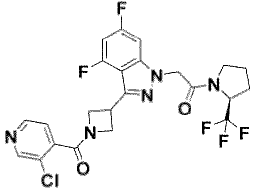 Example 3 RE-2 | 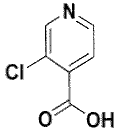 | MS(m/z) 528.2 [M+H]⁺ |
| E-144 | 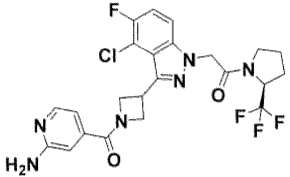 Example 2 step 1 RE-50 | 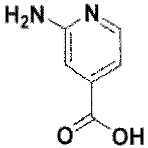 | MS(m/z) 525.2 [M+H]⁺ |
| E-145 | 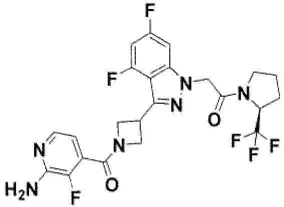 Example 3 RE-2 | 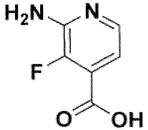 | MS(m/z) 527.2 [M+H]⁺ |

**[Table 15]**

| Compound number | Structural formula of compound (upper row) | Reaction fragment | Data |
|---|---|---|---|
| | Example referred-to (middle row) Compound of Reference Example used (lower row) | | |
| E-146 | 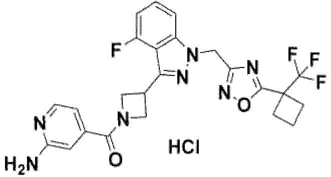 Example 55 RE-102 | 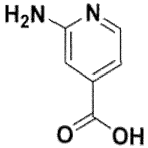 | MS(m/z) 516.2 [M+H]⁺ |
| E-147 | 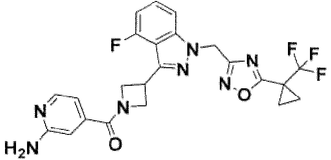 Example 55 RE-103 | 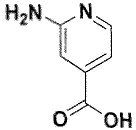 | MS(m/z) 502.2 [M+H]⁺ |
| E-148 | 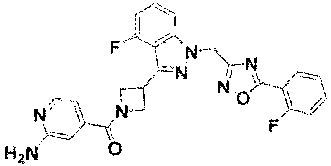 Example 55 RE-104 | 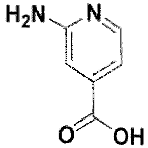 | O MS(m/z) 488.2 [M+H]⁺ |
| E-149 | 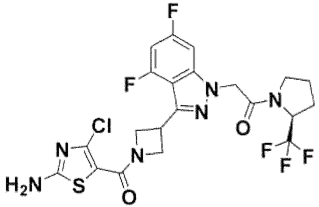 Example 3 RE-2 | 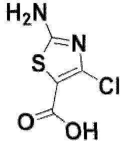 | MS(m/z) 549.1 [M+H]⁺ |
| E-150 | 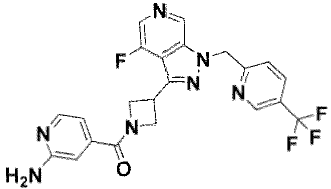 Example 55 RE-82 | 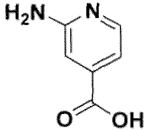 | MS(m/z) 472.2 [M+H]⁺ |

**[Table 16]**

| Compound number | Structural formula of compound (upper row) | Reaction fragment | Data |
|---|---|---|---|
| | Example referred-to (middle row) Compound of Reference Example used (lower row) | | |
| E-151 | 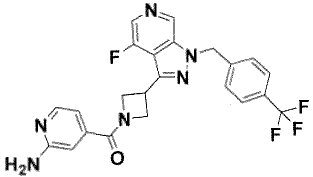 Example 55 RE-83 | 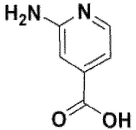 | MS(m/z) 471.2 [M+H]⁺ |
| E-152 | 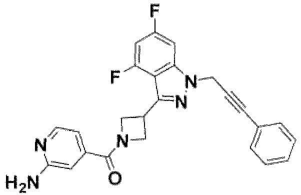 Example 57 RE-56 | 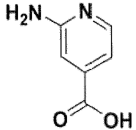 | MS(m/z) 444.2 [M+H]⁺ |
| E-153 | 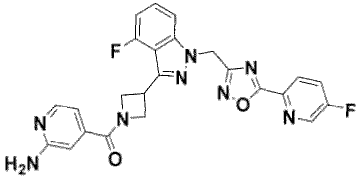 Example 55 RE-105 | 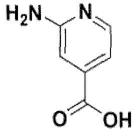 | MS(m/z) 489.2 [M+H]⁺ |
| E-154 | 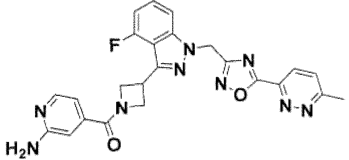 Example 55 RE-106 | 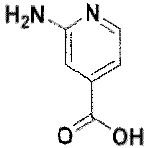 | MS(m/z) 486.2 [M+H]⁺ |
| E-155 | 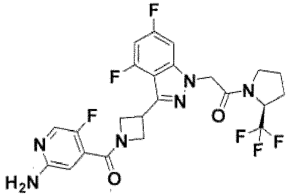 Example 3 RE-2 | RE-24 | MS(m/z) 527.2 [M+H]⁺ |

**[Table 17]**

| Compound number | Structural formula of compound (upper row) | Reaction fragment | Data |
|---|---|---|---|
| | Example referred-to (middle row) Compound of Reference Example used (lower row) | | |
| E-156 | 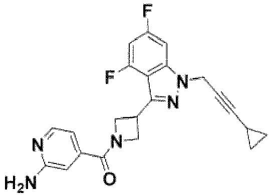 Example 57 RE-57 | 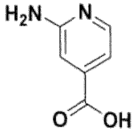 | MS(m/z) 408.2 [M+H]⁺ |
| E-157 | 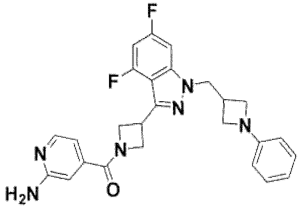 Example 55 RE-58 | 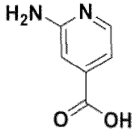 | MS(m/z) 475.2 [M+H]⁺ |
| E-160 | 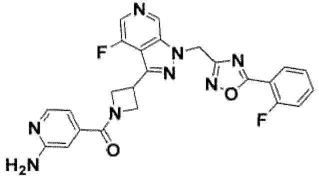 Example 55 RE-84 | 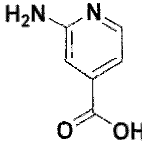 | MS(m/z) 489.2 [M+H]⁺ |
| E-162 | 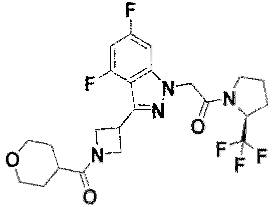 Example 3 RE-3 | 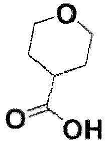 | MS(m/z) 501.2 [M+H]⁺ |
| E-163 | 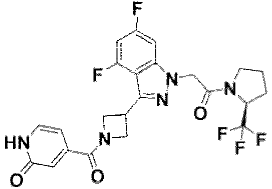 Example 3 RE-2 | 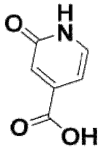 | MS(m/z) 510.2 [M+H]⁺ |

**[Table 18]**

| Compound number | Example referred-to (middle row) Compound of Reference Example used (lower row) | Reaction fragment | Data |
|---|---|---|---|
| | Structural formula of compound (upper row) | | |
| E-164 | 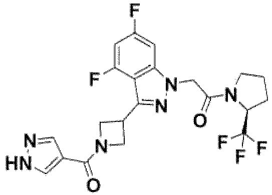 Example 3 RE-2 | 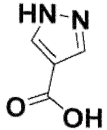 | MS(m/z) 483.2 [M+H]⁺ |
| E-165 | 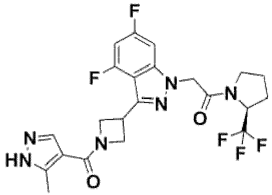 Example 3 RE-2 | 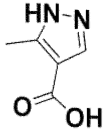 | MS(m/z) 497.2 [M+H]⁺ |
| E-166 | 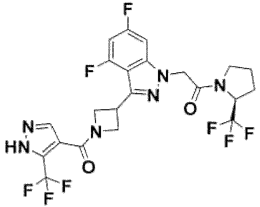 Example 3 RE-2 | 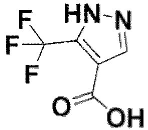 | MS(m/z) 551.2 [M+H]⁺ |
| E-167 | 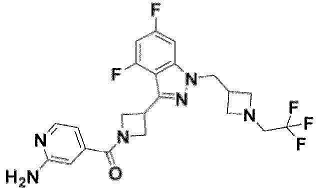 Example 55 RE-59 | 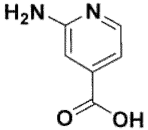 | MS(m/z) 481.2 [M+H]⁺ |
| E-168 | 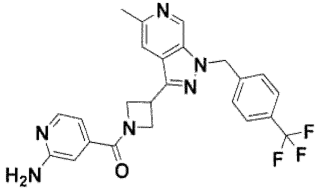 Example 12 RE-35 | 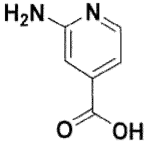 | MS(m/z) 467.2 [M+H]⁺ |

**[Table 19]**

| Compound number | Structural formula of compound (upper row) | Reaction fragment | Data |
|---|---|---|---|
| | Example referred-to (middle row) Compound of Reference Example used (lower row) | | |
| E-169 | 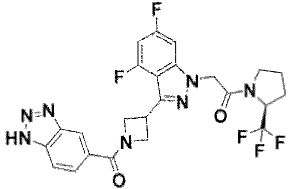 Example 3 RE-2 | 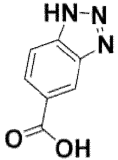 | MS(m/z) 534.2 [M+H]⁺ |
| E-170 | 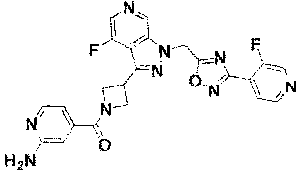 Example 55 RE-85 | 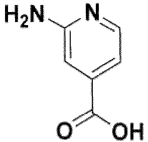 | MS(m/z) 490.2 [M+H]⁺ |
| E-171 | 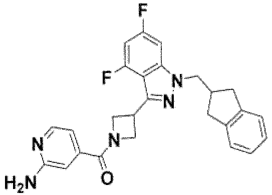 Example 12 RE-60 | 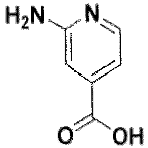 | MS(m/z) 460.3 [M+H]⁺ |
| E-173 | 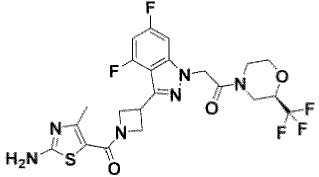 Example 3 RE-61 | 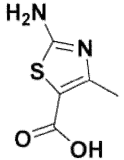 | MS(m/z) 545.2 [M+H]⁺ |
| E-174 | 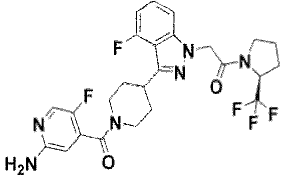 Example 3 RE-71 | RE-24 | MS(m/z) 537.3 [M+H]⁺ |

**[Table 20]**

| Compound number | Structural formula of compound (upper row) | Reaction fragment | Data |
|---|---|---|---|
| | Example referred-to (middle row) Compound of Reference Example used (lower row) | | |
| E-178 | 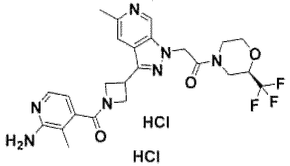 Example 19 RE-36 | RE-25 | MS(m/z) 518.2 [M+H]⁺ |
| E-180 | 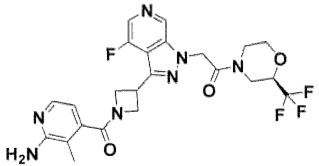 Example 19 RE-86 | RE-25 | MS(m/z) 522.2 [M+H]⁺ |
| E-181 | 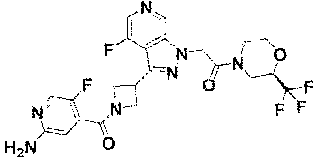 Example 3 RE-86 | RE-24 | MS(m/z) 526.2 [M+H]⁺ |
| E-183 | 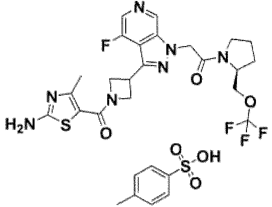 Example 58 RE-87 | 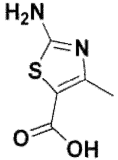 | MS(m/z) 542.2 [M+H]⁺ |
| E-184 | 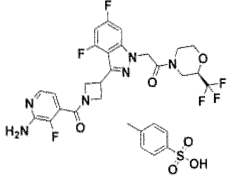 Example 58 RE-61 | 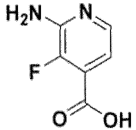 | MS(m/z) 543.2 [M+H]⁺ |

**[Table 21]**

| Compound number | Structural formula of compound (upper row) | Reaction fragment | Data |
|---|---|---|---|
| | Example referred-to (middle row) Compound of Reference Example used (lower row) | | |
| E-185 | 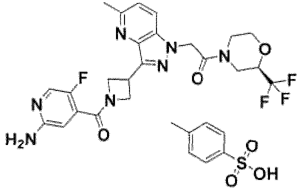 Example 58 RE-78 | RE-24 | MS(m/z) 522.3 [M+H]⁺ |
| E-186 | 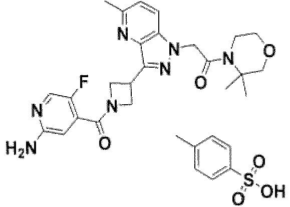 Example 58 RE-79 | RE-24 | MS(m/z) 482.3 [M+H]⁺ |
| E-187 | 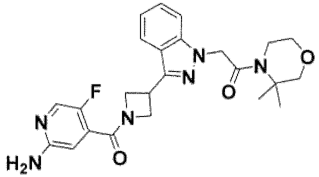 Example 3 RE-8 | RE-24 | MS(m/z) 467.2 [M+H]⁺ |
| E-188 | 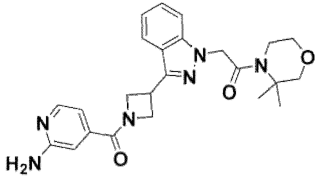 Example 2 step 1 RE-8 | 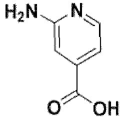 | MS(m/z) 449.2 [M+H]⁺ |
| E-190 | 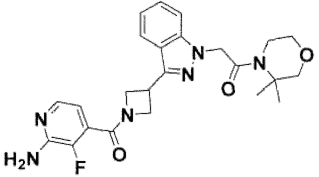 Example 3 RE-8 | 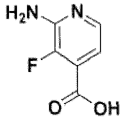 | MS(m/z) 467.2 [M+H]⁺ |

**[Table 22]**

| Compound number | Structural formula of compound (upper row) | Reaction fragment | Data |
|---|---|---|---|
| | Example referred-to (middle row) Compound of Reference Example used (lower row) | | |
| E-191 | 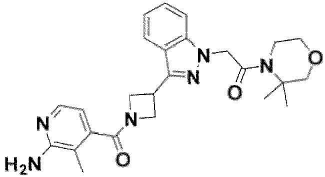 Example 2 step 1 RE-8 | RE-25 | MS(m/z) 463.2 [M+H]⁺ |
| E-192 | 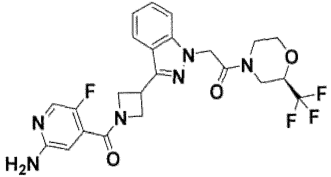 Example 3 RE-9 | RE-24 | MS(m/z) 507.2 [M+H]⁺ |
| E-193 | 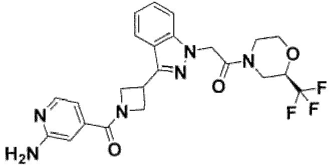 Example 2 step 1 RE-9 | 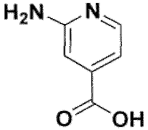 | MS(m/z) 489.2 [M+H]⁺ |
| E-194 | 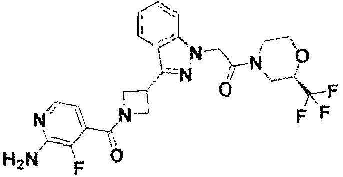 Example 3 RE-9 | 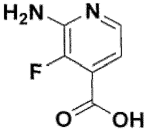 | MS(m/z) 507.2 [M+H]⁺ |
| E-195 | 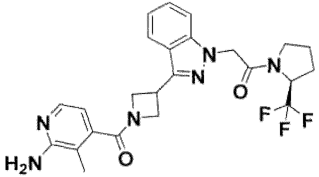 Example 2 step 1 RE-4 | RE-25 | MS(m/z) 487.2 [M+H]⁺ |

**[Table 23]**

| Compound number | Example referred-to (middle row) Compound of Reference Example used (lower row) | Reaction fragment | Data |
|---|---|---|---|
| | Structural formula of compound (upper row) | | |
| E-196 | 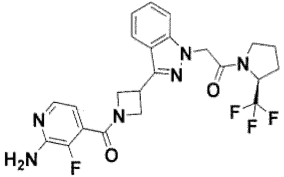 Example 3 RE-4 | 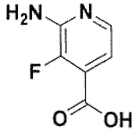 | MS(m/z) **491.1** [M+H]⁺ |
| E-197 | 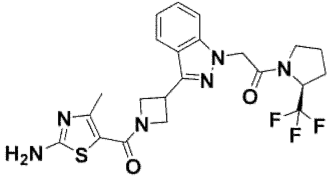 Example 3 RE-4 | 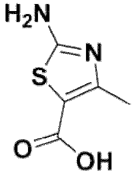 | MS(m/z) 493.1 [M+H]⁺ |
| E-198 | 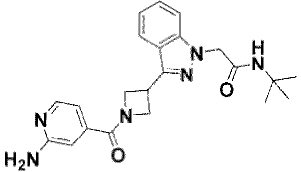 Example 2 step 1 RE-63 | 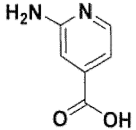 | MS(m/z) 407.2 [M+H]⁺ |
| E-199 | 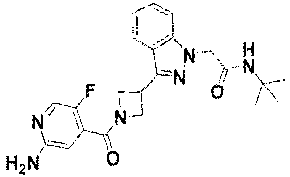 Example 3 RE-63 | RE-24 | MS(m/z) 425.2 [M+H]⁺ |
| E-200 | 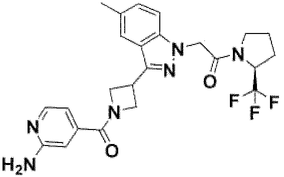 Example 2 step 1 RE-37 | 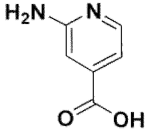 | MS(m/z) 487.2 [M+H]⁺ |

**[Table 24]**

| Compound number | Structural formula of compound (upper row) | Reaction fragment | Data |
|---|---|---|---|
| | Example referred-to (middle row) Compound of Reference Example used (lower row) | | |
| E-201 | 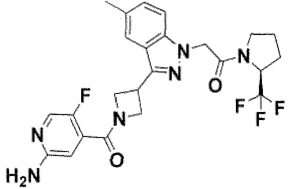 Example 3 RE-37 | RE-24 | MS(m/z) 505.2 [M+H]⁺ |
| E-202 | 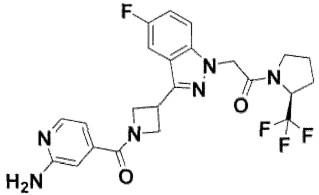 Example 2 step 1 RE-38 | 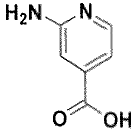 | MS(m/z) 491.2 [M+H]⁺ |
| E-203 | 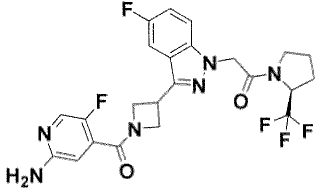 Example 3 RE-38 | RE-24 | MS(m/z) 509.2 [M+H]⁺ |
| E-204 | 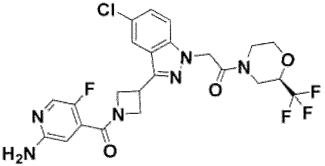 Example 59 RE-67 | RE-24 | MS(m/z) 541.2 [M+H]+ |
| E-205 | 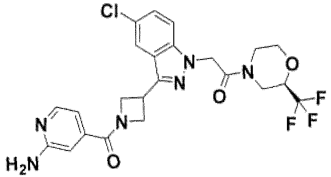 Example 55 RE-67 | 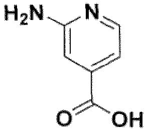 | MS(m/z) 523.2 [M+H]⁺ |

**[Table 25]**

| Compound number | Structural formula of compound (upper row) | Reaction fragment | Data |
|---|---|---|---|
| | Example referred-to (middle row) Compound of Reference Example used (lower row) | | |
| E-206 | 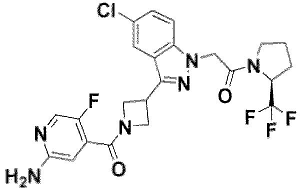 Example 3 RE-68 | RE-24 | MS(m/z) 525.2 [M+H]⁺ |
| E-207 | 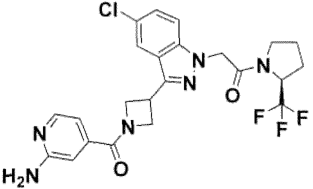 Example 2 step 1 RE-68 | 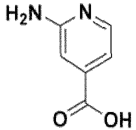 | MS(m/z) 507.2 [M+H]⁺ |
| E-210 | 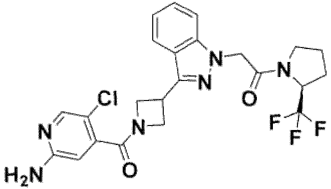 Example 3 RE-4 | 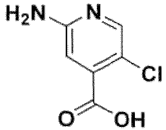 | MS(m/z) 507.2 [M+H]⁺ |
| E-215 | 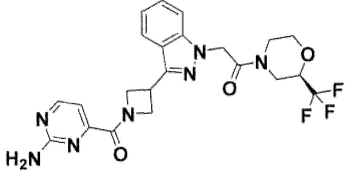 Example 3 RE-9 | 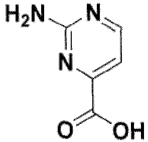 | MS(m/z) 490.2 [M+H]⁺ |
| E-216 | 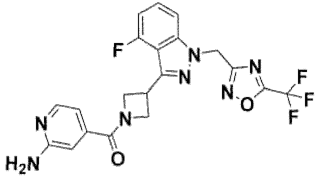 Example 2 step 1 RE-107 | 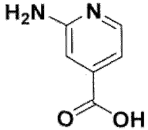 | MS(m/z) 462.2 [M+H]⁺ |

**[Table 26]**

| Compound number | Structural formula of compound (upper row) | Reaction fragment | Data |
|---|---|---|---|
| | Example referred-to (middle row) Compound of Reference Example used (lower row) | | |
| E-217 | 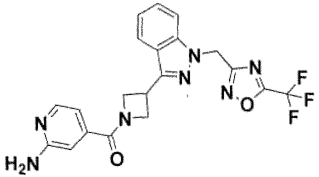 Example 2 step 1 RE-88 | 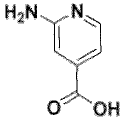 | MS(m/z) 444.1 [M+H]⁺ |
| E-218 | 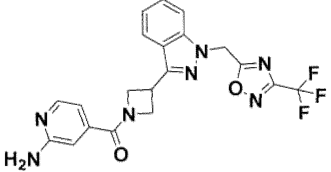 Example 2 step 1 RE-17 | 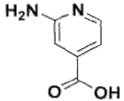 | MS(m/z) 444.1 [M+H]⁺ |
| E-220 | 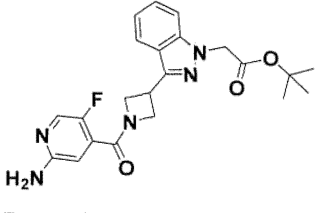 Example 3 RE-39 | RE-24 | MS(m/z) 426.2 [M+H]⁺ |
| E-238 | 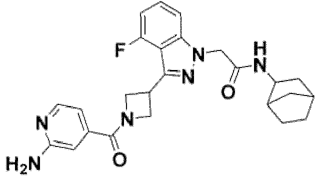 Example 55 RE-112 | 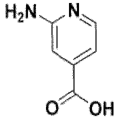 | MS(m/z) 463.3 [M+H]⁺ |
| E-239 | 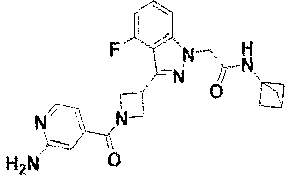 Example 55 RE-113 | 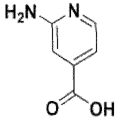 | MS(m/z) 435.3 [M+H]⁺ |

**[Table 27]**

| Compound number | Structural formula of compound (u pper row) | Reaction fragment | Data |
|---|---|---|---|
| | Example referred-to (middle row) Compound of Reference Example used (lower row) | | |
| E-241 | 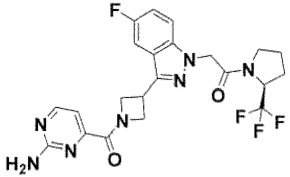 Example 3 RE-38 | 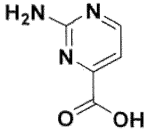 | MS(m/z) 492.2 [M+H]⁺ |
| E-242 | 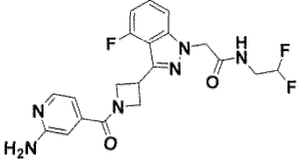 Example 55 RE-114 | 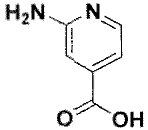 | MS(m/z) 433.2 [M+H]⁺ |
| E-267 | 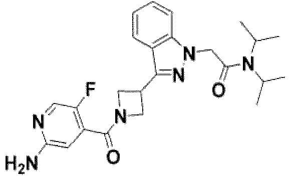 Example 3 RE-64 | RE-24 | MS(m/z) 453.3 [M+H]⁺ |
| E-268 | 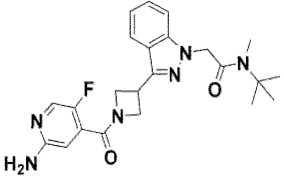 Example 3 RE-65 | RE-24 | MS(m/z) 439.2 [M+H]⁺ |
| E-270 | 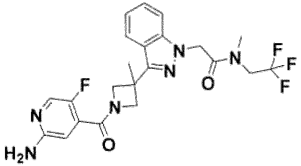 Example 59 RE-70 | RE-24 | MS(m/z) 479.4 [M+H]⁺ |

**[Table 28]**

| Compound number | Structural formula of compound (upper row) | Reaction fragment | Data |
|---|---|---|---|
| | Example referred-to (middle row) Compound of Reference Example used (lower row) | | |
| E-271 | 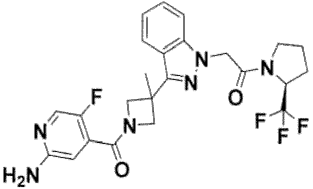 Example 3 RE-7 | RE-24 | MS(m/z) 505.3 [M+H]⁺ |
| E-273 | 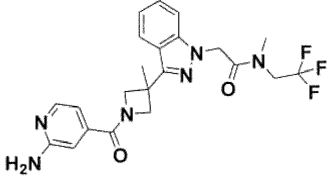 Example 55 RE-70 | 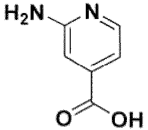 | MS(m/z) 461.2 [M+H]⁺ |
| E-274 | 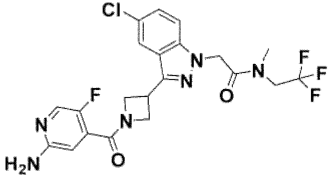 Example 3 RE-5 | RE-24 | MS(m/z) 499.3 [M+H]⁺ |
| E-275 | 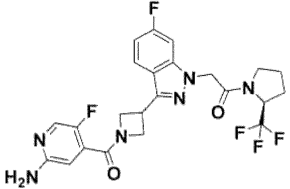 Example 3 RE-69 | RE-24 | MS(m/z) 509.3 [M+H]⁺ |
| E-276 | 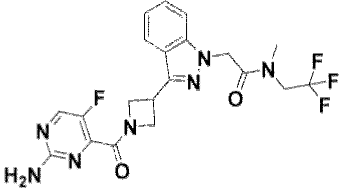 Example 3 RE-66 | RE-26 | MS(m/z) 466.3 [M+H]⁺ |

**[Table 29]**

| Compound number | Structural formula of compound (upper row) | Reaction fragment | Data |
|---|---|---|---|
| | Example referred-to (middle row) Compound of Reference Example used (lower row) | | |
| E-277 | 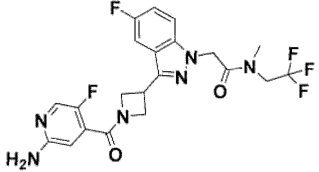 Example 3 RE-42 | RE-24 | MS(m/z) 483.2 [M+H]⁺ |
| E-278 | 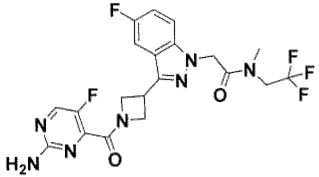 Example 3 RE-42 | RE-26 | MS(m/z) 484.2 [M+H]⁺ |
| E-279 | 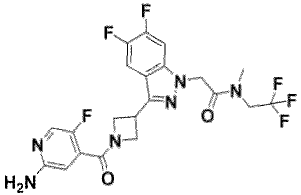 Example 3 RE-43 | RE-24 | MS(m/z) 501.3 [M+H]⁺ |
| E-280 | 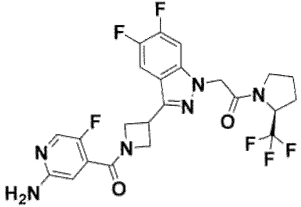 Example 3 RE-44 | RE-24 | MS(m/z) 527.3 [M+H]⁺ |
| E-281 | 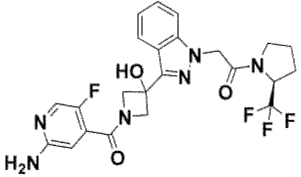 Example 3 RE-45 | RE-24 | MS(m/z) 507.3 [M+H]⁺ |

**[Table 30]**

| Compound number | Structural formula of compound (upper row) | Reaction fragment | Data |
|---|---|---|---|
| | Example referred-to (middle row) Compound of Reference Example used (lower row) | | |
| E-282 | 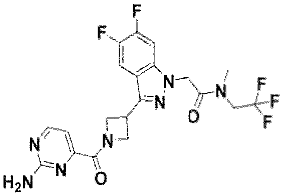 Example 3 RE-43 | 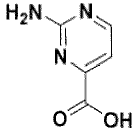 | MS(m/z) 484.2 [M+H]⁺ |
| E-283 | 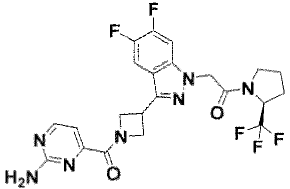 Example 3 RE-44 | 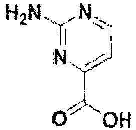 | MS(m/z) 510.2 [M+H]⁺ |
| E-284 | 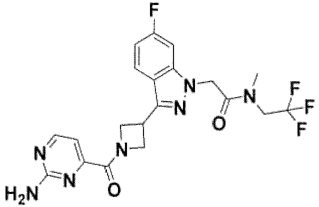 Example 3 RE-6 | 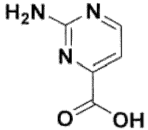 | MS(m/z) 466.2 [M+H]⁺ |
| E-285 | 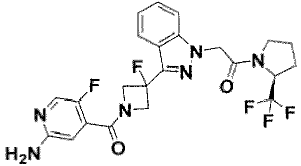 Example 59 RE-46 | RE-24 | MS(m/z) 509.2 [M+H]⁺ |
| E-286 | 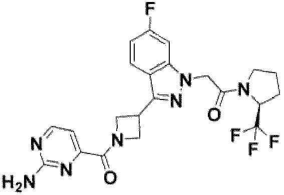 Example 3 RE-69 | 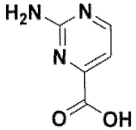 | MS(m/z) 492.2 [M+H]⁺ |

**[Table 31]**

| Compound number | Structural formula of compound (upper row) Example referred-to (middle row) Compound of Reference Example used (lower row) | Reaction fragment | Data |
|---|---|---|---|
| E-287 | 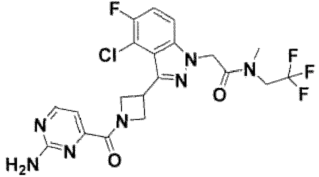 Example 3 | 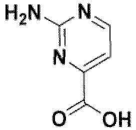 | MS(m/z) 500.2 [M+H]⁺ |
| | RE-51 | | |
| E-288 | 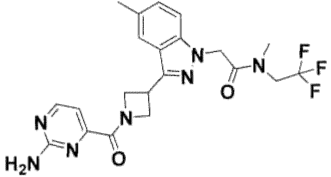 Example 3 | 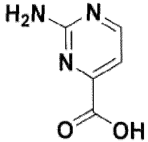 | MS(m/z) 462.3 [M+H]⁺ |
| | RE-47 | | |
| E-289 | 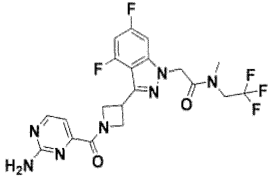 Example 3 | 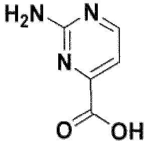 | MS(m/z) 484.2 [M+H]⁺ |
| | RE-62 | | |
| E-290 | 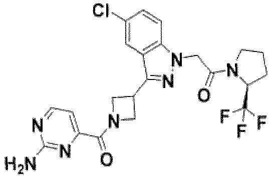 Example 3 | 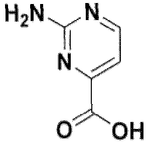 | MS(m/z) 508.2 [M+H]⁺ |
| | RE-68 | | |
| E-291 | 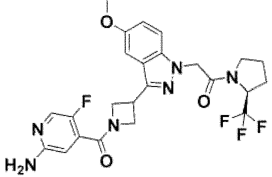 Example 59 | RE-24 | MS(m/z) 521.2 [M+H]⁺ |
| | RE-48 | | |

**[Table 32]**

| Compound number | Structural formula of compound (upper row) Example referred-to (middle row) Compound of Reference Example used (lower row) | Reaction fragment | Data |
|---|---|---|---|
| E-292 | 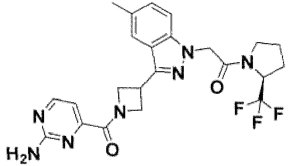 Example 3 | 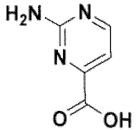 | MS(m/z) 488.2 [M+H]⁺ |
| | RE-37 | | |
| E-293 | 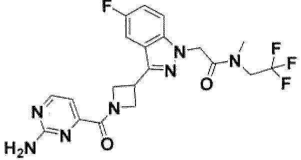 Example 3 | 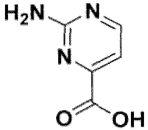 | MS(m/z) 466.2 [M+H]⁺ |
| | RE-42 | | |
| E-294 | 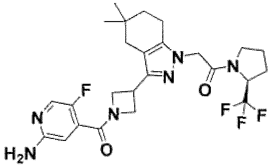 Example 3 | RE-24 | MS(m/z) 523.3 [M+H]⁺ |
| | RE-49 | | |
| E-295 | 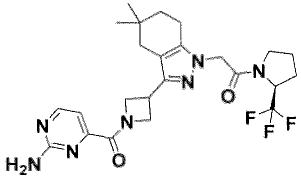 Example 3 | 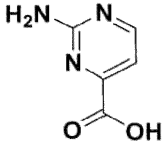 | MS(m/z) 506.4 [M+H]⁺ |
| | RE-49 | | |

Similarly, each of the Example compounds listed in Tables 33 through 48 below (in the Tables, "XXX" in "E-XXX" denotes the Example number) was prepared by combining a Reference Example compound and a reaction fragment through condensation reaction, and if necessary, deprotection, and acid-base reaction, according to procedures similar to those described in the Examples above.

**[Table 33]**

| Compound number | Structural formula of compound (upper row) Example referred-to (middle row) Compound of Reference Example used (lower row) | Reaction fragment | Data |
|---|---|---|---|
| E-73 | 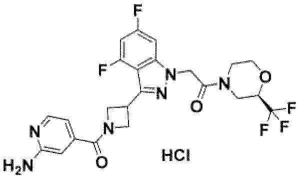 Example 51 | 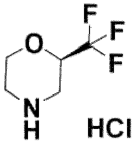 | MS(m/z) 525.1 [M+H]⁺ |
| | RE-116 | | |
| E-76 | 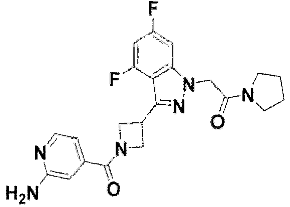 Example 50 | pyrrolidine | MS(m/z) 441.1 [M+H]⁺ |
| | RE-116 | | |
| E-79 | 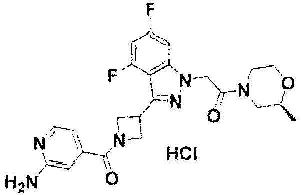 Example 51 | 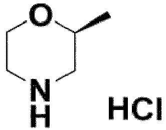 | MS(m/z) 471.2 [M+H]⁺ |
| | RE-116 | | |
| E-80 | 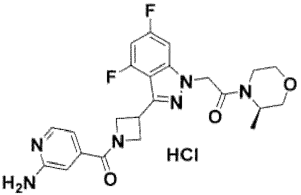 Example 51 | 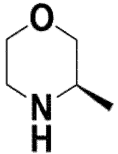 | MS(m/z) 471.2 [M+H]⁺ |
| | RE-116 | | |
| E-82 | 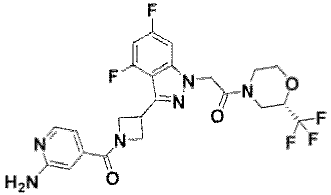 Example 50 | 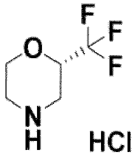 | MS(m/z) 525.1 [M+H]⁺ |
| | RE-116 | | |

**[Table 34]**

| Compound number | Structural formula of compound (upper row) Example referred-to (middle row) Compound of Reference Example used (lower row) | Reaction fragment | Data |
|---|---|---|---|
| E-83 | 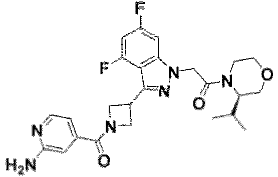 Example 50 | 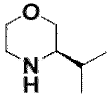 | MS(m/z) 499.2 [M+H]⁺ |
| | RE-116 | | |
| E-84 | 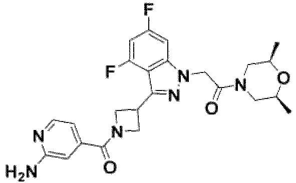 Example 50 | 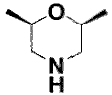 | MS(m/z) 485.2 [M+H]⁺ |
| | RE-116 | | |
| E-89 | 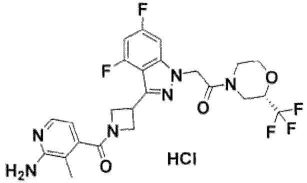 Example 51 | 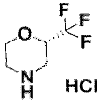 | MS(m/z) 539.2 [M+H]⁺ |
| | RE-119 | | |
| E-95 | 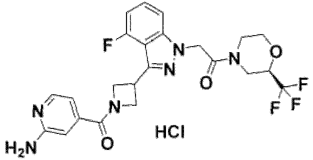 Example 51 | 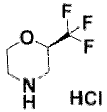 | MS(m/z) 507.2 [M+H]⁺ |
| | RE-118 | | |
| E-96 | 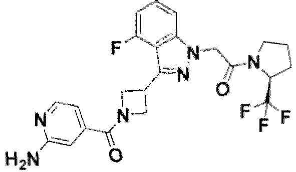 Example 50 | 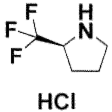 | MS(m/z) 491.2 [M + H] + |
| | RE-118 | | |

**[Table 35]**

| Compound number | Structural formula of compound (upper row) Example referred-to (middle row) Compound of Reference Example used (lower row) | Reaction fragment | Data |
|---|---|---|---|
| E-111 | 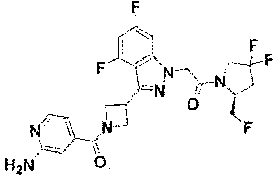 Example 50 | 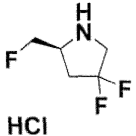 | MS(m/z) 509.2 [M+H]+ |
| | RE-116 | | |
| E-123 | 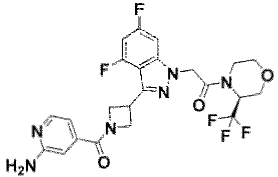 Example 50 | 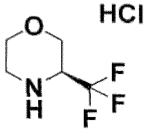 | MS(m/z) 525.2 [M+H]+ |
| | RE-116 | | |
| E-125 | 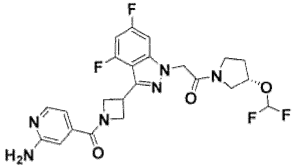 Example 50 | 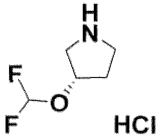 | MS(m/z) 507.3 (M + H) + |
| | RE-116 | | |
| E-126 | 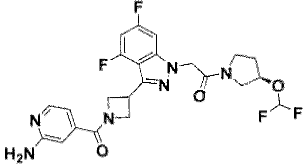 Example 50 | 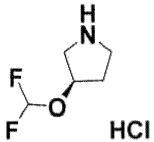 | MS(m/z) 507.3 [M+H]+ |
| | RE-116 | | |
| E-127 | 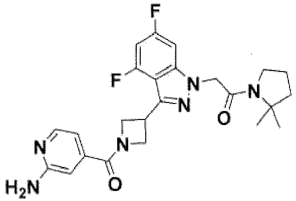 Example 50 | 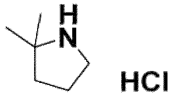 | MS(m/z) 469.3 [M+H]+ |
| | RE-116 | | |

**[Table 36]**

| Compound number | Structural formula of compound (upper row) | Reaction fragment | Data |
|---|---|---|---|
| | Example referred-to (middle row) | | |
| | Compound of Reference Example used (lower row) | | |
| E-158 | 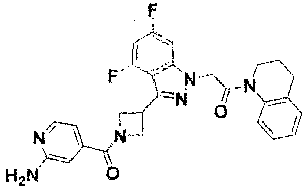 Example 56 | 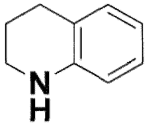 | MS(m/z) 503.3 [M+H]+ |
| | RE-116 | | |
| E-159 | 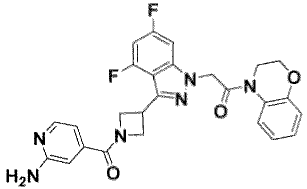 Example 56 | 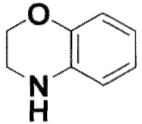 | MS(m/z) 505.2 [M+H]+ |
| | RE-116 | | |
| E-161 | 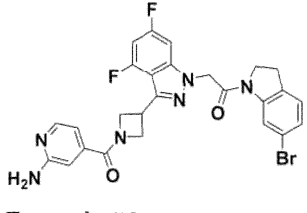 Example 56 | 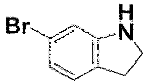 | MS(m/z) 569.0 [M + H) + |
| | RE-116 | | |
| E-172 | 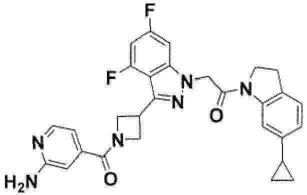 Example 56 | 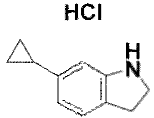 | MS(m/z) 529.2 [M+H]+ |
| | RE-116 | | |
| E-175 | 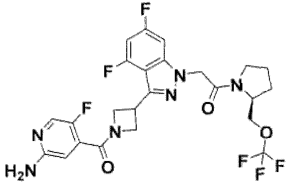 Example 45 | 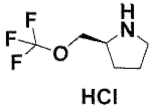 | MS(m/z) 557.2 [M+H]+ |
| | RE-120 | | |

**[Table 37]**

| Compound number | Structural formula of compound (upper row) | Reaction fragment | Data |
|---|---|---|---|
| | Example referred-to (middle row) | | |
| | Compound of Reference Example used (lower row) | | |
| E-176 | 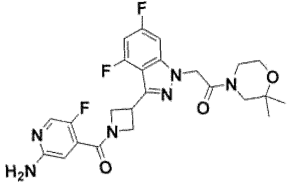 Example 45 | 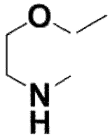 | MS(m/z) 503.2 [M+H]+ |
| | RE-120 | | |
| E-177 | 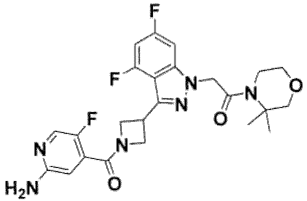 Example 45 | 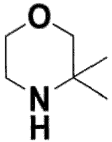 | MS(m/z) 503.2 [M+H]+ |
| | RE-120 | | |
| E-179 | 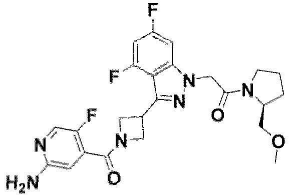 Example 45 | 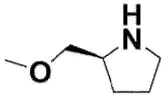 | MS(m/z) 503.2 [M+H]+ |
| | RE-120 | | |
| E-182 | 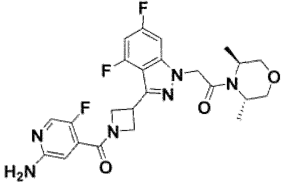 Example 45 | 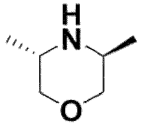 | MS(m/z) 503.3 [M + H] + |
| | RE-120 | | |
| E-189 | 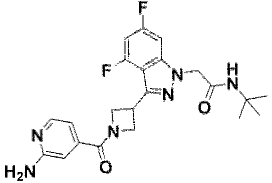 Example 45 | 2-methylpropane-2-amine | MS(m/z) 443.3 [M+H]+ |
| | RE-116 | | |

**[Table 38]**

| Compound number | Structural formula of compound (upper row) | Reaction fragment | Data |
|---|---|---|---|
| | Example referred-to (middle row) Compound of Reference Example used (lower row) | | |
| E-208 | 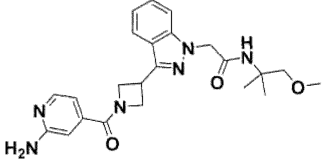 Example 45 | 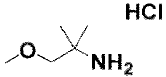 | MS(m/z) 437.2 [M+H]+ |
| | RE-121 | | |
| E-209 | 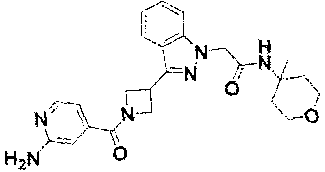 Example 45 | 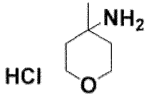 | MS(m/z) 449.2 [M+H]+ |
| | RE-121 | | |
| E-211 | 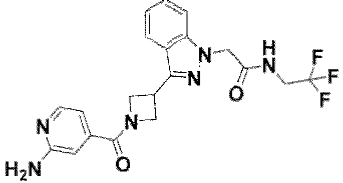 Example 45 | 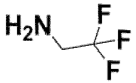 | MS(m/z) 433.1 [M+H]+ |
| | RE-121 | | |
| E-212 | 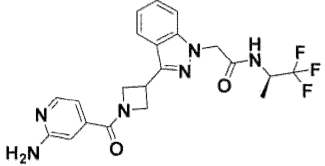 Example 45 | 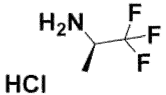 | MS(m/z) 447.1 [M+H]+ |
| | RE-121 | | |
| E-213 | 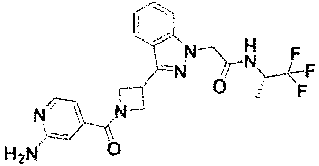 Example 45 | 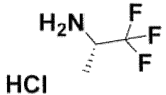 | MS(m/z) 447.1 [M+H]+ |
| | RE-121 | | |

**[Table 39]**

| Compound number | Structural formula of compound (upper row) | Reaction fragment | Data |
|---|---|---|---|
| | Example referred-to (middle row) | | |
| | Compound of Reference Example used (lower row) | | |
| E-214 | 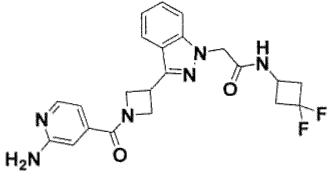 Example 45 | 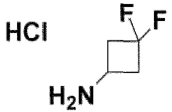 | MS(m/z) **441.2** [M+H]+ |
| | RE-121 | | |
| E-219 | 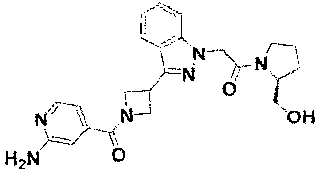 Example 60 | 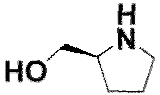 | MS(m/z) 435.2 [M+H]+ |
| | RE-121 | | |
| E-221 | 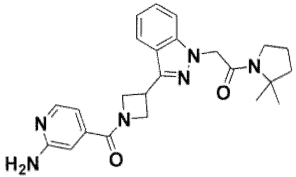 Example 45 | 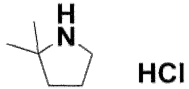 | MS(m/z) 433.3 [M+H]+ |
| | RE-121 | | |
| E-222 | 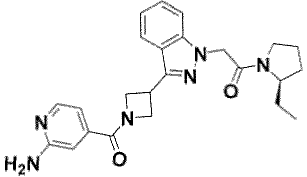 Example 45 | 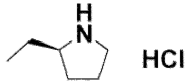 | MS(m/z) 433.3 [M+H]+ |
| | RE-121 | | |
| E-223 | 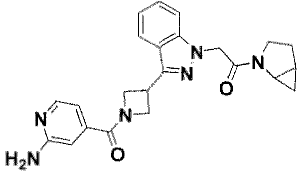 Example 45 | 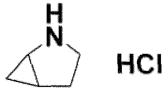 | MS(m/z) 417.2 [M+H]+ |
| | RE-121 | | |

**[Table 40]**

| Compound number | Structural formula of compound (upper row) | Reaction fragment | Data |
|---|---|---|---|
| | Example referred-to (middle row) | | |
| | Compound of Reference Example used (lower row) | | |
| E-224 | 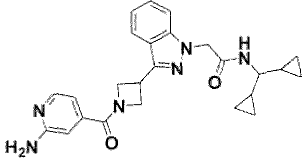 Example 56 | 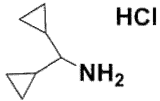 | MS(m/z) 445.3 [M+H]+ |
| | RE-121 | | |
| E-225 | 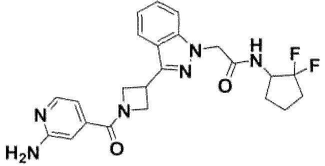 Example 56 | 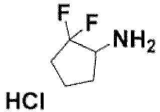 | MS(m/z) 455.3 [M+H]+ |
| | RE-121 | | |
| E-226 | 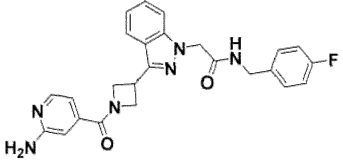 Example 56 | 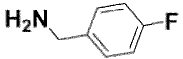 | MS(m/z) 459.2 [M+H]+ |
| | RE-121 | | |
| E-227 | 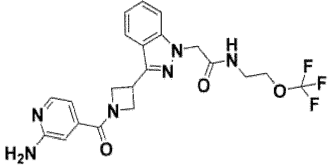 Example 56 | 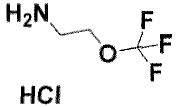 | MS(m/z) 463.2 [M+H]+ |
| | RE-121 | | |
| E-228 | 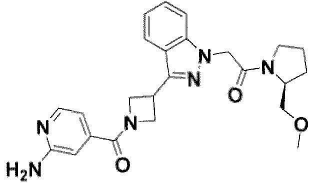 Example 45 | 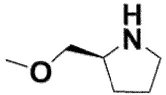 | MS(m/z) 449.2 [M+H]+ |
| | RE-121 | | |

**[Table 41]**

| Compound number | Structural formula of compound (upper row) | Reaction fragment | Data |
|---|---|---|---|
| | Example referred-to (middle row) Compound of Reference Example used (lower row) | | |
| E-229 | 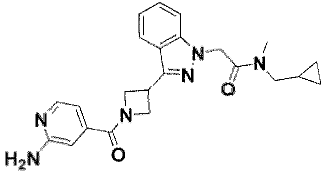 Example 45 | 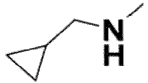 | MS(m/z) 419.2 [M+H]+ |
| | RE-121 | | |
| E-230 | 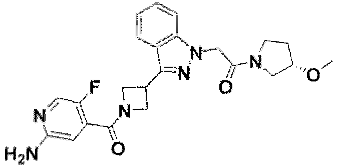 Example 45 | 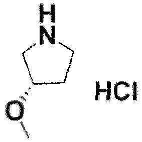 | MS(m/z) 453.2 [M+H]+ |
| | RE-117 | | |
| E-231 | 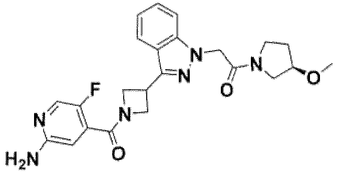 Example 45 | 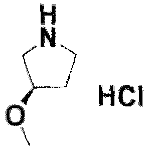 | MS(m/z) 453.2 [M+H] + |
| | RE-117 | | |
| E-232 | 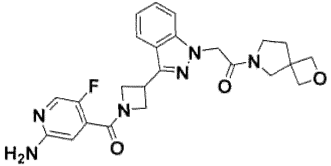 Example 45 | 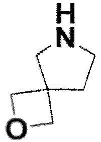 | MS(m/z) 465.2 [M+H]+ |
| | RE-117 | | |
| E-233 | 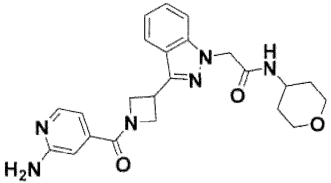 Example 56 | 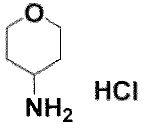 | MS(m/z) 435.2 [M+H]+ |
| | RE-121 | | |

**[Table 42]**

| Compound number | Structural formula of compound (upper row) | Reaction fragment | Data |
|---|---|---|---|
| | Example referred-to (middle row) Compound of Reference Example used (lower row) | | |
| E-234 | 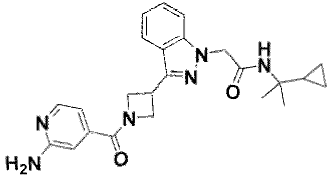 Example 56 | 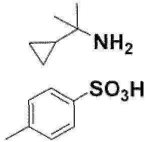 | MS(m/z) 433.3 [M+H]+ |
| | RE-121 | | |
| E-235 | 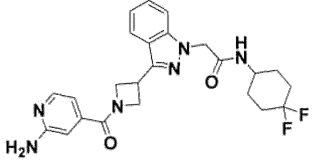 Example 56 | 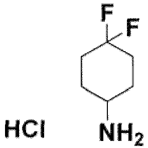 | MS(m/z) 469.3 [M+H]+ |
| | RE-121 | | |
| E-236 | 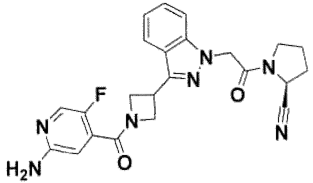 Example 45 | 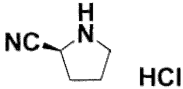 | MS(m/z) 448.2 [M+H]+ |
| | RE-117 | | |
| E-237 | 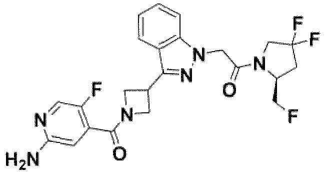 Example 45 | 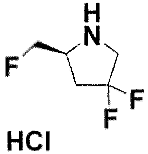 | MS(m/z) 491.2 [M+H]+ |
| | RE-117 | | |
| E-240 | 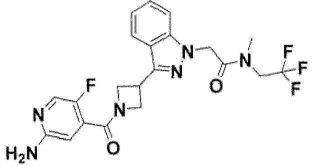 Example 45 | 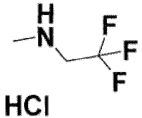 | MS(m/z) 465.2 [M+H]+ |
| | RE-117 | | |

**[Table 43]**

| Compound number | Structural formula of compound (upper row) | Reaction fragment | Data |
|---|---|---|---|
| | Example referred-to (middle row) Compound of Reference Example used (lower row) | | |
| E-243 | 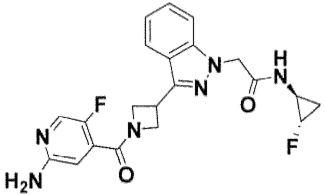 Example 56 | 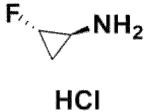 | MS(m/z) 427.2 [M+H]+ |
| | RE-117 | | |
| E-244 | 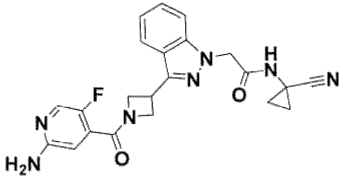 Example 56 | 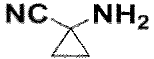 | MS(m/z) 434.2 [M+H]+ |
| | RE-117 | | |
| E-245 | 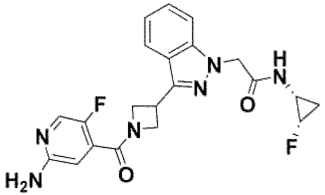 Example 56 | 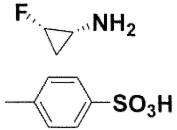 | MS(m/z) 427.2 [M+H]+ |
| | RE-117 | | |
| E-246 | 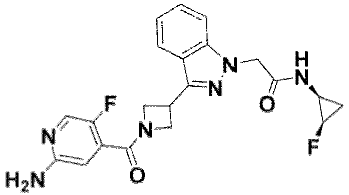 Example 56 | 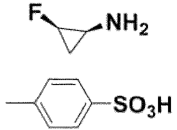 | MS(m/z) 427.2 [M+H]+ |
| | RE-117 | | |
| E-247 | 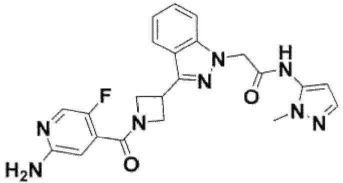 Example 56 | 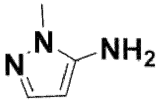 | MS(m/z) 449.2 [M+H]+ |
| | RE-117 | | |

**[Table 44]**

| Compound number | Structural formula of compound (upper row) | Reaction fragment | Data |
|---|---|---|---|
| | Example referred-to (middle row) Compound of Reference Example used (lower row) | | |
| E-248 | 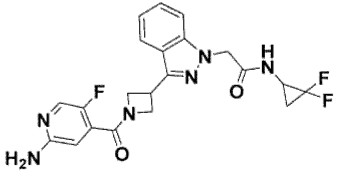 Example 60 | 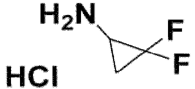 | MS(m/z) 445.2 [M+H]+ |
| | RE-117 | | |
| E-249 | 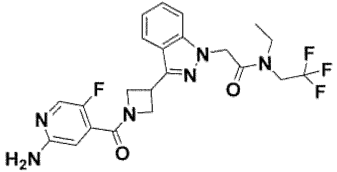 Example 45 | 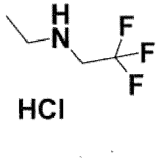 | MS(m/z) 479.2 [M+H]+ |
| | RE-117 | | |
| E-250 | 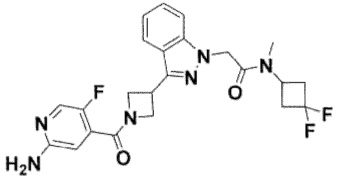 Example 45 | 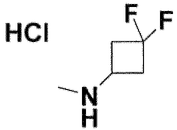 | MS(m/z) 473.2 [M+H]+ |
| | RE-117 | | |
| E-251 | 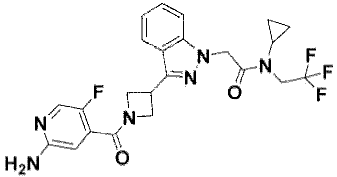 Example 45 | 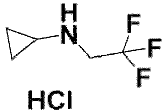 | MS(m/z) 491.3 [M+H]+ |
| | RE-117 | | |
| E-252 | 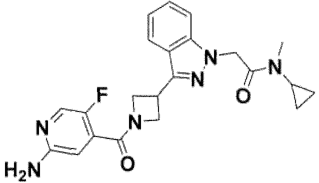 Example 45 | 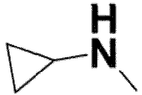 | MS(m/z) 423.2 [M+H]+ |
| | RE-117 | | |

**[Table 45]**

| Compound number | Structural formula of compound (upper row) | Reaction fragment | Data |
|---|---|---|---|
| | Example referred-to (middle row) Compound of Reference Example used (lower row) | | |
| E-253 | 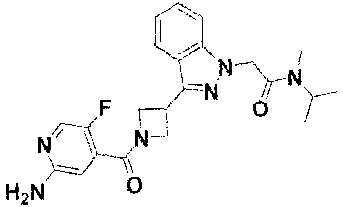 Example 45 | 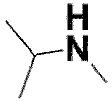 | MS(m/z) 425.3 [M+H]+ |
| | RE-117 | | |
| E-254 | 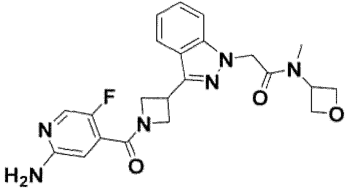 Example 45 | 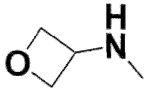 | MS(m/z) 439.2 [M+H]+ |
| | RE-117 | | |
| E-255 | 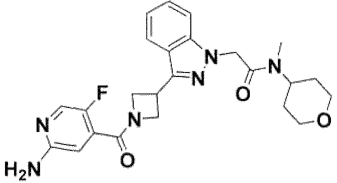 Example 45 | 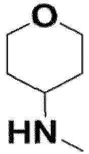 | MS(m/z) 467.2 [M+H]+ |
| | RE-117 | | |
| E-256 | 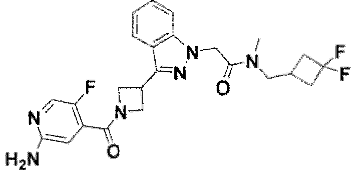 Example 60 | 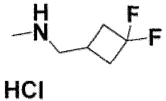 | MS(m/z) 487.2 [M+H]+ |
| | RE-117 | | |
| E-257 | 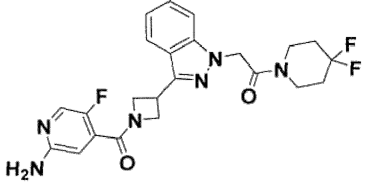 Example 56 | 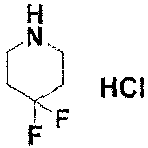 | MS(m/z) 473.3 [M+H]+ |
| | RE-117 | | |

**[Table 46]**

| Compound number | Structural formula of compound (upper row) Example referred-to (middle row) Compound of Reference Example used (lower row) | Reaction fragment | Data |
|---|---|---|---|
| E-258 | 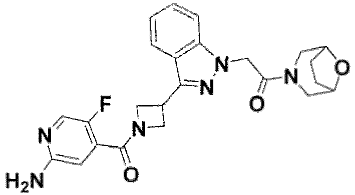 Example 56 | 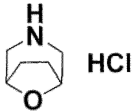 | MS(m/z) 465.4 [M+H]+ |
| | RE-117 | | |
| E-259 | 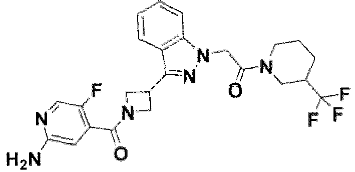 Example 56 | 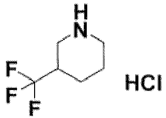 | MS(m/z) 505.3 [M+H]+ |
| | RE-117 | | |
| E-260 | 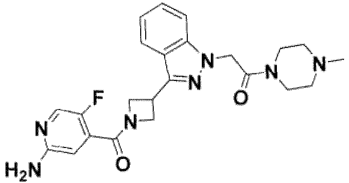 Example 56 | 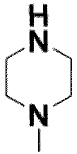 | MS(m/z) 452.4 (M+H]+ |
| | RE-117 | | |
| E-261 | 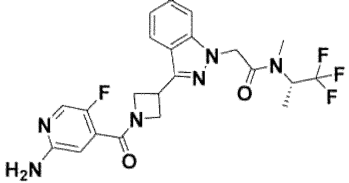 Example 56 | 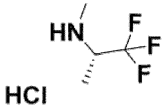 | MS(m/z) 479.2 [M+H]+ |
| | RE-117 | | |
| E-262 | 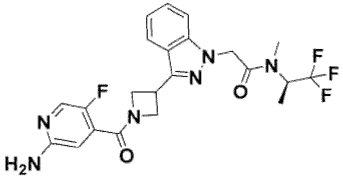 Example 56 | 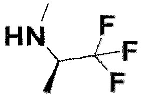 | MS(m/z) 479.2 [M+H]+ |
| | RE-117 | | |

**[Table 47]**

| Compound number | Structural formula of compound (upper row) Example referred-to (middle row) Compound of Reference Example used (lower row) | Reaction fragment | Data |
|---|---|---|---|
| E-263 | 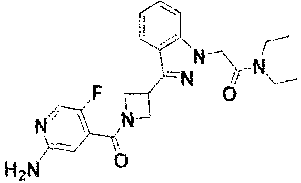 Example 45 | diethylamine | MS(m/z) 425.4 [M+H]+ |
| | RE-117 | | |
| E-264 | 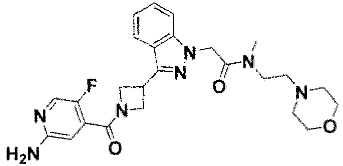 Example 45 | 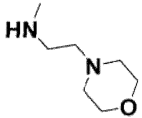 | MS(m/z) 496.4 [M+H]+ |
| | RE-117 | | |
| E-265 | 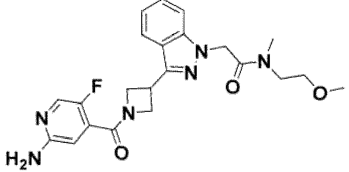 Example 60 | 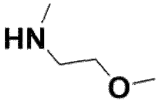 | MS(m/z) 441.4 [M+H]+ |
| | RE-117 | | |
| E-266 | 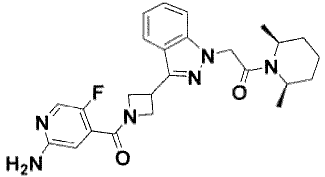 Example 56 | 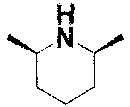 | MS(m/z) 465.3 [M+H]+ |
| | RE-117 | | |
| E-269 | 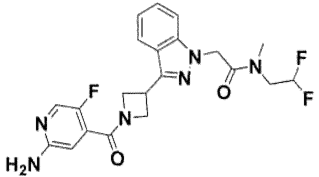 Example 60 | 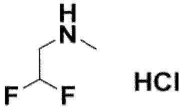 | MS(m/z) 447.3 [M+H]+ |
| | RE-117 | | |

**[Table 48]**

| Compound number | Structural formula of compound (upper row) Example referred-to (middle row) Compound of Reference Example used (lower row) | Reaction fragment | Data |
|---|---|---|---|
| E-272 | 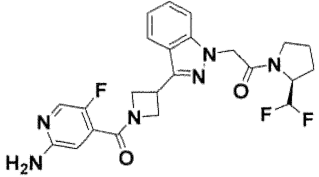 Example 60 | 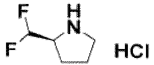 | MS(m/z) 473.3 [M+H]+ |
| | RE-117 | | |

Tables 49 through 72 below show the numbers and chemical names of the Example compounds. In the Tables, the chemical name refers to the name of the compound corresponding to the compound number.

**[Table 49]**

| Compound number | Chemical Name |
|---|---|
| E-1 | 2-(4-chloro-3-{1-[2-(cyclopropylamino)pyridine-4-carbonyl]azetidin-3-yl}-5-fluoro-1H-indazol-1-y1)-N-(2,2,2-trifluoroethyl)acetamide hydrochloride |
| E-2 | 2-{3-[1-(2-aminopyridine-4-carbonyl)azetidin-3-yl]-9,6-difluoro-1H-indazol-1-yl}-1-[(2S)-2-(trifluoromethyl)pyrrolidin-1-yllethan-1-one hydrochloride |
| E-3 | 2-{3-[1-(2-amino-5-fluoropyridine-4-carbonyl)azetidin-3-yl]-1H-indazol-1-y1}-1-[(28)-2-(trifluoromethyl)pyrrolidin-1-yl]ethan-1-one |
| E-4 | 2-{3-[1-(2-aminopyrimidine-4-carbonyl)azetidin-3-yl]-5-chloro-1H-indazol-1-yl}-N-methyl-N-(2,2,2-trifluoroethyl)acetamide |
| E-5 | 2-{3-[1-(2-amino-5-fluoropyridine-4-carbonyl)azetidin-3-yl]-6-fluoro-1H-indazol-1-yl}-N-methyl-N-(2,2,2-trifluoroethyl)acetamide |
| E-6 | 2-{3-[1-(2-aminopyridine-4-carbonyl)-3-methylazetidin-3-yl]-1H-indazol-1-yl]-1-[(2S)-2-(trifluoromethyl)pyrrolidin-1-yl]ethan-1-one |
| E-7 | 2-{3-[1-(2-amino-4-methyl-1,3-thiazole-5-carbonyl)azetidin-3-yl]-1H-indazol-1-yl}-1-(3,3-dimethylmorpholin-4-yl)ethan-1-one |
| E-8 | 2-{3-[1-(2-amino-3-methylpyridine-4-carbonyl)azetidin-3-yl]-1H-indazol-1-yl}-1-[(2R)-2-(trifluoromethyl)morpholin-4-yl]ethan-1-one |
| E-9 | 4-[4-(4-fluoro-1-{[5-(trifluoromethyl)pyridin-2-yl]methyl}-1H-indazol-3-yDpiperidine-1-carbonyl]pyridin-2-amine |
| E-10 | 2-{3-[1-(2-amino-5-fluoropyridine-4-carbonyl)piperidin-4-yl]-4-fluoro-1H-indol-1-yl}-1-[(2S)-2-(trifluoromethyl)pyrrolidin-1-yllethan-1-one |
| E-11 | 2-{3-[1-(2-aminopyridine-4-carbonyl)azetidin-3-yl]-1H-indol-1-yl}-1-[(2S)-2-(trifluoromethyl)pyrrolidin-1-yl]ethan-1-one |
| E-12 | 2-{3-[1-(2-aminopyridine-4-carbonyl)azetidin-3-yl]-5-methyl-1H-pyrazolo[4,3-b]pyridin-1-yl]-1-[(2S)-2-(trifluoromethyl)pyrrolidin-1-yl]ethan-1-one |

**[Table 50]**

| Compound number | Chemical Name |
|---|---|
| E-13 | 2-{3-[1-(2-aminopyridine-4-carbonyl)azetidin-3-yl}-1H-pyrazolo[4,3-clpyridin-1-yl}-1-[(28)-2-(trifluoromethyl)pyrrolidin-1-yllethan-1-one hydrochloride |
| E-14 | 4-(3-[4-fluoro-1-({5-[1-(trifluoromethyl)cyclopropyl]-1,2,4-oxadiazol-3-yl}methyl)-1H-pyrazolo[3,4-c]pyridin-3-yl]azetidine-1-carbonyl}pyridin-2-amine |
| E-15 | 2-{3-[1-(2-aminopyridine-4-carbonyl)azetidin-3-yl]-4-methyl-1H-pyrazolo[3,4-b]pyridin-1-yl}-1-[(2S)-2-(trifluoromethyl)pyrrolidin-1-yl]ethan-1-one |
| E-16 | 2-{3-[1-(6-aminopyridazine-4-carbonyl)azetidin-3-yl]-4,6-difluoro-1H-indazol-1-yl}-1-[(28)-2-(trifluoromethyl)pyrrolidin-1-yl]ethan-1-one |
| E-17 | 2-[4,6-difluoro-3-(1-{1H-pyrazolol3,4-b]pyridine-4-carbonyl]azetidin-3-yl)-1H-indazol-1-y1]-1-[(2S)-2-(trifluoromethyl)pyrrolidin-1-yl]ethan-1-one |
| E-18 | 5-fluoro-4-[3-(1-{[3-(trifluoromethyl)-1,2,4oxadiazol-5-yl]methyl}-1H-indazol-3-yl)azetidine-1-carbonyl]pyridin-2-amine |
| E-19 | 4-{3-[1-({1-1(3,3-difluorocyclobutyl)methyl]-1H-1,2,3-triazol-4-yl}methyl)-4-fluoro-1H-indazol-3-yl]azetidine-1-carbonyl}pyridin-2-amine hydrochloride |
| E-20 | 4-[3-(4-fluoro-1-{[5-(4-fluorophenyl)-1,3,4-oxadiazol-2-yl]methyl]-1H-indazol-3-yl)azetidine-1-carbonyllpyridin-2-amine |
| E-21 | 2-{3-[1-(2-amino-5-fluoropyrimidine-4-carbonyl)azetidin-3-yl]-5-chloro-1H-indazol-1-yl}-N-methyl-N-(2,2,2-trifluoroethyl)acetamide |
| E-22 | 2-{3-[1-(2-amino-5-fluoropyrimidine-4-carbonyl)azetidin-3-yl]-1H-indazol-1-yl}-1-[(28)-2-(trifluoromethyl)pyrrolidin-1-yl]ethan-1-one |
| E-23 | 2-{3-[1-(2-amino-5-fluoropyrimidine-4-carbonyl)azetidin-3-yl]-4-fluoro-1H-indazol-1-yl}-N-methyl-N-(2,2,2-trifluoroethyl)acetamide |
| E-24 | 2-{3-[1-(2-amino-5-fluoropyridine-4-carbonyl)azetidin-3-yl]-4,5,6,7-tetrahydro-1H-indazol-1-yl}-1-[(2S)-2-(trifluoromethyl)pyrrolidin-1-yl]ethan-1-one |

**[Table 51]**

| Compound number | Chemical Name |
|---|---|
| E-25 | 2-{3-[1-(2-amino-5-fluoropyridine-4-carbonyl)azetidin-3-yl]-5,5-difluoro-4,5,6,7-tetrahydro-1H-indazol-1-yl}-1-[(2S)-2-(trifluoromethyl)pyrrolidin-1-yl]ethan-1-one |
| E-26 | 2-(4-chloro-3-{1-[2-(cyclopropylamino)pyridine-4-carbonyl]azetidin-3-yl}-5-fluoro-1H-indazol-1-yl)-N-(2,2,2-trifluorcethyl)acetamide p-toluenesulfonate |
| E-27 | 2-{3-[1-(2-aminopyridine-4-carbonyl)azetidin-3-yl]-4,6-difluoro-1H-indazol-1-yl}-1-[(2S)-2-(trifluoromethyl)pyrrolidin-1-yl]ethan-1-one methanesulfonate |
| E-28 | 2-{S-[1-(2-amino-5-fluoropyridine-4-carbonyl)azetidin-3-yl]-1H-indazol-1-yl}-1-[(2S)-2-(trifluoromethyl)pyrrolidin-1-yl]ethan-1-one hydrochloride |
| E-29 | 2-{3-[1-(2-amino-5-fluoropyridine-4-carbonyl)azetidin-3-yl]-1H-indazol-1-yl]-1-[(2S)-2-(trifluoromethyl)pyrrolidin-1-yl]ethan-1-one methanesulfonate |
| E-30 | 2-(3-{1-[2-(cyclopropylamino)pyridine-4-carbonyl]azetidin-3-yl}-4,6-difluoro-1H-indazol-1-yl)-N-(2,2,2-trifluoroethyl)acetamide hydrochloride |
| E-31 | 2-(3-{1-[6-(cyclopropylamino)pyrimidine-4-carbonyl]azetidin-3-yl]-4,6-difluoro-1H-indazol-1-yl)-N-(2,2,2-trifluoroethyl)acetamide |
| E-32 | 2-[4,6-difluoro-3-(1-{2-[(oxetan-3-yl)amino]pyridine-4-carbonyliazetidin-3-yl)-1H-indazol-1-yl]-N-(2,2,2-trifluoroethyl)acetamide |
| E-33 | 2-[4,6-difluoro-3-(1-{1H-pyrrolo[2,3-b]pyridine-4-carbonyl}azetidin-3-yl)-1H-indazol-1-yl]-1-[(2S)-2-(trifluoromethyl)pyrrolidin-1-yl]ethan-1-one hydrochloride |
| E-34 | 4-(3-{4-fluoro-1-[(1-phenyl-1H-1,2,3-triazol-4-yl)methyl]-1H-indazol-3-yl}azetidine-1-carbonyl)pyridin-2-amine |
| E-35 | 4-(3-{1-[(1-benzyl-1H-1,2,3-triazol-4-yl)methyl]-4-fluoro-1H-indazol-3-yl}azetidine-1-carbonyl)pyridin-2-amine hydrochloride |

**[Table 52]**

| Compound number | Chemical Name |
|---|---|
| E-36 | 2-{3-[1-(2-aminopyridine-4-carbonyl)azetidin-3-yl]-4,6-difluoro-1H-indazol-1-yl}-1-[(2R)-2-(hydroxymethyl)pyrrolidin-1-yl]ethan-1-one |
| E-37 | 4-[3-(1-{[1-(cyclopropylmethyl)-1H-1,2,3-triazol-4-yl]methyl]-4-fluoro-1H-indazol-3-yl)azetidine-1-carbonyl]pyridin-2-amine hydrochloride |
| E-38 | 4-{3-[4.fluoro-1-({1-[(oxetan-3-yl)methyl)-1H-1,2,3-triazol-4-yl}methyl)-1H-indazol-3-yl]azetidine-1-carbonyl]pyridin-2-amine |
| E-39 | 4-{4-[1-({1-1(3,3-difluorocyclobutyl)methyl]-1H-1,2,3-triazol-4-yl}methyl)-4-fluoro-1H-indazol-3-yl]piperidine-1-carbonyl]pyridin-2-amine |
| E-40 | 2-(3-{1-(2-aminopyridine-4-carbonyl)azetidin-3-yl]-1H-indazol-1-yl}-1-[(2S)-2-(trifluoromethyl)pyrrolidin-1-yllethan-1-one |
| E-41 | 2-{3-[1-(2-aminopyridine-4-carbonyl)azetidin-3-yl]-1H-indazol-1-yl}-1-[(2S)-2-(trifluoromethyl)pyrrolidin-1-yl]ethan-1-one hydrochloride |
| E-42 | 2-{3-[1-(2-amino-5-fluoropyridine-4-carbonyl)azetidin-3-yl]-4,6-difluoro-1H-indazol-1-yl}-1-[(2R)-2-(trifluoromethyl)morpholin-4-yllethan-1-one |
| E-43 | 2-(3-[1-(2-amino-5-fluoropyridine-4-carbonyl)azetidin-3-yl]-4,6-difluoro-1H-indazol-1-yl}-1-[(2R)-2-(trifluoromethyl)morpholin-4-yl]ethan-1-one p-toluenesulfonate |
| E-44 | 2-{3-[1-(2-amino-3-methylpyridine-4-carbonyl)azetidin-3-yl]-4-fluoro-1H-pyrazolo[3,4-c]pyridin-1-yl]-1-[(2S)-2-[(trifluoromethoxy)methyl]pyrrolidin-1-yl]ethan-1-one di-p-toluenesulfonate |
| E-45 | 2-{3-(1-(2-aminopyridine-4-carbonyl)azetidin-3-yl]-1H-indazol-1-yl}-N-methyl-N-(2,2,2-trifluoroethyl)acetamide |
| E-46 | 2-{3-[1-(2-aminopyridine-4-carbonylazetidin-3-yl]-1H-indazol-1-yl}-N-methyl-N-(2,2,2-trifluoroethyl)acetamide p-toluenesulfonate |
| E-47 | 2-{3-[1-(2-amino-5-fluoropyridine-4-carbonyl)-1,2,3,6-tetrahydropyridin-4-yl]-1H-indazol-1-yl}-1-[(2S)-2-(trifluoromethyl)pyrrolidin-1-yl]ethan-1-one |

**[Table 53]**

| Compound number | Chemical Name |
|---|---|
| E-48 | 2-{3-[1-(2-amino-5-fluoropyridine-4-carbonyl)piperidin-4-yl]-1H-indazol-1-yl}-N-methyl-N-(2,2,2-trifluoroethyl)acetamide |
| E-49 | 2-{3-[1-(2-amino-5-fluoropyridine-4-carbonyl)piperidin-4-yl]-1H-indazol-1-yl}-1-[(2S)-2-(trifluoromethyl)pyrrolidin-1-yl]ethan-1-one |
| E-50 | 2-{3-[1-(2-aminopyridine-4-carbonyl)azetidin-3-yl]-4,6-difluoro-1H-indazol-1-yl]-1-[(2R)-2-(trifluoromethyl)pyrrolidin-1-yl]ethan-1-one |
| E-51 | 2-{3-[1-(2-amino-3-methylpyridine-4-carbonyl)azetidin-3-yl]-4,6-difluoro-1H-indazol-1-yl}-1-[(2R)-2-(trifluoromethyl)morpholin-4-yl]ethan-1-one hydrochloride |
| E-52 | 4-[3-(1-{[1-(2,2,2-trifluoroethyl)-1H-1,2,3-triazol-4-yl]methyl]-4-fluoro-1H-indazol-3-yl)azetidine-1-carbonyl]pyridin-2-amine hydrochloride |
| E-53 | 2-{3-[1-(2-aminopyridine-4-carbonyl)azetidin-3-yl]-4-methyl-1H-pyrazolo[4,3-c]pyridin-1-yl}-1-[(2S)-2-(trifluoromethyl)pyrrolidin-1-yllethan-1-one |
| E-54 | 2-{3-[1-(2-aminopyridine-4-carbonyl)azetidin-3-yl]-4-fluoro-1H-pyrazolo[3,4-c]pyridin-1-yl}-1-[(2S)-2-(trifluoromethyl)pyrrolidin-1-yllethan-1-one hydrochloride |
| E-55 | 4-{3-[4-fluoro-1-({3-[3-(trifluoromethyl)phenyl]-1,2,4-oxadiazol-5-yl}methyl)-1H-indazol-3-yl]azetidine-1-carbonyl}pyridin-2-amine |
| E-56 | 2-{3-[1-(2-aminopyridine-4-carbonyl)azetidin-3-yl]-4,6-difluoro-1H-indazol-1-yl}-1-[(2S)-2-l(trifluoromethoxy)methyl]pyrrolidin-1-yl]ethan-1-one |
| E-57 | 4-(3-{1-[(4,4-difluorocyclohexyl)methyl]-4,6-difluoro-1H-indazol-3-yl}azetidine-1-carbonyl)pyridin-2-amine |
| E-58 | 2-{3-[1-(2-amino-5-fluoropyridine-4-carbonyl)azetidin-3-yl]-4-fluoro-1H-pyrazolo(3,4-c]pyridin-1-yl}-1-[(2S)-2-[(trifluoromethoxy)methyl]pyrrolidin-1-yl]ethan-1-one di-p-toluenesulfonate |

**[Table 54]**

| Compound number | Chemical Name |
|---|---|
| E-59 | 2-{3-[1-(2-amino-5-fluoropyridine-4-carbonyl)azetidin-3-yl]-4-fluoro-1H-indazol-1-yl}-N-methyl-N-(2,2,2-trifluoroethyl)acetamide |
| E-60 | 2-{3-[1-(2-amino-5-fluoropyridine-4-carbony)azetidin-3-yl]-1H-indazol-1-yl}-1-[(2R)-2-(trifluoromethyl)pyrrolidin-1-yl]ethan-1-one |
| E-61 | 2-[4-chloro-3-(1-(1H-pyrrolo[2,3-b]pyridine-4-carbonyl]azetidin-3-y])-1H-indazol-1-yl]-N-(2,2,2-trifluoroethyl)acetamide |
| E-62 | 2-{3-[1-(2-aminopyridine-4-carbonyl)azetidin-3-yl}-4-chloro-1H-indazol-1-yl}-N-(2,2,2-trifluoroethyl)acetamide |
| E-63 | 2-(4-chloro-3-{1-[2-(methylamino)pyridine-4-carbonyl]azetidin-3-yl]-1H-indazol-1-yl)-N-(2,2,2-trifluoroethyl)acetamide |
| E-64 | 2-(4-chloro-3-(1-[2-(ethylamino)pyridine-4-carbonyl]azetidin-3-yl}-1H-indazol-1-yl)-N-(2,2,2-trifluoroethyl)acetamide hydrochloride |
| E-65 | 2-(3-{1-[2-(cyclopropylamino)pyridine-4-carbonyl]azetidin-3-yl}-4-fluoro-1H-indazol-1-yl)-N-(2,2,2-trifluoroethyl)acetamide hydrochloride |
| E-66 | 2-(3-{1-[2-(cyclopropylamino)pyridine-4-carbonyl]azetidin-3-yl}-4,7-difluoro-1H-indazol-1-yl)-N-(2,2,2-trifluoroethyl)acetamide hydrochloride |
| E-67 | 2-{3-[1-(2-aminopyridine-4-carbonyl)azetidin-3-yl]-4-chloro-5-fluoro-1H-indazol-1-yl}-N-(2,2,2-trifluoroethyl)acetamide hydrochloride |
| E-68 | 2-(3-{1-[2-(cyclobutylamino)pyridine-4-carbonyl]azetidin-3-yl}-4-fluoro-1H-indazol-1-yl)-N-(2,2,2-trifluoroethyl)acetamide hydrochloride |
| E-69 | 2-(4-bromo-3-{ 1-[2-(cyclopropylamino)pyridine-4-carbonyl] azetidin-3-yl}-5-fluoro-1H-indazol-1-yl)-N-(2,2,2-trifluoroethyl)acetamide hydrochloride |
| E-70 | 2-{3-[1-(2-aminopyridine-4-carbonyl)azetidin-3-yl]-4-bromo-5-fluoro-1H-indazol-1-yl}-N-(2,2,2-trifluoroethyl)acetamide hydrochloride |
| E-71 | 2-[4-fluoro-3-(1-{2-{(oxan-4-yl)amino]pyridine-4-carbonyl}azetidin-3-yl)-1H-indazol-1-yl]-N-(2,2,2-trifluoroethyl)acetamide hydrochloride |

**[Table 55]**

| Compound number | Chemical Name |
|---|---|
| E-72 | 2-(3-{1-[2-(cyclopropylamino)pyridine-4-carbonyl]azetidin-3-yl)-4,6-difluoro-1H-indazol-1-yl)-1-[(2S)-2-(trifluoromethyl)pyrrolidin-1 - yllethan-1-one hydrochloride |
| E-73 | 2-(3-[1-(2-aminopyridine-4-carbonyl)azetidin-3-yl]-4,6-difluoro-1H-indazol-1-yl}-1-l(2R)-2-(trifluoromethyl)morpholin-4-yl]ethan-1-one hydrochloride |
| E-74 | 2-[4,6-difluoro-3-(1-{2-[(1-methylcyclopropyl)aminolpyridine-4-carbonyl}azetidin-3-yl)-1H-indazol-1-yl]-1-[(2S)-2-(trifluoromethyl)pyrrolidin-1-yl]ethan-1-one hydrochloride |
| E-75 | 2-[3-(1-{2-[(4,4-difluorocyclohexyl)aminolpyridine-4-carbonyl}azetidin-3-yl)-4,6-difluoro-1H-indazol-1-yl]-1-[(2S)-2-(trifluoromethyl)pyrrolidin-1-yl]ethan-1-one hydrochloride |
| E-76 | 2-{3-[1-(2-aminopyridine-4-carbonyl)azetidin-3-yl]-4,6-difluoro-1H-indazol-1-yl}-1-(pyrrolidin-1-yl)ethan-1-one |
| E-77 | 2-{3-[1-(2-amino-3-methylpyridine-4-carbonyl)azetidin-3-yl]-4,6-difluoro-1H-indazol-1-yl}-1-{(2S)-2-(trifluoromethyl)pyrrolidin-1-yl]ethan-1-one hydrochloride |
| E-78 | 2-{3-[1-(2-amino-5-methylpyridine-4-carbonyl)azetidin-3-yl]-4,6-difluoro-1H-indazol-1-yl}-1-[(2S)-2-(trifluoromethyl)pyrrolidin-1-yl]ethan-1-one hydrochloride |
| E-79 | 2-{3-[1-(2-aminopyridine-4-carbonyl)azetidin-3-yl]-4,6-difluoro-1H-indazol-1-yl}-1-[(2S)-2-methylmorpholin-4-yl]ethan-1-one hydrochloride |
| E-80 | 2-{3-[1-(2-aminopyridine-4-carbonyl)azetidin-3-yl]-4,6-difluoro-1H-indazol-1-yl}-1-[(3R)-3-methylmorpholin-4-yl]ethan-1-one hydrochloride |
| E-81 | 2-{3-[1-(2-aminopyridine-4-carbonyl)piperidin-4-yl]-4-fluoro-1H-indazol-1-yl}-1-[(28)-2-(trifluoromethyl)pyrrolidin-1-yl]ethan-1-one |
| E-82 | 2-{3-[1-(2-aminopyridine-4-carbonyl)azetidin-3-yl]-4,6-difluoro-1H-indazol-1-yl}-1-[(2S)-2-(trifluoromethyl)morpholin-4-yl]ethan-1-one |

**[Table 56]**

| Compound number | Chemical Name |
|---|---|
| E-83 | 2-{3-[1-(2-aminopyridine-4-carbonyl)azetidin-3-yl]-4,6-difluoro-1H-indazol-1-yl]-1-[(3R)-3-(propan-2-yl)morpholin-4-yl]ethan-1-one |
| E-84 | 2-{3-[1-(2-aminopyridine-4-carbonyl)azetidin-3-yl)-4,6-difluoro-1H-indazol-1-yl}-1-[(2R,68)-2,6-dimethylmorpholin-4-yl]ethan-1-one |
| E-85 | 2-{3-(1-(6-aminopyridine-3-carbonyl)piperidin-4-yl]-4-fluoro-1H-indazol-1-yl}-1-[(2S)-2-(trifluoromethyl)pyrrolidin-1-yl]ethan-1-one |
| E-86 | 2-(3-{1-[2-(cyclopropylamino)pyridine-4-carbonyl}piperidin-4-yl]-4-fluoro-1H-indazol-1-yl)-1-[(2S)-2-(trifluoromethyl)pyrrolidin-1-yl]ethan-1-one |
| E-87 | 2-{3-[1-(2-aminopyridine-4-carbonyl)azetidin-3-yl]-4-fluoro-1H-indazol-1-yl}-N-methyl-N-(2,2,2-trifluoroethyl)acetamide |
| E-88 | 2-[4,6-difluoro-3-(1-{2-[(oxan-4-yl)amino]pyridine-4-carbonyl}azetidin-3-yl)-1H-indazol-1-yl]-1-[(2S)-2-(trifluoromethyl)pyrrolidin-1-yl]ethan-1-one |
| E-89 | 2-{3-[1-(2-amino-3-methylpyridine-4-carbonyl)azetidin-3-yl]-4,6-difluoro-1H-indazol-1-yl]-1-[(2S)-2-(trifluoromethyl)morpholin-4-yllethan-1-one hydrochloride |
| E-90 | 2-{3-[1-(2-aminopyridine-4-carbonyl)piperidin-4-yl]-4-fluoro-1H-indazol-1-yl]-1-[(2S)-2-(trifluoromethyl)morpholin-4-yl]ethan-1-one |
| E-91 | 2-{3-[1-(2-aminopyridine-4-carbonyl)piperidin-4-yl]-4-fluoro-1H-indazol-1-yl]-1-[(2R)-2-(trifluoromethyl)morpholin-4-yl]ethan-1-one |
| E-92 | 4-[3-(1-{[1-(cyclobutylmethyl)-1H-1,2,3-triazol-4-yl]methyl}-4-fluoro-1H-indazol-3-yl)azetidine-1-carbonyl]pyridin-2-amine hydrochloride |
| E-93 | 2-(3-{1-[2-(cyclopropylamino)pyridine-4-carbonyl]azetidin-3-yl}-4-methyl-1H-pyrazolo[4,3-c]pyridin-1-yl)-1-[(2S)-2-(trifluoromethyl)pyrrolidin-1-yl]ethan-1-one |
| E-94 | 2-{3-[1-(2-amino-3-methylpyridine-4-carbonyl)azetidin-3-yl]-4-methyl-1H-pyrazolo[4,3-clpyridin-1-yl}-1-[(2S)-2-(trifluoromethyl)pyrrolidin-1-yl]ethan-1-one |

**[Table 57]**

| Compound number | Chemical Name |
|---|---|
| E-95 | 2-{3-[1-(2-aminopyridine-4-carbonyl)azetidin-3-yl]-4-fluoro-1H-indazol-1-yl}-1-[(2R)-2-(trifluoromethyl)morpholin-4-yl]ethan-1-one hydrochloride |
| E-96 | 2-{3-[1-(2-aminopyridine-4-carbonyl)azetidin-3-yl]-4-fluoro-1H-indazol-1-yl}-1-[(2S)-2-(trifluoromethyl)pyrrolidin-1-yl]ethan-1-one |
| E-97 | 2-{3-[1-(2-amino-1,3-thiazole-5-carbonyl)azetidin-3-yl]-4,6-difluoro-1H-indazol-1-yl}-1-[(28)-2-(trifluoromethyl)pyrrolidin-1-yl]ethan-1-one |
| E-98 | 2-(3-{1-[2-(cyclopropylamino)-1,3-thiazole-5-carbonyl]azetidin-3-yl]-4,6-difluoro-1H-indazol-1-yl)-1-[(2S)-2-(trifluoromethyl)pyrrolidin-1-yl]ethan-1-one |
| E-99 | 2-(3-{1-[2-(cyclopropylamino)-4-methyl-1,3-thiazole-5-carbonyl]azetidin-3-yl}-4,6-difluoro-1H-indazol-1-y1)-1-[(2S)-2-(trifluoromethyl)pyrrolidin-1-yl]ethan-1-one |
| E-100 | 2-{3-[1-(2-aminopyridine-4-carbonyl)azetidin-3-yl]-4-fluoro-1H-pyrazolo[3,4-c}pyridin-1-y}}-1-[(2R)-2-(trifluoromethyl)morpholin-4-yl]ethan-1-one |
| E-101 | 2-(4,6-difluoro-3-(1-{imidazol(1,2-alpyridine-3-carbonyl}azetidin-3-yl)-1H-indazol-1-yl]-1-[(2S)-2-(trifluoromethyl)pyrrolidin-1-yl]ethan-1-one |
| E-102 | 2-[4,6-difluoro-3-(1-{pyrazolo[3,2-b][1,3]thiazol-7-carbonyl)azetidin-3-yl)-1H-indazol-1-yl]-1-[(2S)-2-(trifluoromethyl)pyrrolidin-1-yllethan-1-one |
| E-103 | 2-{3-[1-(2-aminopyridine-4-carbonyl)azetidin-3-yl]-4,6-difluoro-1H-indazol-1-yl}-1-[(2S)-2-(trifluoromethyl)pyrrolidin-1-yl}propane-1-one hydrochloride |
| E-104 | 4-[3-(4-fluoro-1-{[3-(4-fluorophenyl)-1,2,4-oxadiazol-5-yl]methyl}-1H-indazol-3-yl)azetidine,1-carbonyl)pyridin-2-amine |
| E-105 | 2-{3-[1-(2-amino-1,3-oxazole-5-carbonyl)azetidin-3-yl]-4,6-difluoro-1H-indazol-1-yl}-1-[(28)-2-(trifluoromethyl)pyrrolidin-1-yl]ethan-1-one |

**[Table 58]**

| Compound number | Chemical Name |
|---|---|
| E-106 | 2-{3-[1-(2-amino-4-methyl-1,3-thiazole-5-carbonyl)azetidin-3-yl}-4,6-difluoro-1H-indazol-1-yl}-1-[(2S)-2-(trifluoromethyl)pyrrolidin-1-yllethan-1-one |
| E-107 | 2-{3-[1-(2-aminopyridine-4-carbonyl)azetidin-3-yl]-4,6-difluoro-1H-indazol-1-yl}-2-fluoro-1-[(2S)-2-(trifluoromethyl)pyrrolidin-1-yl]ethan-1-one |
| E-108 | 2-{3-[1-(2-aminopyridine-4-carbonyp)piperidin-4-yl]-4-fluoro-1H-indol-1-yl}-1-[(2S)-2-(trifluoromethyl)pyrrolidin-1-yl]ethan-1-one |
| E-109 | 4-(3-{4-fluoro-1-[(3-methyl-1,2,4-oxadiazol-5-yl)methyl]-1H-indazol-3-yl}azetidine-1-carbonyl)pyridin-2-amine |
| E-110 | 2-{3-{1-(2-amino-4-methyl-1,3-oxazole-5-carbonyl)azetidin-3-yl}-4,6-difluoro-1H-indazol-1-yl}-1-[(2S)-2-(trifluoromethyl)pyrrolidin-1-yl]ethan-1-one |
| E-111 | 2-{3-[1-(2-aminopyridine-4-carbonylazetidin-3-yl]-4,6-difluoro-1H-indazol-1-y1}-1-[(25)-4,4-difluoro-2-(fluoromethyl)pyrrolidin-1-yl]ethan-1-one |
| E-112 | 4-[3-(4-fluoro-1-{[4-(trifluoromethyl)phenyllmethyl}-1H-indazol-3-yl)azetidine-1-carbonyl]pyridin-2-amine hydrochloride |
| E-113 | 4-[3-(4-fluoro-1-{[3-(trifluoromethyl)phenyllmethyl}-1H-indazol-3-yl)azetidine-1-carbonyl]pyridin-2-amine hydrochloride |
| E-114 | 4-[3-(4-fluoro-1-{[2-(trifluoromethyl)phenyllmethyl}-1H-indazol-3-yl)azetidine-1-carbonyl]pyridin-2-amine hydrochloride |
| E-115 | 4-[3-(4-fluoro-1-{[3-(trifluoromethyn-1,2,4-oxadiazol-5-yl]methyl}-1H-indazol-3-yl)azetidine-1-carbonyl]pyridin-2-amine hydrochloride |
| E-116 | 4-[3-(4-fluoro-1-{[5-(4-fluorophenyl)-1,2,4-oxadiazol-3-yl]methyl}-1H-indazol-3-yl)azetidine-1-carbonyl]pyridin-2-amine |
| E-117 | 4-{3-[4-fluoro-1-({5-[3-(trifluoromethyl)phenyl])-1,2,4-oxadiazol-3-ylimethyl)-1H-indazol-3-yl]azetidine-1-carbonyl}pyridin-2-amine |
| E-118 | 2-{3-[1-(2-amino-1,3-thiazole-5-carbonyl)piperidin-4-yl]-4-fluoro-1H-indazol-1-yl}-1-[(2S)-2-(trifluoromethyl)pyrrolidin-1-yl]ethan-1-one |

**[Table 59]**

| Compound number | Chemical Name |
|---|---|
| E-119 | 2-{3-[1-(2-amino-4-methyl-1,3-thiazole-5-carbonyl)piperidin-4-yl]-4-fluoro-1H-indazol-1-yl}·1-[(2S)-2-(trifluoromethyl)pyrrolidin-1-yl]ethan-1-one |
| E-120 | 2-{3-[1-(2-aminopyridine-4-carbonyl)azetidin-3-yl]-1H-pyrazolo(4,3-blpyridin-1-yl}-1-[(28)-2-(trifluoromethyl)pyrrolidin-1-yl]ethan-1-one |
| E-121 | 2-(3-{1-[2-(cyclopropylamino)pyridine-4-carbonyl]azetidin-3-yl}-1H-pyrazolo[4,3-b]pyridin-1-yl)-1-[(2S)-2-(trifluoromethyl)pyrrolidin-1-yl]ethan-1-one |
| E-122 | 2-{3-[1-(2-amino-4-methyl-1,3-thiazole-5-carbonyl)azetidin-3-yl]-1H-pyrazolo[4,3-blpyridin-1-yl)-1-[(2S)-2-(trifluoromethyl)pyrrolidin-1-yl]ethan-1-one |
| E-123 | 2-{3-[1-(2-aminopyridine-4-carbonyl)azetidin-3-yl]-4,6-difluoro-1H-indazol-1-yl}-1-[(3S)-3-(trifluoromethyl)morpholin-4-yl]ethan-1-one |
| E-124 | 2-{3-[1-(2-aminopyridine-4-carbonyl)-1,2,3,6-tetrahydropyridin-4-yl]-4-fluoro-1H-indol-1-yl}-1-[(2S)-2-(trifluoromethyl)pyrrolidin-1-yllethan-1-one |
| E-125 | 2-13-[1-(2-aminopyridine-4-carbonyl)azetidin-3-yl]-4,6-difluoro-1H-indazol-1-yl}-1-[(38)-3-(difluoromethoxy)pyrrolidin-1-yl]ethan-1-one |
| E-126 | 2-{3-[1-(2-aminopyridine-4-carbonyl)azetidin-3-yl]-4,6-difluoro-1H-indazol-1-yl}-1-[(3R)-3-(difluoromethoxy)pyrrolidin-1-yl]ethan-1-one |
| E-127 | 2-{3-[1-(2-aminopyridine-4-carbonyl)azetidin-3-yl]-4,6-difluoro-1H-indazol-1-yl}-1-(2,2-dimethylpyrrolidin-1-yl)ethan-1-one |
| E-128 | 4-[3-(4-fluoro-1-{[5-(trifluoromethyl)-1,3,4-oxadiazol-2-yl]methyl}-1H-indazol-3-yl)azetidine-1-carbonyl}pyridin-2-amine |
| E-129 | 2-{3-[1-(2-aminopyrimidine-4-carbonyl)azetidin-3-yl}-4,6-difluoro-1H-indazol-1-yl}-1-[(2S)-2-(trifluoromethyl)pyrrolidin-1-yl]ethan-1-one |
| E-130 | 2-(3-(1-[2-(cyclopropylamino)pyridine-4-carbonyl]azetidin-3-yl}-5-methyl-1H-pyrazolo[4,3-b}pyridin-1-yl)-1-[(2S)-2-(trifluoromethyl)pyrrolidin-1-yl]ethan-1-one |

**[Table 60]**

| Compound number | Chemical Name |
|---|---|
| E-131 | 2-{3-[1-(6-aminopyrimidine-4-carbonyl)azetidin-3-yl]-4,6-difluoro-1H-indazol-1-yl}-1-[(28)-2-(trifluoromethyl)pyrrolidin-1-yl]ethan-1-one |
| E-132 | 2-{4,6-difluoro-3-[1-(pyridine-4-carbonyl)azetidin-3-yl]-1H-indazol-1-yl}-1-[(2S)-2-(trifluoromethyl)pyrrolidin-1-yl]ethan-1-one |
| E-133 | 2-{4,6-difluoro-3-[1-(3-methoxypyridine-4-carbonyl)azetidin-3-yl)-1H-indazol-1-yl}-1-[(28)-2-(trifluoromethyl)pyrrolidin-1-yl]ethan-1-one |
| E-134 | 4-[4-(4-fluoro-1-([6-(trifluoromethyl)pyridin-3-yl]methyl)-1H-indazol-3-yl)piperidine-1-carbonyl]pyridin-2-amine |
| E-135 | 2-{3-[1-(2-aminopyridine-4-carbonyl)piperidin-4-yl)-4-fluoro-1H-indol-1-yl}-1-[(2R)-2-(trifluoromethyl)morpholin-4-yl]ethan-1-one |
| E-136 | 2-{3-[1-(2-aminopyridine-4-carbonyl)piperidin-4-yl]-4-fluoro-1H-indol-1-yl}-1-[(2S)-2-(trifluoromethyl)morpholin-4-yl]ethan-1-one |
| E-137 | 4-[3-(4-fluoro-1-{[5-(oxan-4-yl)-1,2,4-oxadiazol-3-yl]methyl}-1H-indazol-3-yl)azetidine-1-carbonyllpyridin-2-amine |
| E-138 | 4-[3-(4-fluoro-1-{(S-(pyridin-4-yl)-1,2,4-oxadiazol-5-yl]methyl}-1H-indazol-3-yl)azetidine-1-carbonyl]pyridin-2-amine |
| E-139 | 2-(3-{1-[2-amino-4-(trifluoromethyl)-1,3-thiazole-5-carbonyl]azetidin-3-yl}-4,6-difluoro-1H-indazol-1-yl)-1-[(2S)-2-(trifluoromethyl)pyrrolidin-1-yl]ethan-1-one |
| E-140 | 2-{4,6-difluoro-3-[1-(1H-indazole-5-carbonyl)azetidin-3-yl]-1H-indazol-1-yl}-1-[(28)-2-(trifluoromethyl)pyrrolidin-1-yl]ethan-1-one |
| E-141 | 2-[4,6-difluoro-3-(1-{1H-pyrazolo[4,3-b]pyridine-5-carbonyl}azetidin-3-yl)-1H-indazol-1-yl]-1-[(2S)-2-(trifluoromethyl)pyrrolidin-1-yllethan-1-one |
| E-142 | 2-{4,6-difluoro-3-[1-(3-fluoropyridine-4-carbonyl)azetidin-3-yl]-1H-indazol-1-yl]-1-[(2S)-2-(trifluoromethyl)pyrrolidin-1-yl]ethan-1-one |

**[Table 61]**

| Compound number | Chemical Name |
|---|---|
| E-143 | 2-{3-[1-(3-chloropyridine-4-carbonyl)azetidin-3-yl]-4,6-difluoro-1H-indazol-1-yl}-1-[(28)-2-(trifluoromethyl)pyrrolidin-1-yl]ethan-1-one |
| E-144 | 2-{3-[1-(2-aminopyridine-4-carbonyl)azetidin-3-yl]-4-chloro-5-fluoro-1H-indazol-1-yl}-1-[(2S)-2-(trifluoromethyl)pyrrolidin-1-yl]ethan-1-one |
| E-145 | 2-{3-[1-(2-amino-3-fluoropyridine-4-carbonyl)azetidin-3-yl]-4,6-difluoro-1H-indazol-1-yl}-1-[(2S)-2-(trifluoromethyl)pyrrolidin-1-yllethan-1-one |
| E-146 | 4-{3-[4-fluoro-1-({5-[1-(trifluoromethyl)cyclobutyl]-1,2,4-oxadiazol-3-yl}methyl)-1H-indazol-3-yl]azetidine-1-carbonyl}pyridin-2-amine hydrochloride |
| E-147 | 4-{3-[4-fluoro-1-({5-[1-(trifluoromethyl)cyclopropyl]-1,2,4-oxadiazol-3-yl}methyl)-1H-indazol-3-yllazetidine-1-carbonyl}pyridin-2-amine |
| E-148 | 4-[3-(4-fluoro-1-i[5-(2-fluorophenyl)-1,2,4-oxadiazol-3-yl] methyl}-1H-indazol-3-yl)azetidine-1-carbonyllpyridin-2-amine |
| E-149 | 2-{3-[1-(2-amino-4-chloro-1,3-thiazole-5-carbonyl)azetidin-3-yl]-4,6-difluoro-1H-indazol-1-yl}-1-[(2S)-2-(trifluoromethyl)pyrrolidin-1-yllethan-1-one |
| E-150 | 4-[3-(4-fluoro-1-{[5-(trifluoromethyl)pyridin-2-yl]methyl}·1H-pyrazolo[3,4-clpyridin-3-yl)azetidine-1-carbonyl]pyridin-2-amine |
| E-151 | 4-[3-(4-fluoro-1-{[4-(trifluromethyl)phenyl]methyl}-1H-pyrazolo[3,4-c]pyridin-3-yl)azetidine-1-carbonyl]pyridin-2-amine |
| E-152 | 4-{3-[4,6-difluoro-1-(3-phenylprop-2-yn-1-yl)-1H-indazol-3-yl]azetidine-1-carbonyl)pyridin-2-amine |
| E-153 | 4-{3-(4-fluoro-1-{[5-(5-fluoropyridin-2-yl)-1,2,4-oxadiazol-3-yl]methyl}-1H-indazol-3-yl)azetidine-1-carbonyl]pyridin-2-amine |
| E-154 | 4-[3-(4-fluoro-1-{[5-(6-methylpyridazin-3-yl)-1,2.4-oxadiazol-3-yl}methyl}-1H-indazol-3-yl)azetidine-1-carbony1]pyridin-2-amine |

**[Table 62]**

| Compound number | Chemical Name |
|---|---|
| E-155 | 2-{3-[1-(2-amino-5-fluoropyridine-4-carbonyl)azetidin-3-yl}-4,6-difluoro-1H-indazol-1-yl}-1-[(28)-2-(trifluoromethylpyrrolidin-1-yllethan-1-one |
| E-156 | 4-{3-[1-(3-cyclopropylprop-2-yn-1-yl)-4,8-difluoro-1H-indazol-3-yl]azetidine-1-carbonyl}pyridin-2-amine |
| E-157 | 4-(3-{4,6-difluoro-1-[(1-phenylazetidin-3-yl)methyl)-1H-indazol-3-yl}azetidine-1-carbonyl)pyridin-2-amine |
| E-158 | 2-{3-[1-(2-aminopyridine-4-carbonyl)azetidin-3-yl]-4,6-difluoro-1H-indazol-1-yl}-1-(1,2,3,4-tetrahydroquinolin-1-yl)ethan-1-one |
| E-159 | 2-{3-{1-(2-aminopyridine-4-carbonyl)azetidin-3-yl]-4,6-difluoro-1H-indazol-1-yl]-1-(3,4-dihydro-2H-1,4-benzoxazin-4-yl)ethan-1-one |
| E-160 | 4-[3-(4-fluoro-1-{[5-(2-fluorophenyl)-1,2,4-oxadiazol-3-yl]methyl}-1H-pyrazolo[3,4-c]pyridin-3-yl)azetidine-1-carbonyl]pyridin-2-amine |
| E-161 | 2-{3-[1-(2-aminopyridine-4-carbonyl)azetidin-3-yl]-4,6-difluoro-1H-indazol-1-yll-1-(6-bromo-2,3-dihydro-1H-indol-1-yl)ethan-1-one |
| E-162 | 2-{4,6-difluoro-3-[1-(oxane-4-carbonyl)azetidin-3-yl]-1H-indazol-1-yl}-1-[(2S)-2-(trifluoromethyl)pyrrolidin-1-yl]ethan-1-one |
| E-163 | 2-{4,6-difluoro-3-[1-(2-hydroxypyridine-4-carbonylazetidin-3-yl]-1H-indazol-1-yl]-1-[(2S)-2-(trifluoromethyl)pyrrolidin-1-yl]ethan-1-one |
| E-164 | 2-(4,6-difluoro-3-[1-(1H-pyrazole-4-carbonyl)azetidin-3-yl]-1H-indazol-1-yl}-1-[(28)-2-(trifluoromethyl)pyrrolidin-1-yl]ethan-1-one |
| E-165 | 2-{4,6-difluoro-3-[1-(5-methyl-1H-pyrazole-4-carbonyl)azetidin-3-yl]-1H-indazol-1-yl}-1-[(28)-2-(trifluoromethyl)pyrrolidin-1-yl]ethan-1-one |
| E-166 | 2-(4,6-difluoro-3-{1-[5-(trifluoromethyl)-1H-pyrazole-4-carbonyl]azetidin-3-yl}-1H-indazol-1-yl)-1-[(2S)-2-(trifluoromethyl)pyrrolidin-1-yl]ethan-1-one |

**[Table 63]**

| Compound number | Chemical Name |
|---|---|
| E-167 | 4-[3-(4,6-difluoro-1-{[1-(2,2,2-trifluoroethyl)azetidin-3-yl]methyl}·1H-indazol-3-yl)azetidine-1-carbonyllpyridin-2-amine |
| E-168 | 4-[3-(5-methyl-1-{[4-(trifluoromethyl)phenyl]methyl]-1H-pyrazolo[3,4-c]pyridin-3-yl)azetidine-1-carbonyl]pyridin-2-amine |
| E-169 | 2-{3-[1-(1H-1,2,3-benztriazole-5-carbonyl)azetidin-3-yl]-4,6-difluoro-1H-indazol-1-yl}-1-[(2S)-2-(trifluoromethyl)pyrrolidin-1-yl]ethan-1-one |
| E-170 | 4-[3-(4-fluoro-1-{[3-(3-fluoropyridin-4-yl)-1,2,4-oxadiazol-5-yl]methyl}-1H-pyrazolo[3,4-clpyridin-3-yl)azetidine-1-carbonyl] pyridin-2-amine |
| E-171 | 4-(3-{1-[(2,3-dihydro-1H-inden-2-yl)methyl]-4,6-difluoro-1H-indazol-3-yl}azetidine-1-carbonyl)pyridin-2-amine |
| E-172 | 2-{3-[1-(2-aminopyridine-4-carbonylazetidin-3-yl]-4,6-difluoro-1H-indazol-1-yl}-1-(6-cyclopropyl-2,3-dihydro-1H-indol-1-yl)ethan-1-one |
| E-173 | 2-(3-[1-(2-amino-4-methyl-1,3-thiazole-5-carbonyl)azetidin-3-yl]-4,6-difluoro-1H-indazol-1-yl}-1-[(2R)-2-(trifluoromethyl)morpholin-4-yl]ethan-1-one |
| E-174 | 2-{3-[1-(2-amino-5-fluoropyridine-4-carbonyl)piperidin-4-yl]-4-fluoro-1H-indazol-1-yl}-1-[(2S)-2-(trifluoromethyl)pyrrolidin-1-yl]ethan-1-one |
| E-175 | 2-{3-[1-(2-amino-5-fluoropyridine-4-carbonyl)azetidin-3-yl]-4,6-difluoro-1H-indazol-1-yl}-1-[(2S)-2-[(trifluoromethoxy)methyl]pyrrolidin-1-yl]ethan-1-one |
| E-176 | 2-{3-[1-(2-amino-5-fluoropyridine-4-carbonyl)azetidin-3-yl)-4,6-difluoro-1H-indazol-1-yl}-1-(2,2-dimethylmorpholin-4-yl)ethan-1-one |
| E-177 | 2-{3-[1-(2-amino-5-fluoropyridine-4-carbonyl)azetidin-3-yl)-4,6-difluoro-1H-indazol-1-yl}-1-(3,3-dimethylmorpholin-4-yl)ethan-1-one |
| E-178 | 2-{3-[1-(2-amino-3-methylpyridine-4-carbonyl)azetidin-3-yl]-5-methyl-1H-pyrazolol3,4-clpyridin-1-yl}-1-[(2R)-2-(trifluoromethyl)morpholin-4-yl]ethan-1-one dihydrochloride |

**[Table 64]**

| Compound number | Chemical Name |
|---|---|
| E-179 | 2-{3-[1-(2-amino-5-fluoropyridine-4-carbonyl)azetidin-3-yl]-4,6-difluoro-1H-indazol-1-yl}-1-f(2S)-2-(methoxymethyl)pyrrolidin-1-yllethan-1-one |
| E-180 | 2-{3-[1-(2-amino-3-methylpyridine-4-carbonyl)azetidin-3-yl]-4-fluoro-1H-pyrazolo{3,4-c)pyridin-1-yl}-1-[(2R)-2-(trifluoromethyl)morpholin-4-yl}ethan-1-one |
| E-181 | 2-{3-[1-(2-amino-5-fluoropyridine-4-carbonyl)azetidin-3-yl]-4-fluoro-1H-pyrazolo[3,4-clpyridin-1-yl}-1-[(2R)-2-(trifluoromethyl)morpholin-4-yl]ethan-1-one |
| E-182 | 2-{3-[1-(2-amino-5-fluoropyridine-4-carbonyl)azetidin-3-yl]-4,6-difluoro-1H-indazol-1-yl}-1-[(3S,5S)-3,5-dimethylmorpholin-4-yl]ethan-1-one |
| E-183 | 2-{3-[1-(2-amino-4-methyl-1,3-thiazole-5-carbonylazetidin-3-yl)-4-fluoro-1H-pyrazolo[3,4-c]pyridin-1-yl}-1-[(2S)-2-[(trifluoromethoxy)methyl]pyrrolidin-1-yl]ethan-1-one p-toluenesulfonate |
| E-184 | 2-{3-[1-(2-amino-3-fluoropyridine-4-carbonyl)azetidin-3-yl]-4,6-difluoro-1H-indazol-1-yl}-1-[(2R)-2-(trifluoromethyl)morpholin-4-yl]ethan-1-one p-toluenesulfonate |
| E-185 | 2-{3-[1-(2-amino-5-fluoropyridine-4-carbonyl)azetidin-3-yl]-5-methyl-1H-pyrazolo(4,3-blpyridin-1-yl}-1-[{(2R)-2-(trifluoromethyl)morpholin-4-yl]ethan-1-one p-toluenesulfonate |
| E-186 | 2-{3-[1-(2-amino-5-fluoropyridine-4-carbonyl)azetidin-3-yl]-5-methyl-1H-pyrazolo[4,3-b]pyridin-1-yl}-1-(3,3-dimethylmorpholin-4-yl)ethan-1-one p-toluenesulfonate |
| E-187 | 2-{3-[1-(2-amino-5-fluoropyridine-4-carbonyl)azetidin-3-yl]-1H-indazol-1-yl}-1-(3,3-dimethylmorpholin-4-yl)ethan-1-one |
| E-188 | 2-{3-[1-(2-aminopyridine-4-carbonyl)azetidin-3-yl]-1H-indazol-1-yl}-1-(3,3-dimethylmorpholin-4-yl)ethan-1-one |
| E-189 | 2-(3-[1-(2-aminopyridine-4-carbonyl)azetidin-3-yl]-4,6-difluoro-1H-indazol-1-yl}-N-tert-butylacetamide |
| E-190 | 2-{3-[1-(2-amino-3-fluoropyridine-4-carbonyl)azetidin-3-yl]-1H-indazol-1-yl}-1-(3,3-dimethylmorpholin-4-yl)ethan-1-one |

**[Table 65]**

| Compound number | Chemical Name |
|---|---|
| E-191 | 2-{3-[1-(2-amino-3-methylpyridine-4-carbonyl)azetidin-3-yl]-1H-indazol-1-yl}-1-(3,3-dimethylmorpholin-4-yl)ethan-1-one |
| E-192 | 2-{3-[1-(2-amino-5-fluoropyridine-4-carbonyl)azetidin-3-yl]-1H-indazol-1-yl}-1-[(2R)-2-(trifluoromethyl)morpholin-4-yl]ethan-1-one |
| E-193 | 2-{3-[1-(2-aminopyridine-4-carbonyl)azetidin-3-yl]-1H-indazol-1-yl}-1-[(2R)-2-(trifluoromethyl)morpholin-4-yl]ethan-1-one |
| E-194 | 2-{3-[1-(2-amino-3-fluoropyridine-4-carbonyl)azetidin-3-yl]-1H-indazol-1-y1}-1-[(2R)-2-(trifluoromethyl)morpholin-4-yl]ethan-1-one |
| E-195 | 2-(3-[1-(2-amino-3-methylpyridine-4-carbonyl)azetidin-3-yl]-1H-indazol-1-yl}-1-[(2S)-2-(trifluoromethyl)pyrrolidin-1-yl]ethan-1-one |
| E-196 | 2-{3-[1-(2-amino-3-fluoropyridine-4-carbonyl)azetidin-3-yl]-1H-indazol-1-yl}-1-[(28)-2-(trifluoromethyl)pyrrolidin-1-yl]ethan-1-one |
| E-197 | 2-(3-[1-(2-amino-4-methyl-1,3-thiazole-5-carbonyl)azetidin-3-yl]-1H-indazol-1-yl}-1-[(2S)-2-(trifluoromethyl)pyrrolidin-1-yl]ethan-1-one |
| E-198 | 2-{3-[1-(2-aminopyridine-4-carbonyl)azetidin-3-yl]-1H-indazol-1-yl}-N-tert-butylacetamide |
| E-199 | 2-{3-[1-(2-amino-5-fluoropyridine-4-carbonyl)azetidin-3-yl]-1H-indazol-1-yl}-N-tert-butylacetamide |
| E-200 | 2-{3-[1-(2-aminopyridine-4-carbonyl)azetidin-3-yl]-5-methyl-1H-indazol-1-yl}-1-[(2S)-2-(trifluoromethyl)pyrrolidin-1-yl]ethan-1-one |
| E-201 | 2-{3-[1-(2-amino-5-fluoropyridine-4-carbonyl)azetidin-3-yl]-5-methyl-1H-indazol-1-yl}-1-[(28)-2-(trifluoromethyl)pyrrolidin-1-yl]ethan-1-one |
| E-202 | 2-{3-[1-(2-aminopyridine-4-carbonyl)azetidin-3-yl]-5-fluoro-1H-indazol-1-yl}-1-[(28)-2-(trifluoromethyl)pyrrolidin-1-yl]ethan-1-one |

**[Table 66]**

| Compound number | Chemical Name |
|---|---|
| E-203 | 2-{3-[1-(2-amino-6-fluoropyridine-4-carbonyl)azetidin-3-yl]-5-fluoro-1H-indazol-1-yl}-1-[(2S)-2-(trifluoromethyl)pyrrolidin-1-yl]ethan-1-one |
| E-204 | 2-{3-[1-(2-amino-5-fluoropyridine-4-carbonylazetidin-3-yl]-5-chloro-1H-indazol-1-yl}-1-[(2R)-2-(trifluoromethyl)morpholin-4-yl}ethan-1-one |
| E-205 | 2-{3-[1-(2-aminopyridine-4-carbonyl)azetidin-3-yl]-5-chloro-1H-indazol-1-yl}-1-[(2R)-2-(trifluoromethyl)morpholin-4-yl]ethan-1-one |
| E-206 | 2-{3-[1-(2-amino-5-fluoropyridine-4-carbonyl)azetidin-3-yl}-5-chloro-1H-indazol-1-yl}-1-[(2S)-2-(trifluoromethyl)pyrrolidin-1-yl]ethan-1-one |
| E-207 | 2-{3-[1-(2-aminopyridine-4-carbonyl)azetidin-3-yl]-6-chloro-1H-indazol-1-yl}-1-[(28)-2-(trifluoromethyl)pyrrolidin-1-yl]ethan-1-one |
| E-208 | 2-{3-[1-(2-aminopyridine-4-carbonyl)azetidin-3-yll-1H-indazol-1-yl}-N-(1-methoxy-2-methylpropan-2-yl)acetamide |
| E-209 | 2-{3-[1-(2-aminopyridine-4-carbonyl)azetidin-3-yl]-1H-indazol-1-yl}-N-(4-methyloxan-4-yl)acetamide |
| E-210 | 2-{3-[1-(2-amino-5-chloropyridine-4-carbonyl)azetidin-3-yl]-1H-indazol-1-yl]-1-[(2S)-2-(trifluoromethyl)pyrrolidin-1-yl]ethan-1-one |
| E-211 | 2-{3-[1-(2-aminopyridine-4-carbonyl)azetidin-3-yl]-1H-indazol-1-yl}-N-(2,2,2-trifluoroethyl)acetamide |
| E-212 | 2-{3-[1-(2-aminopyridine-4-carbonyl)azetidin-3-yl]-1H-indazol-1-yl}-N-[(2R)-1,1,1-trifluoropropan-2-yl]acetamide |
| E-213 | 2-{3-[1-(2-aminopyridine-4-carbonyl)azetidin-3-yl]-1H-indazol-1-yl}-N-[(2S)-1,1,1-trifluoropropan-2-yl]acetamide |
| E-214 | 2-{3-[1-(2-aminopyridine-4-carbonyl)azetidin-3-yl]-1H-indazol-1-yl}-N-(3,3-difluorocyclobutyl)acetamide |
| E-215 | 2-{3-[1-(2-aminopyrimidine-4-carbonyl)azetidin-3-yl]-1H-indazol-1-yl}-1-[(2R)-2-(trifluoromethyl)morpholin-4-yl]ethan-1-one |

**[Table 67]**

| Compound number | Chemical Name |
|---|---|
| E-216 | 4-[3-(4-fluoro-1-([5-(trifluoromethyl)-1,2,4-oxadiazol-3-yl]methyl)-1H-indazol-3-yl)azetidine-1-carbonyl]pyridin-2-amine |
| E-217 | 4-[3-(1-{[5-(trifluoromethyl)-1,2.4-oxadiazol-3-yl]methyl}-1 H-indazol-3-yl)azetidine-1-carbonyl]pyridin-2-amine |
| E-218 | 4-[3-(1-{[3-(trifluoromethyl)-1,2,4-oxadiazol-5-yllmethyl}- 1H-indazol-3-yl)azetidine-1-carbonyl]lpyridin-2-amine |
| E-219 | 2-{3-[1-(2-aminopyridine-4-carbonyl)azetidin-3-yl]-1H-indazol-1-yl]-1-[(2S)-2-(hydroxymethyl)pyrrolidin-1-yl]ethan-1-one |
| E-220 | tert-butyl 2-{3-[1-(2-amino-5-fluoropyridine-4-carbony)azetidin-3-yl]-1H-indazol-1-yl}acetate |
| E-221 | 2-{3-[1-(2-aminopyridine-4-carbonyl)azetidin-3-yl]-1H-indazol-1-yl}-1-(2,2-dimethylpyrrolidin-1-yl)ethan-1-one |
| E-222 | 2-{3-[1-(2-aminopyridine-4-carbonyl)azetidin-3-yl)-1H-indazol-1-yl}-1-[(2R)-2-ethylpyrrolidin-1-yl]ethan-1-one |
| E-223 | 2-{3-[1-(2-aminopyridine-4-carbonyl)azetidin-3-yl]-1H-indazol-1-yl}-1-{2-azabicyclo[3.1.0)hexan-2-yl}ethan-1-one |
| E-224 | 2-{3-[1-(2-aminopyridine-4-carbonyl)azetidin-3-yl]-1H-indazol-1-yl}-N-(dicyclopropylmethyl)acetamide |
| E-225 | 2-{3-[1-(2-aminopyridine-4-carbonyl)azetidin-3-yl}-1H-indazol-1-yl}-N-(2,2-difluorocyclopentyl)acetamide |
| E-226 | 2-{3-[1-(2-aminopyridine-4-carbonyl)azetidin-3-yl]-1H-indazol-1-yl}-N-(4-fluorophenyl)acetamide |
| E-227 | 2-{3-[1-(2-aminopyridine-4-carbonyl)azetidin-3-yl)-1H-indazol-1-yl}-N-[2-(trifluoromethoxy)ethyl]acetamide |
| E-228 | 2-{3-[1-(2-aminopyridine-4-carbonyl)azetidin-3-yl}-1H-indazol-1-yl}-1-[(2S)-2-(methoxymethyl)pyrrolidin-1-yl]ethan-1-one |
| E-229 | 2-{3-[1-(2-aminopyridine-4-carbonyl)azetidin-3-yl]-1H-indazol-1-yl}-N-(cyclopropylmethyl)-N-methylacetamide |
| E-230 | 2-{3-[1-(2-amino-5-fluoropyridine-4-carbonyl)azetidin-3-yl]-1H-indaaol-1-yl}-1-[(3S)-3-methoxypyrrolidin-1-yl]ethan-1-one |

**[Table 68]**

| Compound number | Chemical Name |
|---|---|
| E-231 | 2-{3-[1-(2-amino-5-fluoropyridine-4-carbonyl)azetidin-3-yl]-1H-indazol-1-yl}-1-1(3R)-3-methoxypyrrolidin-1-yllethan-1-one |
| E-232 | 2-{3-[1-(2-amino-5-fluoropyridine-4-carbonyl)azetidin-3-yl]-1H-indazol-1-yl}-1-{2-oxa-6-azaspiro[3.4]octan-6-yl}ethan-1-one |
| E-233 | 2-{3-[1-(2-aminopyridine-4-carbonyl)azetidin-3-yl]-1H-indazol-1-yl}-N-(oxan-4-yl)acetamide |
| E-234 | 2-{3-[1-(2-aminopyridine-4-carbonyl)azetidin-3-yl]-1H-indazol-1-yl}-N-(2-cyclopropylpropan-2-yl)acetamide |
| E-235 | 2-{3-[1-(2-aminopyridine-4-carbonyl)azetidin-3-yl]-1H-indazol-1-yl}-N-(4,4-difluorocyclohexyl)acetamide |
| E-236 | (2S)-1-(2-13-[1-(2-amino-5-fluoropyridine-4-carbonyl)azetidin-3-yl]-1H-indazol-1-yl}acetyl)pyrrolidine-2-carbonitrile |
| E-237 | 2-{3-[1-(2-amino-5-fluoropyridine-4-carbonyl)azetidin-3-yl]-1H-indazol-1-yl]-1-[(2S)-4,4-difluoro-2-(fluoromethyl)pyrrolidin-1-yl]ethan-1-one |
| E-238 | 2-{3-[1-(2-aminopyridine-4-carbonyl)azetidin-3-yl]-4-fluoro-1H-indazol-1-yl}-N-{bicyclo[2.2.1]heptan-2-yl}acetamide |
| E-239 | 2-{3-[1-(2-aminopyridine-4-carbonyl)azetidin-3-yl]-4-fluoro-1H-indazol-1-yl}-N-{bicyclo[1.1.1]pentan-1-yl}acetamide |
| E-240 | 2-{3-[1-(2-amino-5-fluoropyridine-4-carbonyl)azetidin-3-yl}-1H-indazol-1-yl}-N-methyl-N-(2,2,2-trifluoroethyl)acetamide |
| E-241 | 2-{3-[1-(2-aminopyrimidine-4-carbonyl)azetidin-3-yl]-5-fluoro-1H-indazol-1-yl}-1-[(2S)-2-(trifluoromethyl)pyrrolidin-1-yl]ethan-1-one |
| E-242 | 2-{3-[1-(2-aminopyridine-4-carbonyl)azetidin-3-yl]-4-fluoro-1H-indazol-1-yl}-N-(2,2-difluoroethyl)acetamide |
| E-243 | 2-{3-[1-(2-amino-5-fluoropyridine-4-carbonyl)azetidin-3-yl]-1H-indazol-1-yl}-N-[trans-2-fluorocyclopropyl]acetamide |
| E-244 | 2-{3-{1-(2-amino-5-fluoropyridine-4-carbonyl)azetidin-3-yl]-1H-indazol-1-yl}-N-(1-cyanocyclopropyl)acetamide |

**[Table 69]**

| Compound number | Chemical Name |
|---|---|
| E-245 | 2-{3-(1-(2-amino-5-fluoropyridine-4-carbonyl)azetidin-3-yl]-1H-indazol-1-yl}-N-[(1R,2S)-2-fluorocyclopropyl]acetamide |
| E-246 | 2-{3-[1-(2-amino-5-fluoropyridine-4-carbonyl)azetidin-3-yl]-1H-indazol-1-yl}-N-[(1S,2R)-2-fluorocyclopropyl]acetamide |
| E-247 | 2-{3-[1-(2-amino-5-fluoropyridine-4-carbonyl)azetidin-8-yl]-1H-indazol-1-yl}-N-(1-methyl-1H-pyrazol-5-yl)acetamide |
| E-248 | 2-{3-[1-(2-amino-5-fluoropyridine-4-carbonyl)azetidin-3-yl]-1H-indazol-1-yl}-N-(2,2-difluorocyclopropyl)acetamide |
| E-249 | 2-{3-[1-(2-amino-5-fluoropyridine-4-carbonyl)azetidin-3-yl]-1H-indazol-1-yl}-N-ethyl-N-(2,2,2-trifluoroethyl)acetamide |
| E-250 | 2-{3-[1-(2-amino-5-fluoropyridine-4-carbonyl)azetidin-3-yl]-1H-indazol-1-yl}-N-(3,3-difluorocyclobutyl)-N-methylacetamide |
| E-251 | 2-{3-[1-(2-amino-5-fluoropyridine-4-carbonyl)azetidin-3-yl]-1H-indazol-1-yl}-N-cyclopropyl-N-(2,2,2-trifluoroethyl)acetamide |
| E-252 | 2-13-[1-(2-amino-5-fluoropyridine-4-carbonyl)azetidin-3-yl]-1H-indazol-1-yl}-N-cyclopropyl-N-methylacetamide |
| E-253 | 2-{3-[1-(2-amino-5-fluoropyridine-4-carbonyl)azetidin-3-yl]-1H. indazol-1-yl}-N-methyl-N-(propan-2-yl)acetamide |
| E-254 | 2-{3-{1-(2-amino-5-fluoropyridine-4-carbonyl)azetidin-3-yl]-1H-indazol-1-yl}-N-methyl-N-(oxetan-3-yl)acetamide |
| E-255 | 2-{8-[1-(2-amino-5-fluoropyridine-4-carbonyl)azetidin-8-yl]-1H-indazol-1-yl}-N-methyl-N-(oxan-4-yl)acetamide |
| E-256 | 2-{3-[1-(2-amino-5-fluoropyridine-4-carbonyl)azetidin-3-yl]-1H-indazol-1-yl]-N-[(3,3-difluorocyclobutyl)methyl]-N-methylacetamide |
| E-257 | 2-{3-[1-(2-amino-5-fluoropyridine-4-carbonyl)azetidin-3-yl]-1H-indazol-1-yl}-1-(4,4-difluoropiperidin-1-yl)ethan-1-one |
| E-258 | 2-{3-[1-(2-amino-5-fluoropyridine-4-carbonyl)azetidin-3-yl]-1H-indazol-1-yll-1-18-oxa-3-azabicyclo[3.2.1}octan-3-yl]ethan-1-one |

**[Table 70]**

| Compound number | Chemical Name |
|---|---|
| E-259 | 2-{3-[1-(2-amino-5-fluoropyridine-4-carbonyl)azetidin-3-yl}-1H-indazol-1-yl}-1-[3-(trifluoromethyl)piperidin-1-yl]ethan-1-one |
| E-260 | indazol-1-yl}-1-(4-methylpiperazin-1-yl)ethan-1-one |
| E-261 | 2-{3-[1-(2-amino-5-fluoropyridine-4-carbonyl)azetidin-3-yl]-1H-indazol-1-yl}-N-methyl-N-[(2S)-1,1,1-trifluoropropan-2-yl]acetamide |
| E- 262 | 2-{3-[1-(2-amino-5-fluoropyridine-4-carbonyl)azetidin-3-yl]-1H-indazol-1-yl}-N-methyl-N-[(2R)-1,1,1-trifluoropropan-2-yl]acetamide |
| E-263 | 2-{3-[1-(2-amino-5-fluoropyridine-4-carbonyl)azetidin-3-yl]-1H-indazol-1-yl}-N,N-diethylacetamide |
| E-264 | 2-{3-[1-(2-amino-5-fluoropyridine-4-carbonyl)azetidin-3-yl]-1H-indazol-1-yl}-N-methyl-N-[2-(morpholin-4-yl)ethyllacetamide |
| E-265 | 2-{3-[1-(2-amino-5-fluoropyridine-4-carbonyl)azetidin-3-yl]-1H-indazol-1-yl}-N-(2-methoxyethyl)-N-methylacetamide |
| E-266 | 2·{3-[1-(2-amino-5-fluoropyridine-4-carbonyl)azetidin-3-yl]-1H-indazol-1-yl}-1-[(2R,68)-2,6-dimethylpiperidin-1-yl]ethan-1-one |
| E-267 | 2-{3-[1-(2-amino-5-fluoropyridine-4-carbonyl)azetidin-3-yl]-1H-indazol-1-yl}-N,N-bis(propan-2-yl)acetamide |
| E-268 | 2-{3-[1-(2-amino-5-fluoropyridine-4-carbonyl)azetidin-3-yl]-1H-indazol-1-yl}-N-tert-butyl-N-methylacetamide |
| E-269 | 2-{3-[1-(2-amino-5-fluoropyridine-4-carbonyl)azetidin-3-yl]-1H-indazol-1-yl}-N-(2,2-difluoroethyl)-N-methylacetamide |
| E-270 | 2-{3-(1-(2-amino-5-fluoropyridine-4-carbonyl)-3-methylazetidin-3-yl]-1H-indazol-1-yl}-N-methyl-N-(2,2,2-trifluoroethyl)acetamide |
| E-271 | 2-{3-[1-(2-amino-5-fluoropyridine-4-carbonyl)-3-methylazetidin-3-yl]-1H-indazol-1-yl}-1-[(2S)-2-(trifluoromethyl)pyrrolidin-1-yl]ethan-1-one |
| E-272 | 2-{3-[1-(2-amino-5-fluoropyridine-4-carbonyl)azetidin-3-yl]-1H-indazol-1-yl}-1-[(2S)-2-(difluoromethyl)pyrrolidin-1-yl]ethan-1-one |

**[Table 71]**

| Compound number | Chemical Name |
|---|---|
| E-273 | 2-{3-[1-(2-aminopyridine-4-carbonyl)-3-methylazetidin-3-yl]-1H-indazol-1-yl}-N-methyl-N-(2,2,2-trifluoroethyl)acetamide |
| E-274 | 2-{3-[1-(2-amino-5-fluoropyridine-4-carbonyl)azetidin-3-yl]-5-chloro-1H-indazol-1-yl}-N-methyl-N-(2,2,2-trifluoroethyl)acetamide |
| E-275 | 2-{3-[1-(2-amino-5-fluoropyridine-4-carbonyl)azetidin-3-yl]-6-fluoro-1H-indazol-1-yl}-1-[(2S)-2-(trifluoromethyl)pyrrolidin-1-yl]ethan-1-one |
| E-276 | 2-{3-[1-(2-amino-5-fluoropyrimidine-4-carbonyl)azetidin-3-yl)-1H-indazol-1-yl}-N-methyl-N-(2,2,2-trifluoroethyl)acetamide |
| E-277 | 2-{3-[1-(2-amino-5-fluoropyridine-4-carbonyl)azetidin-3-yl-5-fluoro-1H-indazol-1-yl}-N-methyl-N-(2,2,2-trifluoroethyl)acetamide |
| E-278 | 2-{3-[1-(2-amino-5-fluoropyrimidine-4-carbonyl)azetidin-3-yl]-5-fluoro-1H-indazol-1-yl}-N-methyl-N-(2,2,2-trifluoroethyl)acetamide |
| E-279 | 2-{3-[1-(2-amino-5-fluoropyridine-4-carbonyl)azetidin-3-yl]-5,6-difluoro-1H-indazol-1-yl}-N-methyl-N-(2,2,2-trifluoroethyl)acetamide |
| E-280 | 2-{3-[1-(2-amino-5-fluoropyridine-4-carbonylazetidin-3-yl]-5,6-difluoro-1H-indazol-1-yl}-1-[(2S)-2-(trifluoromethyl)pyrrolidin-1-yllethan-1-one |
| E-281 | 2-{3-[1-(2-amino-5-fluoropyridine-4-carbonyl)-3-hydroxyazetidin-3-yl]-1H-indazol-1-yl}-1-[(28)-2-(trifluoromethyl)pyrrolidin-1-yl]ethan-1-one |
| E-282 | 2-{3-[1-(2-aminopyrimidine-4-carbonyl)azetidin-3-yl]-5,6-difluoro-1H-indazol-1-yl}-N-methyl-N-(2,2,2-trifluoroethyl)acetamide |
| E-283 | 2-{3-[1-(2-aminopyrimidine-4-carbonyl)azetidin-3-yl]-5,6-diffuoro-1H-indazol-1-yl}-1-[(2S)-2-(trifluoromethyl)pyrrolidin-1-yl}ethan-1-one |
| E-284 | 2-{3-[1-(2-aminopyrimidine-4-carbonyl)azetidin-3-yl]-6-fluoro-1H-indazol-1-yl}-N-methyl-N-(2,2,2-trifluoroethyl)acetamide |

**[Table 72]**

| Compound number | Chemical Name |
|---|---|
| E-285 | 2-(3-[1-(2-amino-5-fluoropyridine-4-carbonyl)-3-fluoroazetidin-3-yl]-1H-indazol-1-yl}-1-[(2S)-2-(trifluoromethyl)pyrrolidin-1-yl]ethan-1-one |
| E-286 | 2-{3-[1-(2-aminopyrimidine-4-carbonyl)azetidin-3-yl]-6-fluoro-1H-indazol-1-yl}-1-[(2S)-2-(trifluoromethyl)pyrrolidin-1-yl]ethan-1-one |
| E-287 | 2-{3-(1-(2-aminopyrimidine-4-carbonyl)azetidin-3-yl]-4-chloro-5-fluoro-1H-indazol-1-yl}-N-methyl-N-(2,2,2-trifluoroethyl)acetamide |
| E-288 | 2-{3-[1-(2-aminopyrimidine-4-carbonyl)azetidin-3-yl]-5-methyl-1H-indazol-1-yl}-N-methyl-N-(2,2,2-trifluoroethyl)acetamide |
| E-289 | 2-{3-[1-(2-aminopyrimidine-4-carbonyl)azetidin-3-yl]-4,6-difluoro-1H-indazol-1-yl)-N-methyl-N-(2,2,2-trifluoroethyl)acetamide |
| E-290 | 2-{3-[1-(2-aminopyrimidine-4-carbonyl)azetidin-3-yl]-5-chloro-1H-indazol-1-yl}-1-[(28)-2-(trifluoromethyl)pyrrolidin-1-yl]ethan-1-one |
| E-291 | 2-(3-(1-(2-amino-5-fluoropyridine-4-carbonyl)azetidin-3-yl]-5-methoxy-1H-indazol-1-yl}·1-[(2S)-2-(trifluoromethyl)pyrrolidin-1-yl]ethan-1-one |
| E-292 | 2-{3-[1-(2-aminopyrimidine-4-carbonyl)azetidin-3-yl]-5-methyl-1H-indazol-1-yl]-1-[(2S)-2-(trifluoromethyl)pyrrolidin-1-yl]ethan-1-one |
| E-293 | 2-{3-[1-(2-aminopyrimidine-4-carbonyl)azetidin-3-yl]-5-fluoro-1H-indazol-1-yl]-N-methyl-N-(2,2,2-trifluoroethyl)acetamide |
| E-294 | 2-{3-[1-(2-amino-5-fluoropyridine-4-carbonyl)azetidin-3-yl]-5,5-dimethyl-4,5,6,7-tetrahydro-1H-indazol-1-yl}-1-[(2S)-2-(trifluoromethyl)pyrrolidin-1-yl]ethan-1-one |
| E-295 | 2-{3-[1-(2-aminopyrimidine-4-carbonyl)azetidin-3-yl]-5,5-dimethyl-4,5,6,7-tetrahydro-1H-indazol-1-yl}-1-[(2S)-2-(trifluoromethyl)pyrrolidin-1-yl]ethan-1-one |

The following are examples of biological tests of compounds used in the present invention.

### <Test Example 1 Evaluation of DDR1 kinase inhibition>

### 1. Preparation of test substance

The test substance was prepared to a solution at 10 mM in dimethyl sulfoxide (DMSO, Nacalai Tesque, 13445-74), and then the solution was diluted in DMSO to six final concentrations for each compound, selected from 1000, 100, 10, 1, 0.1, 0.01, and 0.001 pM. The solution was further diluted in a buffer containing 50 mM HEPES (Fujifilm Wako Pure Chemicals, 340-01371), 1 mM EGTA (DOJINDO, G002), 10 mM magnesium chloride hexahydrate (Nacalai Tesque, 20908-65), 2 mM dithiothreitol (Nacalai Tesque, 14112-52) and 0.01 % Tween 20 (Tokyo Kasei Kogyo, T0543) to six concentrations for each compound, selected from 330000, 33000, 330, 33, 3, 0.3, and 0.03 nM, to prepare test substance solutions.

### 2. Measurement of the inhibitory effect on kinase activity of DDR1 kinase

The test substance solutions prepared at six concentrations for each compound were added in 384-well plates (Corning, 4512) at 3 µL/well to final concentrations of 10000, 1000, 100, 10, 1, 0.1, and 0.01 nM, respectively. DDR1 protein (Carna Biosciences, 08 -113) was added to the wells at 2 µL/well to a final concentration of 0.5 nM. A LANCE Ultra ULight(TM)-poly GAT (PerkinElmer, TRF0101) and ATP (Sigma-Aldrich, A6559) were mixed as substrates to final concentrations of 50 nM and 100 pM, respectively, and added to the wells at 5 µL/well. After incubation at room temperature for 2 hours, a LANCE Detection Buffer (Perkin Elmer, CR97-100) containing EDTA (DOJINDO, K001) at a final concentration of 10 mM and Europium (Perkin Elmer, AD0068) at a final concentration of 1 nM were added to the wells at 10 µL/well. After incubation at room temperature for 1 hour, a fluorescence intensity was measured at 615 nm and 665 nm with Envision (Perkin Elmer) or SpectraMax iD5 (Molecular Devices). Tests were performed in duplicate.

### 3. Analysis of measurement results

The fluorescence intensity at 665 nM was divided by the fluorescence intensity at 615 nM to calculate the inhibition rate by the test substance, with the maximum inhibition rate at 100% and the minimum inhibition rate at 0% for each test substance. The IC₅₀ value (50% inhibitory concentration) of the test substance was calculated by logistic regression analysis. The results for each Example compound are shown in Tables 73 through 78 below.

**[Table 73]**

| Example (Compound number) | DDR-1 IC₅₀ (nM) | Example (Compound number) | DDR-1 IC₅₀ (nM) |
|---|---|---|---|
| E-1 | 0.4 | E-30 | 4.1 |
| E-2 | 1.6 | E-31 | 21.0 |
| E-3 | 0.4 | E-32 | 160.0 |
| E-4 | 2.0 | E-33 | 1.6 |
| E-5 | 9.1 | E-34 | 120.0 |
| E-6 | 7.1 | E-35 | 31.0 |
| E-7 | 15.0 | E-36 | 270.0 |
| E-8 | 5.2 | E-37 | 50.0 |
| E-9 | 14.0 | E-38 | 190.0 |
| E-10 | 0.3 | E-39 | 88.0 |
| E-11 | 6.1 | E-40 | 5.7 |
| E-12 | 7.5 | E-41 | - |
| E-13 | 47.0 | E-42 | 3.2 |
| E-14 | 5.1 | E-43 | - |
| E-15 | 210.0 | E-44 | 9.2 |
| E-16 | 15.0 | E-45 | 4.4 |
| E-17 | 0.3 | E-46 | - |
| E-18 | 6.9 | E-47 | 140.0 |
| E-19 | 56.0 | E-48 | 23.0 |
| E-20 | 84.0 | E-49 | 9.9 |
| E-21 | 1.5 | E-50 | 28.0 |
| E-22 | 5.6 | E-51 | 12.0 |
| E-23 | 15.0 | E-52 | 43.0 |
| E-24 | 5.1 | E-53 | 160.0 |
| E-25 | 9.0 | E-54 | 6.2 |
| E-26 | - | E-55 | 23.0 |
| E-27 | - | E-56 | 0.8 |
| E-28 | - | E-57 | 140.0 |
| E-29 | - | E-58 | 4.4 |

**[Table 74]**

| Example (Compound number) | DDR-1 ICso (nM) | Example (Compound number) | DDR-1 IC₅₀ (nM) |
|---|---|---|---|
| E-59. | 5.4 | E-88 | 21.0 |
| E-60 | 6.5 | E-89 | 45.0 |
| E-61 | 2.8 | E-90 | 7.4 |
| E-62 | 9.3 | E-91 | 11.0 |
| E-63 | 4.3 | E-92 | 71.0 |
| E-64 | 62.0 | E-93 | 110.0 |
| E-65 | 1.9 | E-94 | 150.0 |
| E-66 | 3.9 | E-95 | 7.7 |
| E-67 | 1.0 | E-96 | 0.9 |
| E-68 | 3.8 | E-97 | 68.0 |
| E-69 | 1.1 | E-98 | 86.0 |
| E-70 | 1.7 | E-99 | 40.0 |
| E-71 | 32.0 | E-100 | 34.0 |
| E-72 | 8.5 | E-101 | 21.0 |
| E-73 | 5.6 | E-102 | 13.0 |
| E-74 | 0.4 | E-103 | 14.0 |
| E-75 | 15.0 | E-104 | 5.2 |
| E-76 | 100.0 | E-105 | 79.0 |
| E-77 | 3.5 | E-106 | 5.2 |
| E-78 | 13.0 | E-107 | 91.0 |
| E-79 | 35.0 | E-108 | 1.0 |
| E-80 | 36.0 | E-109 | 210.0 |
| E-81 | 1.5 | E-110 | 45.0 |
| E-82 | 0.8 | E-111 | 9.6 |
| E-83 | 0.3 | E-112 | 3.7 |
| E-84 | 0.3 | E-113 | 25.0 |
| E-85 | 210.0 | E-114 | 56.0 |
| E-86 | 2.0 | E-115 | 8.6 |
| E-87 | 22.0 | E-116 | 4.4 |

**[Table 75]**

| Example (Compound number) | DDR-1 IC₅₀ (nM) | Example (Compound number) | DDR-1 IC₅₀ (nM) |
|---|---|---|---|
| E-117 | 56.0 | E-146 | 0.4 |
| E-118 | 31.0 | E-147 | 0.3 |
| E-119 | 4.4 | E-148 | 1.9 |
| E-120 | 60.0 | E-149 | 1.7 |
| E-121 | 24.0 | E-150 | 350.0 |
| E-122 | 140.0 | E-151 | 160.0 |
| E-123 | 8.8 | E-152 | 90.0 |
| E-124 | 2.2 | E-153 | 140.0 |
| E-125 | 57.0 | E-154 | 270.0 |
| E-126 | 12.0 | E-155 | 2.4 |
| E-127 | 11.0 | E-156 | 12.0 |
| E-128 | 180.0 | E-157 | 350.0 |
| E-129 | 6.3 | E-158 | 0.6 |
| E-130 | 5.7 | E-159 | 1.2 |
| E-131 | 7.5 | E-160 | 140.0 |
| E-132 | 10.0 | E-161 | 0.3 |
| E-133 | 23.0 | E-162 | 320.0 |
| E-134 | 110.0 | E-163 | 14.0 |
| E-135 | 5.0 | E-164 | 200.0 |
| E-136 | 5.4 | E-165 | 29.0 |
| E-137 | 34.0 | E-166 | 150.0 |
| E-138 | 50.0 | E-167 | 190.0 |
| E-139 | 18.0 | E-168 | 64.0 |
| E-140 | 0.8 | E-169 | 9.8 |
| E-141 | 5.0 | E-170 | 220.0 |
| E-142 | 7.9 | E-171 | 34.0 |
| E-143 | 4.1 | E-172 | 1.3 |
| E-144 | 1.4 | E-173 | 70.0 |
| E-145 | 4.6 | E-174 | 1.1 |

**[Table 76]**

| Example (Compound number) | DDR-1 IC₅₀ (nM) | Example (Compound number) | DDR-1 IC₅₀ (nM) |
|---|---|---|---|
| E-175 | 0.5 | E-204 | 2.0 |
| E-176 | 8.4 | E-205 | 1.6 |
| E-177 | 2.1 | E-206 | 1.6 |
| E-178 | 54.0 | E-207 | 1.6 |
| E-179 | 2.9 | E-208 | 170.0 |
| E-180 | 22.0 | E-209 | 280.0 |
| E-181 | 14.0 | E-210 | 3.5 |
| E-182 | 12.0 | E-211 | 27.0 |
| E-183 | 8.8 | E-212 | 7.1 |
| E-184 | 3.0 | E-213 | 7.0 |
| E-185 | 22.0 | E-214 | 10.0 |
| E-186 | 23.0 | E-215 | 56.0 |
| E-187 | 0.6 | E-216 | 13.0 |
| E-188 | 7.9 | E-217 | 32.0 |
| E-189 | 11.0 | E-218 | 13.0 |
| E-190 | 7.5 | E-219 | 160.0 |
| E-191 | 5.1 | E-220 | 3.6 |
| E-192 | 1.3 | E-221 | 4.4 |
| E-193 | 1.5 | E-222 | 2.1 |
| E-194 | 4.8 | E-223 | 82.0 |
| E-195 | 4.9 | E-224 | 18.0 |
| E-196 | 2.8 | E-225 | 17.0 |
| E-197 | 2.3 | E-226 | 150.0 |
| E-198 | 31.0 | E-227 | 60.0 |
| E-199 | 5.2 | E-228 | 9.0 |
| E-200 | 2.2 | E-229 | 6.0 |
| E-201 | 1.7 | E-230 | 54.0 |
| E-202 | 1.1 | E-231 | 30.0 |
| E-203 | 1.5 | E-232 | 38.0 |

**[Table 77]**

| Example (Compound number) | DDR-1 IC₅₀ (nM) | Example (Compound number) | DDR-1 IC₅₀ (nM) |
|---|---|---|---|
| E-233 | 500.0 | E-262 | 9.3 |
| E-234 | 7.9 | E-263 | 15.0 |
| E-235 | 59.0 | E-264 | 170.0 |
| E-236 | 4.8 | E-265 | 35.0 |
| E-237 | 3.8 | E-266 | 4.5 |
| E-238 | 5.6 | E-267 | 6.2 |
| E-239 | 3.3 | E-268 | 3.9 |
| E-240 | 5.0 | E-269 | 4.8 |
| E-241 | 4.3 | E-270 | 35.0 |
| E-242 | 30.0 | E-271 | 4.0 |
| E-243 | 29.0 | E-272 | 1.4 |
| E-244 | 41.0 | E-273 | 60.0 |
| E-245 | 440.0 | E-274 | 1.0 |
| E-246 | 69.0 | E-275 | 4.8 |
| E-247 | 47.0 | E-276 | 19.0 |
| E-248 | 11.0 | E-277 | 4.0 |
| E-249 | 3.5 | E-278 | 6.9 |
| E-250 | 2.0 | E-279 | 3.1 |
| E-251 | 2.5 | E-280 | 0.9 |
| E-252 | 19.0 | E-281 | 11.0 |
| E-253 | 9.5 | E-282 | 52.0 |
| E-254 | 21.0 | E-283 | 4.5 |
| E-255 | 22.0 | E-284 | 54.0 |
| E-256 | 2.0 | E-285 | 1.3 |
| E-257 | 13.0 | E-286 | 5.0 |
| E-258 | 74.0 | E-287 | 7.8 |
| E-259 | 2.1 | E-288 | 14.0 |
| E-260 | 240.0 | E-289 | 45.0 |
| E-261 | 4.6 | E-290 | 1.6 |

**[Table 78]**

| Example (Compound number) | DDR-1 IC₅₀ (nM) | Example (Compound number) | DDR-1 IC₅₀ (nM) |
|---|---|---|---|
| E-291 | 2.6 | E-294 | 9.1 |
| E-292 | 2.3 | E-295 | 160.0 |
| E-293 | 26.0 | | |

### <Test Example 2 Evaluation of DDR1 kinase inhibition in DDR1-expressing U2OS cells>

### 1. Preparation of test substance

The test substance was prepared to a solution at 10 mM in DMSO and then the solution was diluted in DMSO to final concentrations of 1000, 100, 10, 1, and 0.1 µM. The solution was further diluted in an E-MEM medium (Fujifilm Wako Pure Chemicals, 051-07615) containing 10% fetal bovine serum (FBS, Gibco, 10270106) to 60000, 6000, 600, 60, 6, and 0.6 nM to prepare test substance solutions.

### 2. Measurement of DDR1 kinase inhibition in DDR1-expressing U2OS cells

DDR1-expressing U2OS cells (DiscoverX, 93-0894C3) suspended at 2.5× 10^5 cells/mL in 10% FBS + E-MEM medium were seeded in 384-well plates (DiscoverX, 92-0013) and cultured overnight at 37°C, 5% CO₂.

The next day, the prepared test substance solutions were added to the wells at 5 µL/well to final concentrations of 10000, 1000, 100, 10, 1, and 0.1 nM, respectively. After incubation at 37°C, 5% CO₂ for 1 hour, type I collagen (Nitta gelatin, 631-00771) was added to the wells at 5 µL/well to a final concentration of 50 µg/mL and then incubated overnight at 37°C, 5% CO₂.

On the following day, after the plate was left at room temperature for 1 hour, a detection solution was added to the wells at 5 pL/well according to the handling instructions for the PathHunter^{®} Detection Kit (DiscoverX, 93-0001), and then incubated at room temperature for 2 hours. After the incubation, a luminescence intensity was measured by Envision (Perkin-Elmer). Tests were performed in duplicate.

### 3. Analysis of measurement results

The inhibition rate by the test substance was calculated using Blank (type I collagen stimulation only) as 100% and the negative control group (no type I collagen stimulation) as 0%. The IC₅₀ value (50% inhibitory concentration) of the test substance was calculated by logistic regression analysis. The results for each Example compound are shown in Tables 79 through 81 below.

**[Table 79]**

| Example (Compound number) | DDR-1 IC₅₀ (nM)I | Example (Compound number) | DDR-1 IC₅₀ (nM) |
|---|---|---|---|
| E-1 | 78 | E-55 | 150 |
| E-2 | 37 | E-59 | 170 |
| E-3 | 53 | E-61 | 960 |
| E-4 | 55 | | 1600 |
| E-5 | 63 | E-63 | 140 |
| E-8 | 230 | E-64 | 180 |
| E-11 | 110 | E-65 | 41 |
| E-12 | 330 | E-66 | 340 |
| E-13 | 1200 | E-67 | 250 |
| E-15 | 2600 | E-68 | 8 |
| E-16 | 720 | E-69 | 120 |
| E-18 | 26 | E-70 | 120 |
| E-19 | 84 | E-71 | 1300 |
| E-21 | 140 | E-72 | 200 |
| E-22 | 18 | E-73 | 13 |
| E-23 | 170 | E-74 | 6 |
| E-30 | 74 | E-75 | 33 |
| E-31 | 690 | E-76 | 1300 |
| E-33 | 390 | E-77 | 320 |
| E-35 | 2000 | E-78 | 380 |
| E-37 | 2600 | E-79 | 3400 |
| E-40 | 69 | E-80 | 4200 |
| E-42 | 150 | E-81 | 61 |
| E-45 | 140 | E-82 | 130 |
| E-48 | 390 | E-83 | 74 |
| E-50 | 1900 | E-84 | 2300 |
| E-51 | 400 | E-86 | 34 |
| E-54 | 180 | E-87 | 93 |

**[Table 80]**

| Example (Compound number) | DDR-1 IC₅₀ (nM) | Example (Compound number) | DDR-1 IC₅₀ (nM) |
|---|---|---|---|
| E-88 | 1400 | E-131 | 56 |
| E-89 | 400 | E-139 | 850 |
| E-90 | 96 | E-163 | 780 |
| E-91 | 610 | E-169 | 1100 |
| E-92 | 1400 | E-180 | 3000 |
| E-95 | 480 | E-183 | 500 |
| E-96 | 84 | E-185 | 3300 |
| E-97 | 2800 | E-186 | 690 |
| E-98 | 3700 | E-188 | 89 |
| E-99 | 150 | E-189 | 460 |
| E-100 | 110 | E-191 | 620 |
| E-101 | 270 | E-194 | 370 |
| E-102 | 3300 | E-195 | 69 |
| E-104 | 110 | E-196 | 43 |
| E-105 | 370 | E-197 | 150 |
| E-106 | 180 | E-206 | 6 |
| E-107 | 670 | E-211 | 290 |
| E-108 | 100 | E-212 | 60 |
| E-110 | 110 | E-213 | 91 |
| E-111 | 85 | E-214 | 380 |
| E-112 | 170 | E-218 | 74 |
| E-113 | 470 | E-225 | 450 |
| E-115 | 250 | E-228 | 95 |
| E-116 | 800 | E-229 | 81 |
| E-121 | 170 | E-236 | 120 |
| E-124 | 140 | E-237 | 34 |
| E-126 | 520 | E-240 | 140 |
| E-129 | 70 | E-242 | 740 |
| E-130 | 82 | E-243 | 600 |

**[Table 81]**

| Example (Compound number) | DDR-1 IC₅₀ (nM) | Example (Compound number) | DDR-1 IC₅₀ (nM) |
|---|---|---|---|
| E-244 | 420 | E-261 | 6 |
| E-247 | 300 | E-262 | 20 |
| E-248 | 510 | E-263 | 65 |
| E-250 | 17 | E-265 | 600 |
| E-251 | 19 | E-269 | 38 |
| E-252 | 240 | E-270 | 140 |
| E-253 | 170 | E-272 | 3 |
| E-254 | 390 | E-275 | 21 |
| E-255 | 180 | E-276 | 430 |
| E-257 | 42 | | |

### <Test Example 3 Investigation of in vivo drug efficacy using an anti-glomerular basement membrane antibody-induced nephritis model>

### 1. Preparation of test substance

The test substance was weighed and suspended in 0.5% methylcellulose (Shin-Etsu Chemical Co., Ltd.) + 99.5% Otsuka distilled water (Otsuka Pharmaceutical Factory) to prepare the test substance administration solution.

### 2. Creation of animal models

WKY/NCrlCrlj rats (7-week-old males) were administered a tail vein injection of 300 pL of phosphate-buffered saline (Wako, 045-29795) containing 33.3 to 100 pg/mL anti-glomerular basement membrane antibody (Chondrex, 70221). After 7 days, the test substance was administered twice daily for 7 days, and on days 6 and 7 of administration, the rats were moved to a metabolic and urine collection cage, and urine was collected for 24 hours. The weight of the collected urine was measured.

### 3. Measurement of urinary albumin level

150 µL of urine was transferred to a sample cup (650-M, BMBio ST). The albumin concentration in the urine was quantified by using a JCA-BM6050 automated analyzer (JEOL). The amount of albumin in the urine was corrected by the measured urine weight and the amount of albumin in the urine was calculated.

### 4. Analysis of measurement results

The ratio of the amount of albumin in the urine in the test substance-treated group was calculated using the amounts of albumin in the urine of the anti-glomerular basement membrane antibody untreated group and the medium-treated group as 0% and 100%, respectively. The experimental results are shown in Table X.

**[Table 82]**

| Example (Compound number) | Dose (mg/kg) | Amount (%) of albumin in urine compared with medium-treated group |
|---|---|---|
| E-2 | 200 | 73 |
| E-3 | 100 | 67 |

## Claims

1. A compound of formula (I): 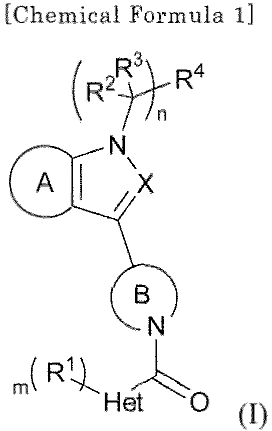 wherein:
A is 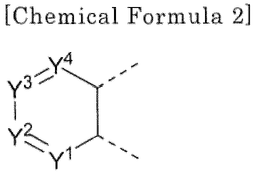
wherein
Y¹, Y², Y³ and Y⁴ are each independently N or C-R⁵, wherein at most two of the Y¹, Y², Y³ and Y⁴ are N, and the others are C-R⁵,
R⁵ is hydrogen, halogen, cyano, alkoxy, or optionally-substituted C₁ - C₆ alkyl, and
dashed lines represent bonding points,
or 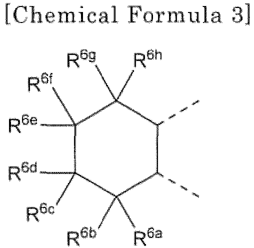
wherein
R^{6a}, R^{6b}, R^{6c}, R^{6d}, R^{6e}, R^{6f}, R^{6g} and R^{6h} are each independently hydrogen, halogen, cyano or optionally-substituted C₁ -C₆ alkyl, and
dashed lines represent bonding points;
B is optionally-substituted, 3- to 8-membered saturated or unsaturated monocyclic nitrogen-containing heterocyclic ring;
X is N or C-R⁷, wherein R⁷ is hydrogen, halogen, cyano or optionally-substituted C₁ -C₆ alkyl;
R¹ s are each independently selected from the group consisting of hydrogen, halogen, cyano, oxo, C₁ -C₆ alkyl, C₂-C₆ alkenyl, C₂-C₆ alkynyl, C₁-C₆ alkoxy, hydroxy, carboxy, alkylcarbonyloxy, amino, monoalkylamino, dialkylamino, aminoalkyl, alkylcarbonylamino, C₃-C₁₀ cycloalkyl, C₃-C₆ cycloalkenyl, heterocycloalkyl, aryl, and heteroaryl, each of which may be optionally substituted;
R² and R³ are each independently hydrogen, halogen or optionally-substituted C₁-C₆ alkyl;
R⁴ is selected from the group consisting of hydrogen, halogen, cyano, C₁-C₆ alkyl, C₂-C₆ alkenyl, C₂-C₆ alkynyl. C₁-C₆ alkoxy, hydroxy, carboxy, alkylcarbonyloxy, amino, monoalkylamino, dialkylamino, aminoalkyl, alkylcarbonylamino, monocyclic or bicyclic saturated carbocyclic ring, monocyclic or bicyclic unsaturated carbocyclic ring, monocyclic or bicyclic saturated heterocyclic ring, and monocyclic or bicyclic unsaturated heterocyclic ring, each of which may be optionally substituted; or
R⁴ is -CONR⁸R⁹, wherein:
R⁸ and R⁹ are each independently selected from the group consisting of hydrogen, C₁-C₆ alkyl, C₁-C₆ alkoxy, C₂-C₆ alkenyl, C₂-C₆ alkynyl, C₃-C₁₀ cycloalkyl, C₃-C₆ cycloalkenyl, heterocycloalkyl, aryl, and heteroaryl, each of which may be optionally substituted; or
R⁸ and R⁹, together with the atom to which they are attached, form optionally-substituted, 5- to 10-membered monocyclic or bicyclic saturated heterocyclic ring or 5- to 10-membered monocyclic or bicyclic unsaturated heterocyclic ring;
m is an integer of 0 to 3;
n is an integer of 0 to 3; and
Het is 5- to 10-membered monocyclic or bicyclic saturated heterocyclic ring or 5- to 10-membered monocyclic or bicyclic unsaturated heterocyclic ring,
or a pharmaceutically acceptable salt thereof, or a solvate thereof.

2. The compound according to claim 1, wherein the formula (I) is formula (II): 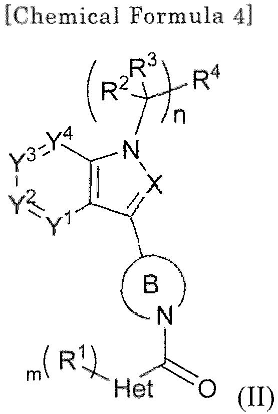
wherein B, R¹, R², R³, R⁴, Het, X, Y¹, Y², Y³, Y⁴, m and n are as defined in the formula (I),
or a pharmaceutically acceptable salt thereof, or a solvate thereof.

3. The compound according to claim 1, wherein the formula (I) is formula (III): 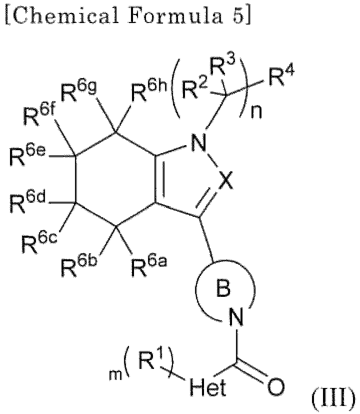
wherein B, R¹, R², R³, R⁴, R^{6a}, R^{6b}, R^{6c}, R^{6d}, R^{6e}, R^{6f}, R^{6g}, R^{6h}, Het, X, m and n are as defined in the formula (I),
or a pharmaceutically acceptable salt thereof, or a solvate thereof.

4. The compound according to any one of claims 1 to 3, wherein R¹ s are each independently selected from the group consisting of hydrogen, halogen, oxo, C₁-C₆ alkyl, C₁-C₆ alkoxy, amino, monoalkylamino, dialkylamino, aminoalkyl, alkylcarbonylamino, C₃-C₁₀ cycloalkyl, heterocycloalkyl, aryl, and heteroaryl, each of which may be optionally substituted,
or a pharmaceutically acceptable salt thereof, or a solvate thereof.

5. The compound according to any one of claims 1 to 4, wherein R⁴ is selected from the group consisting of hydrogen, halogen, cyano, C₁-C₆ alkyl, C₂-C₆ alkenyl, C₂-C₆ alkynyl, C₁-C₆ alkoxy, hydroxy, carboxy, alkylcarbonyloxy, amino, monoalkylamino, dialkylamino, aminoalkyl, alkylcarbonylamino, C₃-C₁₀ cycloalkyl, C₃-C₆ cycloalkenyl, heterocycloalkyl, aryl, and heteroaryl, each of which may be optionally substituted,
or a pharmaceutically acceptable salt thereof, or a solvate thereof.

6. The compound according to any one of claims 1 to 4, wherein R⁴ is - CONR⁸R⁹, wherein:
R⁸ and R⁹ are each independently selected from the group consisting of hydrogen, C₁-C₆ alkyl, C₁-C₆ alkoxy, C₂-C₆ alkenyl, C₂-C₆ alkynyl, C₃-C₁₀ cycloalkyl, C₃-C₆ cycloalkenyl, heterocycloalkyl, aryl, and heteroaryl, each of which may be optionally substituted; or
R⁸ and R⁹, together with the atom to which they are attached, form optionally-substituted, 5- to 10-membered monocyclic or bicyclic saturated heterocyclic ring or 5- to 10-membered monocyclic or bicyclic unsaturated heterocyclic ring,
or a pharmaceutically acceptable salt thereof, or a solvate thereof.

7. The compound according to any one of claims 1 to 6, wherein X is N,
or a pharmaceutically acceptable salt thereof, or a solvate thereof.

8. The compound according to any one of claims 1 to 6, wherein X is C-R⁷, wherein R⁷ is hydrogen, halogen, cyano or optionally-substituted C₁-C₆ alkyl,
or a pharmaceutically acceptable salt thereof, or a solvate thereof.

9. A compound selected from the group consisting of the following compounds (1) to (295), or a pharmaceutically acceptable salt thereof, or a solvate thereof:
(1) 2-(4-chloro-3-{1-[2-(cyclopropylamino)pyridine-4-carbonyl]azetidin-3-yl}-5-fluoro-1H-indazol-1-yl)-N-(2,2,2-trifluoroethyl)acetamide hydrochloride
(2) 2-{3-[1-(2-aminopyridine-4-carbonyl)azetidin-3-yl]-4,6-difluoro-1H-indazol-1-yl}-1-[(2S)-2-(trifluoromethyl)pyrrolidin-1-yl]ethan-1-one hydrochloride
(3) 2-{3-[1-(2-amino-5-fluoropyridine-4-carbonyl)azetidin-3-yl]-1H-indazol-1-yl}-1-[(2S)-2-(trifluoromethyl)pyrrolidin-1-yl]ethan-1-one
(4) 2-{3-[1-(2-aminopyrimidine-4-carbonyl)azetidin-3-yl]-5-chloro-1H-indazol-1-yl}-N-methyl-N-(2,2,2-trifluoroethyl)acetamide
(5) 2-{3-[1-(2-amino-5-fluoropyridine-4-carbonyl)azetidin-3-yl]-6-fluoro-1H-indazol-1-yl}-N-methyl-N-(2,2,2-trifluoroethyl)acetamide
(6) 2-{3-[1-(2-aminopyridine-4-carbonyl)-3-methylazetidin-3-yl]-1H-indazol-1-yl}-1-((2S)-2-(trifluoromethyl)pyrrolidin-1-yl]ethan-1-one
(7) 2-{3-[1-(2-amino-4-methyl-1,3-thiazole-5-carbonyl)azetidin-3-yl]-1H-indazol-1-yl}-1-(8,3-dimethylmorpholin-4-yl)ethan-1-one
(8) 2-{3-[1-(2-amino-3-methylpyridine-4-carbonyl)azetidin-3-yl]-1H-indazol-1-yl}-1-[(2R)-2-(trifluoromethyl)morpholin-4-yl]ethan-1-one
(9) 4-[4-(4-fluoro-1-{[5-(trifluoromethyl)pyridin-2-yl]methyl}-1H-indazol-3-yl)piperidine-1-carbonyl]pyridin-2-amine
(10) 2-{3-[1-(2-amino-5-fluoropyridine-4-carbonyl)piperidin-4-yl]-4-fluoro-1H-indol-1-yl}-1-[(2S)-2-(trifluoromethyl)pyrrolidin-1-yl]ethan-1-one
(11) 2-{3-[1-(2-aminopyridine-4-carbonyl)azetidin-3-yl]-1H-indol-1-yl}-1-[(2S)-2-(trifluoromethyl)pyrrolidin-1-yl]ethan-1-one
(12) 2-{3-[1-(2-aminopyridine-4-carbonyl)azetidin-3-yl]-5-methyl-1H-pyrazolo[4,3-b]pyridin-1-yl}-1-[(2S)-2-(trifluoromethyl)pyrrolidin-1-yl]ethan-1-one
(13) 2-{3-[1-(2-aminopyridine-4-carbonyl)azetidin-3-yl]-1H-pyrazolo[4,3-c]pyridin-1-yl}-1-[(2S)-2-(trifluoromethyl)pyrrolidin-1-yl)ethan-1-one hydrochloride
(14) 4-{3-[4-fluoro-1-({5-[1-(trifluoromethyl)cyclopropyl]-1,2,4-oxadiazol-3-yl}methyl)-1H-pyrazolo[3,4-c]pyridin-3-yl]azetidine-1-carbonyl}pyridin-2-amine
(15) 2-{3-(1-(2-aminopyridine-4-carbonyl)azetidin-3-yl]-4-methyl-1H-pyrazolo[3,4-b]pyridin-1-yl}-1-[(2S)-2-(trifluoromethyl)pyrrolidin-1-yl]ethan-1-one
(16) 2-{3-[1-(6-aminopyridazine-4-carbonyl)azetidin-3-yl]-4,6-difluoro-1H-indazol-1-yl}-1-[(2S)-2-(trifluoromethyl)pyrrolidin-1-yl]ethan-1-one
(17) 2-[4,6-difluoro-3-(1-{1H-pyrazolo[3,4-b]pyridine-4-carbonyl}azetidin-3-yl)-1H-indazol-1-yl]-1-[(2S)-2-(trifluoromethyl)pyrrolidin-1-yl]ethan-1-one
(18) 5-fluoro-4-[3-(1-{[3-(trifluoromethyl)-1,2,4-oxadiazol-5-yl]methyl}-1H-indazol-3-yl)azetidine-1-carbonyl]pyridin-2-amine
(19) 4-{3-[1-({1-[(3,3-difluorocyclobutyl)methyl]-1H-1,2,3-triazol-4-yl}methyl)-4-fluoro-1H-indazol-3-yl]azetidine-1-carbonyl}pyridin-2-amine hydrochloride
(20) 4-[3-(4-fluoro-1-{[5-(4-fluorophenyl)-1,3,4-oxadiazol-2-yl]methyl}-1H-indazol-3-yl)azetidine-1-carbonyl]pyridin-2-amine
(21) 2-{3-[1-(2-amino-5-fluoropyrimidine-4-carbonyl)azetidin-3-yl]-5-chloro-1H-indazol-1-yl)-N-methyl-N-(2,2,2-trifluoroethyl)acetamide
(22) 2-{3-[1-(2-amino-5-fluoropyrimidine-4-carbonyl)azetidin-3-yl]-1H-indazol-1-yl}-1-[(2S)-2-(trifluoromethyl)pyrrolidin-1-yl]ethan-1-one
(23) 2-{3-[1-(2-amino-5-fluoropyrimidine-4-carbonyl)azetidin-3-yl]-4-fluoro-1H-indazol-1-yl)-N-methyl-N-(2,2,2-trifluoroethyl)acetamide
(24) 2-{3-[1-(2-amino-5-fluoropyridine-4-carbonyl)azetidin-3-yl]-4,5,8,7-tetrahydro-1H-indazol-1-yl}-1-[(2S)-2-(trifluoromethyl)pyrrolidin-1-yl]ethan-1-one
(25) 2-{3-[1-(2-amino-5-fluoropyridine-4-carbonyl)azetidin-3-yl]-5,5-difluoro-4,5,6,7-tetrahydro-1H-indazol-1-yl}-1-[(2S)-2-(trifluoromethyl)pyrrolidin-1-yl]ethan-1-one
(26) 2-(4-chloro-3-{1-[2-(cyclopropylamino)pyridine-4-carbonyl]azetidin-3-yl}-5-fluoro-1H-indazol-1-yl)-N-(2,2,2-trifluoroethyl)acetamide p-toluenesulfonate
(27) 2-{3-[1-(2-aminopyridine-4-carbonyl)azetidin-3-yl]-4,6-difluoro-1H-indazol-1-yl}-1-[(2S)-2-(trifluoromethyl)pyrrolidin-1-yl]ethan-1-one methanesulfonate
(28) 2-{3-[1-(2-amino-5-fluoropyridine-4-carbonyl)azetidin-3-yl)-1H-indazol-1-yl}-1-[(2S)-2-(trifluoromethyl)pyrrolidin-1-yl]ethan-1-one hydrochloride
(29) 2-{8-[1-(2-amino-5-fluoropyridine-4-carbonyl)azetidin-3-yl]-1H-indazol-1-yl}-1-[(2S)-2-(trifluoromethyl)pyrrolidin-1-yl]ethan-1-one methanesulfonate
(30) 2-(3-{1-[2-(cyclopropylamino)pyridine-4-carbonyl]azetidin-3-yl}-4,6-difluoro-1H-indazol-1-yl)-N-(2,2,2-trifluoroethyl)acetamide hydrochloride
(31) 2-(3-{1-[6-(cyclopropylamino)pyrimidine-4-carbonyl]azetidin-3-yl}-4,6-difluoro-1H-indazol-1-yl)-N-(2,2,2-trifluoroethyl)acetamide
(32) 2-[4,6-difluoro-3-(1-{2-[(oxetan-3-yl)amino]pyridine-4-carbonyl}azetidin-3-yl)-1H-indazol-1-yl]-N-(2,2,2-trifluoroethyl)acetamide
(33) 2-[4,6-difluoro-3-(1-{1H-pyrrolo[2,3-b]pyridine-4-carbonyl}azetidin-3-yl)-1H-indazol-1-yl]-1-[(2S)-2-(trifluoromethyl)pyrrolidin-1-yl]ethan-1-one hydrochloride
(34) 4-(3-{4-fluoro-1-[(1-phenyl-1H-1,2,3-triazol-4-yl)methyl]-1H-indazol-3-yl}azetidine-1-carbonyl)pyridin-2-amine
(35) 4-(3-{1-[(1-benzyl-1H-1,2,3-triazol-4-yl)methyl]-4-fluoro-1H-indazol-3-yl}azetidine-1-carbonyl)pyridin-2-amine hydrochloride
(36) 2-{3-[1-(2-aminopyridine-4-carbonyl)azetidin-3-yl]-4,6-difluoro-1H-indazol-1-yl}-1-[(2R)-2-(hydroxymethyl)pyrrolidin-1-yl]ethan-1-one
(37) 4-[3-(1-{[1-(cyclopropylmethyl)-1H-1,2,3-triazol-4-yl]methyl}-4-fluoro-1H-indazol-3-yl)azetidine-1-carbonyl]pyridin-2-amine hydrochloride
(38) 4-{3-[4-fluoro-1-({1-[(oxetan-3-yl)methyl]-1H-1,2,3-triazol-4-yl}methyl)-1H-indazol-3-yl]azetidine-1-carbonyl}pyridin-2-amine
(39) 4-{4-[1-({1-[(3,3-difluorocyclobutyl)methyl]-1H-1,2,3-triazol-4-yl}methyl)-4-fluoro-1H-indazol-3-yl]piperidine-1-carbonyl}pyridin-2-amine
(40) 2-{3-[1-(2-aminopyridine-4-carbonyl)azetidin-3-yl]-1H-indazol-1-yl}-1-[(2S)-2-(trifluoromethyl)pyrrolidin-1-yl]ethan-1-one
(41) 2-{3-[1-(2-aminopyridine-4-carbonyl)azetidin-3-yl]-1H-indazol-1-yl}-1-[(2S)-2-(trifluoromethyl)pyrrolidin-1-yl]ethan-1-one hydrochloride
(42) 2-{3-[1-(2-amino-5-fluoropyridine-4-carbonyl)azetidin-3-yl]-4,6-difluoro-1H-indazol-1-yl}-1-[(2R)-2-(trifluoromethyl)morpholin-4-yl]ethan-1-one
(43) 2-{3-[1-(2-amino-5-fluoropyridine-4-carbonyl)azetidin-3-yl]-4,6-difluoro-1H-indazol-1-yl}-1-[(2R)-2-(trifluoromethyl)morpholin-4-yl]ethan-1-one p-toluenesulfonate
(44) 2-{3-[1-(2-amino-3-methylpyridine-4-carbonyl)azetidin-3-yl]-4-fluoro-1H-pyrazolo[3,4-c]pyridin-1-yl}-1-[(2S)-2-[(trifluoromethoxy)methyl]pyrrolidin-1-yllethan-1-one di-p-toluenesulfonate
(45) 2-{3-[1-(2-aminopyridine-4-carbonyl)azetidin-3-yl]-1H-indazol-1-yl}-N-methyl-N-(2,2,2-trifluoroethyl)acetamide
(46) 2-{3-[1-(2-aminopyridine-4-carbonyl)azetidin-3-yl]-1H-indazol-1-yl}-N-methyl-N-(2,2,2-trifluoroethyl)acetamide p-toluenesulfonate
(47) 2-{3-[1-(2-amino-5-fluoropyridine-4-carbonyl)-1,2,3,6-tetrahydropyridin-4-yl}-1H-indazol-1-yl}-1-[(2S)-2-(trifluoromethyl)pyrrolidin-1-yl]ethan-1-one
(48) 2-{3-[1-(2-amino-5-fluoropyridine-4-carbonyl)piperidin-4-yl]-1H-indazol-1-yl}-N-methyl-N-(2,2,2-trifluoroethyl)acetamide
(49) 2-13-[1-(2-amino-5-fluoropyridine-4-carbonyl)piperidin-4-yl]-1H-indazol-1-yl}-1-[(2S)-2-(trifluoromethyl)pyrrolidin-1-yl]ethan-1-one
(50) 2-{3-[1-(2-aminopyridine-4-carbonyl)azetidin-3-yl]-4,6-difluoro-1H-indazol-1-yl}-1-[(2R)-2-(trifluoromethyl)pyrrolidin-1-yl]ethan-1-one
(51) 2-{3-[1-(2-amino-3-methylpyridine-4-carbonyl)azetidin-3-yl]-4,6-difluoro-1H-indazol-1-yl}-1-[(2R)-2-(trifluoromethyl)morpholin-4-yl]ethan-1-one hydrochloride
(52) 4-[3-(1-{[1-(2,2,2-trifluoroethyl)-1H-1,2,3-triazol-4-yl]methyl}-4-fluoro-1H-indazol-3-yl)azetidine-1-carbonyl]pyridin-2-amine hydrochloride
(53) 2-{3-[1-(2-aminopyridine-4-carbonyl)azetidin-3-yl]-4-methyl-1H-pyrazolo[4,3-c]pyridin-1-yl}-1-[(2S)-2-(trifluoromethyl)pyrrolidin-1-yl]ethan-1-one
(54) 2-{3-[1-(2-aminopyridine-4-carbonyl)azetidin-3-yl]-4-fluoro-1H-pyrazolo[3,4-c]pyridin-1-yl}-1-[(2S)-2-(trifluoromethyl)pyrrolidin-1-yl]ethan-1-one hydrochloride
(55) 4-{3-[4-fluoro-1-({3-[3-(trifluoromethyl)phenyl]-1,2,4-oxadiazol-5-yl}methyl)-1H-indazol-3-yl]azetidine-1-carbonyl}pyridin-2-amine
(56) 2-{3-[1-(2-aminopyridine-4-carbonyl)azetidin-3-yl]-4,6-difluoro-1H-indazol-1-yl}-1-[(2S)-2-[(trifluoromethoxy)methyl]pyrrolidin-1-yl]ethan-1-one
(57) 4-(3-{1-[(4,4-difluorocyclohexyl)methyl]-4,6-difluoro-1H-indazol-3-yl}azetidine-1-carbonyl)pyridin-2-amine
(58) 2-13-[1-(2-amino-5-fluoropyridine-4-carbonyl)azetidin-3-yl]-4-fluoro-1H-pyrazolo[3,4-c]pyridin-1-y]}-1-[(2S)-2-[(trifluoromethoxy)methyl]pyrrolidin-1-yl]ethan-1-one di-p-toluenesulfonate
(59) 2-{3-[1-(2-amino-5-fluoropyridine-4-carbonyl)azetidin-3-yl]-4-fluoro-1H-indazol 1-yl}-N-methyl-N-(2,2,2-trifluoroethyl)acetamide
(60) 2-{3-[1-(2-amino-5-fluoropyridine-4-carbonyl)azetidin-3-yl]-1H-indazol-1-yl}-1-[(2R)-2-(trifluoromethyl)pyrrolidin-1-yl]ethan-1-one
(61) 2-[4-chloro-3-(1-{1H-pyrrolo[2,3-b]pyridine-4-carbonyl}azetidin-3-yl)-1H-indazol-1-yl]-N-(2,2,2-trifluoroethyl)acetamide
(62) 2-{3-[1-(2-aminopyridine-4-carbonyl)azetidin-3-yl]-4-chloro-1H-indazol-1-yl}-N-(2,2,2-trifluoroethyl)acetamide
(63) 2-(4-chloro-3-{1-[2-(methylamino)pyridine-4-carbonyl]azetidin-3-yl}-1H-indazol-1-yl)-N-(2,2,2-trifluoroethyl)acetamide
(64) 2-(4-chloro-3-{1-[2-(ethylamino)pyridine-4-carbonyl]azetidin-3-yl}-1H-indazol-1-yl)-N-(2,2,2-trifluoroethyl)acetamide hydrochloride
(65) 2-(3-{1-[2-(cyclopropylamino)pyridine-4-carbonyl]azetidin-3-yl}-4-fluoro-1H-indazol-1-yl)-N-(2,2,2-trifluoroethyl)acetamide hydrochloride
(66) 2-(3-{1-[2-(cyclopropylamino)pyridine-4-carbonyl]azetidin-3-yl}-4,7-difluoro-1H-indazol-1-yl)-N-(2,2,2-trifluoroethyl)acetamide hydrochloride
(67) 2-{3-[1-(2-aminopyridine-4-carbonyl)azetidin-3-yl]-4-chloro-5-fluoro-1H-indazol-1-yl}-N-(2,2,2-trifluoroethyl)acetamide hydrochloride
(68) 2-(3-{1-[2-(cyclobutylamino)pyridine-4-carbonyl]azetidin-3-yl}-4-fluoro-1H-indazol-1-yl)-N-(2,2,2-trifluoroethyl)acetamide hydrochloride
(69) 2-(4-bromo-3-{1-[2-(cyclopropylamino)pyridine-4-carbonyl]azetidin-3-yl}-5-fluoro-1H-indazol-1-yl)-N-(2,2,2-trifluoroethyl)acetamide hydrochloride
(70) 2-{3-[1-(2-aminopyridine-4-carbonyl)azetidin-3-yl]-4-bromo-5-fluoro-1H-indazol-1-yl}-N-(2,2,2-trifluoroethyl)acetamide hydrochloride
(71) 2-[4-fluoro-3-(1-{2-[(oxan-4-yl)amino]pyridine-4-carbonyl}azetidin-3-yl)-1H-indazol-1-yl]-N-(2,2,2-trifluoroethyl)acetamide hydrochloride
(72) 2-(3-{1-[2-(cyclopropylamino)pyridine-4-carbonyl]azetidin-3-yl}-4,6-difluoro-1H-indazol-1-yl)-1-[(2S)-2-(trifluoromethyl)pyrrolidin-1-yl]ethan-1-one hydrochloride
(73) 2-{3-[1-(2-aminopyridine-4-carbonyl)azetidin-3-yl]-4,6-difluoro-1H-indazol-1-yl}-1-[(2R)-2-(trifluoromethyl)morpholin-4-yl]ethan-1-one hydrochloride
(74) 2-[4,6-difluoro-3-(1-{2-[(1-methylcyclopropyl)aminolpyridine-4-carbonyl}azetidin-3-yl)-1H-indazol-1-yl]-1-[(2S)-2-(trifluoromethyl)pyrrolidin-1-yllethan-1-one hydrochloride
(75) 2-[3-(1-{2-[(4,4-difluorocyclohexyl)amino]pyridine-4-carbonyl}azetidin-3-yl)-4,6-difluoro-1H-indazol-1-yl]-1-[(2S)-2-(trifluoromethyl)pyrrolidin-1-yl]ethan-1-one hydrochloride
(76) 2-{3-[1-(2-aminopyridine-4-carbonyl)azetidin-3-yl]-4,6-difluoro-1H-indazol-1-yl}-1-(pyrrolidin-1-yl)ethan-1-one
(77) 2-{3-[1-(2-amino-3-methylpyridine-4-carbonyl)azetidin-3-yl]-4,6-difluoro-1H-indazol-1-yl}-1-[(2S)-2-(trifluoromethyl)pyrrolidin-1-yl]ethan-1-one hydrochloride
(78) 2-{3-[1-(2-amino-5-methylpyridine-4-carbonyl)azetidin-3-yl]-4,6-difluoro-1H-indazol-1-yl}-1-[(2S)-2-(trifluoromethyl)pyrrolidin-1-yl]ethan-1-one hydrochloride
(79) 2-{3-[1-(2-aminopyridine-4-carbonyl)azetidin-3-yl]-4,6-difluoro-1H-indazol-1-yl}-1-[(2S)-2-methylmorpholin-4-yl]ethan-1-one hydrochloride
(80) 2-{3-[1-(2-aminopyridine-4-carbonyl)azetidin-3-yl]-4,6-difluoro-1H-indazol-1-yl}-1-[(3R)-3-methylmorpholin-4-yl]ethan-1-one hydrochloride
(81) 2-{3-[1-(2-aminopyridine-4-carbonyl)piperidin-4-yl])-4-fluoro-1H-indazol-1-yl}-1-[(2S)-2-(trifluoromethyl)pyrrolidin-1-yl]ethan-1-one
(82) 2-{3-[1-(2-aminopyridine-4-carbonyl)azetidin-3-yl]-4,6-difluoro-1H-indazol-1-yl)-1-[(2S)-2-(trifluoromethyl)morpholin-4-yl]ethan-1-one
(83) 2-{3-[1-(2-aminopyridine-4-carbonyl)azetidin-3-yl}-4,6-difluoro-1H-indazol-1-yl}-1-[(3R)-3-(propan-2-yl)morpholin-4-yl]ethan-1-one
(84) 2-{3-(1-(2-aminopyridine-4-carbonyl)azetidin-3-yl]-4,6-difluoro-1H-indazol-1-yl}-1-[(2R,6S)-2,6-dimethylmorpholin-4-yl]ethan-1-one
(85) 2-{3-[1-(6-aminopyridine-3-carbonyl)piperidin-4-yl]-4-fluoro-1H-indazol-1-yl}-1-[(2S)-2-(trifluoromethyl)pyrrolidin-1-yl]ethan-1-one
(86) 2-(3-{1-[2-(cyclopropylamino)pyridine-4-carbonyl]piperidin-4-yl}-4-fluoro-1H-indazol-1-yl)-1-[(2S)-2-(trifluoromethyl)pyrrolidin-1-yl]ethan-1-one
(87) 2-{3-[1-(2-aminopyridine-4-carbonyl)azetidin-3-yl]-4-fluoro-1H-indazol-1-yl}-N-methyl-N-(2,2,2-trifluoroethyl)acetamide
(88) 2-[4,6-difluoro-3-(1-{2-[(oxan-4-yl)amino]pyridine-4-carbonyl}azetidin-3-yl)-1H-indazol-1-yl]-1-[(2S)-2-(trifluoromethyl)pyrrolidin-1-yl]ethan-1-one
(89) 2-{3-[1-(2-amino-3-methylpyridine-4-carbonyl)azetidin-3-yl]-4,6-difluoro-1H-indazol-1-yl]-1-[(2S)-2-(trifluoromethyl)morpholin-4-yl]ethan-1-one hydrochloride
(90) 2-{3-[1-(2-aminopyridine-4-carbonyl)piperidin-4-yl]-4-fluoro-1H-indazol-1-yl}-1-[(2S)-2-(trifluoromethyl)morpholin-4-yl]ethan-1-one
(91) 2-{3-[1-(2-aminopyridine-4-carbonyl)piperidin-4-yl]-4-fluoro-1H-indazol-1-yl}-1-[(2R)-2-(trifluoromethyl)morpholin-4-yl]ethan-1-one
(92) 4-[3-(1-{[1-(cyclobutylmethyl)-1H-1,2,3-triazol-4-yl)methyl}-4-fluoro-1H-indazol-3-yl)azetidine-1-carbonyl]pyridin-2-amine hydrochloride
(93) 2-(3-{1-[2-(cyclopropylamino)pyridine-4-carbonyl]azetidin-3-yl}-4-methyl-1H-pyrazolo[9,3-c]pyridin-1-yl)-1-[(2S)-2-(trifluoromethyl)pyrrolidin-1-yl]ethan-1-one
(94) 2-{3-[1-(2-amino-3-methylpyridine-4-carbonyl)azetidin-3-yl]-4-methyl-1H-pyrazolo[4,3-c]pyridin-1-yl}-1-[(2S)-2-(trifluoromethyl)pyrrolidin-1-yl]ethan-1-one
(95) 2-{3-[1-(2-aminopyridine-4-carbonyl)azetidin-3-yl]-4-fluoro-1H-indazol-1-yl}-1-[(2R)-2-(trifluoromethyl)morpholin-4-yl]ethan-1-one hydrochloride
(96) 2-{3-[1-(2-aminopyridine-4-carbonyl)azetidin-3-yl]-4-fluoro-1H-indazol-1-yl}-1-[(2S)-2-(trifluoromethyl)pyrrolidin-1-yl]ethan-1-one
(97) 2-{3-[1-(2-amino-1,3-thiazole-5-carbonyl)azetidin-3-yl]-4,6-difluoro-1H-indazol-1-yl}-1-[(2S)-2-(trifluoromethyl)pyrrolidin-1-yl]ethan-1-one
(98) 2-(3-{1-[2-(cyclopropylamino)-1,3-thiazole-5-carbonyl]azetidin-3-yl}-4,6-difluoro-1H-indazol-1-yl)-1-[(2S)-2-(trifluoromethyl)pyrrolidin-1-yl]ethan-1-one
(99) 2-(3-{1-[2-(cyclopropylamino)-4-methyl-1,3-thiazole-5-carbonyl]azetidin-3-yl}-4,6-difluoro-1H-indazol-1-yl)-1-[(2S)-2-(trifluoromethyl)pyrrolidin-1-yl]ethan-1-one
(100) 2-{3-[1-(2-aminopyridine-4-carbonyl)azetidin-3-yl]-4-fluoro-1H-pyrazolo[3,4-c]pyridin-1-yl}-1-[(2R)-2-(trifluoromethyl)morpholin-4-yl]ethan-1-one
(101) 2-[4,6-difluoro-3-(1-{imidazo[1,2-a]pyridine-3-carbonyl}azetidin-3-yl)-1H-indazol-1-yl]-1-[(2S)-2-(trifluoromethyl)pyrrolidin-1-yl]ethan-1-one
(102) 2-[4,6-difluoro-3-(1-{pyrazolo[3,2-b][1,3]thiazol-7-carbonyl}azetidin-3-yl)-1H-indazol-1-yl]-1-[(2S)-2-(trifluoromethyl)pyrrolidin-1-yl]ethan-1-one
(103) 2-{3-[1-(2-aminopyridine-4-carbonyl)azetidin-3-yl]-4,6-difluoro-1H-indazol-1-yl}-1-[(2S)-2-(trifluoromethyl)pyrrolidin-1-yl]propane-1-one hydrochloride
(104) 4-[3-(4-fluoro-1-{[3-(4-fluorophenyl)-1,2,4-oxadiazol-5-yl]methyl}-1H-indazol-3-yl)azetidine-1-carbonyl]pyridin-2-amine
(105) 2-{3-[1-(2-amino-1,3-oxazole-5-carbonyl)azetidin-3-yl]-4,6-difluoro-1H-indazol-1-yl}-1-[(2S)-2-(trifluoromethy])pyrrolidin-1-yl]ethan-1-one
(106) 2-{3-[1-(2-amino-4-methyl-1,3-thiazole-5-carbonyl)azetidin-3-yl]-4,6-difluoro-1H-indazol-1-yl}-1-[(2S)-2-(trifluoromethyl)pyrrolidin-1-yl]ethan-1-one
(107) 2-{3-[1-(2-aminopyridine-4-carbonyl)azetidin-3-yl]-4,6-difluoro-1H-indazol-1-yl}-2-fluoro-1-[(2S)-2-(trifluoromethyl)pyrrolidin-1-yl]ethan-1-one
(108) 2-{3-[1-(2-aminopyridine-4-carbonyl)piperidin-4-yl]-4-fluoro-1H-indol-1-yl}-1-[(2S)-2-(trifluoromethyl)pyrrolidin-1-yl]ethan-1-one
(109) 4-(3-{4-fluoro-1-[(3-methyl-1,2,4-oxadiazol-5-yl)methyl]-1H-indazol-3-yl}azetidine-1-carbonyl)pyridin-2-amine
(110) 2-{3-[1-(2-amino-4-methyl-1,3-oxazole-5-carbonyl)azetidin-3-yl]-4,6-difluoro-1H-indazol-1-yl}-1-[(2S)-2-(trifluoromethyl)pyrrolidin-1-yl]ethan-1-one
(111) 2-{3-[1-(2-aminopyridine-4-carbonyl)azetidin-3-yl]-4,6-difluoro-1H-indazol-1-yl}-1-((2S)-4,4-difluoro-2-(fluoromethyl)pyrrolidin-1-yl]ethan-1-one
(112) 4-[3-(4-fluoro-1-{[4-(trifluoromethyl)phenyl]methyl}-1H-indazol-3-yl)azetidine-1-carbonyl]pyridin-2-amine hydrochloride
(113) 4-[3-(4-fluoro-1-{[3-(trifluoromethyl)phenyl]methyl}-1H-indazol-3-yl)azetidine-1-carbonyl]pyridin-2-amine hydrochloride
(114) 4-[3-(4-fluoro-1-{[2-(trifluoromethyl)phenyl]methyl}-1H-indazol-3-yl)azetidine-1-carbonyl]pyridin-2-amine hydrochloride
(115) 4-[3-(4-fluoro-1-{[3-(trifluoromethyl)-1,2,4-oxadiazol-5-yl]methyl}-1H-indazol-3-yl)azetidine-1-carbonyl]pyridin-2-amine hydrochloride
(116) 4-[3-(4-fluoro-1-{[5-(4-fluorophenyl)-1,2,4-oxadiazol-3-yl]methyl}-1H-indazol-3-yl)azetidine-1-carbonyl]pyridin-2-amine
(117) 4-{3-[4-fluoro-1-({5-[3-(trifluoromethyl)phenyl]-1,2,4-oxadiazol-3-yl}methyl)-1H-indazol-3-yl]azetidine-1-carbonyl}pyridin-2-amine
(118) 2-{3-[1-(2-amino-1,3-thiazole-5-carbonyl)piperidin-4-yl]-4-fluoro-1H-indazol-1-yl}-1-[(2S)-2-(trifluoromethyl)pyrrolidin-1-yl]ethan-1-one
(119) 2-{3-[1-(2-amino-4-methyl-1,3-thiazole-5-carbonyl)piperidin-4-yl]-4-fluoro-1H-indazol-1-yl}-1-[(2S)-2-(trifluoromethyl)pyrrolidin-1-yl]ethan-1-one
(120) 2-{3-[1-(2-aminopyridine-4-carbonyl)azetidin-3-yl]-1H-pyrazolo[4,3-b]pyridin-1-yl}-1-[(2S)-2-(trifluoromethyl)pyrrolidin-1-yl]ethan-1-one
(121) 2-(3-{1-[2-(cyclopropylamino)pyridine-4-carbonyl]azetidin-3-yl}-1H-pyrazolo[4,3-b]pyridin-1-yl)-1-[(2S)-2-(trifluoromethyl)pyrrolidin-1-yl]ethan-1-one
(122) 2-{3-[1-(2-amino-4-methyl-1,3-thiazole-5-carbonyl)azetidin-3-yl]-1H-pyrazolo[4,3-b]pyridin-1-yl}-1-[(2S)-2-(trifluoromethyl)pyrrolidin-1-yl]ethan-1-one
(123) 2-{3-[1-(2-aminopyridine-4-carbonyl)azetidin-3-yl]-4,6-difluoro-1H-indazol-1-yl}-1-[(3S)-3-(trifluoromethyl)morpholin-4-yl]ethan-1-one
(124) 2-{3-[1-(2-aminopyridine-4-carbonyl)-1,2,3,6-tetrahydropyridin-4-yl]-4-fluoro-1H-indol-1-yl}-1-[(2S)-2-(trifluoromethyl)pyrrolidin-1-yl]ethan-1-one
(125) 2-{3-[1-(2-aminopyridine-4-carbonyl)azetidin-3-yl]-4,6-difluoro-1H-indazol-1-yl)-1-[(3S)-3-(difluoromethoxy)pyrrolidin-1-yl]ethan-1-one
(126) 2-{3-[1-(2-aminopyridine-4-carbonyl)azetidin-3-yl}-4,6-difluoro-1H-indazol-1-yl}-1-[(3R)-3-(difluoromethoxy)pyrrolidin-1-yl]ethan-1-one
(127) 2-{3-[1-(2-aminopyridine-4-carbonyl)azetidin-3-yl]-4,6-difluoro-1H-indazol-1-yl}-1-(2,2-dimethylpyrrolidin-1-yl)ethan-1-one
(128) 4-[3-(4-fluoro-1-{[5-(trifluoromethyl)-1,3,4-oxadiazol-2-yl]methyl}-1H-indazol-3-yl)azetidine-1-carbonyl]pyridin-2-amine
(129) 2-{3-[1-(2-aminopyrimidine-4-carbonyl)azetidin-3-yl]-4,6-difluoro-1H-indazol-1-yl}-1-[(2S)-2-(trifluoromethyl)pyrrolidin-1-yl]ethan-1-one
(130) 2-(3-{1-[2-(cyclopropylamino)pyridine-4-carbonyl]azetidin-3-yl}-5-methyl-1H-pyrazolo[4,3-b]pyridin-1-yl)-1-[(2S)-2-(trifluoromethyl)pyrrolidin-1-yl]ethan-1-one
(131) 2-{3-[1-(6-aminopyrimidine-4-carbonyl)azetidin-3-yl]-4,6-difluoro-1H-indazol-1-yl}-1-[(2S)-2-(trifluoromethyl)pyrrolidin-1-yl}ethan-1-one
(132) 2-{4,6-difluoro-3-[1-(pyridine-4-carbonyl)azetidin-3-yl]-1H-indazol-1-yl}-1-[(2S)-2-(trifluoromethyl)pyrrolidin-1-yl]ethan-1-one
(133) 2-{4,6-difluoro-3-[1-(3-methoxypyridine-4-carbonylazetidin-3-yl]-1H-indazol-1-yl}-1-[(2S)-2-(trifluoromethyl)pyrrolidin-1-yl}ethan-1-one
(134) 4-[4-(4-fluoro-1-{[6-(trifluoromethyl)pyridin-3-yl]methyl}-1H-indazol-3-yl)piperidine-1-carbonyl]pyridin-2-amine
(135) 2-{3-[1-(2-aminopyridine-4-carbonyl)piperidin-4-yl]-4-fluoro-1H-indol-1-yl}-1-[(2R)-2-(trifluoromethyl)morpholin-4-yl]ethan-1-one
(136) 2-{3-[1-(2-aminopyridine-4-carbonyl)piperidin-4-yl)-4-fluoro-1H-indol-1-yl}-1-[(2S)-2-(trifluoromethyl)morpholin-4-yl]ethan-1-one
(137) 4-[3-(4-fluoro-1-{[5-(oxan-4-yl)-1,2,4-oxadiazol-3-yl]methyl}-1H-indazol-3-yl)azetidine-1-carbonyl]pyridin-2-amine
(138) 4-[3-(4-fluoro-1-{[3-(pyridin-4-yl)-1,2,4-oxadiazol-5-yl]methyl}-1H-indazol-3-yl)azetidine-1-carbonyl]pyridin-2-amine
(139) 2-(3-{1-[2-amino-4-(trifluoromethyl)-1,3-thiazole-5-carbonyl]azetidin-3-yl}-4,6-difluoro-1H-indazol-1-yl)-1-[(2S)-2-(trifluoromethyl)pyrrolidin-1-yl]ethan-1-one
(140) 2-{4,6-difluoro-3-[1-(1H-indazole-5-carbonyl)azetidin-3-yl]-1H-indazol-1-yl}-1-[(2S)-2-(trifluoromethyl)pyrrolidin-1-yl}ethan-1-one
(141) 2-[4,6-difluoro-3-(1-{1H-pyrazolo[4,3-b]pyridine-5-carbonyl}azetidin-3-yl)-1H-indazol-1-yl]-1-[(2S)-2-(trifluoromethyl)pyrrolidin-1-yl]ethan-1-one
(142) 2-{4,6-difluoro-3-[1-(3-fluoropyridine-4-carbonyl)azetidin-3-yl]-1H-indazol-1-yl}-1-[(2S)-2-(trifluoromethyl)pyrrolidin-1-yl]ethan-1-one
(143) 2-{3-[1-(3-chloropyridine-4-carbonyl)azetidin-3-yl]-4,6-difluoro-1H-indazol-1-yl}-1-[(2S)-2-(trifluoromethyl)pyrrolidin-1-yl]ethan-1-one
(144) 2-{3-[1-(2-aminopyridine-4-carbonyl)azetidin-3-yl]-4-chloro-5-fluoro-1H-indazol-1-yl}-1-[(2S)-2-(trifluoromethyl)pyrrolidin-1-yl]ethan-1-one
(145) 2-{3-[1-(2-amino-3-fluoropyridine-4-carbonyl)azetidin-3-yl)-4,6-difluoro-1H-indazol-1-yl]-1-[(2S)-2-(trifluoromethyl)pyrrolidin-1-yl]ethan-1-one
(146) 4-{3-[4-fluoro-1-({5-[1-(trifluoromethyl)cyclobutyl]-1,2,4-oxadiazol-3-yl}methyl)-1H-indazol-3-yl]azetidine-1-carbonyl}pyridin-2-amine hydrochloride
(147) 4-{3-[4-fluoro-1-({5-[1-(trifluoromethyl)cyclopropyl]-1,2,4-oxadiazol-3-yl}methyl)-1H-indazol-3-yl]azetidine-1-carbonyl}pyridin-2-amine
(148) 4-[3-(4-fluoro-1-{[5-(2-fluorophenyl)-1,2,4-oxadiazol-3-yl]methyl}-1H-indazol-3-yl)azetidine-1-carbonyl]pyridin-2-amine
(149) 2-{3-(1-(2-amino-4-chloro-1,3-thiazole-5-carbonyl)azetidin-3-yl]-4,6-difluoro-1H-indazol-1-yl}-1-[(2S)-2-(trifluoromethyl)pyrrolidin-1-yl]ethan-1-one
(150) 4-[3-(4-fluoro-1-{[5-(trifluoromethy)pyridin-2-yl]methyl}-1H-pyrazolo[3,4-c]pyridin-3-yl)azetidine-1-carbonyl]pyridin-2-amine
(151) 4-[3-(4-fluoro-1-{[4-(trifluoromethyl)phenyl]methyl}-1H-pyrazolo[3,4-c]pyridin-3-yl)azetidine-1-carbonyl]pyridin-2-amine
(152) 4-{3-[4,6-difluoro-1-(3-phenylprop-2-yn-1-yl)-1H-indazol-3-yl]azetidine-1-carbonyl}pyridin-2-amine
(153) 4-[3-(4-fluoro-1-{[5-(5-fluoropyridin-2-yl)-1,2,4-oxadiazol-3-yl]methyl}-1H-indazol-3-yl)azetidine-1-carbonyl]pyridin-2-amine
(154) 4-[3-(4-fluoro-1-{[5-(6-methylpyridazin-3-yl)-1,2,4-oxadiazol-3-yl)methyl}-1H-indazol-3-yl)azetidine-1-carbonyl]pyridin-2-amine
(155) 2-{3-[1-(2-amino-5-fluoropyridine-4-carbonyl)azetidin-3-yl]-4,6-difluoro-1H-indazol-1-yl}-1-[(2S)-2-(trifluoromethyl)pyrrolidin-1-yl]ethan-1-one
(156) 4-{3-[1-(3-cyclopropylprop-2-yn-1-yl)-4,6-difluoro-1H-indazol-3-yl]azetidine-1-carbonyl}pyridin-2-amine
(157) 4-(3-{4,6-difluoro-1-[(1-phenylazetidin-3-yl)methyl]-1H-indazol-3-yl}azetidine-1-carbonyl)pyridin-2-amine
(158) 2-{3-[1-(2-aminopyridine-4-carbonyl)azetidin-3-yl]-4,6-difluoro-1H-indazol-1-yl}-1-(1,2,3,4-tetrahydroquinolin-1-yl)ethan-1-one
(159) 2-{3-[1-(2-aminopyridine-4-carbonyl)azetidin-3-yl]-4,6-difluoro-1H-indazol-1-yl}-1-(3,4-dihydro-2H-1,4-benzoxazin-4-yl)ethan-1-one
(160) 4-[3-(4-fluoro-1-{[5-(2-fluorophenyl)-1,2,4-oxadiazol-3-yl]methyl)-1H-pyrazolo[3,4-c]pyridin-3-yl)azetidine-1-carbonyl]pyridin-2-amine
(161) 2-{3-[1-(2-aminopyridine-4-carbonyl)azetidin-3-yl}-4,6-difluoro-1H-indazol-1-yl}-1-(6-bromo-2,3-dihydro-1H-indol-1-yl)ethan-1-one
(162) 2-{4,6-difluoro-3-[1-(oxane-4-carbonyl)azetidin-3-yl]-1H-indazol-1-yl}-1-[(2S)-2-(trifluoromethyl)pyrrolidin-1-yl]ethan-1-one
(163) 2-{4,6-difluoro-3-[1-(2-hydroxypyridine-4-carbonyl)azetidin-3-yl]-1H-indazol-1-yl}-1-[(2S)-2-(trifluoromethyl)pyrrolidin-1-yl]ethan-1-one
(164) 2-{4,6-difluoro-3-[1-(1H-pyrazole-4-carbonyl)azetidin-3-yl]-1H-indazol-1-yl}-1-((2S)-2-(trifluoromethyl)pyrrolidin-1-yl)ethan-1-one
(165) 2-{4,6-difluoro-3-[1-(5-methyl-1H-pyrazole-4-carbonylazetidin-3-yl]-1H-indazol-1-yl}-1-[(2S)-2-(trifluoromethyl)pyrrolidin-1-yl]ethan-1-one
(166) 2-(4,6-difluoro-3-{1-[5-(trifluoromethyl)-1H-pyrazole-4-carbonyl]azetidin-3-yl})-1H-indazol-1-yl)-1-[(2S)-2-(trifluoromethyl)pyrrolidin-1-yl]ethan-1-one
(167) 4-[3-(4,6-difluoro-1-{[1-(2,2,2-trifluoroethyl)azetidin-3-yl]methyl}-1H-indazol-3-yl)azetidine-1-carbonyl]pyridin-2-amine
(168) 4-[3-(5-methyl-1-{[4-(trifluoromethyl)phenyl]methyl}-1H-pyrazolo[3,4-c]pyridin-3-yl)azetidine-1-carbonyl]pyridin-2-amine
(169) 2-{3-[1-(1H-1,2,3-benztriazole-5-carbonyl)azetidin-3-yl]-4,6-difluoro-1H-indazol-1-yl}-1-[(2S)-2-(trifluoromethyl)pyrrolidin-1-yl]ethan-1-one
(170) 4-[3-(4-fluoro-1-{[3-(3-fluoropyridin-4-yl)-1,2,4-oxadiazol-5-yl]methyl}-1H-pyrazolo[3,4-c]pyridin-3-yl)azetidine-1-carbonyl]pyridin-2-amine
(171) 4-(3-{1-[(2,3-dihydro-1H-inden-2-yl)methyl]-4,6-difluoro-1H-indazol-3-yl}azetidine-1-carbonyl)pyridin-2-amine
(172) 2-13-[1-(2-aminopyridine-4-carbonyl)azetidin-3-yl]-4,6-difluoro-1H-indazol-1-yl}-1-(6-cyclopropyl-2,3-dihydro-1H-indol-1-yl)ethan-1-one
(173) 2-{3-[1-(2-amino-4-methyl-1,3-thiazole-5-carbonyl)azetidin-3-yl]-4,6-difluoro-1H-indazol-1-yl}-1-[(2R)-2-(trifluoromethyl)morpholin-4-yl]ethan-1-one
(174) 2-{3-[1-(2-amino-5-fluoropyridine-4-carbonyl)piperidin-4-yl]-4-fluoro-1H-indazol-1-yl}-1-[(2S)-2-(trifluoromethyl)pyrrolidin-1-yl]ethan-1-one
(175) 2-{3-[1-(2-amino-5-fluoropyridine-4-carbonyl)azetidin-3-yl]-4,6-difluoro-1H-indazol-1-yl}-1-[(2S)-2-[(trifluoromethoxy)methyl]pyrrolidin-1-yl]ethan-1-one
(176) 2-{3-[1-(2-amino-5-fluoropyridine-4-carbonyl)azetidin-3-yl]-4,6-difluoro-1H-indazol-1-yl}-1-(2,2-dimethylmorpholin-4-yl)ethan-1-one
(177) 2-{3-[1-(2-amino-5-fluoropyridine-4-carbonyl)azetidin-3-yl]-4,6-difluoro-1H-indazol-1-yl}-1-(3,3-dimethylmorpholin-4-yl)ethan-1-one
(178) 2-{3-[1-(2-amino-3-methylpyridine-4-carbonyl)azetidin-3-yl]-5-methyl-1H-pyrazolo[3,4-c]pyridin-1-yl}-1-[(2R)-2-(trifluoromethyl)morpholin-4-yl]ethan-1-one dihydrochloride
(179) 2-{3-[1-(2-amino-5-fluoropyridine-4-carbonyl)azetidin-3-yl]-4,6-difluoro-1H-indazol-1-yl}-1-[(2S)-2-(methoxymethyl)pyrrolidin-1-yl]ethan-1-one
(180) 2-{3-[1-(2-amino-3-methylpyridine-4-carbonyl)azetidin-3-yl]-4-fluoro-1H-pyrazolo[3,4-c]pyridin-1-yl}-1-[(2R)-2-(trifluoromethyl)morpholin-4-yl]ethan-1-one
(181) 2-{3-[1-(2-amino-5-fluoropyridine-4-carbonyl)azetidin-3-yl]-4-fluoro-1H-pyrazolo[3,4-clpyridin-1-yl}-1-[(2R)-2-(trifluoromethyl)morpholin-4-yllethan-1-one
(182) 2-{3-[1-(2-amino-5-fluoropyridine-4-carbonyl)azetidin-3-yl]-4,6-difluoro-1H-indazol-1-yl}-1-[(38,58)-3,5-dimethylmorpholin-4-yl]ethan-1-one
(183) 2-{3-[1-(2-amino-4-methyl-1,3-thiazole-5-carbonyl)azetidin-3-yl]-4-fluoro-1H-pyrazolo[3,4-c]pyridin-1-yl}-1-[(2S)-2-[(trifluoromethoxy)methyl]pyrrolidin-1-yllethan-1-one p-toluenesulfonate
(184) 2-{3-[1-(2-amino-3-fluoropyridine-4-carbonyl)azetidin-3-yl]-4,6-difluoro-1H-indazol-1-yl}-1-[(2R)-2-(trifluoromethyl)morpholin-4-yl]ethan-1-one p-toluenesulfonate
(185) 2-{3-[1-(2-amino-5-fluoropyridine-4-carbonyl)azetidin-3-yl]-5-methyl-1H-pyrazolo[4,3-b]pyridin-1-yl}-1-[(2R)-2-(trifluoromethyl)morpholin-4-yl]ethan-1-one p-toluenesulfonate
(186) 2-{3-[1-(2-amino-5-fluoropyridine-4-carbonyl)azetidin-3-yl)-5-methyl-1H-pyrazolo[4,3-b]pyridin-1-yl}-1-(3,3-dimethylmorpholin-4-yl)ethan-1-one p-toluenesulfonate
(187) 2-{3-[1-(2-amino-5-fluoropyridine-4-carbonyl)azetidin-3-yl]-1H-indazol-1-yl}-1-(3,3-dimethylmorpholin-4-yl)ethan-1-one
(188) 2-{3-[1-(2-aminopyridine-4-carbonyl)azetidin-3-yl]-1H-indazol-1-y1}-1-(3,3-dimethylmorpholin-4-yl)ethan-1-one
(189) 2-{3-[1-(2-aminopyridine-4-carbonyl)azetidin-3-yl]-4,6-difluoro-1H-indazol-1-yl}-N-tert-butylacetamide
(190) 2-{3-[1-(2-amino-3-fluoropyridine-4-carbonyl)azetidin-3-yl]- 1H-indazol-1-yl}-1-(3,3-dimethylmorpholin-4-yl)ethan-1-one
(191) 2-{3-[1-(2-amino-3-methylpyridine-4-carbonyl)azetidin-3-yl]-1H-indazol-1-yl}-1-(3,3-dimethylmorpholin-4-yl)ethan-1-one
(192) 2-{3-[1-(2-amino-5-fluoropyridine-4-carbonyl)azetidin-3-yl]-1H-indazol-1-yl}-1-[(2R)-2-(trifluoromethyl)morpholin-4-yl]ethan-1-one
(193) 2-{3-[1-(2-aminopyridine-4-carbonyl)azetidin-3-yl)-1H-indazol-1-yl}-1-[(2R)-2-(trifluoromethyl)morpholin-4-yl]ethan-1-one
(194) 2-{3-[1-(2-amino-3-fluoropyridine-4-carbonyl)azetidin-3-yl]-1H-indazol-1-yl}-1-[(2R)-2-(trifluoromethyl)morpholin-4-y]ethan-1-one
(195) 2-{3-[1-(2-amino-3-methylpyridine-4-carbonyl)azetidin-3-yl]-1H-indazol-1-yl}-1-[(2S)-2-(trifluoromethyl)pyrrolidin-1-yl]ethan-1-one
(196) 2-{3-(1-(2-amino-3-fluoropyridine-4-carbonyl)azetidin-3-yl]-1H-indazol-1-yl}-1-[(2S)-2-(trifluoromethyl)pyrrolidin-1-yl]ethan-1-one
(197) 2-{3-(1-(2-amino-4-methyl-1,3-thiazole-5-carbonyl)azetidin-3-yl]-1H-indazol-1-yl}-1-[(2S)-2-(trifluoromethyl)pyrrolidin-1-yl]ethan-1-one
(198) 2-{3-[1-(2-aminopyridine-4-carbonyl)azetidin-3-yl]-1H-indazol-1-yl}-N-tert-butylacetamide
(199) 2-{3-[1-(2-amino-5-fluoropyridine-4-carbonyl)azetidin-3-yl]-1H-indazol-1-yl}-N-tert-butylacetamide
(200) 2-{3-[1-(2-aminopyridine-4-carbonyl)azetidin-3-yl]-5-methyl-1H-indazol-1-yl}-1-[(2S)-2-(trifluoromethyl)pyrrolidin-1-yl]ethan-1-one
(201) 2-{3-[1-(2-amino-5-fluoropyridine-4-carbonyl)azetidin-3-yl]-5-methyl-1H-indazol-1-yl}-1-[(2S)-2-(trifluoromethyl)pyrrolidin-1-yl]ethan-1-one
(202) 2-{3-[1-(2-aminopyridine-4-carbonyl)azetidin-3-yl]-5-fluoro-1H-indazol-1-yl}-1-[(2S)-2-(trifluoromethyl)pyrrolidin-1-yl]ethan-1-one
(203) 2-{3-[1-(2-amino-5-fluoropyridine-4-carbonyl)azetidin-3-yl]-5-fluoro-1H-indazol-1-yl}-1-[(2S)-2-(trifluoromethyl)pyrrolidin-1-yl]ethan-1-one
(204) 2-{3-[1-(2-amino-5-fluoropyridine-4-carbonyl)azetidin-3-yl]-5-chloro-1H-indazol-1-yl}-1-[(2R)-2-(trifluoromethyl)morpholin-4-yl]ethan-1-one
(205) 2-{3-[1-(2-aminopyridine-4-carbonyl)azetidin-3-yl]-5-chloro-1H-indazol-1-yl}-1-[(2R)-2-(trifluoromethyl)morpholin-4-yl]ethan-1-one
(206) 2-{3-[1-(2-amino-5-fluoropyridine-4-carbonyl)azetidin-3-yl]-5-chloro-1H-indazol-1-yl}-1-[(2S)-2-(trifluoromethyl)pyrrolidin-1-yl]ethan-1-one
(207) 2-{3-[1-(2-aminopyridine-4-carbonyl)azetidin-3-yl]-5-chloro-1H-indazol-1-yl}-1-[(2S)-2-(trifluoromethyl)pyrrolidin-1-yl]ethan-1-one
(208) 2-{3-[1-(2-aminopyridine-4-carbonyl)azetidin-3-yl]-1H-indazol-1-yl}-N-(1-methoxy-2-methylpropan-2-yl)acetamide
(209) 2-{3-[1-(2-aminopyridine-4-carbonyl)azetidin-3-yl]-1H-indazol-1-yl}-N-(4-methyloxan-4-yl)acetamide
(210) 2-{3-(1-(2-amino-5-chloropyridine-4-carbonyl)azetidin-3-yl]-1H-indazol-1-yl}-1-((2S)-2-(trifluoromethyl)pyrrolidin-1-yl]ethan-1-one
(211) 2-{3-(1-(2-aminopyridine-4-carbonyl)azetidin-3-yl]-1H-indazol-1-yl}-N-(2,2,2-trifluoroethyl)acetamide
(212) 2-{3-[1-(2-aminopyridine-4-carbonyl)azetidin-3-yl]-1H-indazol-1-yl}-N-[(2R)-1,1,1-trifluoropropan-2-yl]acetamide
(213) 2-{3-[1-(2-aminopyridine-4-carbonyl)azetidin-3-yl]-1H-indazol-1-yl}-N-[(2S)-1,1,1-trifluoropropan-2-yl]acetamide
(214) 2-{3-[1-(2-aminopyridine-4-carbonyl)azetidin-3-yl]-1H-indazol-1-yl}-N-(3,3-difluorocyclobutyl)acetamide
(215) 2-{3-[1-(2-aminopyrimidine-4-carbonyl)azetidin-3-yl)-1H-indazol-1-yl}-1-[(2R)-2-(trifluoromethyl)morpholin-4-yl]ethan-1-one
(216) 4-[3-(4-fluoro-1-{[5-(trifluoromethyl)-1,2,4-oxadiazol-3-yl]methyl}-1H-indazol-3-yl)azetidine-1-carbonyl]pyridin-2-amine
(217) 4-[3-(1-{[5-(trifluoromethyl)-1,2,4-oxadiazol-3-yl]methyl}-1H-indazol-3-yl)azetidine-1-carbonyl]pyridin-2-amine
(218) 4-[3-(1-{[3-(trifluoromethyl)-1,2,4-oxadiazol-5-yl]methyl}-1H-indazol-3-yl)azetidine-1-carbonyl]pyridin-2-amine
(219) 2-{3-[1-(2-aminopyridine-4-carbonyl)azetidin-3-yl]-1H-indazol-1-yl}-1-[(2S)-2-(hydroxymethyl)pyrrolidin-1-yl]ethan-1-one
(220) tert-butyl 2-{3-[1-(2-amino-5-fluoropyridine-4-carbonyl)azetidin-3-yl]-1H-indazol-1-yl}acetate
(221) 2-{3-[1-(2-aminopyridine-4-carbonyl)azetidin-3-yl]-1H-indazol-1-yl}-1-(2,2-dimethylpyrrolidin-1-yl)ethan-1-one
(222) 2-{3-[1-(2-aminopyridine-4-carbonyl)azetidin-3-yl]-1H-indazol-1-yl}-1-[(2R)-2-ethylpyrrolidin-1-yl]ethan-1-one
(223) 2-{3-[1-(2-aminopyridine-4-carbonyl)azetidin-3-yl]-1H-indazol-1-yl}-1-{2-azabicyclo[3.1.0]hexan-2-yl}ethan-1-one
(224) 2-{3-[1-(2-aminopyridine-4-carbonyl)azetidin-3-yl]-1H-indazol-1-yl}-N-(dicyclopropylmethyl)acetamide
(225) 2-{3-[1-(2-aminopyridine-4-carbonyl)azetidin-3-yl}-1H-indazol-1-yl}-N-(2,2-difluorocyclopentyl)acetamide
(226) 2-{3-[1-(2-aminopyridine-4-carbonyl)azetidin-3-yl]-1H-indazol-1-yl}-N-(4-fluorophenyl)acetamide
(227) 2-{3-[1-(2-aminopyridine-4-carbonyl)azetidin-3-yl]-1H-indazol-1-yl}-N-[2-(trifluoromethoxy)ethyl] acetamide
(228) 2-{3-[1-(2-aminopyridine-4-carbonyl)azetidin-3-yl]-1H-indazol-1-yl}-1-[(2S)-2-(methoxymethyl)pyrrolidin-1-yl]ethan-1-one
(229) 2-{3-[1-(2-aminopyridine-4-carbonyl)azetidin-3-yl]-1H-indazol-1-yl}-N-(cyclopropylmethyl)-N-methylacetamide
(230) 2-{3-[1-(2-amino-5-fluoropyridine-4-carbonyl)azetidin-3-yl)-1H-indazol-1-yl}-1-[(3S)-3-methoxypyrrolidin-1-yl]ethan-1-one
(231) 2-{3-[1-(2-amino-5-fluoropyridine-4-carbonyl)azetidin-3-yl]-1H-indazol-1-yl}-1-[(3R)-3-methoxypyrrolidin-1-yl]ethan-1-one
(232) 2-{3-[1-(2-amino-5-fluoropyridine-4-carbonyl)azetidin-3-yl]-1H-indazol-1-yl}-1-{2-oxa-6-azaspiro[3.4]octan-6-yl}ethan-1-one
(233) 2-{3-[1-(2-aminopyridine-4-carbonyl)azetidin-3-yl]-1H-indazol-1-yl}-N-(oxan-4-yl)acetamide
(234) 2-{3-[1-(2-aminopyridine-4-carbonyl)azetidin-3-yl]-1H-indazol-1-yl}-N-(2-cyclopropylpropan-2-yl)acetamide
(235) 2-{3-[1-(2-aminopyridine-4-carbonyl)azetidin-3-yl]-1H-indazol-1-yl}-N-(4,4-difluorocyclohexyl)acetamide
(236) (2S)-1-(2-{3-[1-(2-amino-5-fluoropyridine-4-carbonyl)azetidin-3-yl]-1H-indazol-1-yl}acetyl)pyrrolidine-2-carbonitrile
(237) 2-{3-[1-(2-amino-5-fluoropyridine-4-carbonyl)azetidin-3-yl]-1H-indazol-1-yl}-1-[(2S)-4,4-difluoro-2-(fluoromethyl)pyrrolidin-1-yl]ethan-1-one
(238) 2-{3-[1-(2-aminopyridine-4-carbonyl)azetidin-3-yl]-4-fluoro-1H-indazol-1-yl}-N-{bicyclo[2.2.1]heptan-2-yl}acetamide
(239) 2-{3-[1-(2-aminopyridine-4-carbonyl)azetidin-3-yl]-4-fluoro-1H-indazol-1-yl}-N-{bicyclo[1.1.1]pentan-1-yl}acetamide
(240) 2-{3-(1-(2-amino-5-fluoropyridine-4-carbonyl)azetidin-3-yl]-1H-indazol-1-yl}-N-methyl-N-(2,2,2-trifluoroethyl)acetamide
(241) 2-{3-[1-(2-aminopyrimidine-4-carbonyl)azetidin-3-yl]-5-fluoro-1H-indazol-1-yl}-1-[(2S)-2-(trifluoromethyl)pyrrolidin-1-yl]ethan-1-one
(242) 2-{3-(1-(2-aminopyridine-4-carbonyl)azetidin-3-yl]-4-fluoro-1H-indazol-1-yl}-N-(2,2-difluoroethyl)acetamide
(243) 2-{3-[1-(2-amino-5-fluoropyridine-4-carbonyl)azetidin-3-yl]-1H-indazol-1-yl}-N-[trans-2-fluorocyclopropyl]acetamide
(244) 2-{3-[1-(2-amino-5-fluoropyridine-4-carbonyl)azetidin-3-yl]-1H-indazol-1-yl}-N-(1-cyanocyclopropyl)acetamide
(245) 2-{3-[1-(2-amino-5-fluoropyridine-4-carbonyl)azetidin-3-yl]-1H-indazol-1-yl}-N-[(1R,2S)-2-fluorocyclopropyl]acetamide
(246) 2-{3-[1-(2-amino-5-fluoropyridine-4-carbonyl)azetidin-3-yl]-1H-indazol-1-yl}-N-[(1S,2R)-2-fluorocyclopropyl]acetamide
(247) 2-{3-[1-(2-amino-5-fluoropyridine-4-carbonyl)azetidin-3-yl]-1H-indazol-1-yl}-N-(1-methyl-1H-pyrazol-5-yl)acetamide
(248) 2-{3-[1-(2-amino-5-fluoropyridine-4-carbonyl)azetidin-3-yl]-1H-indazol-1-yl}-N-(2,2-difluorocyclopropyl)acetamide
(249) 2-{3-[1-(2-amino-5-fluoropyridine-4-carbonyl)azetidin-3-yl]-1H-indazol-1-yl}-N-ethyl-N-(2,2,2-trifluoroethyl)acetamide
(250) 2-{3-[1-(2-amino-5-fluoropyridine-4-carbonyl)azetidin-3-yl]-1H-indazol-1-yl}-N-(3,3-difluorocyclobutyl)-N-methylacetamide
(251) 2-{3-[1-(2-amino-6-fluoropyridine-4-carbonyl)azetidin-3-yl]-1H-indazol-1-yl}-N-cyclopropyl-N-(2,2,2-trifluoroethyl)acetamide
(252) 2-{3-[1-(2-amino-5-fluoropyridine-4-carbonyl)azetidin-3-yl]-1H-indazol-1-yl}-N-cyclopropyl-N-methylacetamide
(253) 2-{3-[1-(2-amino-5-fluoropyridine-4-carbonyl)azetidin-3-yl]-1H-indazol-1-yl}-N-methyl-N-(propan-2-yl)acetamide
(254) 2-{3-[1-(2-amino-5-fluoropyridine-4-carbonyl)azetidin-3-yl]-1H-indazol-1-yl}-N-methyl-N-(oxetan-3-yl)acetamide
(255) 2-{3-[1-(2-amino-5-fluoropyridine-4-carbonyl)azetidin-3-yl]-1H-indazol-1-yl}-N-methyl-N-(oxan-4-yl)acetamide
(256) 2-{3-[1-(2-amino-5-fluoropyridine-4-carbonyl)azetidin-3-yl}-1H-indazol-1-yl}-N-[(3,3-difluorocyclobutyl)methyl]-N-methylacetamide
(257) 2-{3-[1-(2-amino-5-fluoropyridine-4-carbonyl)azetidin-3-yl]-1H-indazol-1-yl}-1-(4,4-difluoropiperidin-1-yl)ethan-1-one
(258) 2-{3-[1-(2-amino-5-fluoropyridine-4-carbonyl)azetidin-3-yl]-1H-indazol-1-yl}-1-{8-oxa-3-azabicyclo[3.2.1]octan-3-yl}ethan-1-one
(259) 2-{3-[1-(2-amino-5-fluoropyridine-4-carbonyl)azetidin-3-yl]-1H-indazol-1-yl}-1-[3-(trifluoromethyl)piperidin-1-yl]ethan-1-one
(260) 2-{3-[1-(2-amino-5-fluoropyridine-4-carbonyl)azetidin-3-yl]-1H-indazol-1-yl}-1-(4-methylpiperazin-1-yl)ethan-1-one
(261) 2-{3-[1-(2-amino-5-fluoropyridine-4-carbonyl)azetidin-3-yl]-1H-indazol-1-yl}-N-methyl-N-[(2S)-1,1,1-trifluoropropan-2-yl]acetamide
(262) 2-{3-[1-(2-amino-5-fluoropyridine-4-carbonyl)azetidin-3-yl]-1H-indazol-1-yl}-N-methyl-N-[(2R)-1,1,1-trifluoropropan-2-yl]acetamide
(263) 2-{3-[1-(2-amino-5-fluoropyridine-4-carbonyl)azetidin-3-yl]-1H-indazol-1-yl}-N,N-diethylacetamide
(264) 2-{3-[1-(2-amino-5-fluoropyridine-4-carbonyl)azetidin-3-yl]-1H-indazol-1-yl}-N-methyl-N-[2-(morpholin-4-yl)ethyl]acetamide
(265) 2-{3-[1-(2-amino-5-fluoropyridine-4-carbonyl)azetidin-3-yl]-1H-indazol-1-yl}-N-(2-methoxyethyl)-N-methylacetamide
(266) 2-1{3-[1-(2-amino-5-fluoropyridine-4-carbonyl)azetidin-3-yl]-1H-indazol-1-yl}-1-[(2R,6S)-2,6-dimethylpiperidin-1-yl]ethan-1-one
(267) 2-{3-[1-(2-amino-5-fluoropyridine-4-carbonyl)azetidin-3-yl]-1H-indazol-1-yl}-N,N-bis(propan-2-yl)acetamide
(268) 2-{3-[1-(2-amino-5-fluoropyridine-4-carbonyl)azetidin-3-yl]-1H-indazol-1-yl}-N-tert-butyl-N-methylacetamide
(269) 2-{3-[1-(2-amino-5-fluoropyridine-4-carbonyl)azetidin-3-yl]-1H-indazol-1-yl}-N-(2,2-difluoroethyl)-N-methylacetamide
(270) 2-{3-(1-(2-amino-5-fluoropyridine-4-carbonyl)-3-methylazetidin-3-yl]-1H-indazol-1-yl}-N-methyl-N-(2,2,2-trifluoroethyl)acetamide
(271) 2-{3-[1-(2-amino-5-fluoropyridine-4-carbonyl)-3-methylazetidin-3-yl]-1H-indazol-1-yl}-1-[(2S)-2-(trifluoromethyl)pyrrolidin-1-yl]ethan-1-one
(272) 2-{3-[1-(2-amino-5-fluoropyridine-4-carbonyl)azetidin-3-yl]-1H-indazol-1-yl}-1-[(2S)-2-(difluoromethyl)pyrrolidin-1-yl]ethan-1-one
(273) 2-{3-[1-(2-aminopyridine-4-carbonyl)-3-methylazetidin-3-yl]-1H-indazol-1-yl}-N-methyl-N-(2,2,2-trifluoroethyl)acetamide
(274) 2-{3-[1-(2-amino-5-fluoropyridine-4-carbonyl)azetidin-3-yl]-5-chloro-1H-indazol-1-yl}-N-methyl-N-(2,2,2-trifluoroethyl)acetamide
(275) 2-{3-[1-(2-amino-5-fluoropyridine-4-carbonyl)azetidin-3-yl]-6-fluoro-1H-indazol-1-yl}-1-[(2S)-2-(trifluoromethyl)pyrrolidin-1-yl]ethan-1-one
(276) 2-{3-[1-(2-amino-5-fluoropyrimidine-4-carbonyl)azetidin-3-yl]-1H-indazol-1-yl}-N-methyl-N-(2,2,2-trifluoroethyl)acetamide
(277) 2-{3-[1-(2-amino-5-fluoropyridine-4-carbonyl)azetidin-3-yl]-5-fluoro-1H-indazol-1-yl}-N-methyl-N-(2,2,2-trifluoroethyl)acetamide
(278) 2-{3-[1-(2-amino-5-fluoropyrimidine-4-carbonyl)azetidin-3-yl]-5-fluoro-1H-indazol-1-yl)-N-methyl-N-(2,2,2-trifluoroethyl)acetamide
(279) 2-{3-[1-(2-amino-5-fluoropyridine-4-carbonyl)azetidin-3-yl]-5,6-difluoro-1H-indazol-1-yl}-N-methyl-N-(2,2,2-trifluoroethyl)acetamide
(280) 2-{3-[1-(2-amino-5-fluoropyridine-4-carbonyl)azetidin-3-yl]-5,6-difluoro-1H-indazol-1-yl}-1-[(2S)-2-(trifluoromethyl)pyrrolidin-1-yl]ethan-1-one
(281) 2-{3-[1-(2-amino-5-fluoropyridine-4-carbonyl)-3-hydroxyazetidin-3-yl]-1H-indazol-1-yl}-1-[(2S)-2-(trifluoromethyl)pyrrolidin-1-yl]ethan-1-one
(282) 2-{3-[1-(2-aminopyrimidine-4-carbonyl)azetidin-3-yl]-5,6-difluoro-1H-indazol-1-yl}-N-methyl-N-(2,2,2-trifluoroethyl)acetamide
(283) 2-{3-[1-(2-aminopyrimidine-4-carbonyl)azetidin-3-yl]-5,6-difluoro-1H-indazol-1-yl}-1-[(2S)-2-(trifluoromethyl)pyrrolidin-1-yl]ethan-1-ane
(284) 2-{3-[1-(2-aminopyrimidine-4-carbonyl)azetidin-3-yl]-6-fluoro-1H-indazol-1-yl}-N-methyl-N-(2,2,2-trifluoroethyl)acetamide
(285) 2-{3-[1-(2-amino-5-fluoropyridine-4-carbonyl)-3-fluoroazetidin-3-yl]-1H-indazol-1-yl}-1-[(2S)-2-(trifluoromethyl)pyrrolidin-1-yl]ethan-1-one
(286) 2-{3-[1-(2-aminopyrimidine-4-carbonyl)azetidin-3-yl]-6-fluoro-1H-indazol-1-yl}-1-[(2S)-2-(trifluoromethyl)pyrrolidin-1-yl]ethan-1-one
(287) 2-{3-[1-(2-aminopyrimidine-4-carbonyl)azetidin-3-yl]-4-chloro-5-fluoro-1H-indazol-1-yl}-N-methyl-N-(2,2,2-trifluoroethyl)acetamide
(288) 2-{3-[1-(2-aminopyrimidine-4-carbonyl)azetidin-3-yl]-5-methyl-1H-indazol-1-yl}-N-methyl-N-(2,2,2-trifluoroethyl)acetamide
(289) 2-{3-[1-(2-aminopyrimidine-4-carbonyl)azetidin-3-yl]-4,6-difluoro-1H-indazol-1-yl}-N-methyl-N-(2,2,2-trifluoroethyl)acetamide
(290) 2-{3-(1-(2-aminopyrimidine-4-carbonyl)azetidin-3-yl]-5-chloro-1H-indazol-1-yl}-1-[(2S)-2-(trifluoromethyl)pyrrolidin-1-yl]ethan-1-one
(291) 2-{3-[1-(2-amino-5-fluoropyridine-4-carbonyl)azetidin-8-yl]-5-methoxy-1H-indazol-1-yl}-1-[(2S)-2-(trifluoromethyl)pyrrolidin-1-yl]ethan-1-one
(292) 2-{3-[1-(2-aminopyrimidine-4-carbonyl)azetidin-3-yl)-5-methyl-1H-indazol-1-yl}-1-[(2S)-2-(trifluoromethyl)pyrrolidin-1-yl]ethan-1-one
(293) 2-{3-[1-(2-aminopyrimidine-4-carbonyl)azetidin-3-yl]-5-fluoro-1H-indazol-1-yl}-N-methyl-N-(2,2,2-trifluoroethyl)acetamide
(294) 2-{3-[1-(2-amino-5-fluoropyridine-4-carbonyl)azetidin-3-yl]-5,5-dimethyl-4,5,6,7-tetrahydro-1H-indazol-1-yl}-1-[(2S)-2-(trifluoromethyl)pyrrolidin-1-yl]ethan-1-one, and
(295) 2-{3-[1-(2-aminopyrimidine-4-carbonyl)azetidin-3-yl]-5,5-dimethyl-4,5,6,7-tetrahydro-1H-indazol-1-yl}-1-[(2S)-2-(trifluoromethyl)pyrrolidin-1-yl]ethan-1-one.

10. 2-{3-[1-(2-Aminopyridine-4-carbonyl)azetidin-3-yl]-4,6-difluoro-1H-indazol-1-yl}-1-[(2S)-2-(trifluoromethyl)pyrrolidin-1-yl]ethan-1-one, or a pharmaceutically acceptable salt thereof, or a solvate thereof.

11. 2-{3-[1-(2-Aminopyridine-4-carbonyl)azetidin-3-yl]-4,6-difluoro-1H-indazol-1-yl}-1-[(2S)-2-(trifluoromethyl)pyrrolidin-1-yl]ethan-1-one hydrochloride, or a solvate thereof.

12. 2-{3-[1-(2-Amino-5-fluoropyridine-4-carbonyl)azetidin-3-yl]-6-fluoro-1H-indazol-1-yl)-N-methyl-N-(2,2,2-trifluoroethyl)acetamide, or a pharmaceutically acceptable salt thereof, or a solvate thereof.

13. 2-{3-[1-(2-Aminopyridine-4-carbonyl)-3-methylazetidin-3-yl]-1H-indazol-1-yl}-1-[(2S)-2-(trifluoromethyl)pyrrolidin-1-yl]ethan-1-one, or a pharmaceutically acceptable salt thereof, or a solvate thereof.

14. 2-{3-{1-(2-Amino-5-fluoropyridine-4-carbonyl)azetidin-3-yl]-1H-indazol-1-yl}-1-[(2S)-2-(trifluoromethyl)pyrrolidin-1-yl]ethan-1-one, or a pharmaceutically acceptable salt thereof, or a solvate thereof.

15. 2-{3-[1-(2-Amino-5-fluoropyridine-4-carbonyl)azetidin-3-yl)-1H-indazol-1-yl}-1-[(2S)-2-(trifluoromethyl)pyrrolidin-1-yl]ethan-1-one methanesulfonate, or a solvate thereof.

16. A pharmaceutical composition comprising the compound according to any one of claims 1 to 15, or a pharmaceutically acceptable salt thereof, or a solvate thereof.

17. A DDR1 kinase inhibitor comprising the compound according to any one of claims 1 to 15, or a pharmaceutically acceptable salt thereof, or a solvate thereof as an active ingredient.

18. A method for inhibiting a DDR1 kinase, which comprises administering the compound according to any one of claims 1 to 15, or a pharmaceutically acceptable salt thereof, or a solvate thereof to a subject in need thereof.

19. A prophylactic or therapeutic agent for a disease in which a DDR1 kinase is involved, comprising the compound according to any one of claims 1 to 15, or a pharmaceutically acceptable salt thereof, or a solvate thereof.

20. A therapeutic agent for Alport syndrome, IgA nephropathy, Goodpasture syndrome, anti-glomerular basement membrane nephritis, lupus nephritis, ANCA-related nephritis, diabetic nephropathy, purpura nephritis, focal segmental glomerulosclerosis, chronic kidney failure, minimal change nephrotic syndrome, mesangial proliferative glomerulonephritis, membranoproliferative glomerulonephritis, crescentic glomerulonephritis, membranous nephropathy, pulmonary fibrosis, myelofibrosis, liver fibrosis, acute myeloid leukemia, chronic myelogenous leukemia, acute lymphoid leukemia, chronic lymphocytic leukemia, Hodgkin lymphoma, non-Hodgkin lymphoma, glioma, non-small-cell lung cancer, small-cell lung cancer, breast cancer, ovarian cancer, prostate cancer, colorectal cancer or osteoarthrosis, comprising the compound according to any one of claims 1 to 15, or a pharmaceutically acceptable salt thereof, or a solvate thereof as an active ingredient.

21. A method for preventing or treating a disease in which a DDR1 kinase is involved, which comprises administering the compound according to any one of claims 1 to 15, or a pharmaceutically acceptable salt thereof, or a solvate thereof to a subject in need thereof.

22. A method for preventing or treating Alport syndrome, IgA nephropathy, Goodpasture syndrome, anti-glomerular basement membrane nephritis, lupus nephritis, ANCA-related nephritis, diabetic nephropathy, purpura nephritis, focal segmental glomerulosclerosis, chronic kidney failure, minimal change nephrotic syndrome, mesangial proliferative glomerulonephritis, membranoproliferative glomerulonephritis, crescentic glomerulonephritis, membranous nephropathy, pulmonary fibrosis, myelofibrosis, liver fibrosis, acute myeloid leukemia, chronic myelogenous leukemia, acute lymphoid leukemia, chronic lymphocytic leukemia, Hodgkin lymphoma, non-Hodgkin lymphoma, glioma, non-small-cell lung cancer, small-cell lung cancer, breast cancer, ovarian cancer, prostate cancer, colorectal cancer or osteoarthrosis, which comprises administering the compound according to any one of claims 1 to 15, or a pharmaceutically acceptable salt thereof, or a solvate thereof to a subject in need thereof.

23. The compound according to any one of claims 1 to 15, or a pharmaceutically acceptable salt thereof, or a solvate thereof for use in preventing or treating a disease in which a DDR1 kinase is involved.

24. The compound according to any one of claims 1 to 15, or a pharmaceutically acceptable salt thereof, or a solvate thereof for use in preventing or treating Alport syndrome, IgA nephropathy, Goodpasture syndrome, anti-glomerular basement membrane nephritis, lupus nephritis, ANCA-related nephritis, diabetic nephropathy, purpura nephritis, focal segmental glomerulosclerosis, chronic kidney failure, minimal change nephrotic syndrome, mesangial proliferative glomerulonephritis, membranoproliferative glomerulonephritis, crescentic glomerulonephritis, membranous nephropathy, pulmonary fibrosis, myelofibrosis, liver fibrosis, acute myeloid leukemia, chronic myelogenous leukemia, acute lymphoid leukemia, chronic lymphocytic leukemia, Hodgkin lymphoma, non-Hodgkin lymphoma, glioma, non-small-cell lung cancer, small-cell lung cancer, breast cancer, ovarian cancer, prostate cancer, colorectal cancer or osteoarthrosis.

25. Use of the compound according to any one of claims 1 to 15, or a pharmaceutically acceptable salt thereof, or a solvate thereof for the manufacture of a prophylactic agent or therapeutic agent for a disease in which a DDR1 kinase is involved.

26. Use of the compound according to any one of claims 1 to 15, or a pharmaceutically acceptable salt thereof, or a solvate thereof for the manufacture of a prophylactic agent or therapeutic agent for Alport syndrome, IgA nephropathy. Goodpasture syndrome, anti-glomerular basement membrane nephritis, lupus nephritis, ANCA-related nephritis, diabetic nephropathy, purpura nephritis, focal segmental glomerulosclerosis, chronic kidney failure, minimal change nephrotic syndrome, mesangial proliferative glomerulonephritis, membranoproliferative glomerulonephritis, crescentic glomerulonephritis, membranous nephropathy, pulmonary fibrosis, myelofibrosis, liver fibrosis, acute myeloid leukemia, chronic myelogenous leukemia, acute lymphoid leukemia, chronic lymphocytic leukemia, Hodgkin lymphoma, non-Hodgkin lymphoma, glioma, non-small-cell lung cancer, small-cell lung cancer, breast cancer, ovarian cancer, prostate cancer, colorectal cancer or osteoarthrosis.
